# EUROPEAN PATENT APPLICATION

(11) **EP 4 485 076 A1**
(43) Date of publication of application: **01.01.2025**
(21) Application number: 24184618.7
(22) Date of filing: 26.06.2024
(51) Int. Cl.: G03F 7/004, G03F 7/039

(54) **ACETAL MODIFIER, POLYMER, CHEMICALLY AMPLIFIED POSITIVE RESIST COMPOSITION, AND RESIST PATTERN FORMING PROCESS**

(30) Priority: 28.06.2023 JP 2023106108
(71) Applicant: SHIN-ETSU CHEMICAL CO., LTD., Tokyo (JP)
(72) Inventor: FUKUSHIMA, Masahiro, Joetsu-shi (JP); WATANABE, Satoshi, Joetsu-shi (JP); FUNATSU, Kenji, Joetsu-shi (JP); MASUNAGA, Keiichi, Joetsu-shi (JP); KOTAKE, Masaaki, Joetsu-shi (JP); MATSUZAWA, Yuta, Joetsu-shi (JP)
(74) Representative: Ter Meer Steinmeister & Partner

(57) **Abstract**

An acetal modifier affording a group having an oxygen or sulfur-containing cyclic saturated heterocycle serving as a protective group for an aliphatic or aromatic hydroxy group is provided as well as a polymer adapted to turn alkali soluble as a result of deprotection under the action of acid, the polymer comprising repeat units A1 having on side chain a structure including an aromatic hydroxy group protected with an acid labile group. A chemically amplified positive resist composition comprising the polymer is also provided.

## Description

### TECHNICAL FIELD

This invention relates to an acetal modifier, polymer, chemically amplified positive resist composition, and resist pattern forming process.

### BACKGROUND ART

To meet the recent demand for higher integration in integrated circuits, pattern formation to a smaller feature size is required. Acid-catalyzed chemically amplified resist compositions are most often used in forming resist patterns with a feature size of 0.2 µm or less. High-energy radiation such as UV, deep-UV, EUV or EB is used as the energy source for exposure of these resist compositions. In particular, the EB lithography, which is utilized as the ultra-fine microfabrication technique, is also indispensable in processing a photomask blank into a photomask for use in the fabrication of semiconductor devices.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB having a minute area. The operation of successively scanning all finely divided regions on the work surface takes a long time as compared with one-shot exposure through a photomask. To avoid any throughput decline, a resist film having a high sensitivity is required. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate. A small line edge roughness (LER) is another important performance factor. In recent years, the multibeam mask writing (MBMW) process is used in the processing of mask blanks to achieve further miniaturization. The resist used in the MBMW process is a low-sensitivity resist composition (or high-dose region) which is advantageous in roughness while a spotlight is brought to the optimization of the resist composition in the high-dose region.

Various improvements in the control of resist sensitivity and pattern profile have been made by a proper selection and combination of resist material components and processing conditions. One improvement pertains to the diffusion of acid that largely affects the resolution of a resist film. In the processing of photomasks, it is required that the profile of a resist pattern formed do not change with a lapse of time from the end of exposure to bake. The major cause of such a change of resist pattern profile with time is diffusion of an acid generated upon exposure. The problem of acid diffusion has been widely studied not only in terms of photomask processing, but also in terms of general resist compositions because the acid diffusion has a significant impact on sensitivity and resolution.

Patent Documents 1 and 2 describe acid generators capable of generating bulky acids for controlling acid diffusion and reducing LER. Since these acid generators are still insufficient to control acid diffusion, it is desired to have an acid generator with shorter acid diffusion.

Patent Document 3 discloses a resist composition comprising a base polymer having introduced therein repeat units having a sulfonium structure capable of generating a sulfonic acid upon light exposure. This approach of controlling acid diffusion by introducing repeat units capable of generating acid upon exposure into a base polymer is effective in forming a pattern with small LER. However, the base polymer having introduced therein repeat units capable of generating acid upon exposure sometimes encounters a problem with respect to its solubility in organic solvent, depending on the structure and proportion of the repeat units.

Polymers comprising a major proportion of aromatic structure having an acidic side chain, for example, polyhydroxystyrene are useful as a base polymer in resist materials for the KrF excimer laser lithography. These polymers are not used in resist materials for the ArF excimer laser lithography because they exhibit strong absorption to radiation of wavelength around 200 nm. These polymers, however, are expected to form useful resist materials for the EB and EUV lithography for forming patterns of smaller size than the processing limit of ArF excimer laser because they offer high etching resistance.

Often used as the base polymer in positive resist compositions for EB and EUV lithography is a polymer having an acidic functional group on phenol side chain masked with an acid labile group. Upon exposure to high-energy radiation, a photoacid generator generates an acid and the acid labile group is deprotected by the catalysis of the generated acid whereby the polymer turns soluble in alkaline developer. Typical of the acid labile group are tertiary alkyl and tert-butoxycarbonyl. Acetal groups are also used as an acid labile group requiring a relatively low level of activation energy. See Patent Documents 4 to 8.

The acetal group has the advantage that a resist film having a high sensitivity is obtainable. In the MBMW image writing process by the EB lithography for the fabrication of advanced photomasks of 10 nm node or less, images are written in thin-film regions where a resist film has a thickness of 100 nm or less and in high-dose regions having a high level of irradiation energy. If acetal has a high reactivity and a bulky structure, deprotection reaction can occur even in the unexposed region of the resist film and residues are left even in the exposed region. There arise such problems as degradations of resolution of isolated spaces and LER, which are regarded important for positive resist compositions, and formation of defects.

It is known that a develop loading phenomenon arises in the development step of the photomask fabrication process. That is, the finish size of pattern features differs between a grouped region and an isolated region on a photomask. Due to the develop loading, the distribution of pattern finish size becomes non-uniform depending on the surrounding pattern feature distribution. This is caused by a difference in elimination reaction during acid generation due to an energy difference of EB and a difference of dissolution rate in alkaline developer between grouped and isolated images. As one solution, Patent Document 9 discloses a beam dose computing method of an EB writing apparatus comprising the steps of adjusting an input dose in the EB writing apparatus so as to correct develop loading effects, and irradiating EB in the adjusted dose for thereby writing a pattern on a photomask. However, since the prior art correcting method has not fully taken into account the develop loading phenomenon for correction, the accuracy of correcting develop loading effects is not so high. To solve such problems, Patent Document 10 discloses an imaging method and Patent Document 11 discloses a method of improving a development mode after patterning. These methods are insufficient for establishing a uniform distribution of grouped and isolated features in the lithography of advanced generation. It is desired to improve a resist composition so as to achieve a high resolution and reductions of develop loading and residue defects in the lithography of advanced generation.

### Citation List

Patent Document 1: JP-A 2009-053518
Patent Document 2: JP-A 2010-100604
Patent Document 3: JP-A 2011-022564
Patent Document 4: JP 3981830
Patent Document 5: JP 5385017
Patent Document 6: WO 2019/167419
Patent Document 7: JP 6987873
Patent Document 8: JP 5696254
Patent Document 9: JP-A 2007-150243 (USP 7740991)
Patent Document 10: JP 5443548
Patent Document 11: JP 6281244

### SUMMARY OF INVENTION

An object of the invention is to provide a chemically amplified positive resist composition which is lithographically processed into a resist pattern with a very high resolution of isolated spaces, reduced LER, improved rectangularity, and minimized influences of develop loading and residue defects, as well as etching resistance and restrained pattern collapse. More particularly, an object of the invention is to provide an acetal modifier; a polymer modified with the acetal modifier; a chemically amplified positive resist composition comprising the polymer; and a resist pattern forming process using the composition.

The inventors have found that when a resist composition comprises a base polymer having acid labile groups derived from an acetal modifier having an oxygen or sulfur-containing cyclic saturated heterocycle, a resist pattern with satisfactory isolated-space resolution, pattern profile and LER is formed even in high-dose regions while controlling the influences of develop loading and residue defects.

Accordingly, the invention provides an acetal modifier, polymer, chemically amplified positive resist composition, and resist pattern forming process, as defined below.
1. An acetal modifier providing a group having an oxygen or sulfur-containing cyclic saturated heterocycle serving as a protective group for an aliphatic or aromatic hydroxy group.
2. The acetal modifier of 1, providing a group serving as a protective group for an aromatic hydroxy group.
3. The acetal modifier of 1 or 2, which is a compound having the formula (AC-1) or (AC-2): 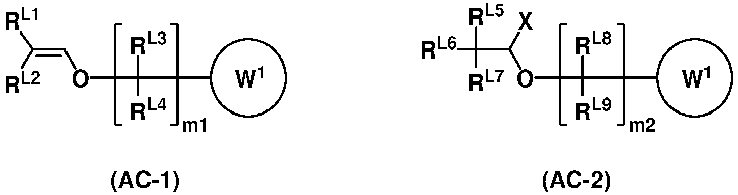 wherein R^{L1}, R^{L2}, R^{L5}, R^{L6} and R^{L7} are each independently hydrogen or a C₁-C₁₅ hydrocarbyl group, R^{L1} and R^{L2} may bond together to form a ring with the carbon atom to which they are attached, and any two of R^{L5} to R^{L7} may bond together to form a ring with the carbon atom to which they are attached,
   R^{L3}, R^{L4}, R^{L8} and R^{L9} are each independently hydrogen or a C₁-C₂₀ hydrocarbyl group which may contain a heteroatom, R^{L3} and R^{L4} may bond together to form a ring with the carbon atom to which they are attached, and R^{L8} and R^{L9} may bond together to form a ring with the carbon atom to which they are attached,
   W¹ is each independently a group having an oxygen or sulfur-containing cyclic saturated heterocycle,
   X is chlorine, bromine or iodine, and
   m1 and m2 are each independently an integer of 0 to 3.
4. A polymer adapted to turn alkali soluble as a result of deprotection under the action of acid, the polymer comprising repeat units A1 having on side chain a structure including an aromatic hydroxy group protected with an acid labile group having the formula (AL-1) or (AL-2): 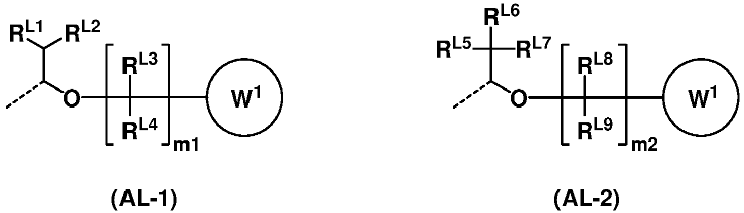 wherein R^{L1}, R^{L2}, R^{L5}, R^{L6} and R^{L7} are each independently hydrogen or a C₁-C₁₅ hydrocarbyl group, R^{L1} and R^{L2} may bond together to form a ring with the carbon atom to which they are attached, and any two of R^{L5} to R^{L7} may bond together to form a ring with the carbon atom to which they are attached,
   R^{L3}, R^{L4}, R^{L8} and R^{L9} are each independently hydrogen or a C₁-C₂₀ hydrocarbyl group which may contain a heteroatom, R^{L3} and R^{L4} may bond together to form a ring with the carbon atom to which they are attached, and R^{L8} and R^{L9} may bond together to form a ring with the carbon atom to which they are attached,
   W¹ is each independently a group having an oxygen or sulfur-containing cyclic saturated heterocycle,
   m1 and m2 are each independently an integer of 0 to 3, and
   the broken line designates a point of attachment to the oxygen atom of the aromatic hydroxy group.
5. The polymer of 4 wherein the repeat unit A1 has the formula (A1): 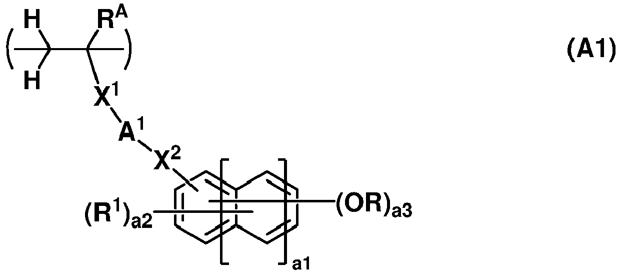 wherein a1 is 0 or 1, a2 is an integer of 0 to 4 in case of a1=0, and an integer of 0 to 6 in case of a1=1, a3 is an integer of 1 to 3, with the proviso that a2+a3 is from 1 to 5 in case of a1=0 and a2+a3 is from 1 to 7 in case of a1=1,
   R^{A} is hydrogen, fluorine, methyl or trifluoromethyl,
   X¹ is a single bond, *-C(=O)-O- or *-C(=O)-NH-, * designates a point of attachment to the carbon atom in the backbone,
   X² is a single bond, ether bond, ester bond, carbonyl group, sulfonate ester bond, carbonate bond or carbamate bond,
   A¹ is a single bond or a C₁-C₁₀ saturated hydrocarbylene group in which some -CH₂-may be replaced by -O-,
   R¹ is halogen or a C₁-C₂₀ hydrocarbyl group which may contain a heteroatom, and
   R is an acid labile group having formula (AL-1) or (AL-2).
6. The polymer of 4 or 5, further comprising phenolic hydroxy group-bearing repeat units A2 having the formula (A2): 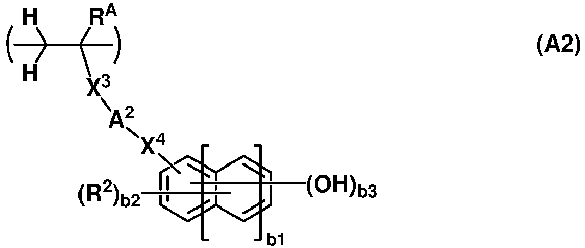 wherein b1 is 0 or 1, b2 is an integer of 0 to 4 in case of b1=0, and an integer of 0 to 6 in case of b 1 = 1, b3 is an integer of 1 to 3, with the proviso that b2+b3 is from 1 to 5 in case of b1=0 and b2+b3 is from 1 to 7 in case of b1=1,
   R^{A} is hydrogen, fluorine, methyl or trifluoromethyl,
   X³ is a single bond, *-C(=O)-O- or *-C(=O)-NH-, * designates a point of attachment to the carbon atom in the backbone,
   X⁴ is a single bond, ether bond, ester bond, carbonyl group, sulfonate ester bond, carbonate bond or carbamate bond,
   A² is a single bond or a C₁-C₁₀ saturated hydrocarbylene group in which some -CH₂-may be replaced by -O-, and
   R² is halogen or a C₁-C₂₀ hydrocarbyl group which may contain a heteroatom.
7. The polymer of 6 wherein repeat unit A2 has the formula (A2-1): 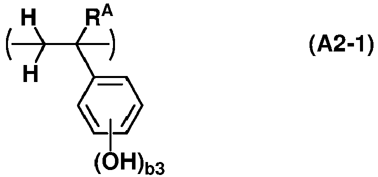 wherein R^{A} and b3 are as defined above.
8. The polymer of any one of 4 to 7, further comprising repeat units of at least one type selected from repeat units having the formula (A3-1) and repeat units having the formula (A3-2): 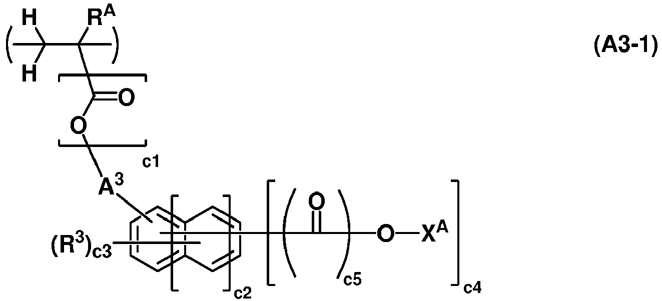 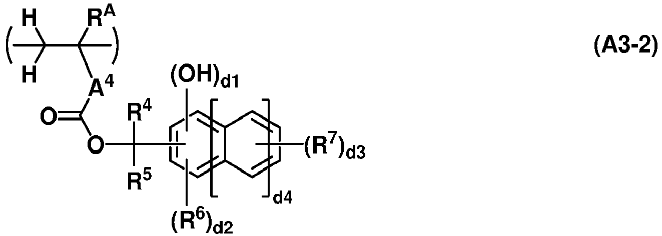 wherein c1 is 0 or 1, c2 is an integer of 0 to 2, c3 is an integer satisfying 0 ≤ c3 ≤ 5+2(c2)-c4, c4 is an integer of 1 to 3, c5 is 0 or 1,
   d1 is an integer of 0 to 2, d2 is an integer of 0 to 2, d3 is an integer of 0 to 5, d4 is an integer of 0 to 2,
   R^{A} is each independently hydrogen, fluorine, methyl or trifluoromethyl,
   A³ is a single bond or a C₁-C₁₀ saturated hydrocarbylene group in which some -CH₂-may be replaced by -O-,
   A⁴ is a single bond, phenylene group, naphthylene group or *-C(=O)-O-A⁴¹-, A⁴¹ is a C₁-C₂₀ aliphatic hydrocarbylene group which may contain hydroxy, ether bond, ester bond or lactone ring, or a phenylene group or naphthylene group, * designates a point of attachment to the carbon atom in the backbone,
   R³ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
   R⁴ and R⁵ are each independently a C₁-C₁₀ hydrocarbyl group which may contain a heteroatom, R⁴ and R⁵ may bond together to form a ring with the carbon atom to which they are attached,
   R⁶ is each independently fluorine or a C₁-C₅ fluorinated alkyl group or C₁-C₅ fluorinated alkoxy group,
   R⁷ is each independently a C₁-C₁₀ hydrocarbyl group which may contain a heteroatom, and
   X^{A} is an acid labile group in case of c4=1 and X^{A} is hydrogen or an acid labile group, at least one being an acid labile group, in case of c4=2 or 3.
9. The polymer of any one of 4 to 8, further comprising repeat units of at least one type selected from repeat units having the formula (B1), repeat units having the formula (B2), and repeat units having the formula (B3): 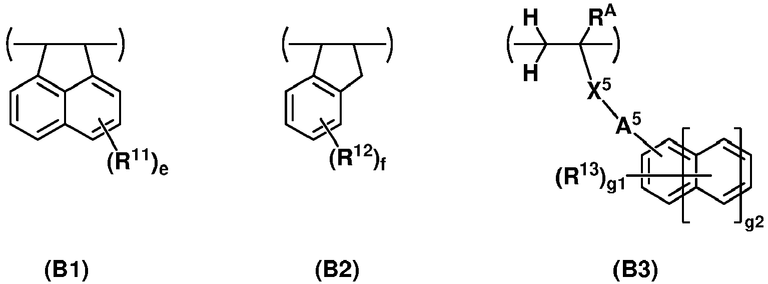 wherein e and f are each independently an integer of 0 to 4, g1 is an integer of 0 to 5, g2 is an integer of 0 to 2,
   R^{A} is hydrogen, fluorine, methyl or trifluoromethyl,
   X⁵ is a single bond, *-C(=O)-O- or *-C(=O)-NH-, * designates a point of attachment to the carbon atom in the backbone,
   A⁵ is a single bond or a C₁-C₁₀ saturated hydrocarbylene group in which some -CH₂-may be replaced by -O-, and
   R¹¹ and R¹² are each independently a hydroxy group, halogen, an optionally halogenated C₂-C₈ saturated hydrocarbylcarbonyloxy group, optionally halogenated C₁-C₈ saturated hydrocarbyl group, or optionally halogenated C₁-C₈ saturated hydrocarbyloxy group,
   R¹³ is an acetyl group, C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group or cyano group, R¹³ may also be a hydroxy group in case of g2=1 or 2.
10. The polymer of any one of 4 to 9, further comprising repeat units of at least one type selected from repeat units having the formulae (C1) to (C4): 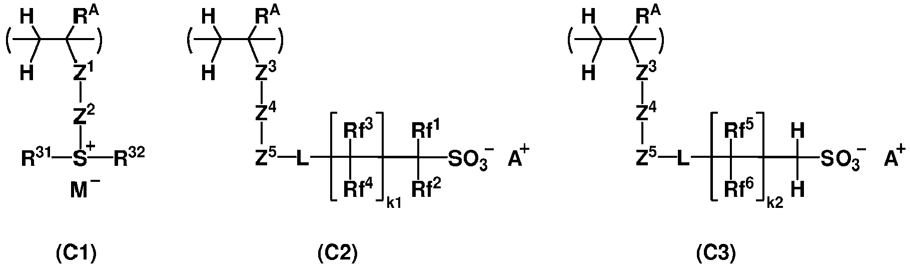 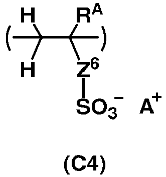 wherein R^{A} is each independently hydrogen, fluorine, methyl or trifluoromethyl,
   Z¹ is a single bond or phenylene group,
   Z² is *¹-C(=O)-O-Z²¹-, *¹-C(=O)-NH-Z²¹-, or *¹-O-Z²¹-, Z²¹ is a C₁-C₆ aliphatic hydrocarbylene group, phenylene group or divalent group obtained by combining the foregoing, which may contain a carbonyl moiety, ester bond, ether bond or hydroxy moiety, *¹ designates a point of attachment to Z¹,
   Z³ is each independently a single bond, phenylene group, naphthylene group or *²-C(=O)-O-Z³¹-, Z³¹ is a C₁-C₁₀ aliphatic hydrocarbylene group, phenylene group or naphthylene group, the aliphatic hydrocarbylene group may contain a hydroxy moiety, ether bond, ester bond or lactone ring, *² designates a point of attachment to the carbon atom in the backbone,
   Z⁴ is each independently a single bond, *³-Z⁴¹-C(=O)-O-, *³-C(=O)-NH-Z⁴¹-, or *³-O-Z⁴¹-, Z⁴¹ is a C₁-C₂₀ hydrocarbylene group which may contain a heteroatom, *³ designates a point of attachment to Z³,
   Z⁵ is each independently a single bond, *⁴-Z⁵¹-C(=O)-O-, *⁴-C(=O)-NH-Z⁵¹-, or *⁴-O-Z⁵¹-, Z⁵¹ is a C₁-C₂₀ hydrocarbylene group which may contain a heteroatom, *⁴ designates a point of attachment to Z⁴,
   Z⁶ is each independently a single bond, methylene, ethylene, phenylene, fluorinated phenylene, trifluoromethyl-substituted phenylene, *²-C(=O)-O-Z⁶¹-, *²-C(=O)-N(H)-Z⁶¹-, or *²-O-Z⁶¹-, Z⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, *² designates a point of attachment to the carbon atom in the backbone,
   R³¹ and R³² are each independently a C₁-C₂₀ hydrocarbyl group which may contain a heteroatom, R³¹ and R³² may bond together to form a ring with the sulfur atom to which they are attached,
   L is a single bond, ether bond, ester bond, carbonyl group, sulfonate ester bond, carbonate bond or carbamate bond,
   Rf¹ and Rf² are each independently fluorine or a C₁-C₆ fluorinated saturated hydrocarbyl group,
   Rf³ and Rf⁴ are each independently hydrogen, fluorine or a C₁-C₆ fluorinated saturated hydrocarbyl group,
   Rf⁵ and Rf⁶ are each independently hydrogen, fluorine or a C₁-C₆ fluorinated saturated hydrocarbyl group, excluding that all Rf⁵ and Rf⁶ are hydrogen at the same time,
   M⁻ is a non-nucleophilic counter ion,
   A⁺ is an onium cation,
   k1 and k2 are each independently an integer of 0 to 3.
11. A chemically amplified positive resist composition comprising the polymer of any one of 4 to 10.
12. The resist composition of 11, further comprising an organic solvent.
13. The resist composition of 11 0r 12, further comprising a photoacid generator capable of generating an acid having an acid strength (pKa) of -2.0 or larger.
14. The resist composition of 13 wherein the photoacid generator contains an anion having the formula (P1): 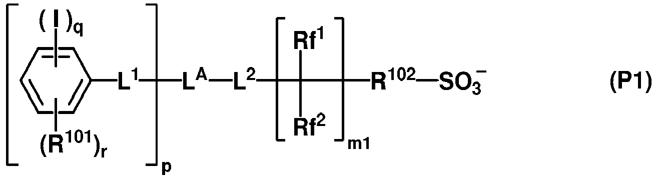 wherein m1 is 0 or 1, p is an integer of 1 to 3, q is an integer of 1 to 5, r is an integer of 0 to 3,
   L¹ is a single bond, ether bond, ester bond, sulfonate ester bond, carbonate bond or carbamate bond,
   L² is an ether bond, ester bond, sulfonate ester bond, carbonate bond or carbamate bond,
   L^{A} is a single bond or C₁-C₂₀ hydrocarbylene group in case of p=1, and a C₁-C₂₀ (p+1)-valent hydrocarbon group in case of p=2 or 3, the hydrocarbylene and (p+1)-valent hydrocarbon groups may contain at least one moiety selected from an ether bond, carbonyl moiety, ester bond, amide bond, sultone ring, lactam ring, carbonate bond, halogen, hydroxy moiety and carboxy moiety,
   Rf¹ and Rf² are each independently hydrogen, fluorine or trifluoromethyl, at least one thereof being fluorine or trifluoromethyl,
   R¹⁰¹ is a hydroxy group, carboxy group, C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{101A})(R^{101B}), -N(R^{101C})-C(=O)-R^{101D}, -N(R^{101C})-C(=O)-O-R^{101D}, R^{101A} and R^{101B} are each independently hydrogen or a C₁-C₆ saturated hydrocarbyl group, R^{101C} is hydrogen or a C₁-C₆ saturated hydrocarbyl group, R^{101D} is a C₁-C₆ saturated hydrocarbyl group or C₂-C₈ unsaturated aliphatic hydrocarbyl group, and
   R¹⁰² is a C₁-C₂₀ saturated hydrocarbylene group or C₆-C₂₀ arylene group, some or all of the hydrogen atoms in the saturated hydrocarbylene group may be substituted by halogen exclusive of fluorine, and some or all of the hydrogen atoms in the arylene group may be substituted by at least one substituent selected from a C₁-C₂₀ saturated hydrocarbyl moiety, C₁-C₂₀ saturated hydrocarbyloxy moiety, C₆-C₂₀ aryl moiety, halogen, and hydroxy moiety.
15. The resist composition of any one of 11 to 14, further comprising a quencher.
16. The resist composition of any one of 11 to 15, further comprising a fluorinated polymer comprising repeat units of at least one type selected from repeat units having the formula (D1), repeat units having the formula (D2), repeat units having the formula (D3), and repeat units having the formula (D4): 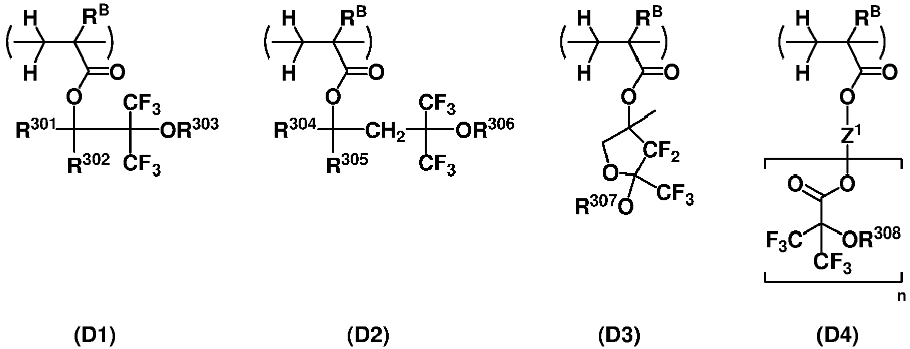 wherein R^{B} is each independently hydrogen, fluorine, methyl or trifluoromethyl,
   R³⁰¹, R³⁰², R³⁰⁴ and R³⁰⁵ are each independently hydrogen or a C₁-C₁₀ saturated hydrocarbyl group,
   R³⁰³, R³⁰⁶, R³⁰⁷ and R³⁰⁸ are each independently hydrogen, a C₁-C₁₅ hydrocarbyl group, C₁-C₁₅ fluorinated hydrocarbyl group, or acid labile group, with the proviso that when R³⁰³, R³⁰⁶, R³⁰⁷ and R³⁰⁸ each are a hydrocarbyl or fluorinated hydrocarbyl group, an ether bond or carbonyl moiety may intervene in a carbon-carbon bond,
   n is an integer of 1 to 3, and
   Z¹ is a C₁-C₂₀ (n+1)-valent hydrocarbon group or C₁-C₂₀ (n+1)-valent fluorinated hydrocarbon group.
17. The resist composition of 16 wherein the fluorinated polymer further comprises repeat units of at least one type selected from repeat units having the formula (D5) and repeat units having the formula (D6): 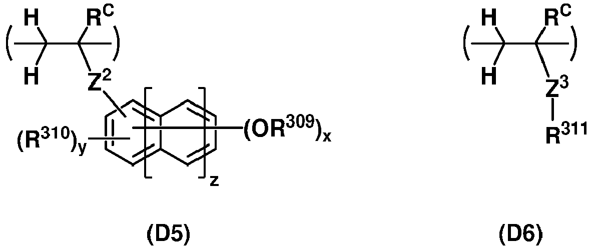 wherein R^{C} is each independently hydrogen or methyl,
   R³⁰⁹ is hydrogen or a C₁-C₅ straight or branched hydrocarbyl group in which a heteroatom-containing moiety may intervene in a carbon-carbon bond,
   R³¹⁰ is a C₁-C₅ straight or branched hydrocarbyl group in which a heteroatom-containing moiety may intervene in a carbon-carbon bond,
   R³¹¹ is a C₁-C₂₀ saturated hydrocarbyl group in which at least one hydrogen is substituted by fluorine, and in which some -CH₂- may be replaced by an ester bond or ether bond,
   x is an integer of 1 to 3, y is an integer satisfying 0 ≤ y ≤ 5+2z-x, z is 0 or 1,
   Z² is a single bond, *-C(=O)-O- or *-C(=O)-NH-,
   Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.
18. The resist composition of any one of 11 to 17, wherein the polymer has a dissolution rate in alkaline developer of up to 10 nm/min.
19. The resist composition of any one of 11 to 18, which forms a resist film, the resist film in an unexposed region having a dissolution rate in alkaline developer of up to 10 nm/min.
20. The resist composition of any one of 11 to 19, which forms a resist film, the resist film in an exposed region having a dissolution rate in alkaline developer of at least 50 nm/sec.
21. A resist pattern forming process comprising the steps of:
   applying the chemically amplified positive resist composition of any one of 11 to 20, onto a substrate to form a resist film thereon,
   exposing the resist film to a pattern of high-energy radiation, and
   developing the exposed resist film in an alkaline developer.
22. The process of 21 wherein the high-energy radiation is KrF excimer laser, ArF excimer laser, EB or EUV of wavelength 3 to 15 nm.
23. The process of 21 or 22 wherein the substrate has the outermost surface of a material containing at least one element selected from chromium, silicon, tantalum, molybdenum, cobalt, nickel, tungsten, and tin.
24. The process of any one of 21 to 23, wherein the substrate is a mask blank of transmission or reflection type.
25. A mask blank of transmission or reflection type which is coated with the chemically amplified positive resist composition of any one of 11 to 20.

### ADVANTAGEOUS EFFECTS OF INVENTION

The chemically amplified positive resist composition comprising a polymer modified with an acetal modifier can be processed to form a resist pattern of good profile with a high resolution, reduced LER, and improved rectangularity while controlling the influence of residue defects. It is thus suited as a resist composition for forming a resist film which is sensitive to EB and useful in the processing of semiconductor substrates and photomask blanks. The pattern forming process using the positive resist composition can form a resist pattern with a high resolution, reduced LER, etch resistance, and minimized influence of residue defects and is thus best suited in the micropatterning technology, typically EUV or EB lithography.

### DESCRIPTION OF EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, Me stands for methyl, Ac stands for acetyl, and the broken line (---) or asterisk (*) designates a valence bond or point of attachment. As used herein, the term "fluorinated" refers to a fluorine-substituted or fluorine-containing compound or group. The terms "group" and "moiety" are interchangeable.

The abbreviations and acronyms have the following meaning.
- EB:: electron beam
- EUV:: extreme ultraviolet
- Mw:: weight average molecular weight
- Mn:: number average molecular weight
- Mw/Mn:: molecular weight distribution or dispersity
- GPC:: gel permeation chromatography
- PEB:: post-exposure bake
- PAG:: photoacid generator
- LER:: line edge roughness
- CDU:: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Acetal modifier

One embodiment of the invention is an acetal modifier providing a group having an oxygen or sulfur-containing cyclic saturated heterocycle serving as a protective group for an aliphatic or aromatic hydroxy group.

The preferred acetal modifier has the formula (AC-1) or (AC-2).

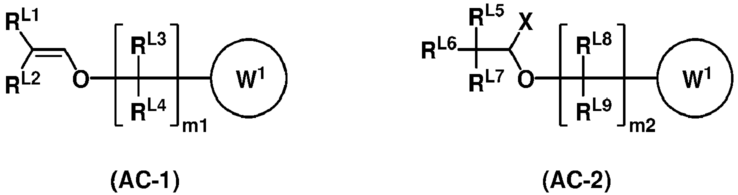

In formulae (AC-1) and (AC-2), R^{L1}, R^{L2}, R^{L5}, R^{L6} and R^{L7} are each independently hydrogen or a C₁-C₁₅ hydrocarbyl group. The hydrocarbyl group may be saturated or unsaturated and straight, branched or cyclic. Examples thereof include, but are not limited to, C₁-C₁₅ alkyl groups such as methyl, ethyl, n-propyl, isopropyl, n-butyl, and tert-butyl, C₃-C₁₅ cyclic saturated hydrocarbyl groups such as cyclopropyl, cyclopentyl, cyclohexyl, and cyclopropylmethyl; C₂-C₁₅ alkenyl groups such as vinyl, allyl, propenyl, butenyl and hexenyl; and C₃-C₁₅ cyclic unsaturated hydrocarbyl groups such as cyclohexenyl.

R^{L1} and R^{L2} may bond together to form a ring with the carbon atom to which they are attached. Any two of R^{L5} to R^{L7} may bond together to form a ring with the carbon atom to which they are attached. Exemplary rings include cyclopropane, cyclobutane, cyclopentane, cyclohexane, norbornane, and adamantane rings.

R^{L1}, R^{L2}, R^{L5}, R^{L6} and R^{L7} are properly selected depending on the design of sensitivity of decomposable group to acid. For example, if the decomposable group is designed so as to be decomposed with strong acid while ensuring relatively high stability, then hydrogen is selected. If the decomposable group is designed to have a high sensitivity relative to pH changes by utilizing a relatively high reactivity and to restrain residue defects, then a straight alkyl group is selected. Specific examples of R^{L1}, R^{L2}, R^{L5}, R^{L6} and R^{L7} include methyl, ethyl, propyl, isopropyl, cyclopentyl, cyclohexyl, and tert-butyl, with methyl being preferred in view of an optimum acid-elimination ability.

In the acetal modifier having formula (AC-2), the carbon atom to which R^{L5}, R^{L6} and R^{L7} are attached is preferably a secondary carbon atom in view of the stability and reactivity with acid of a polymer.

In formulae (AC-1) and (AC-2), R^{L3}, R^{L4}, R^{L8} and R^{L9} are each independently hydrogen or a C₁-C₂₀ hydrocarbyl group which may contain a heteroatom. The hydrocarbyl group may be saturated or unsaturated and straight, branched or cyclic. Examples thereof are as exemplified above for the hydrocarbyl group represented by R^{L1}, R^{L2}, R^{L5}, R^{L6} and R^{L7}.

R^{L3} and R^{L4} may bond together to form a ring with the carbon atom to which they are attached. R^{L8} and R^{L9} may bond together to form a ring with the carbon atom to which they are attached. Examples of the ring are as exemplified for the ring that R^{L1} and R^{L2}, taken together, form with the carbon atom to which they are attached.

In formulae (AC-1) and (AC-2), W¹ is a group having an oxygen or sulfur-containing cyclic saturated heterocycle. Preferred examples of the cyclic saturated heterocycle include cyclic ether, cyclic sulfide, cyclic sulfoxide, and cyclic sulfone, with the cyclic ether and cyclic sulfide being more preferred. Lactone and sulfone structures are unfavorable because they have too high an acid diffusion suppressing effect and thus substantially lower the sensitivity of a resist film.

Examples of the group having an oxygen or sulfur-containing cyclic saturated heterocycle, represented by W¹, are shown below, but not limited thereto.

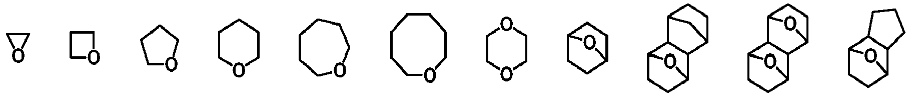

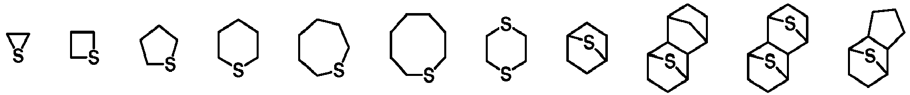

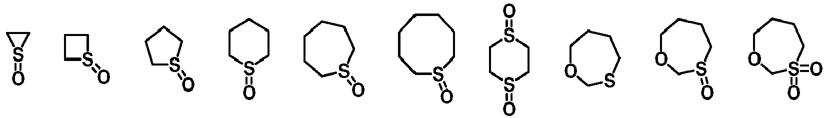

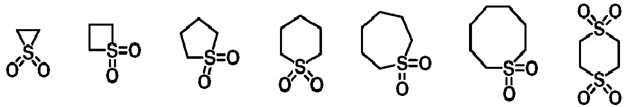

In formula (AC-2), X is chlorine, bromine or iodine, with chlorine being preferred from the aspect of synthesis.

In formulae (AC-1) and (AC-2), m1 and m2 are each independently an integer of 0 to 3. Preferably each of m1 and m2 is 1 or 2, most preferably 1 from the aspect of smooth progress of modifying reaction on a polymer.

Examples of the acetal modifier having formula (AC-1) are shown below, but not limited thereto.

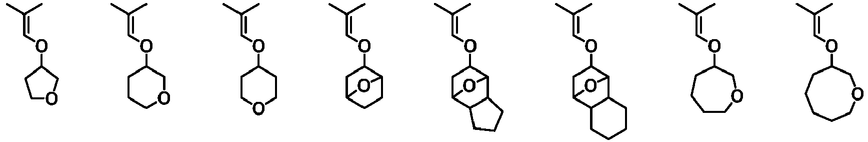

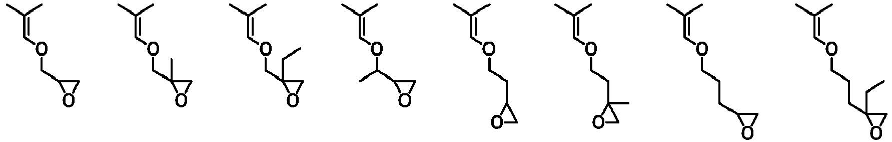

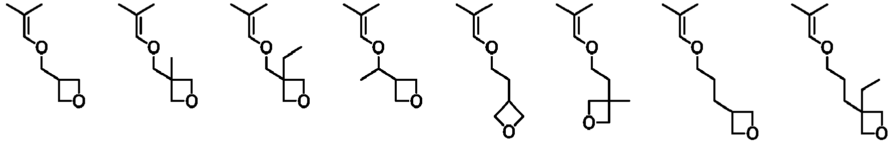

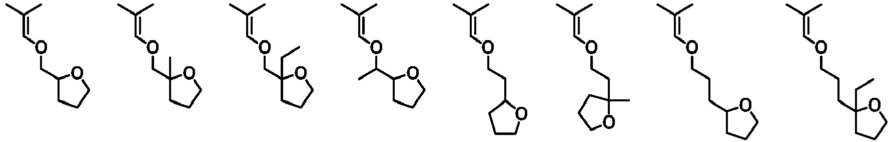

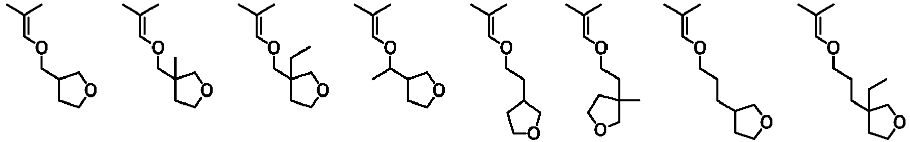

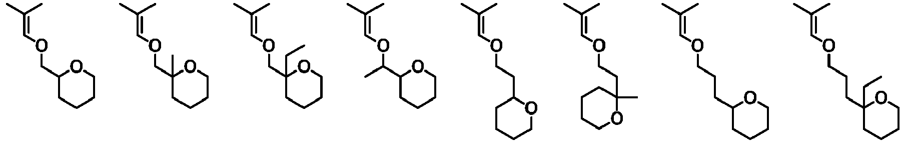

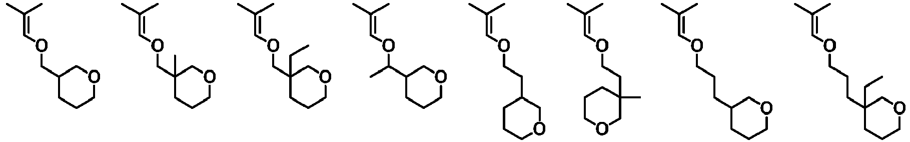

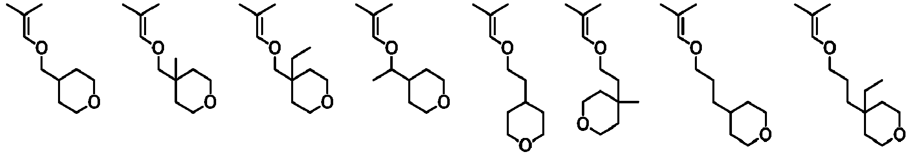

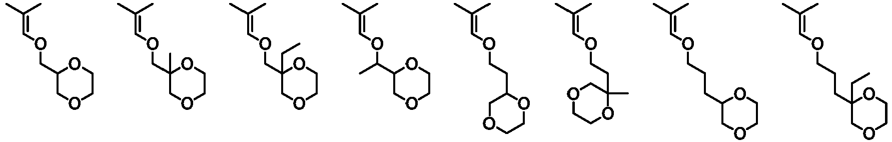

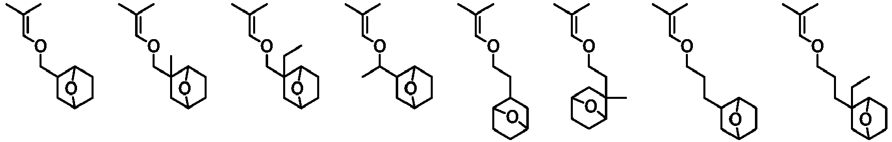

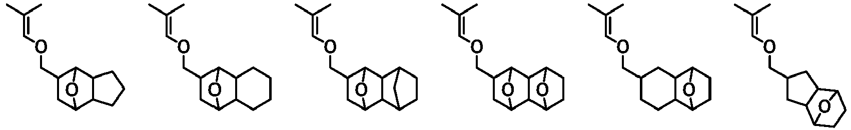

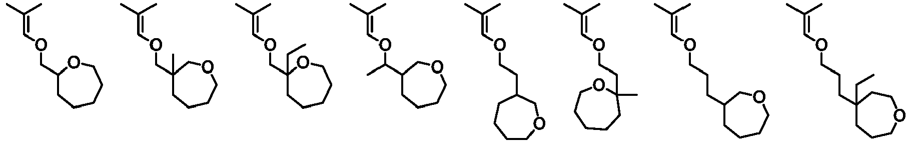

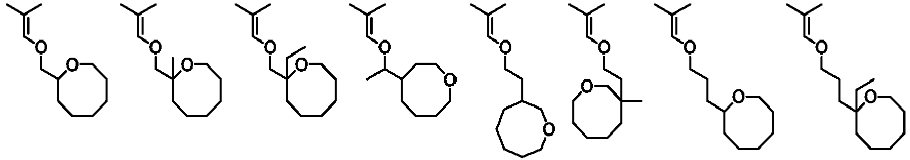

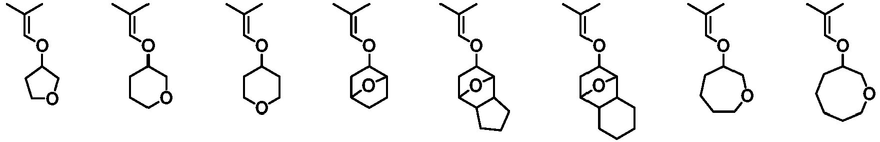

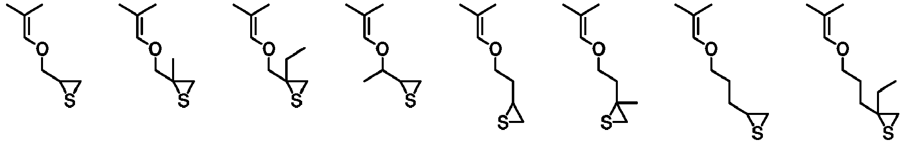

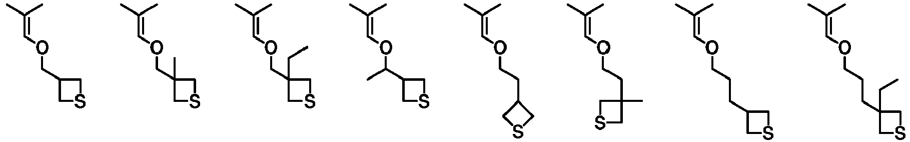

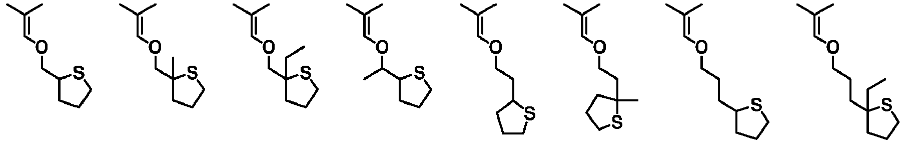

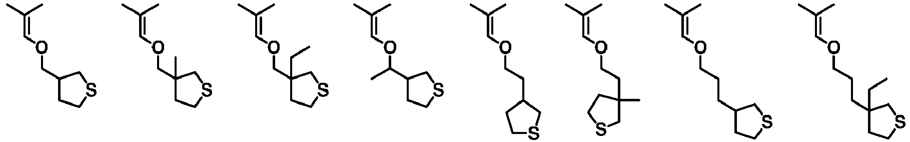

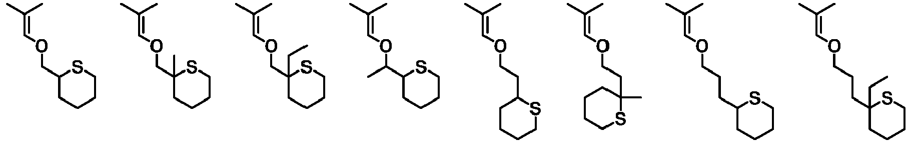

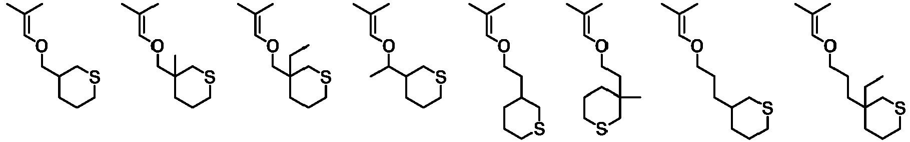

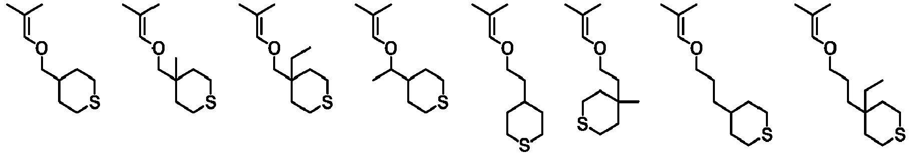

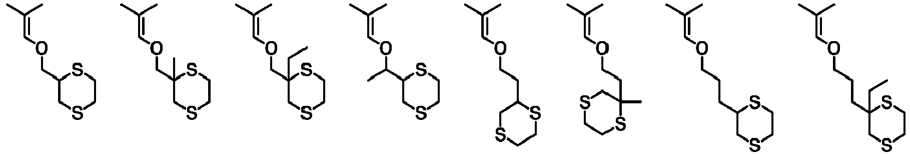

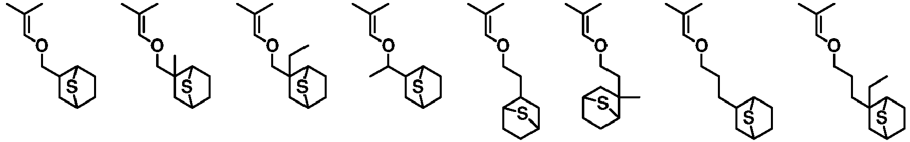

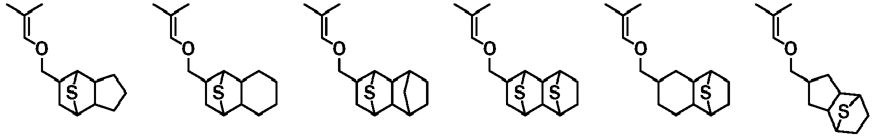

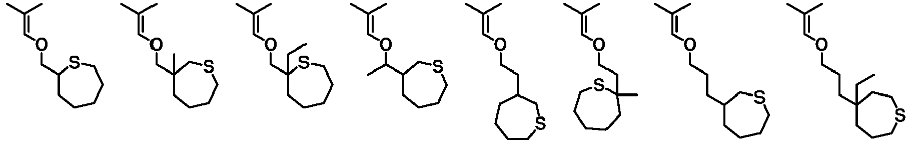

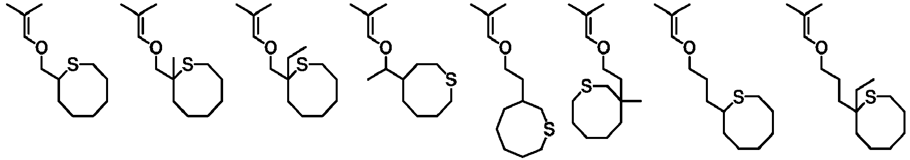

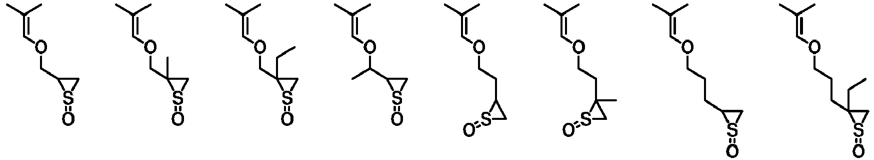

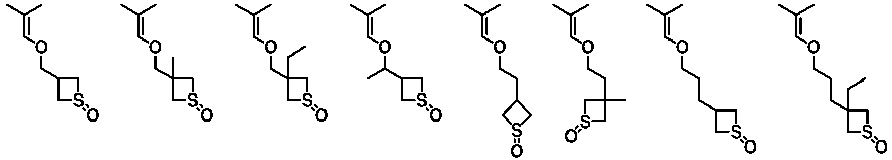

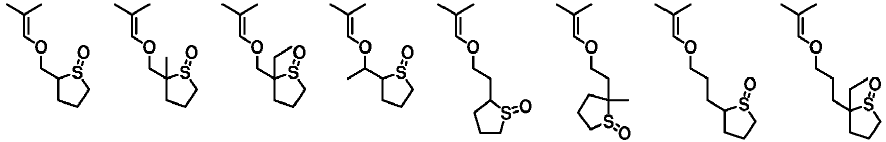

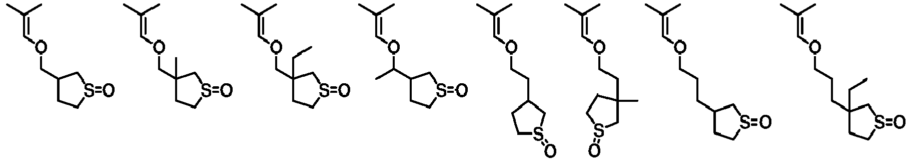

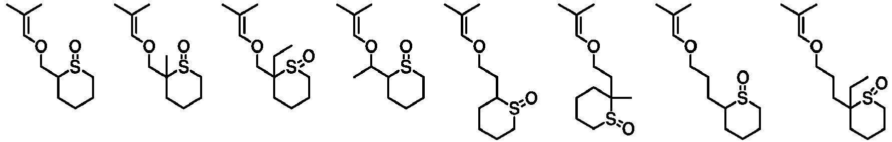

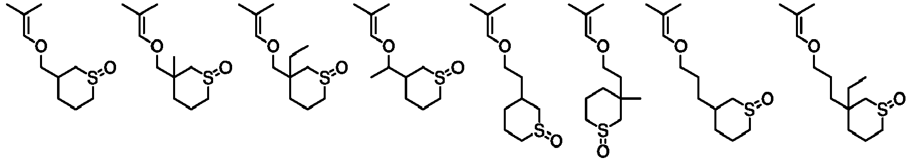

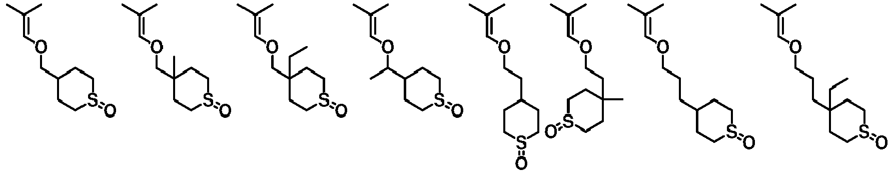

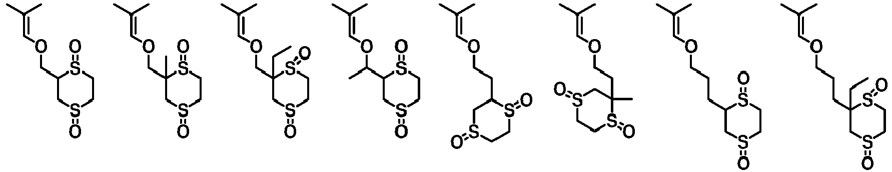

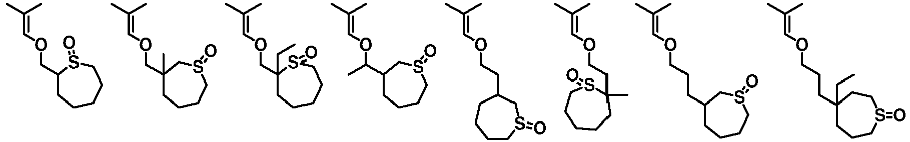

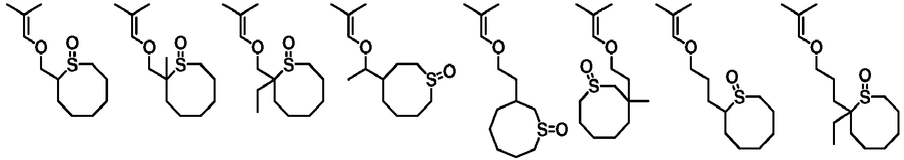

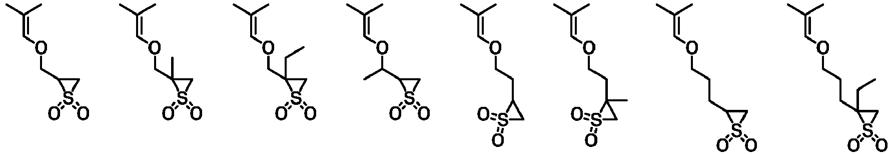

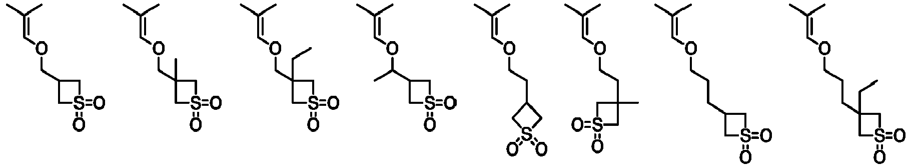

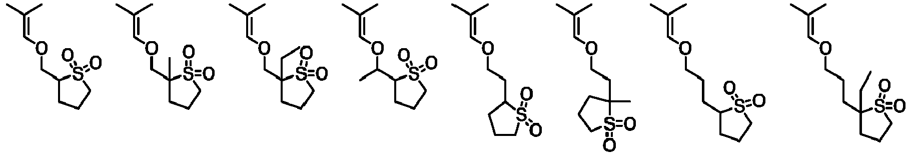

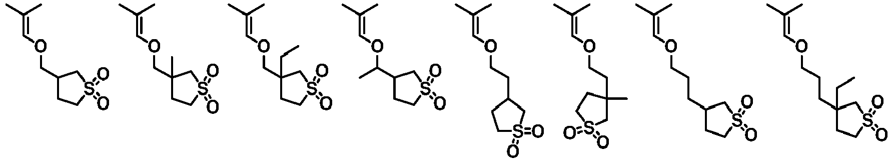

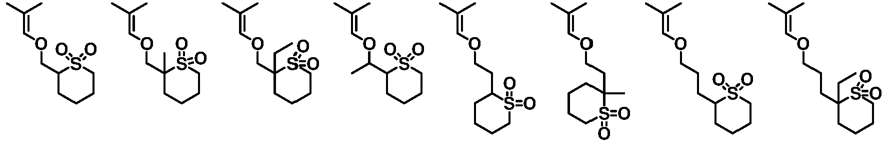

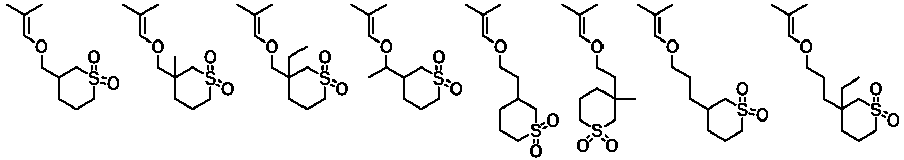

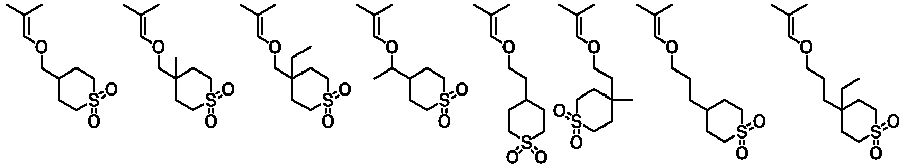

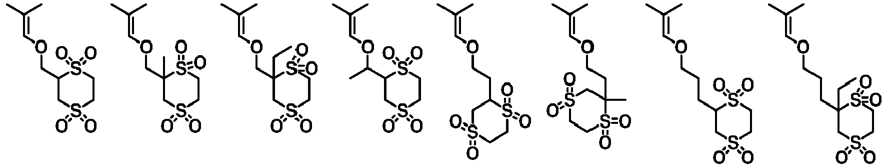

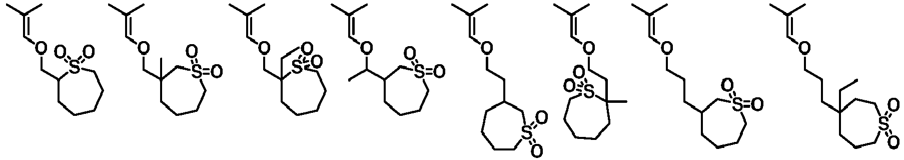

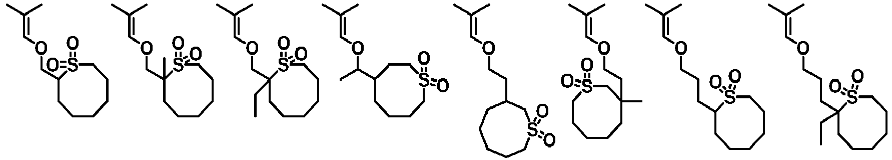

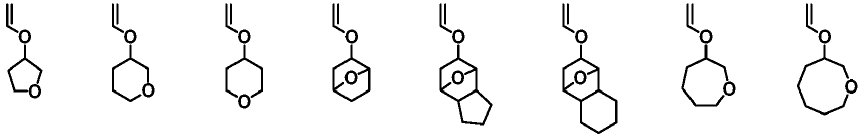

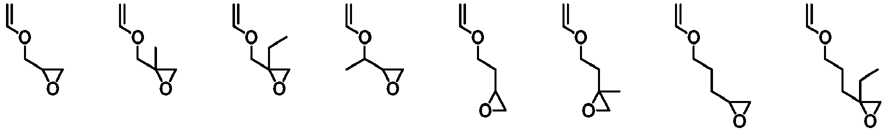

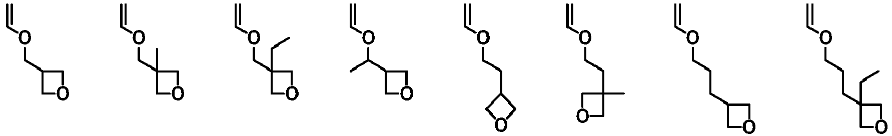

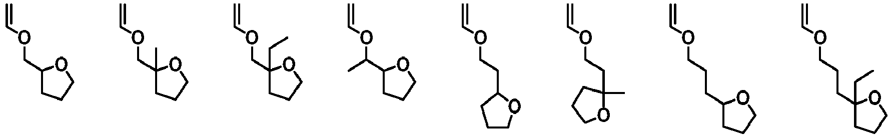

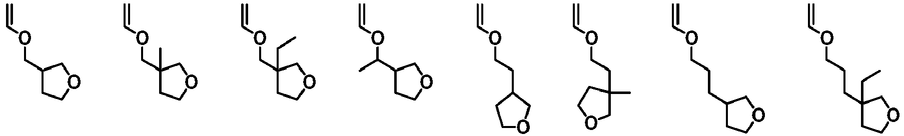

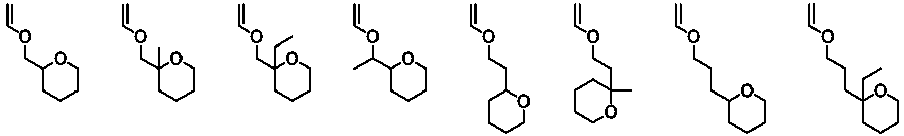

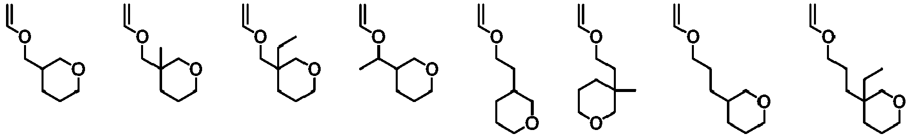

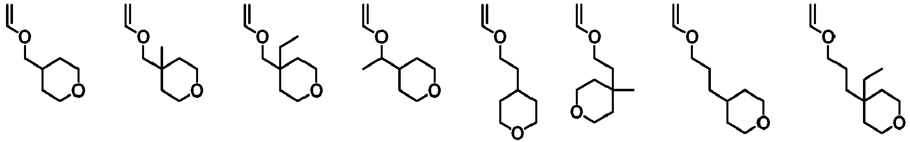

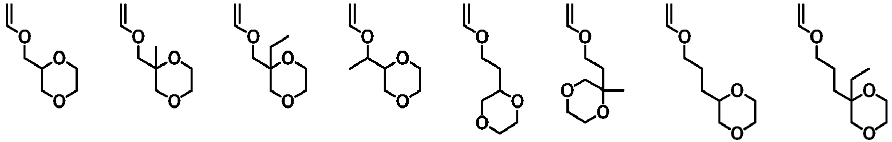

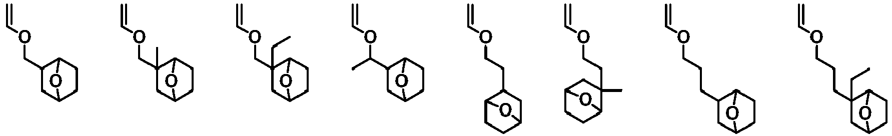

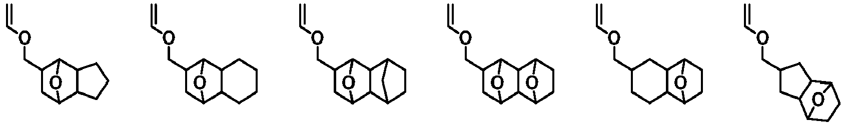

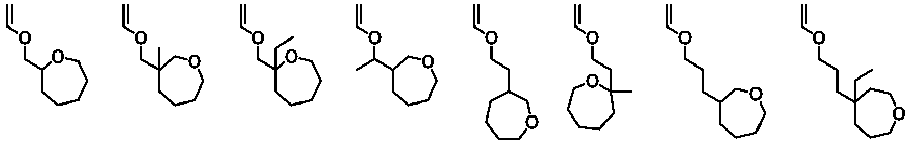

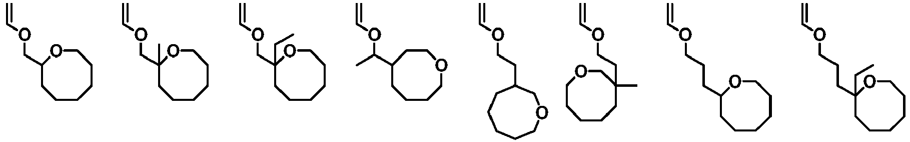

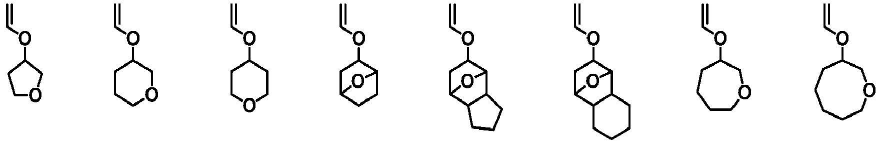

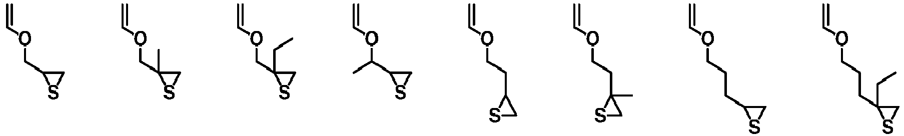

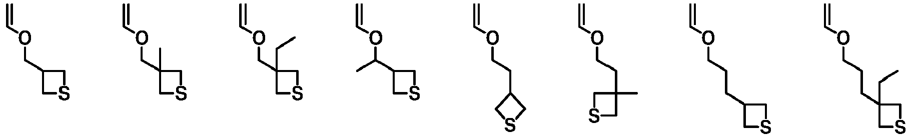

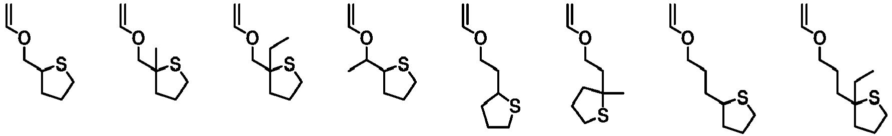

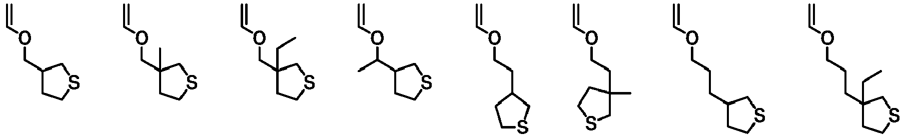

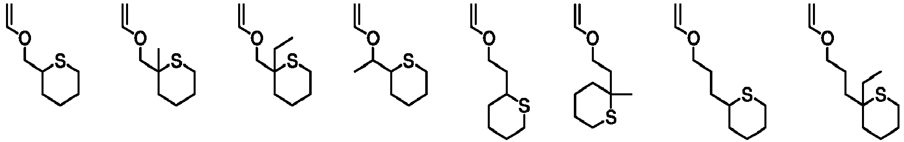

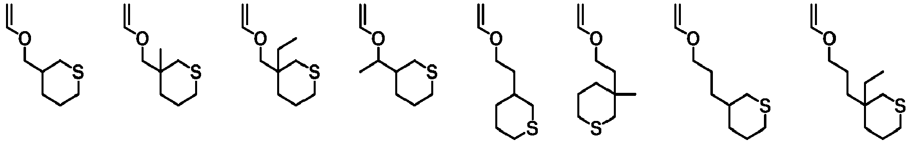

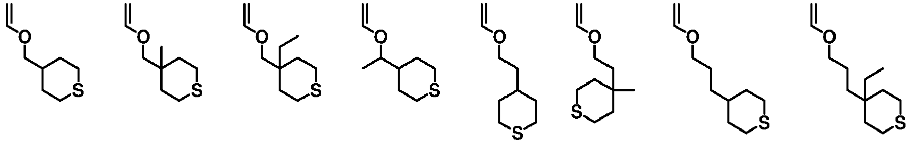

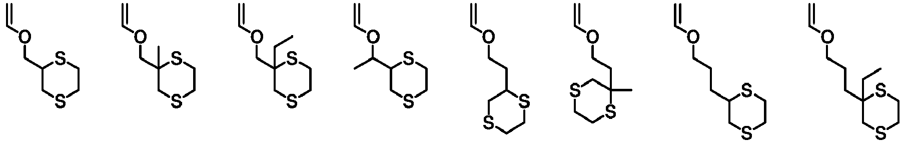

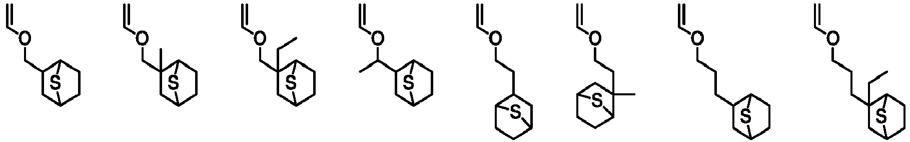

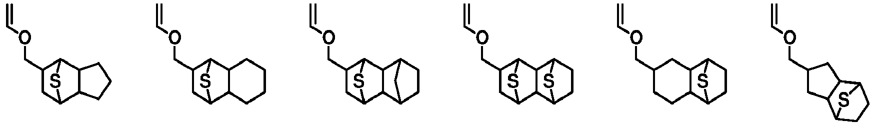

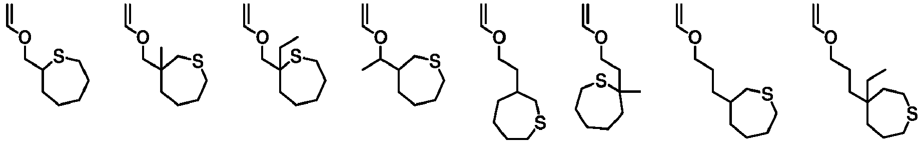

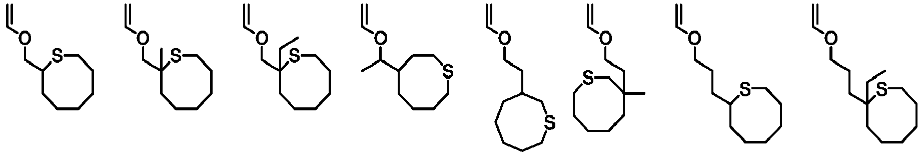

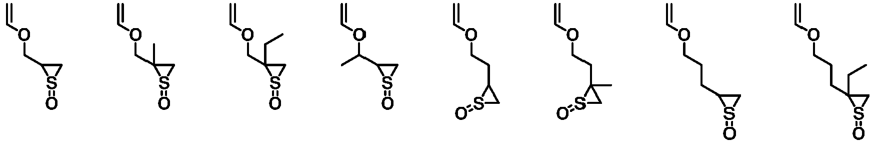

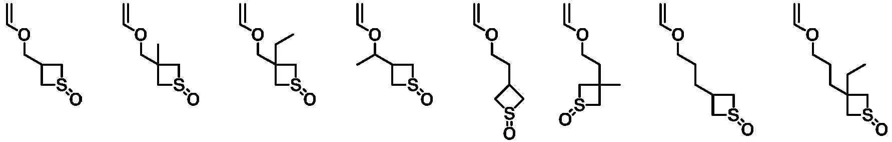

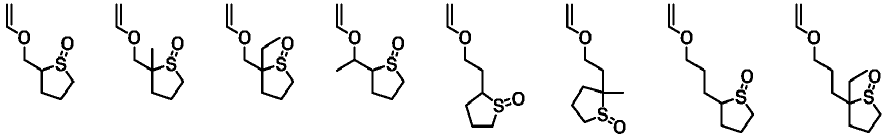

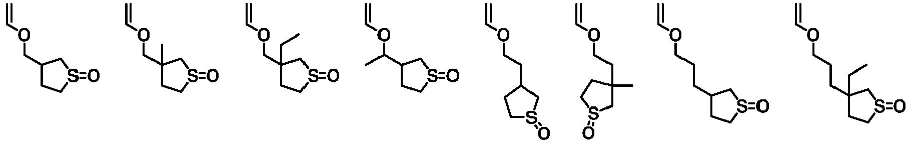

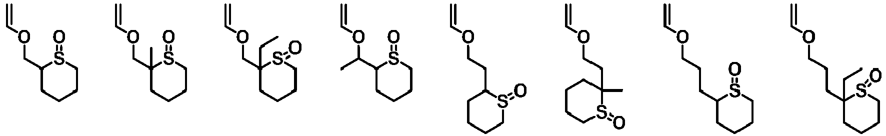

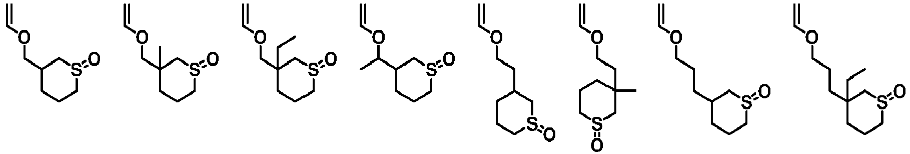

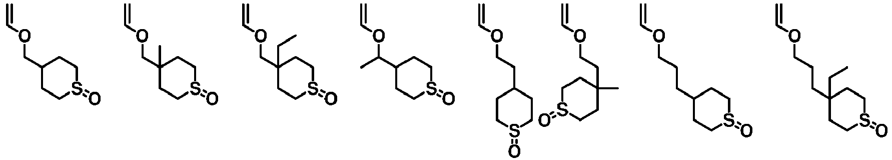

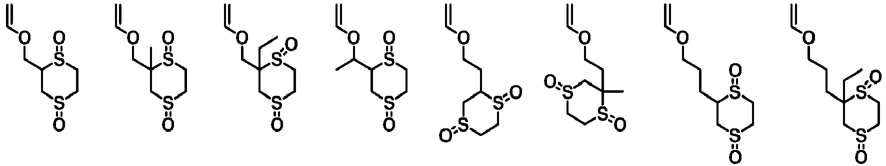

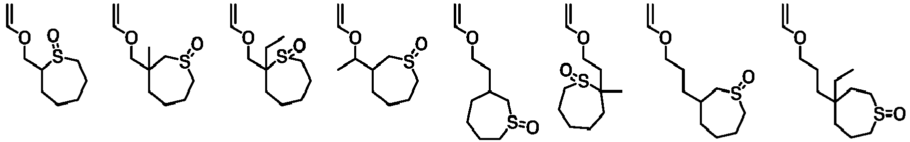

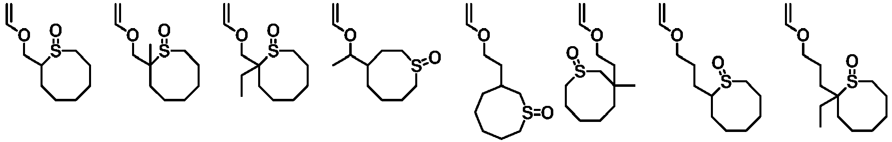

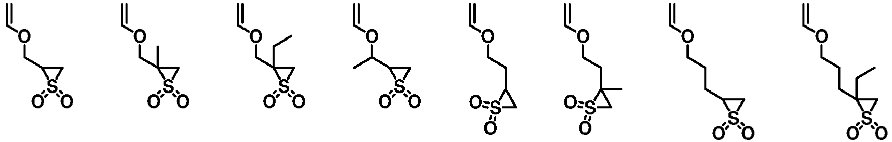

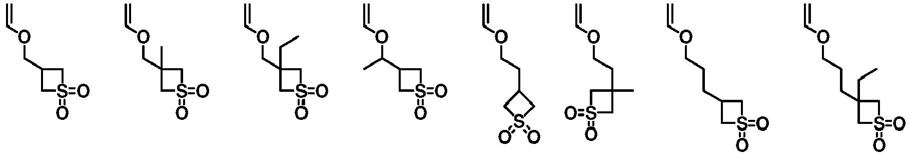

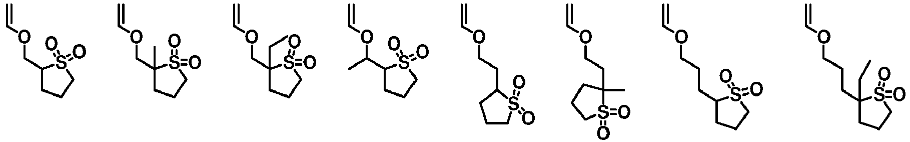

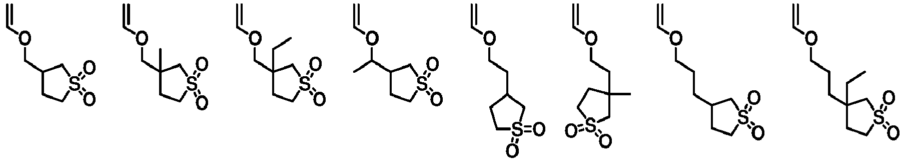

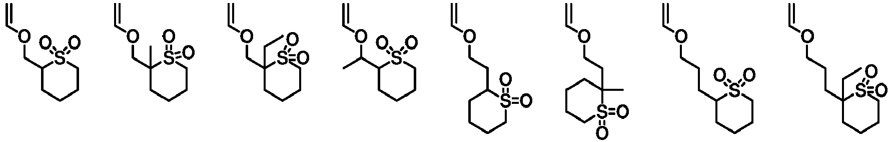

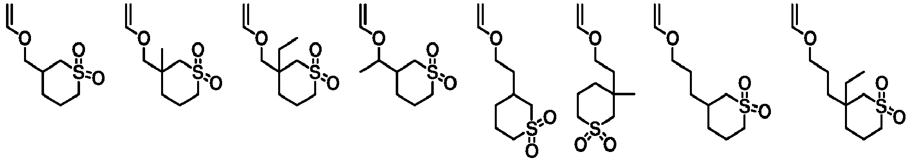

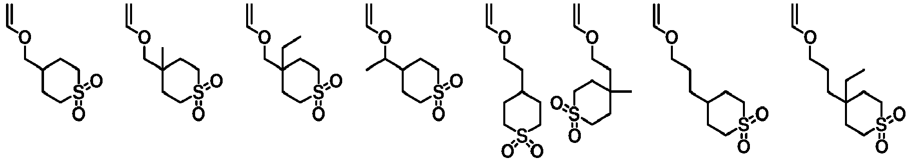

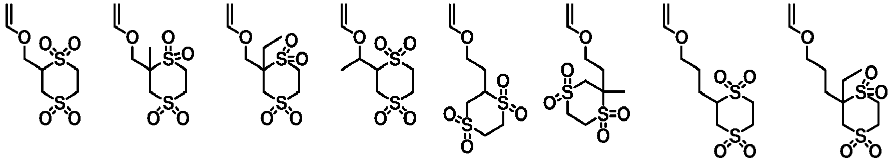

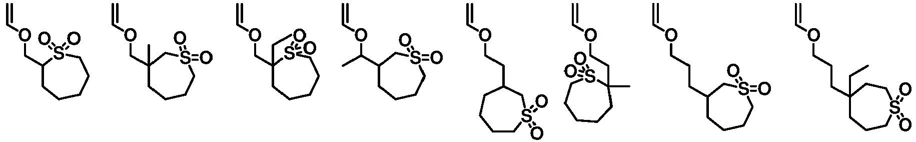

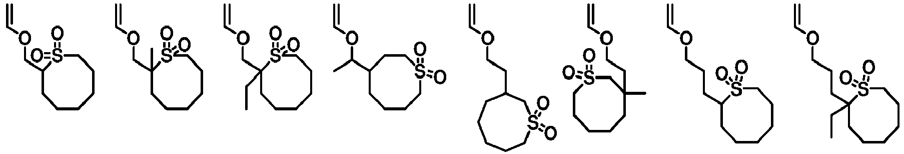

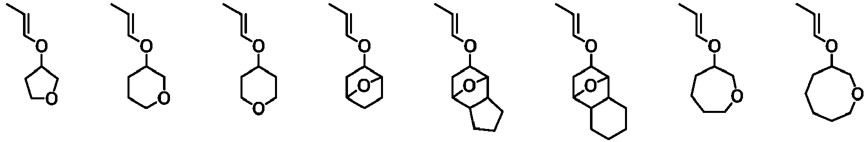

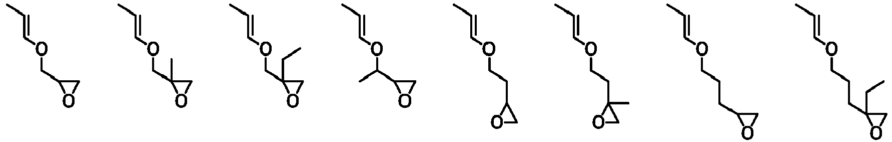

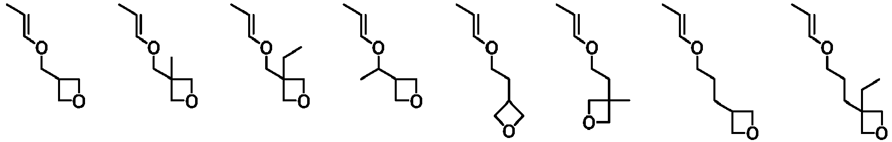

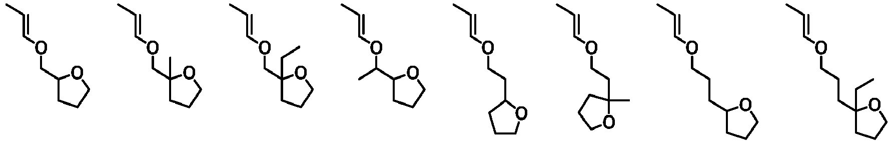

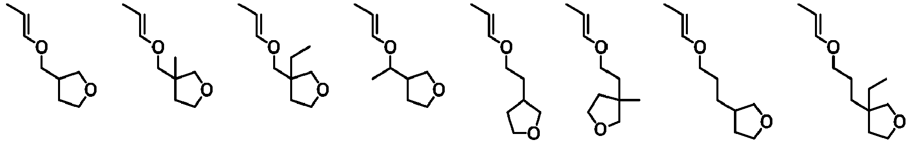

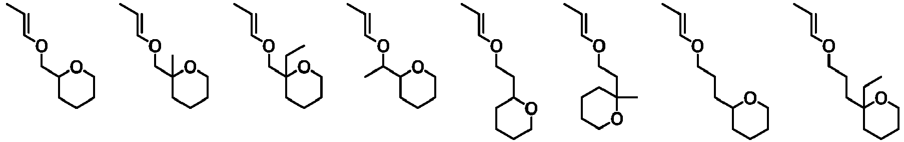

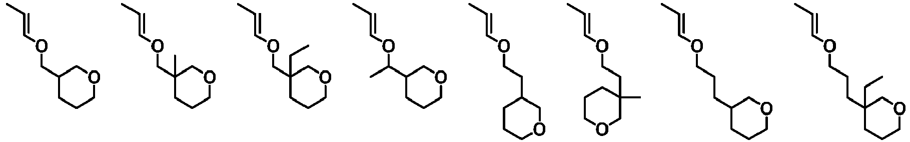

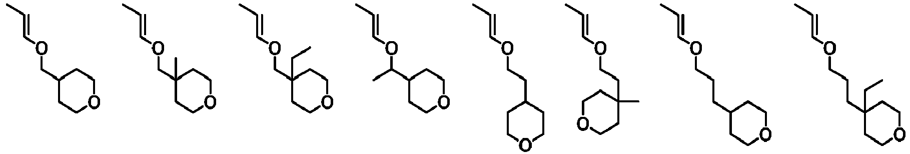

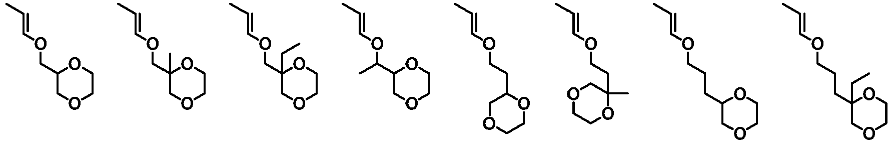

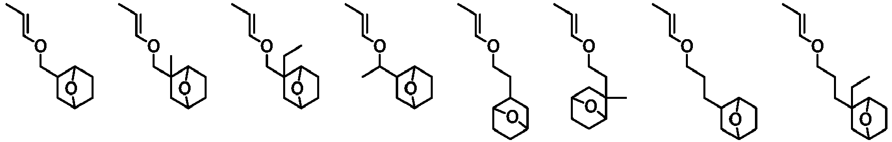

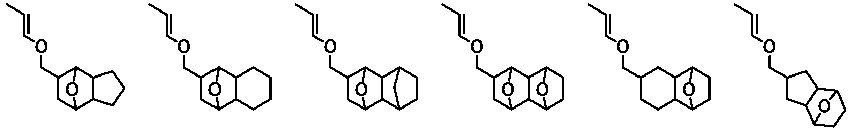

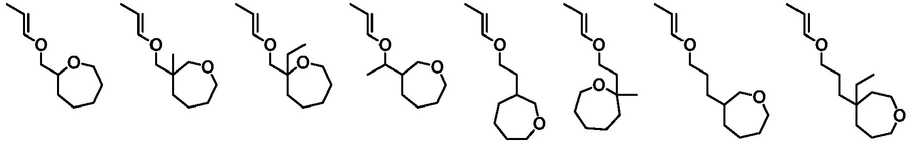

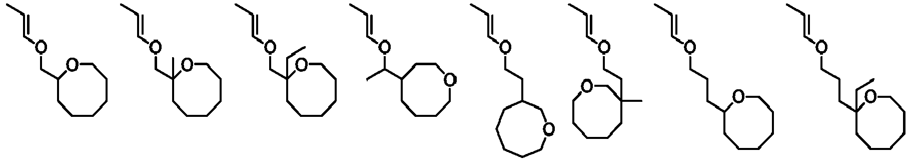

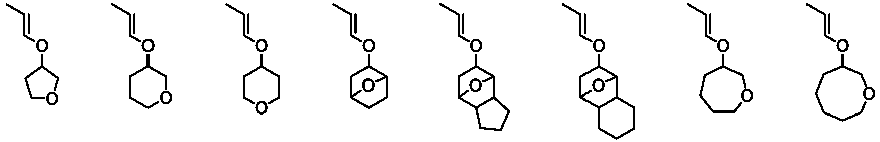

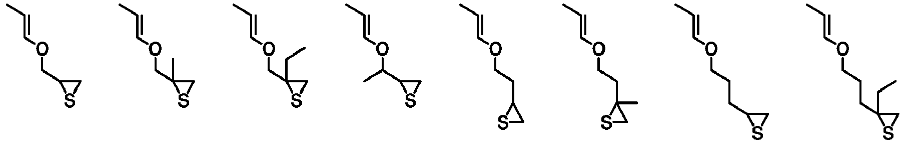

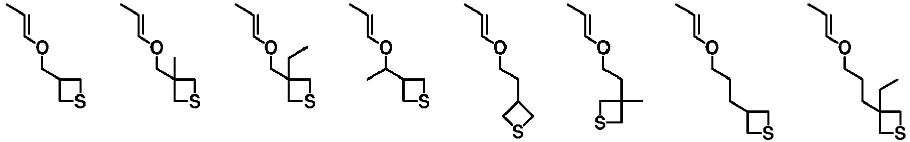

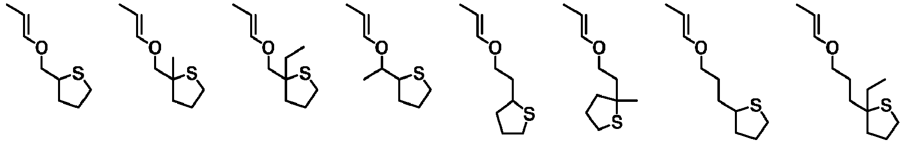

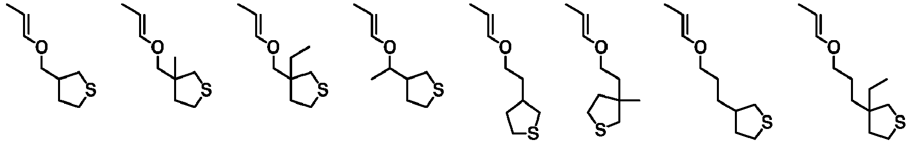

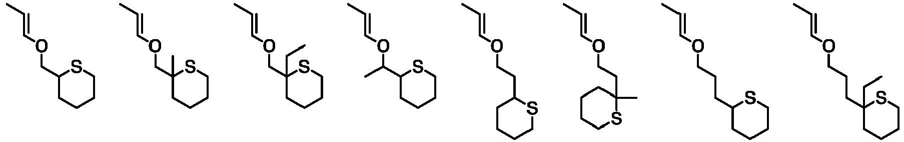

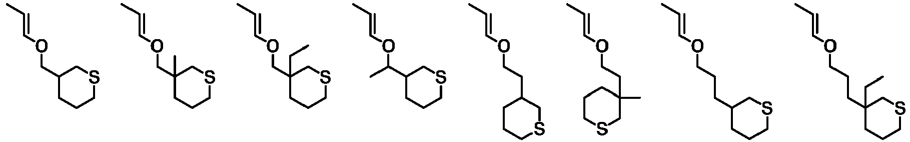

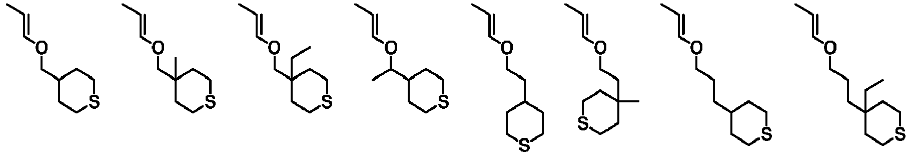

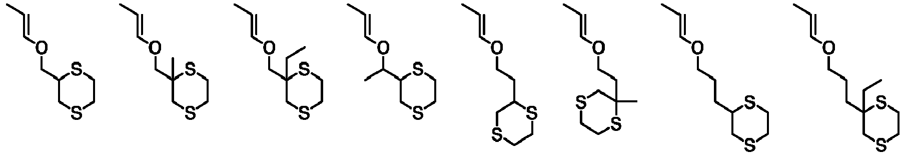

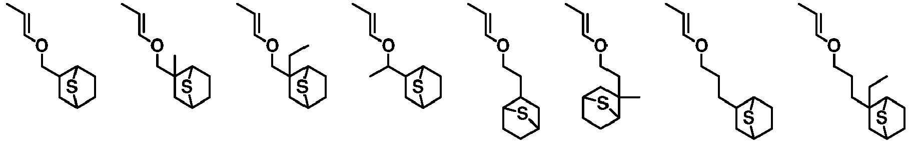

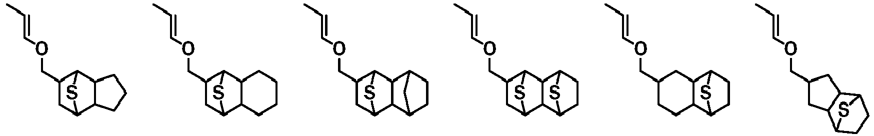

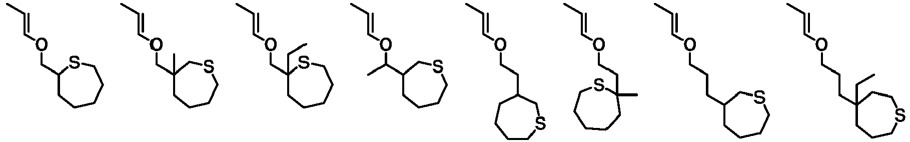

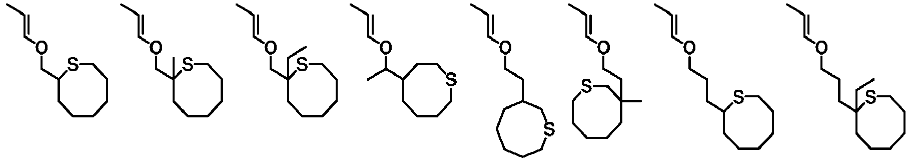

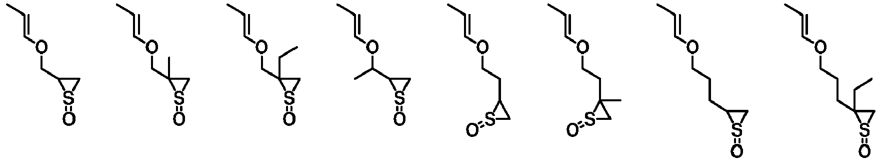

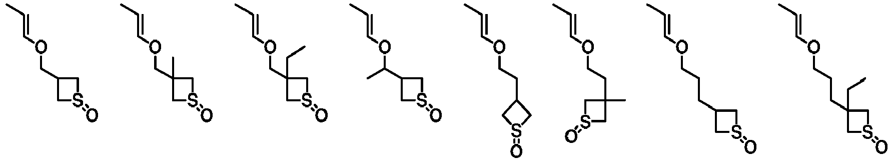

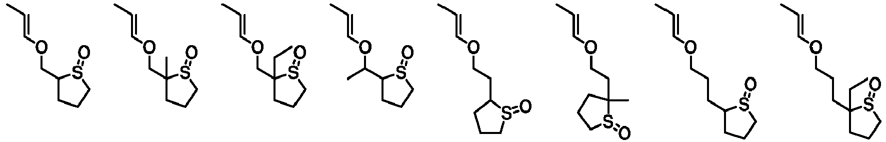

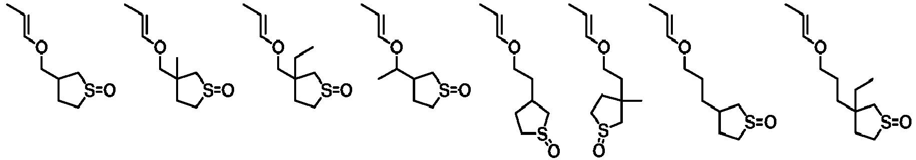

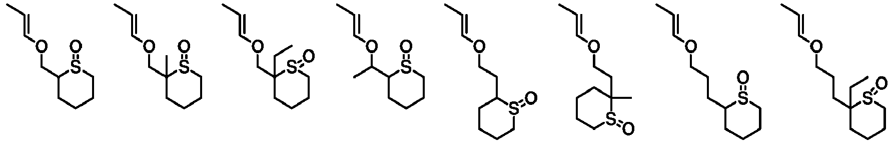

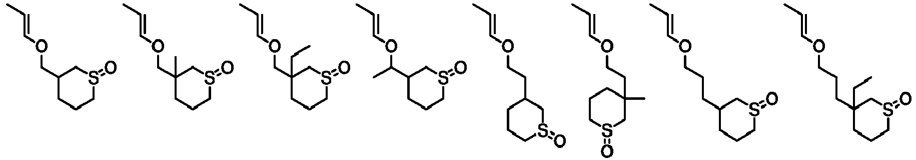

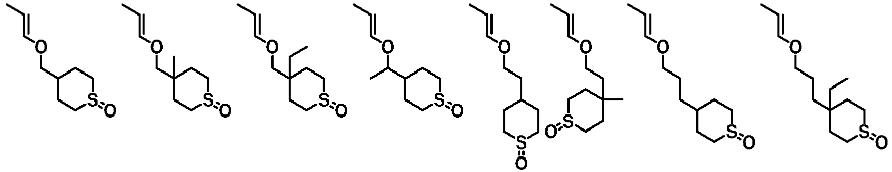

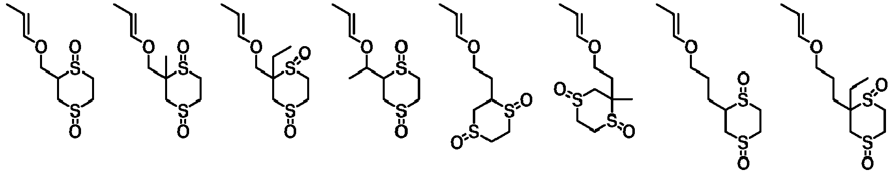

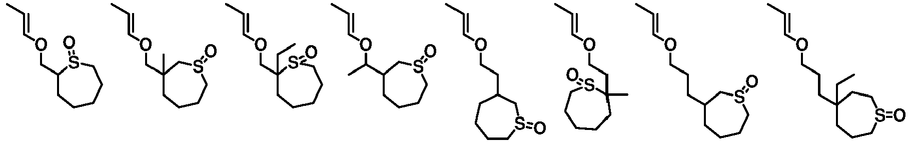

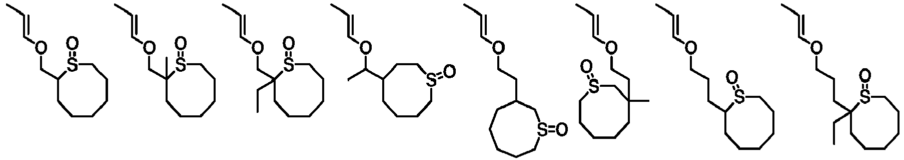

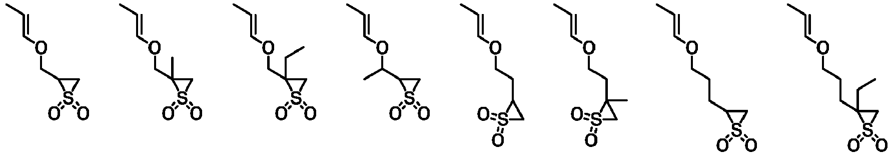

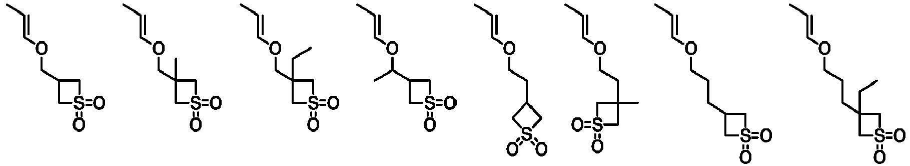

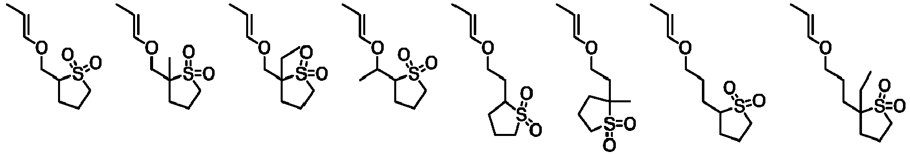

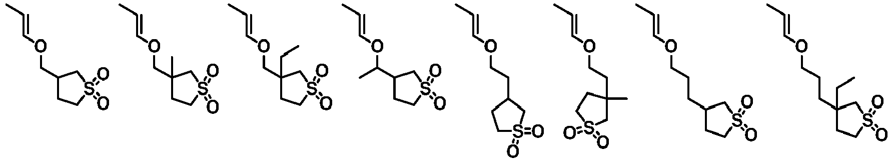

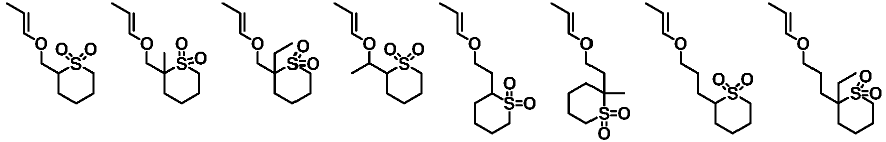

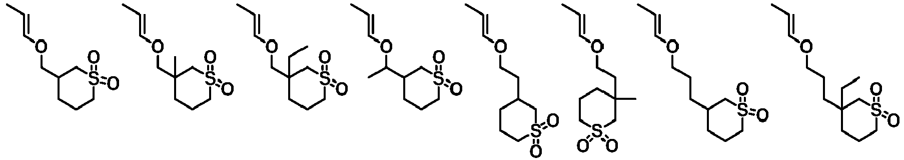

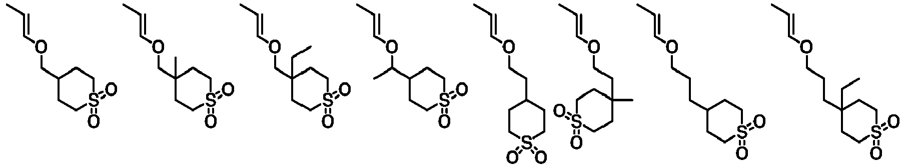

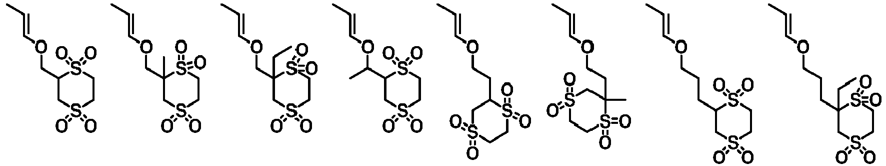

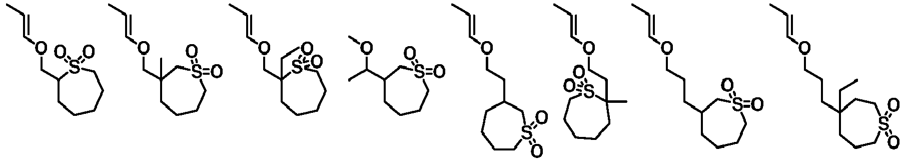

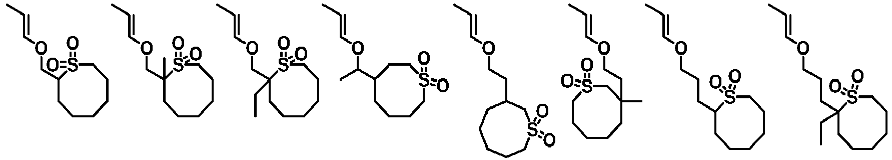

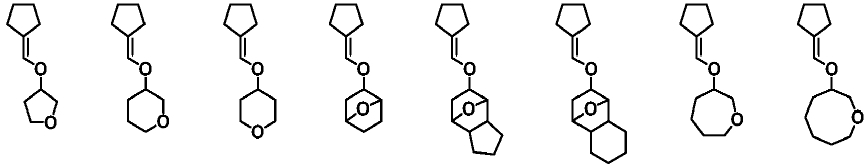

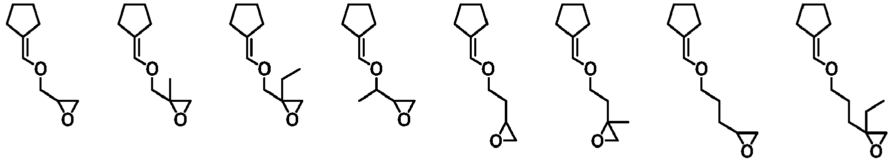

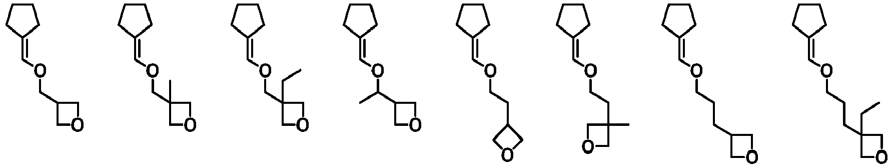

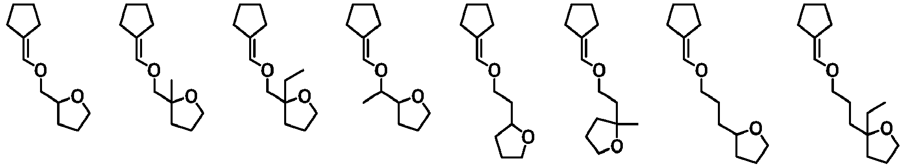

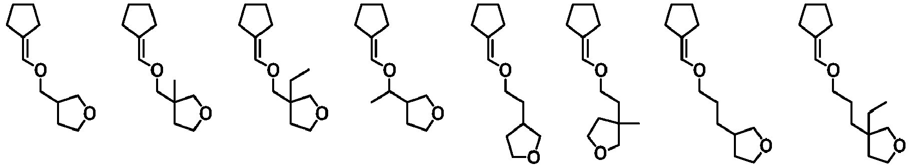

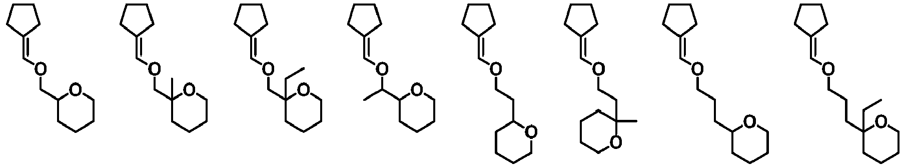

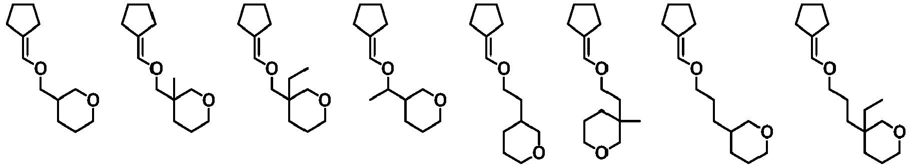

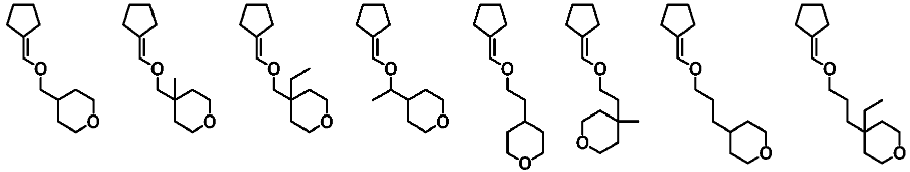

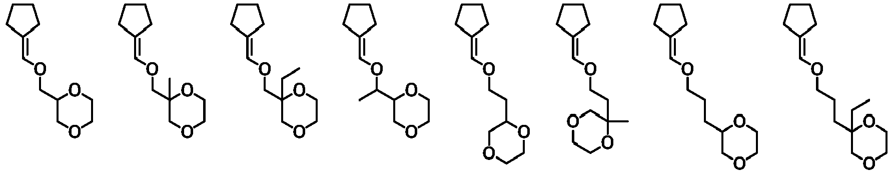

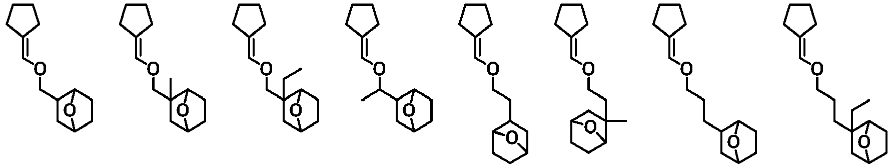

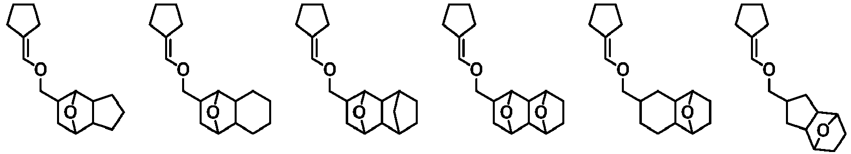

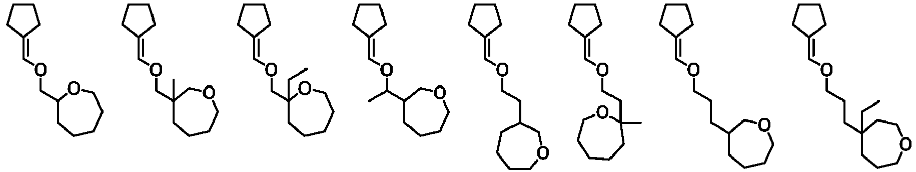

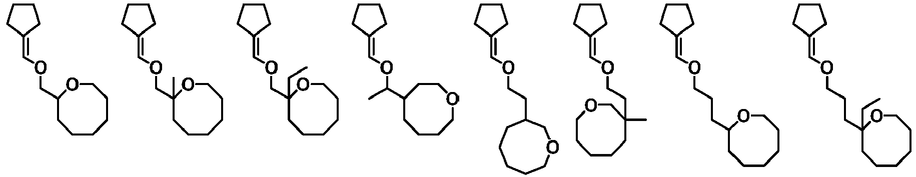

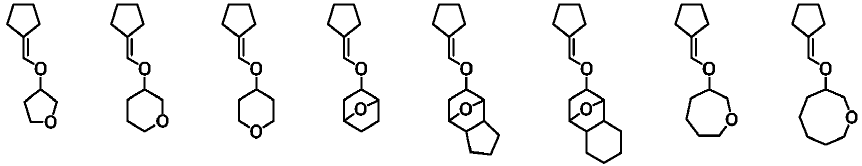

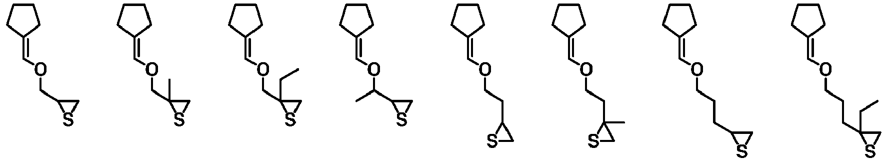

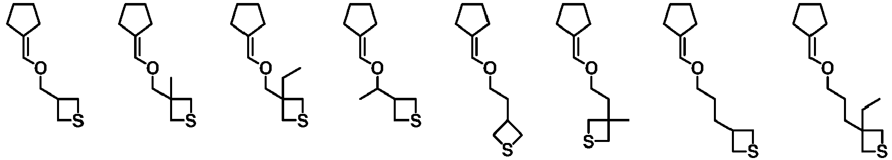

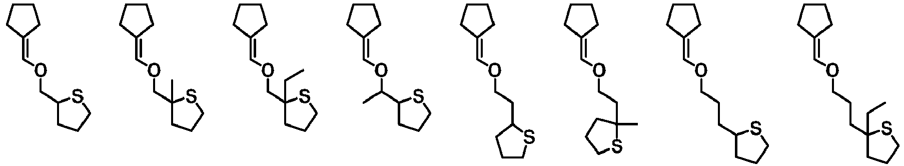

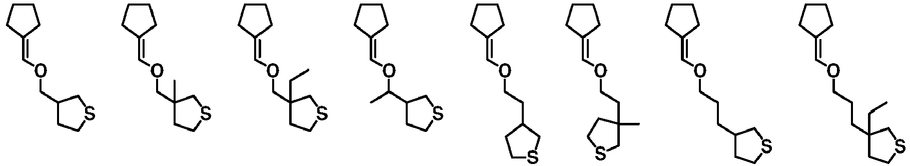

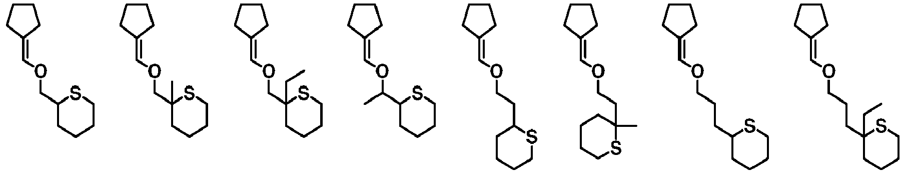

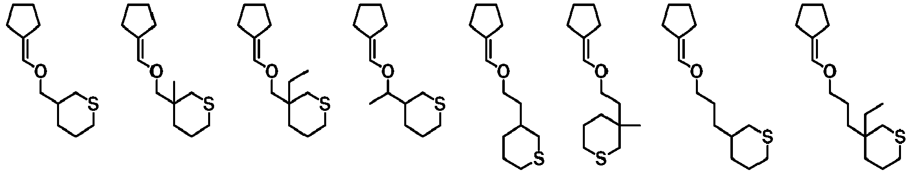

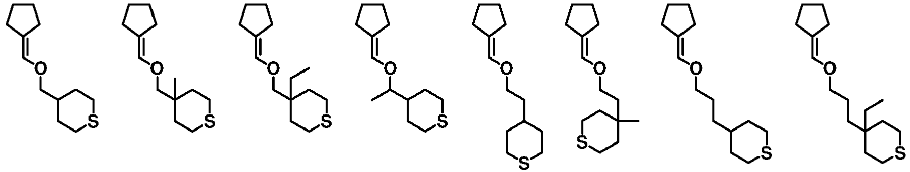

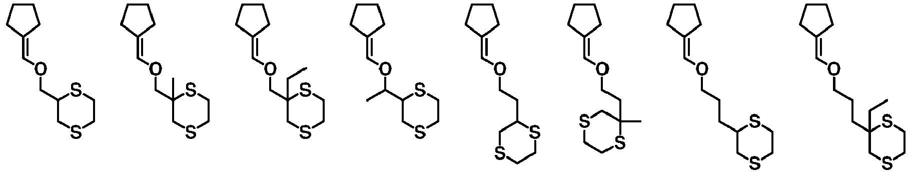

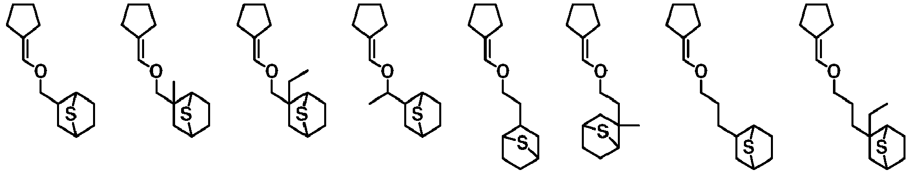

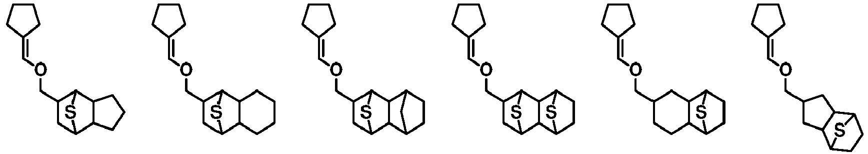

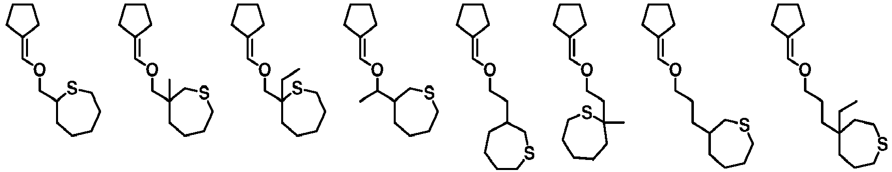

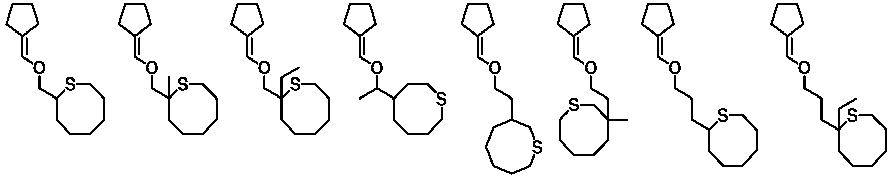

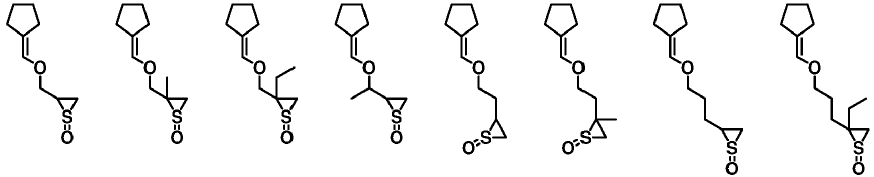

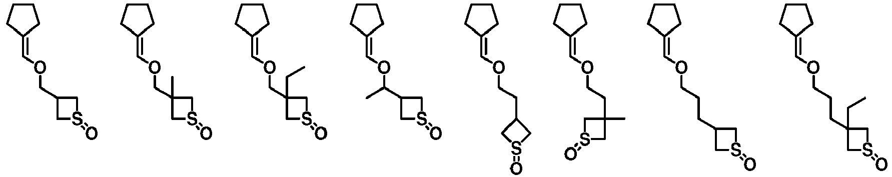

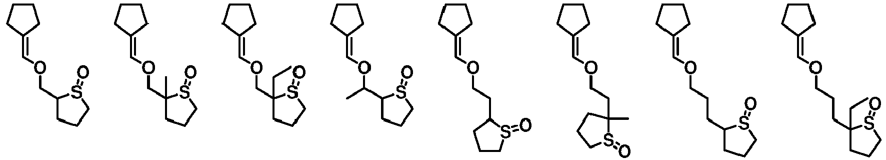

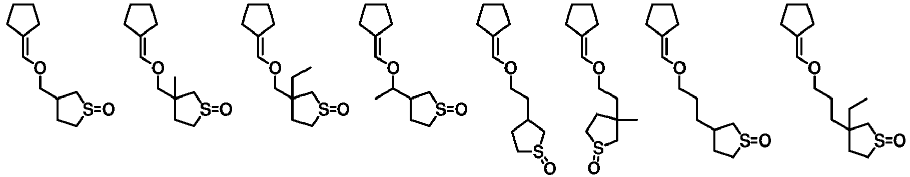

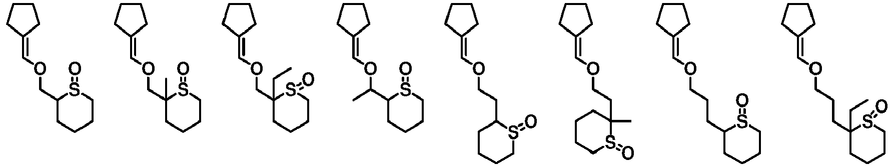

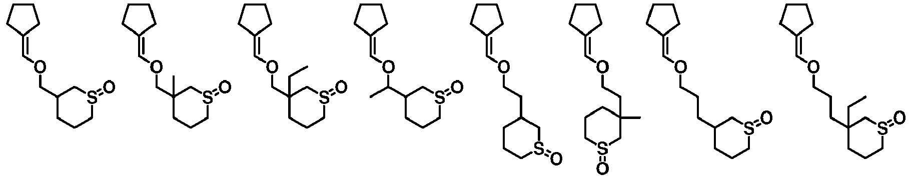

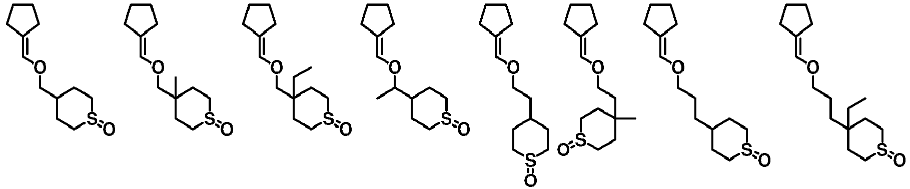

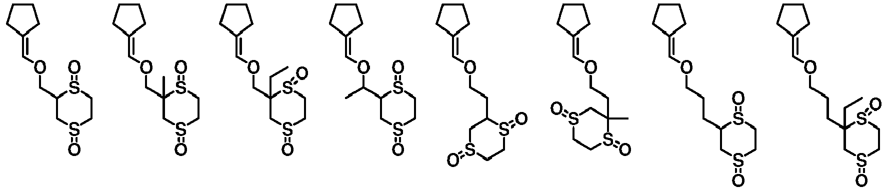

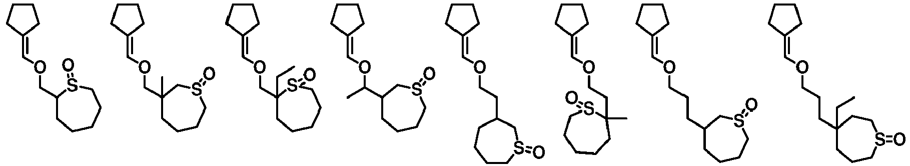

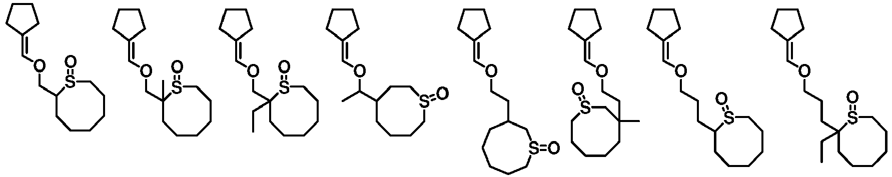

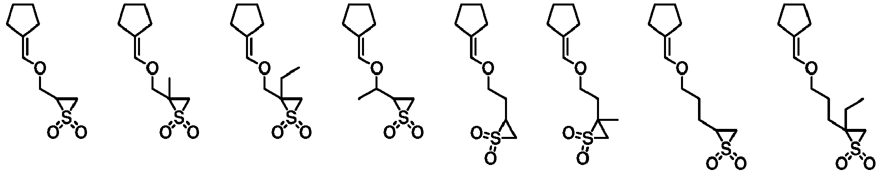

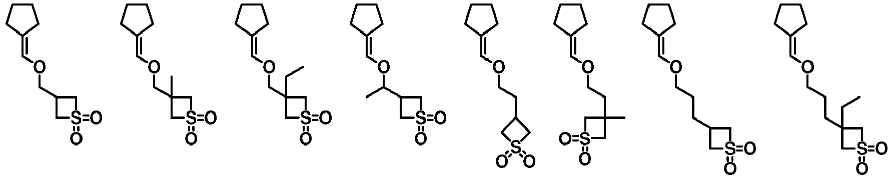

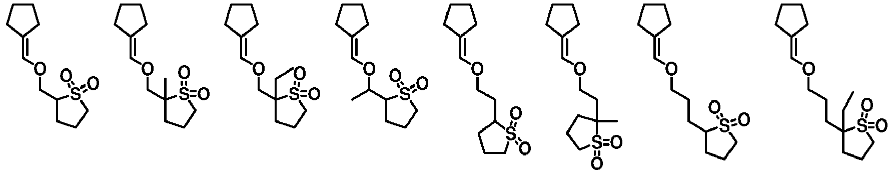

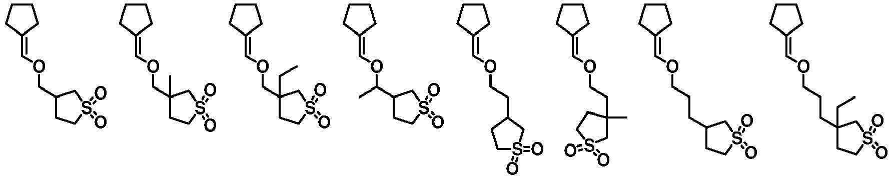

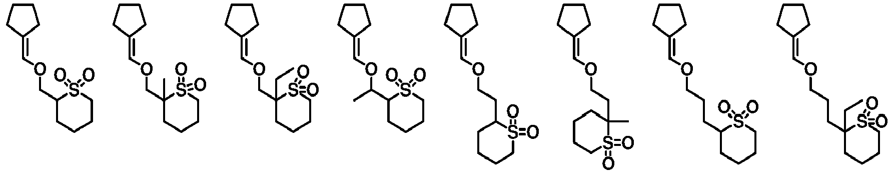

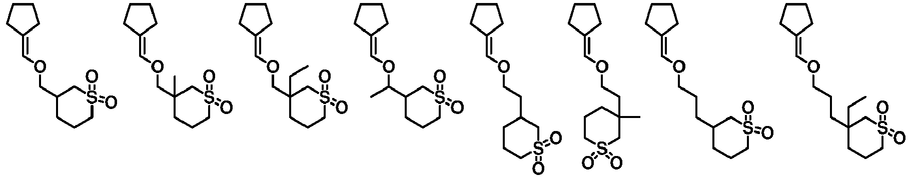

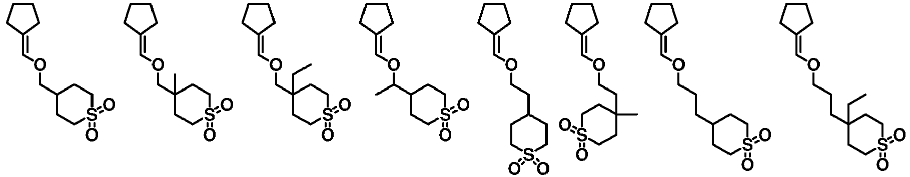

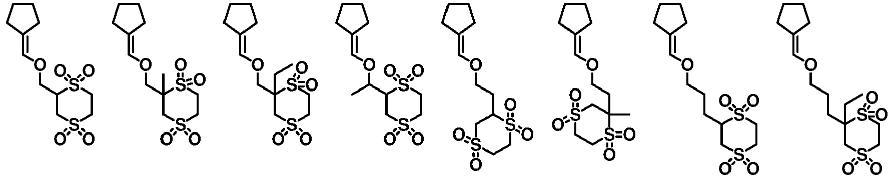

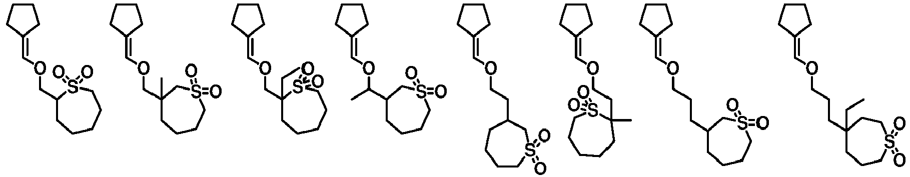

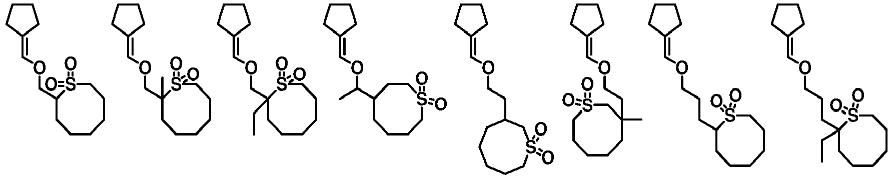

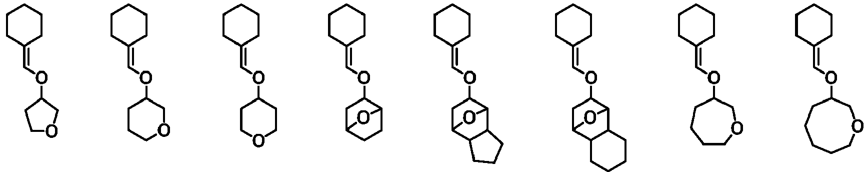

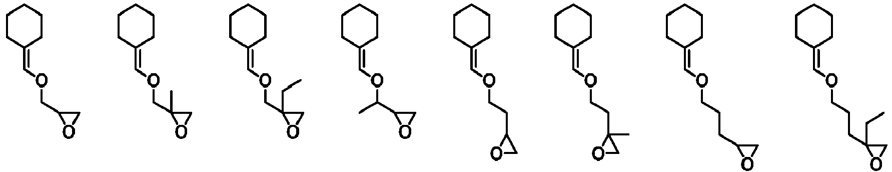

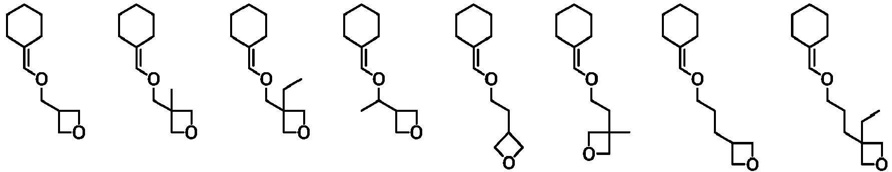

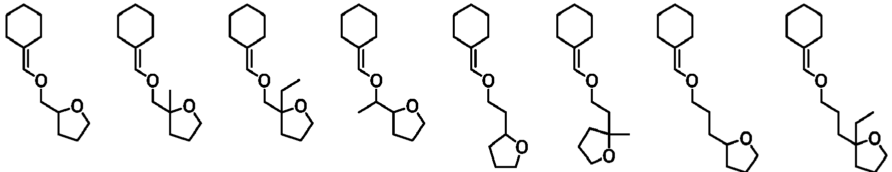

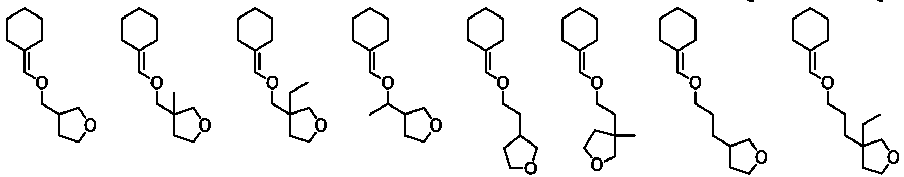

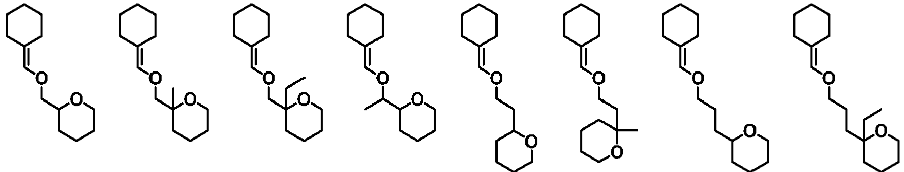

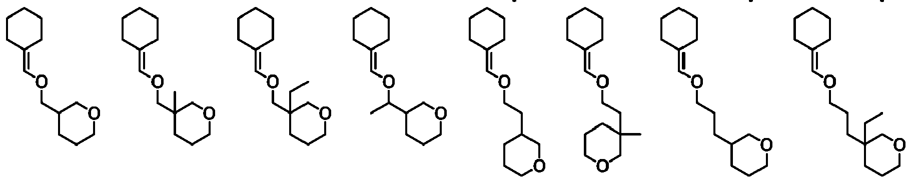

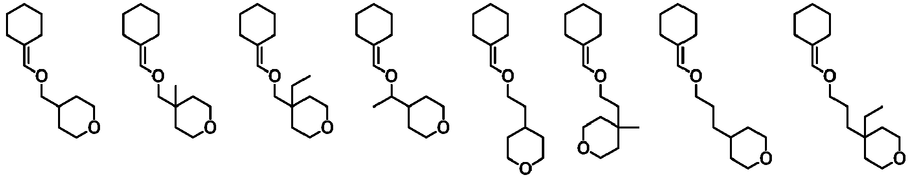

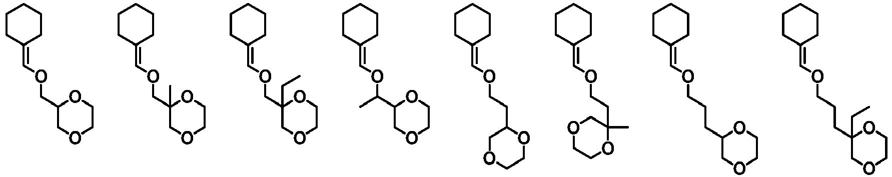

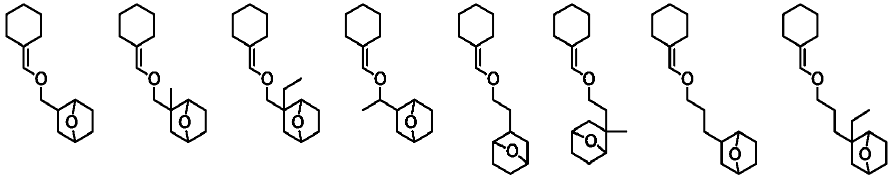

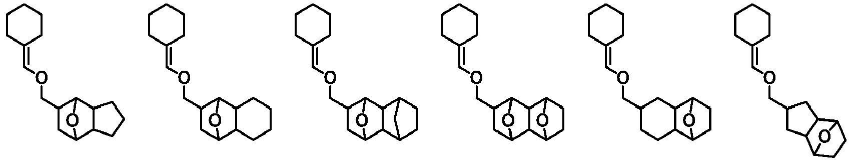

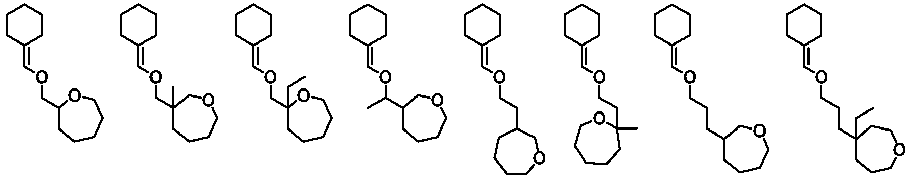

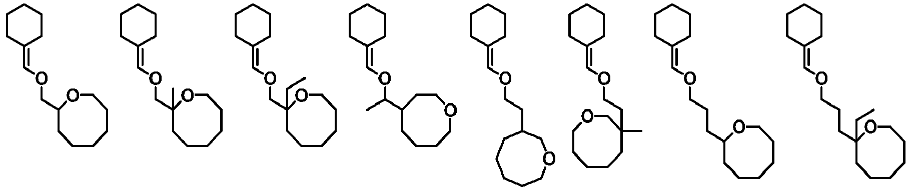

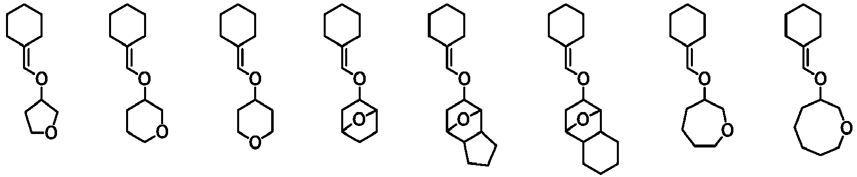

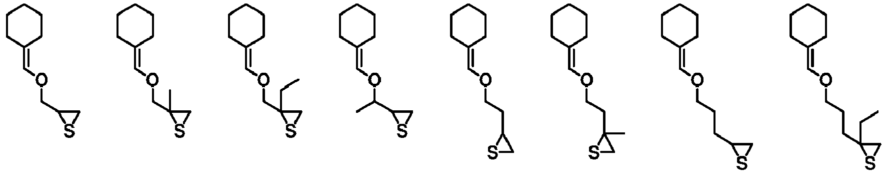

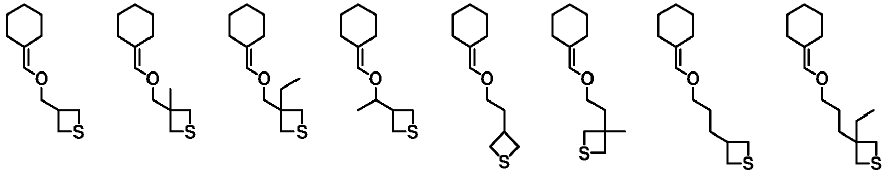

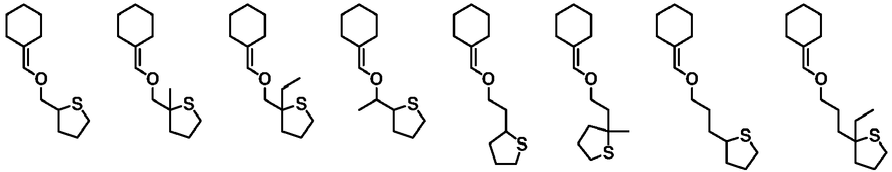

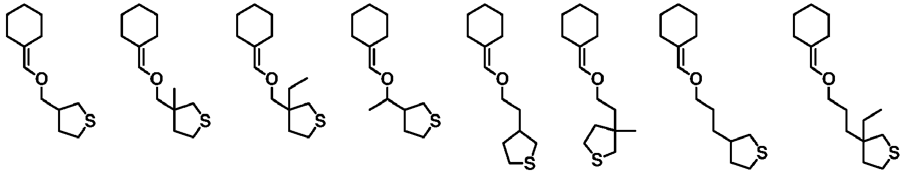

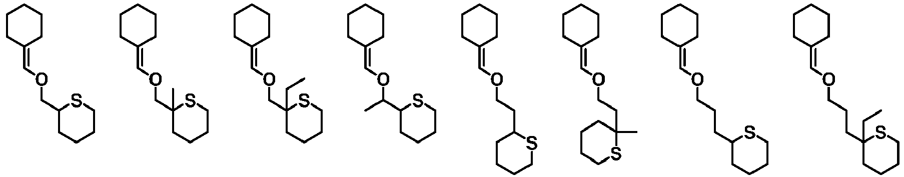

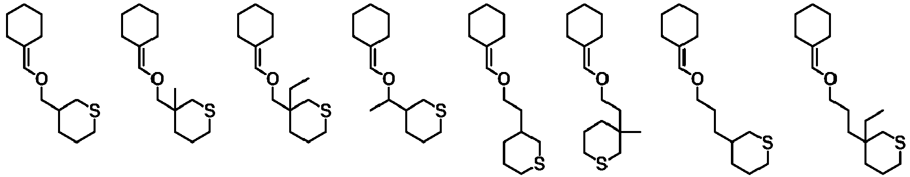

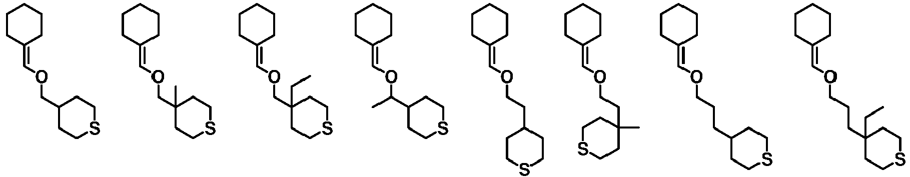

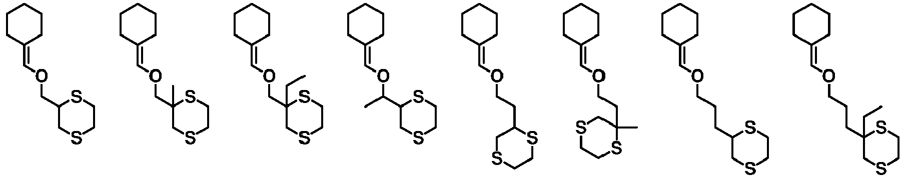

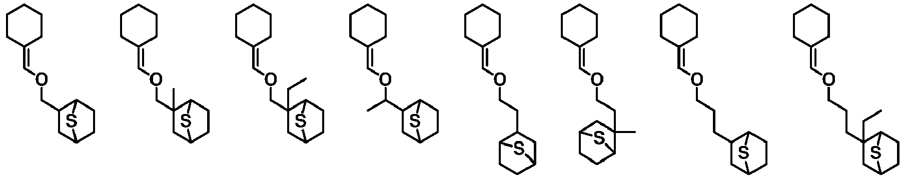

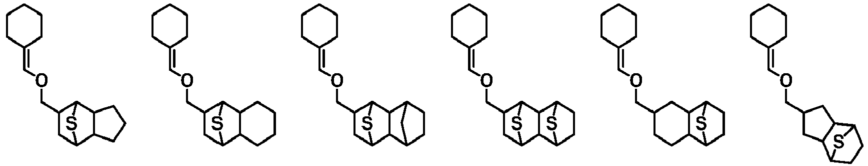

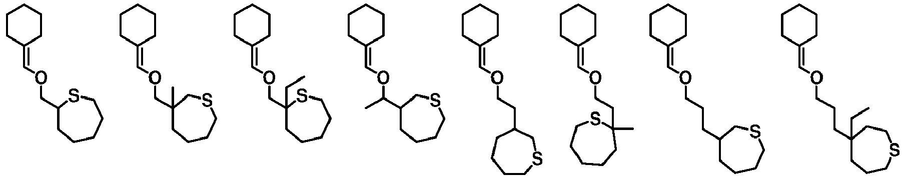

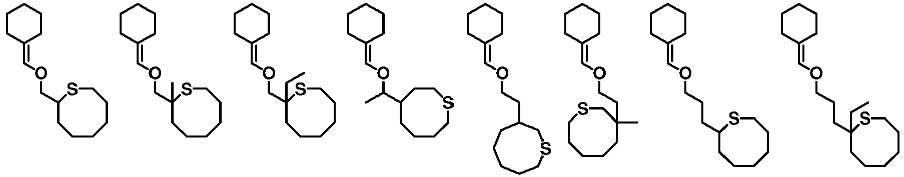

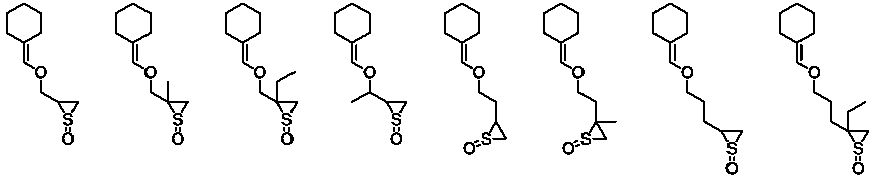

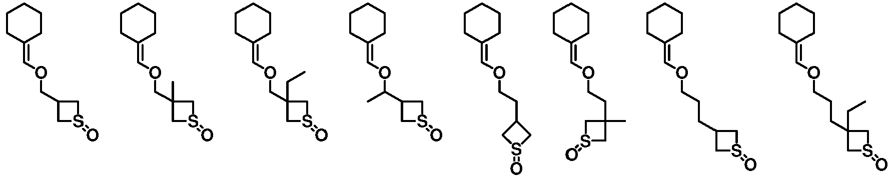

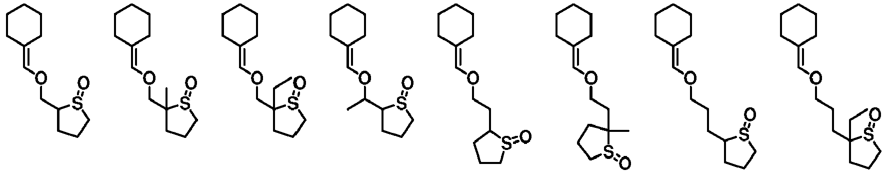

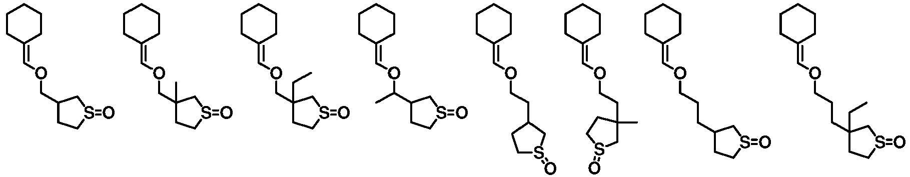

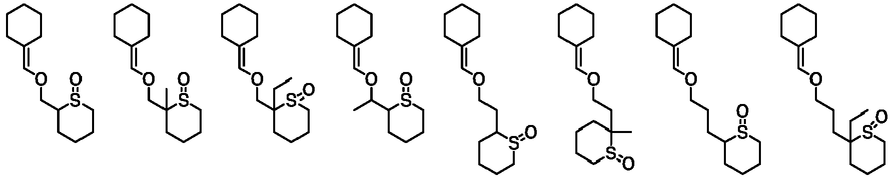

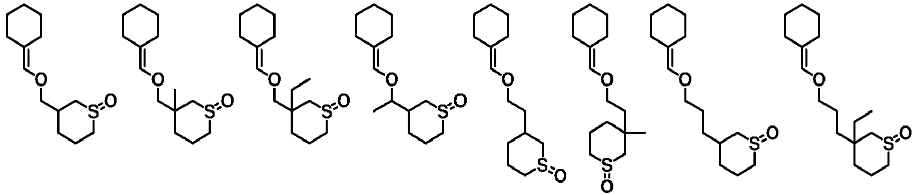

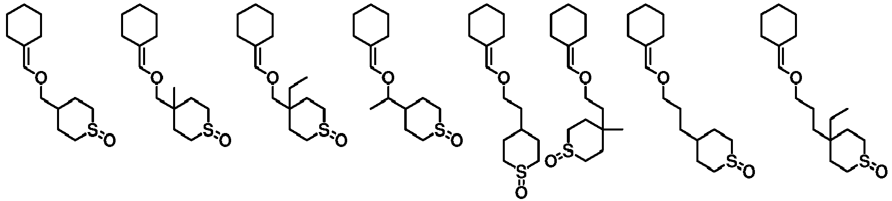

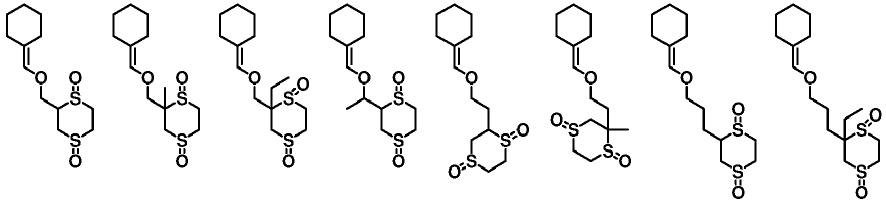

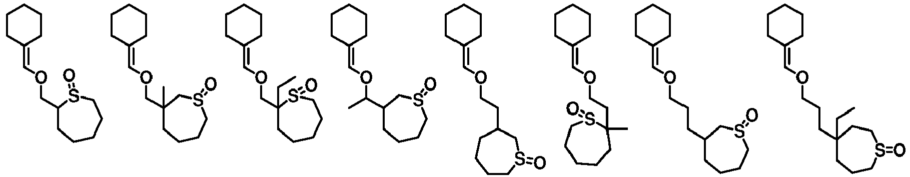

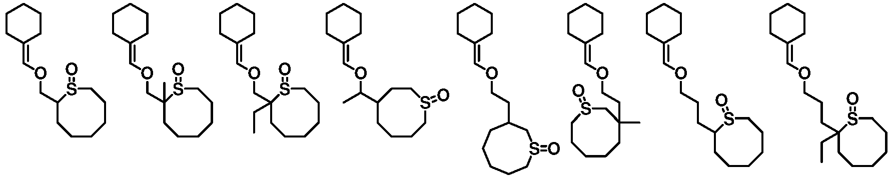

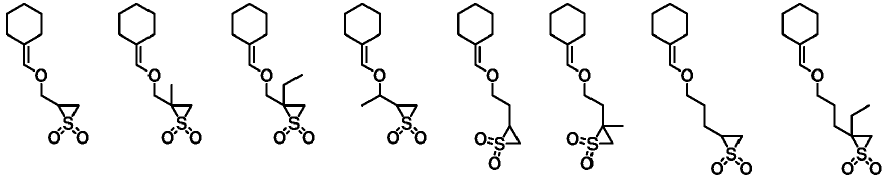

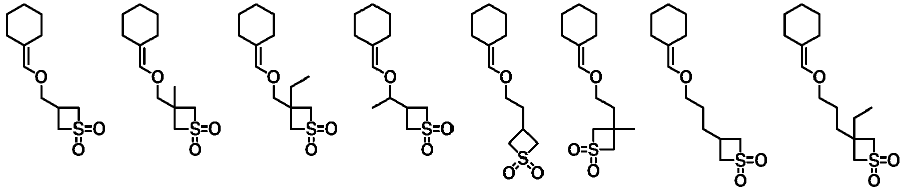

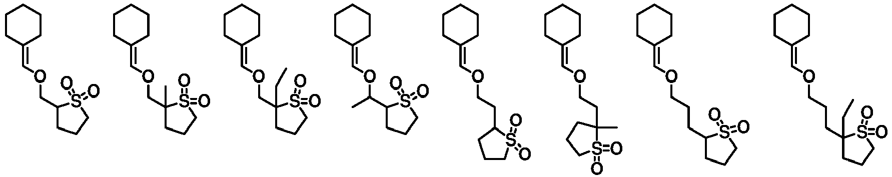

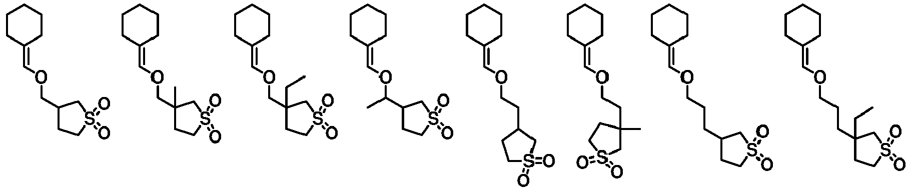

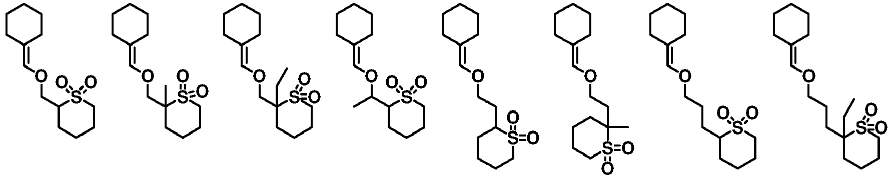

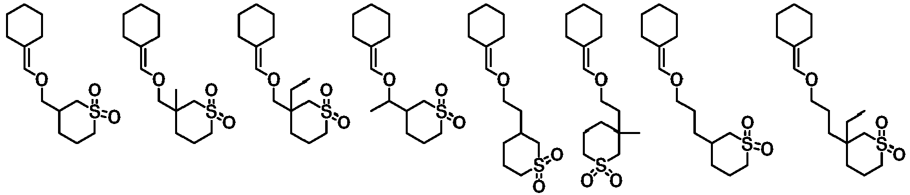

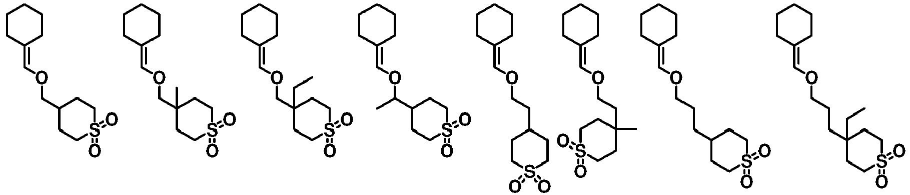

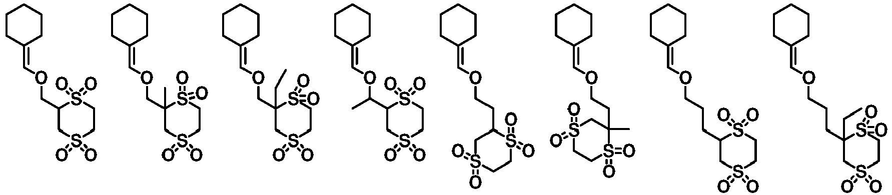

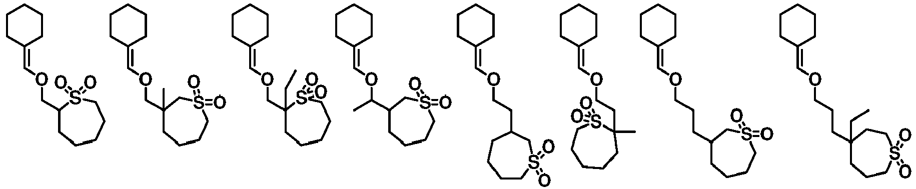

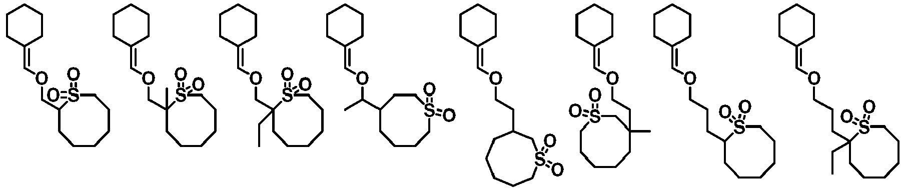

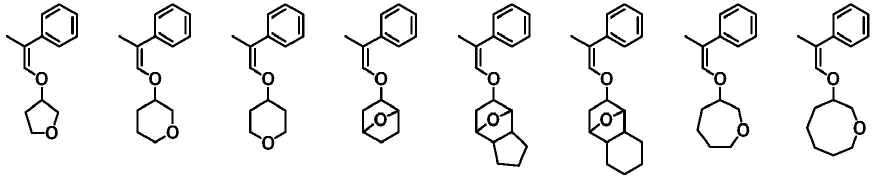

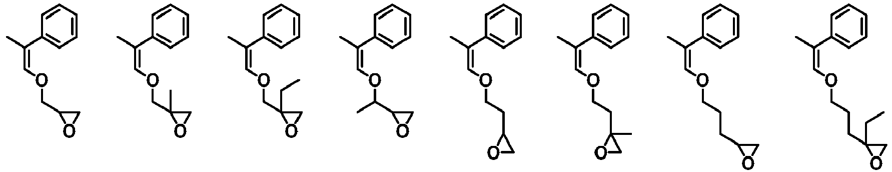

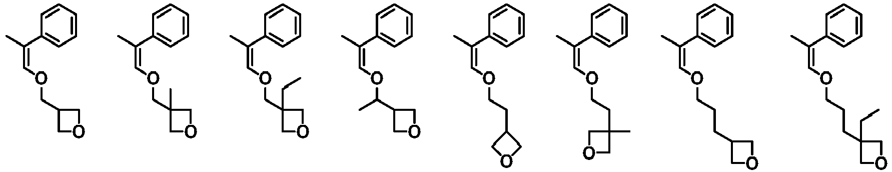

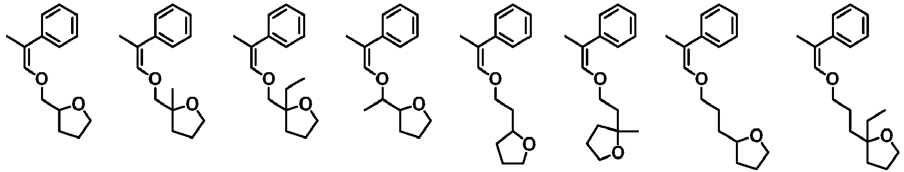

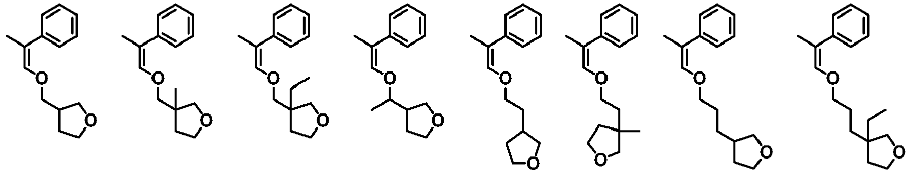

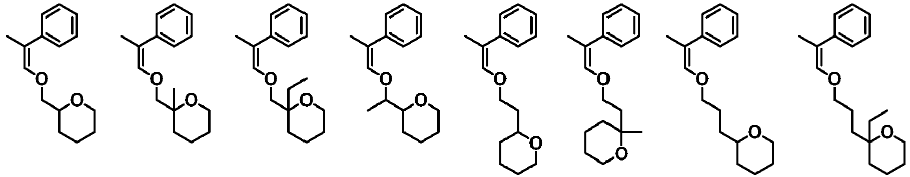

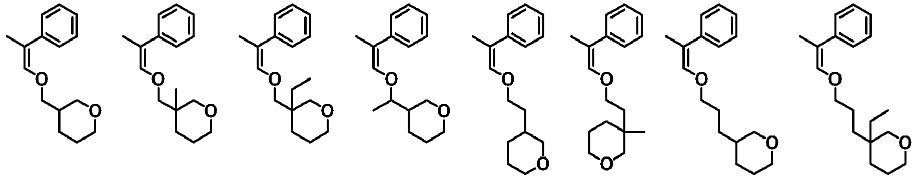

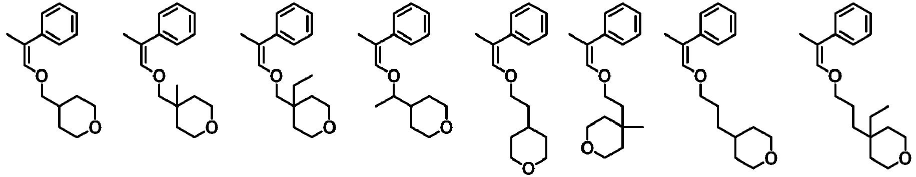

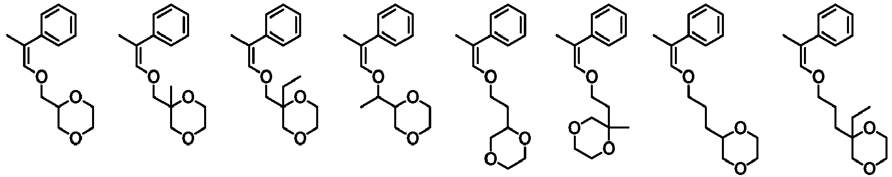

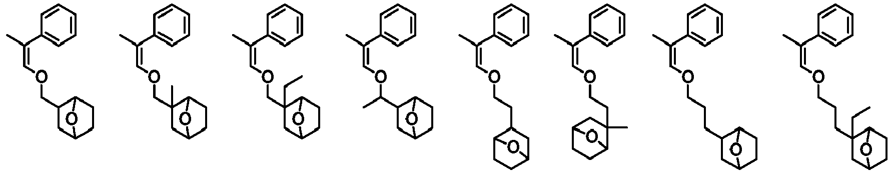

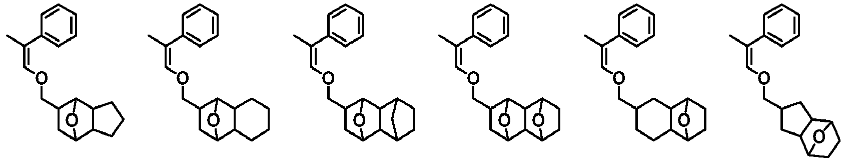

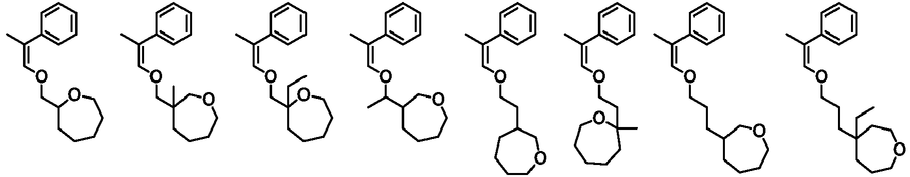

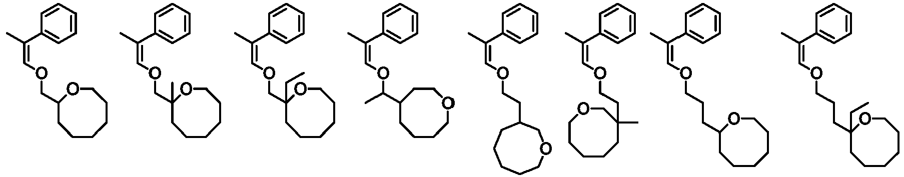

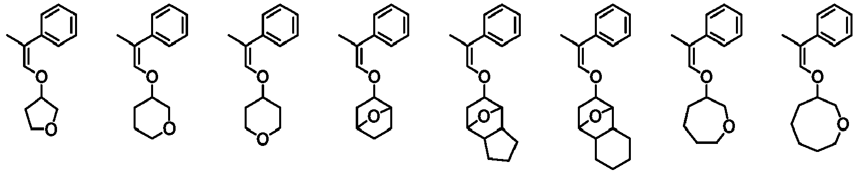

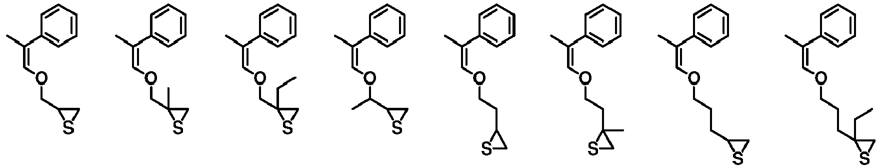

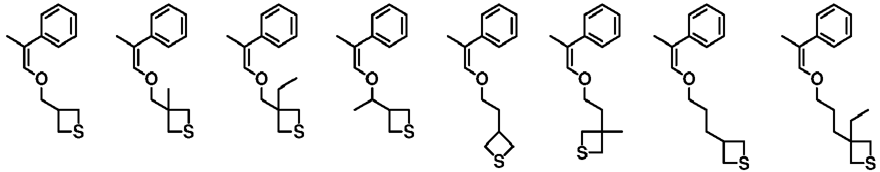

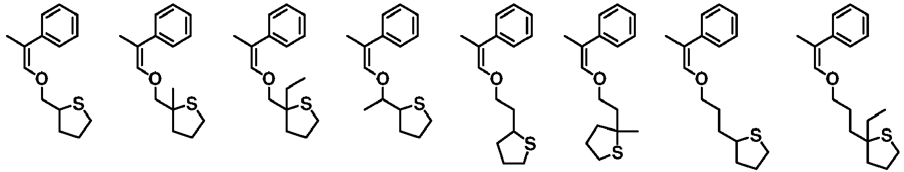

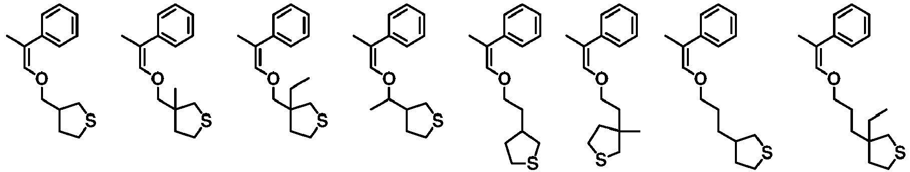

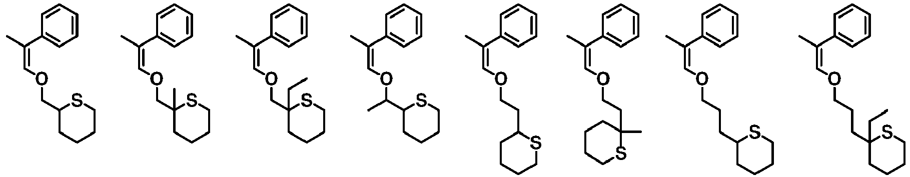

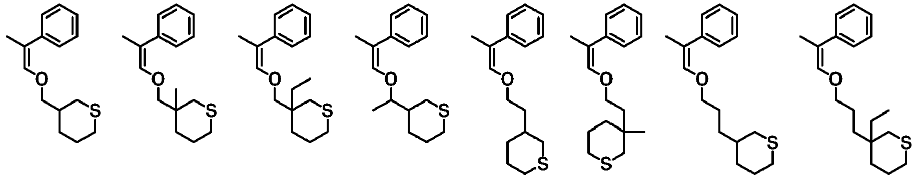

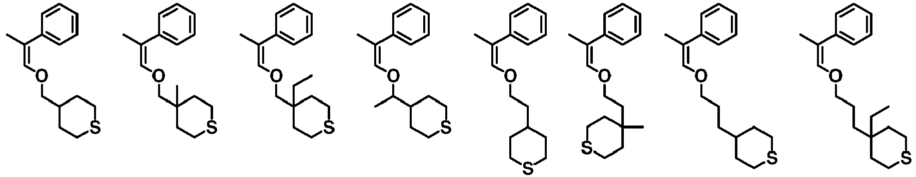

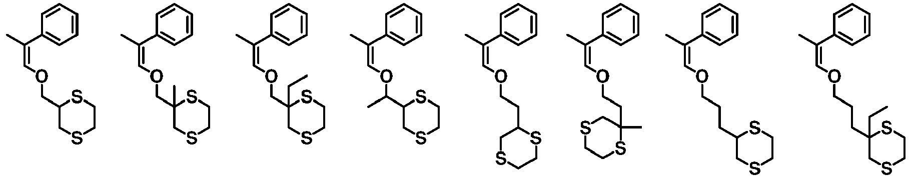

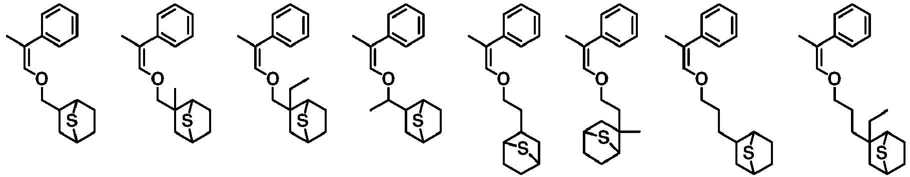

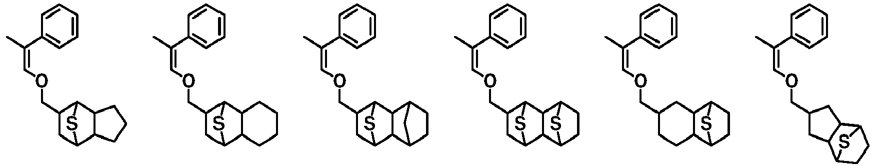

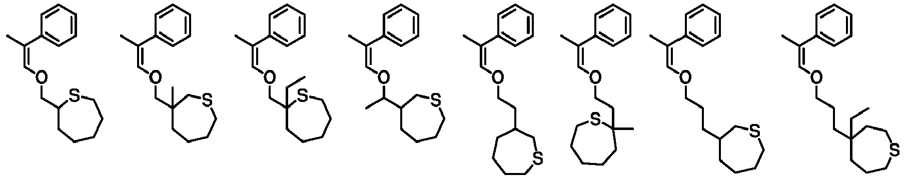

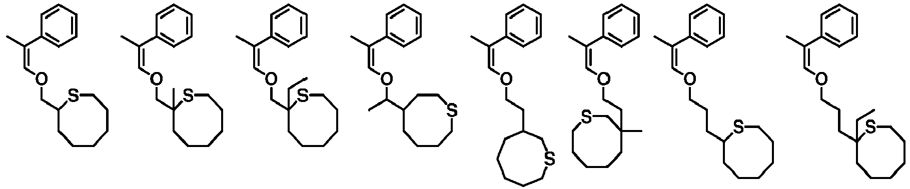

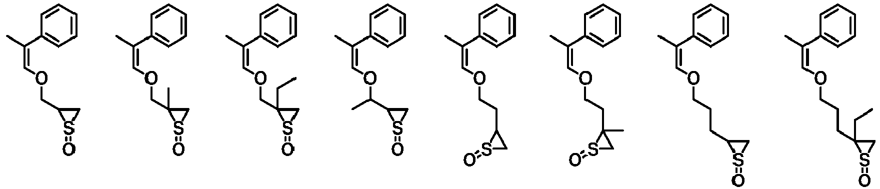

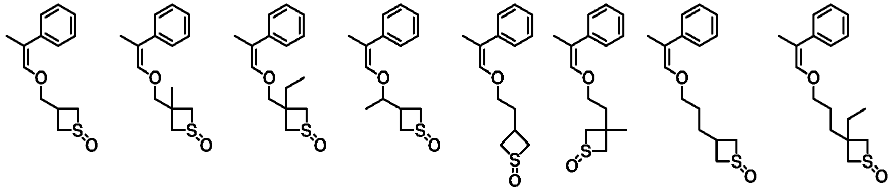

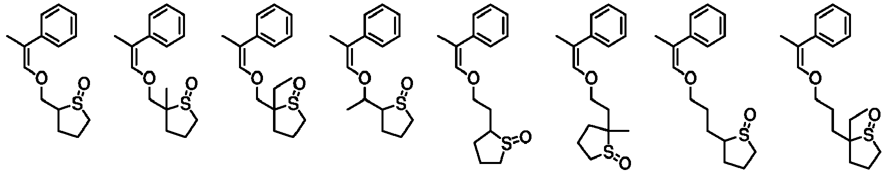

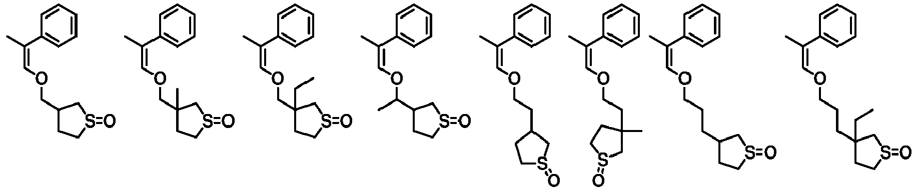

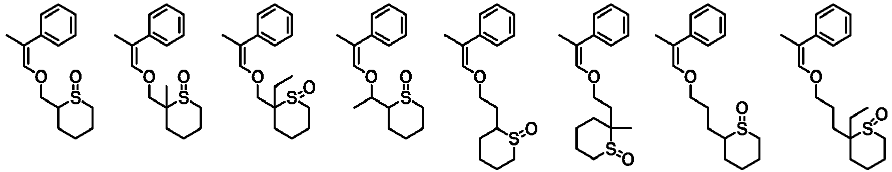

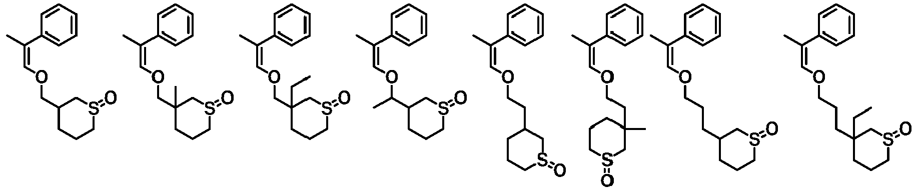

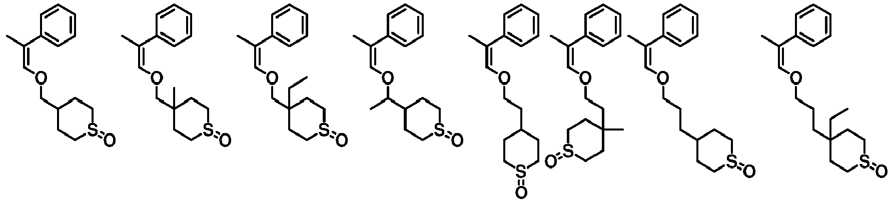

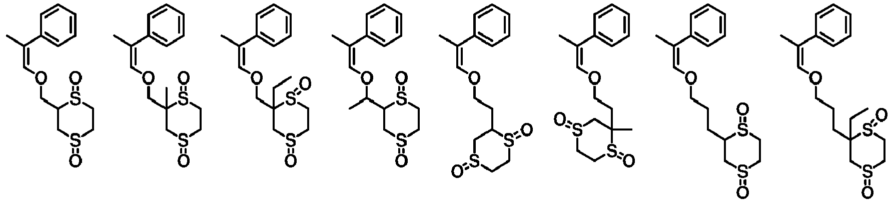

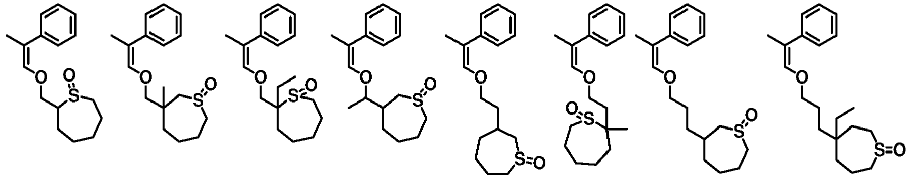

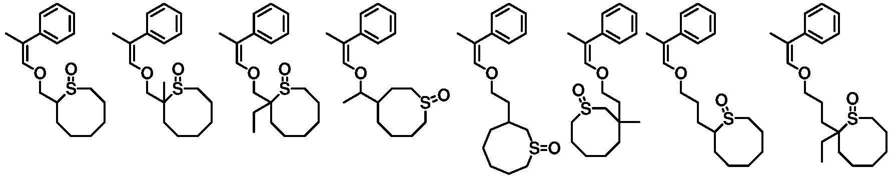

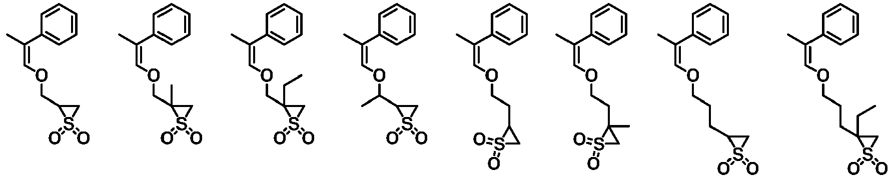

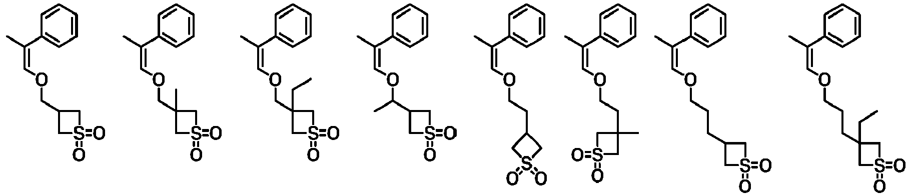

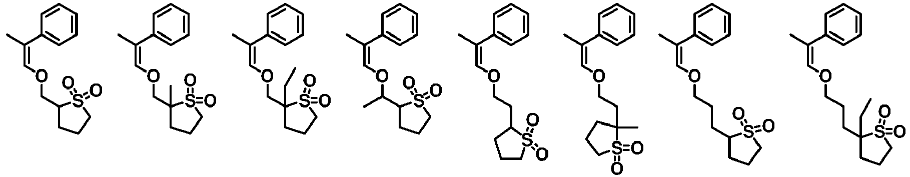

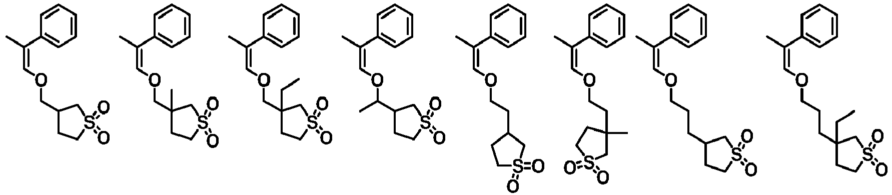

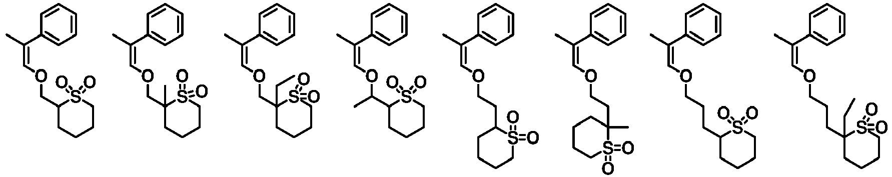

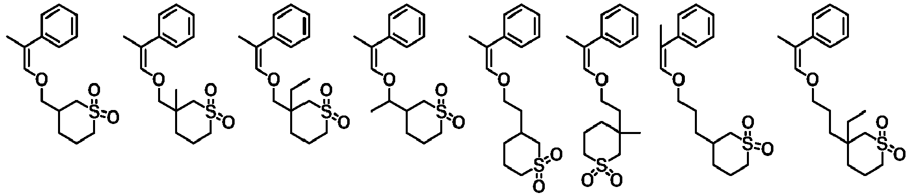

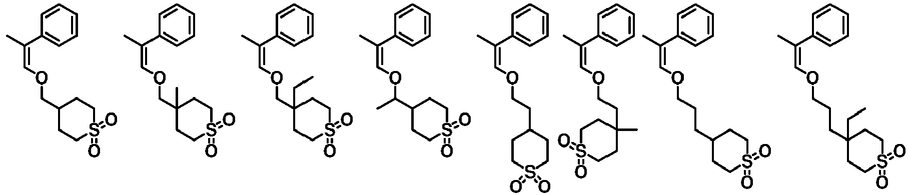

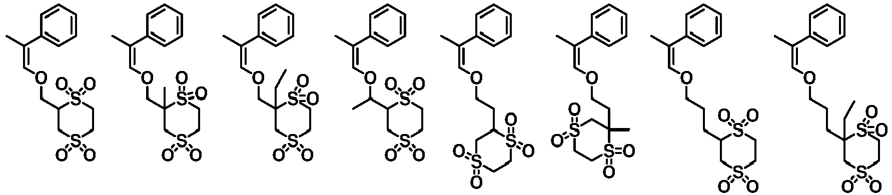

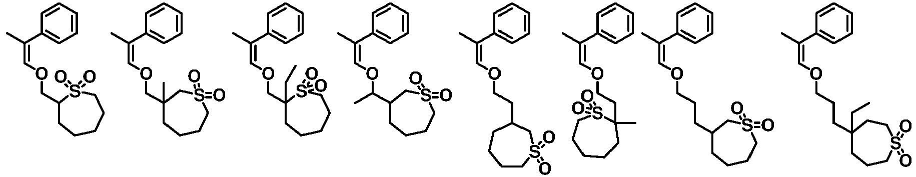

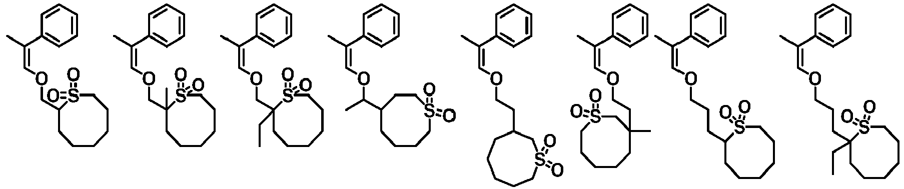

Examples of the acetal modifier having formula (AC-2) are shown below, but not limited thereto.

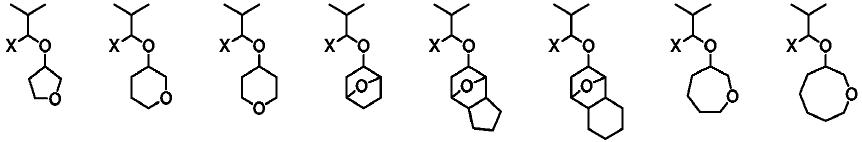

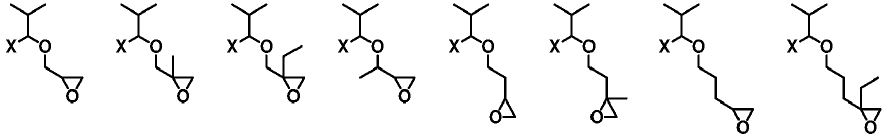

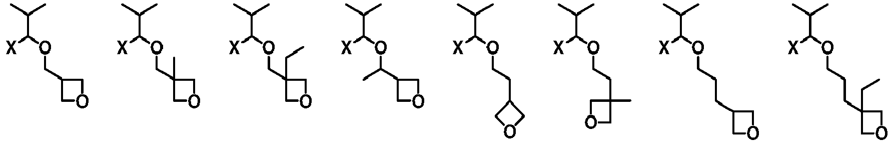

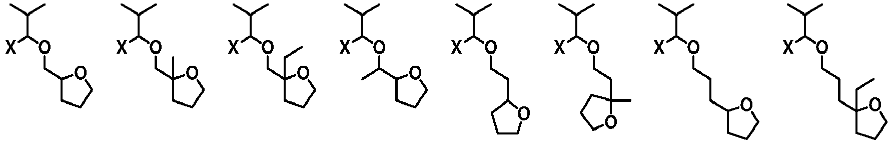

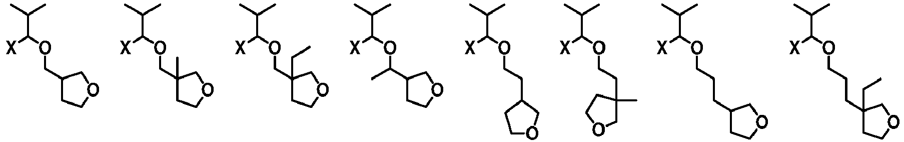

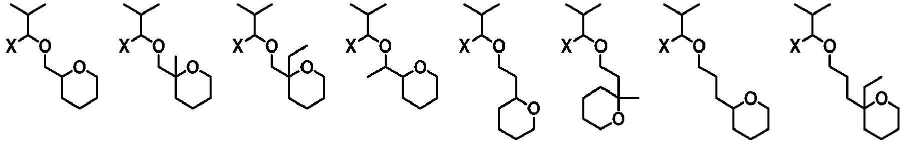

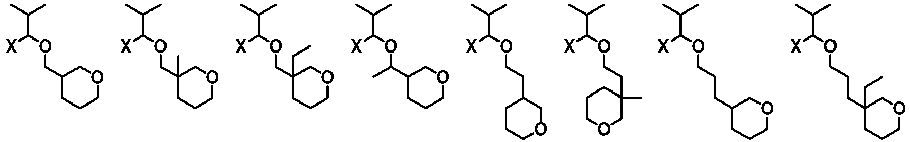

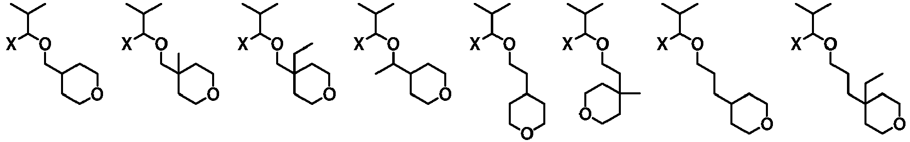

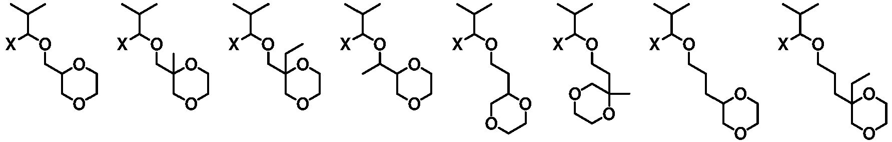

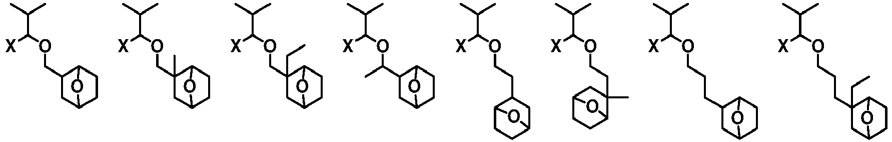

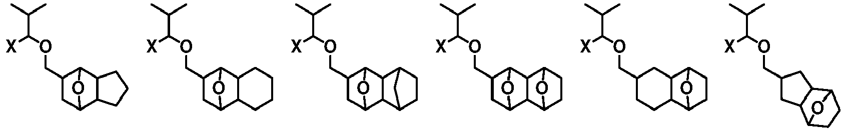

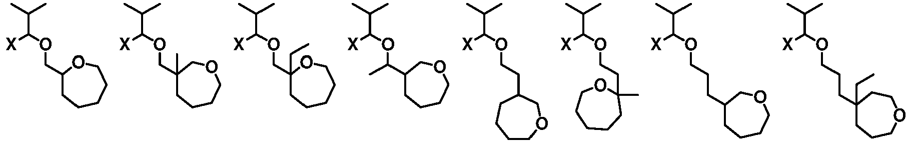

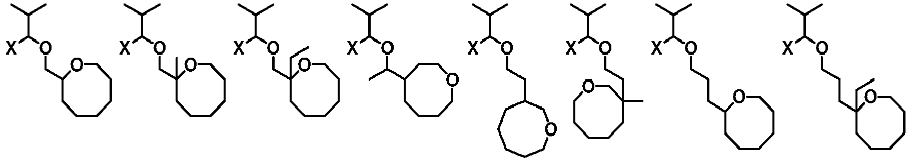

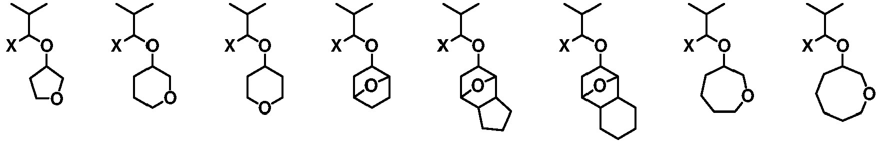

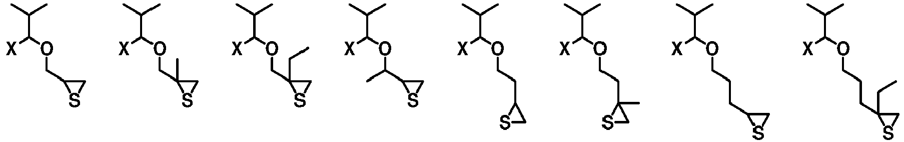

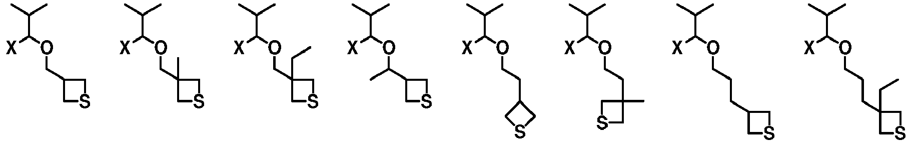

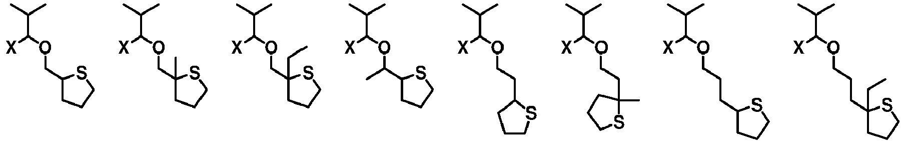

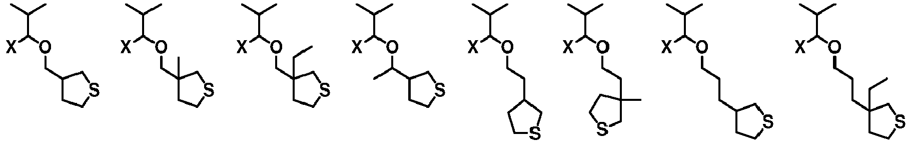

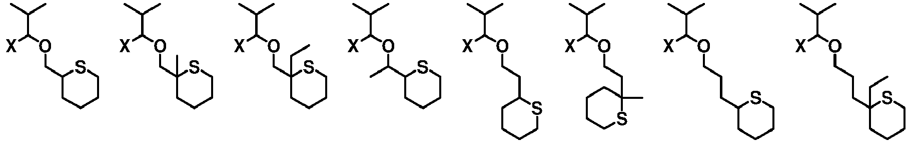

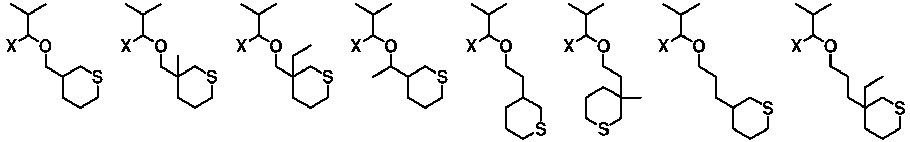

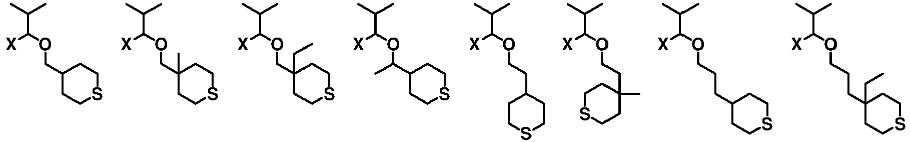

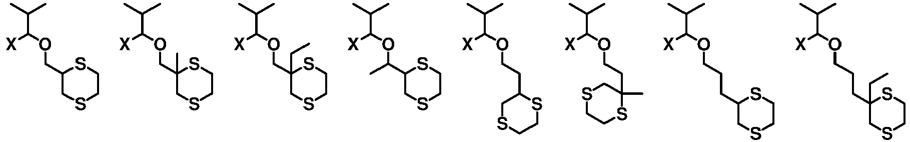

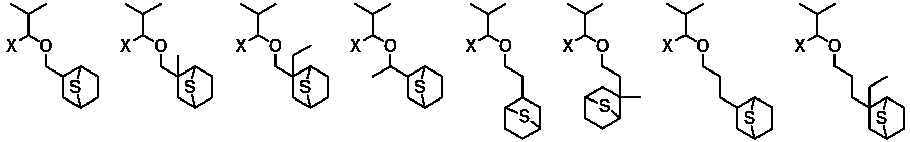

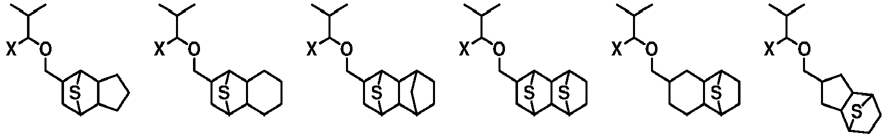

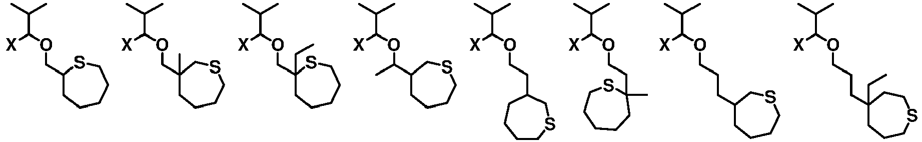

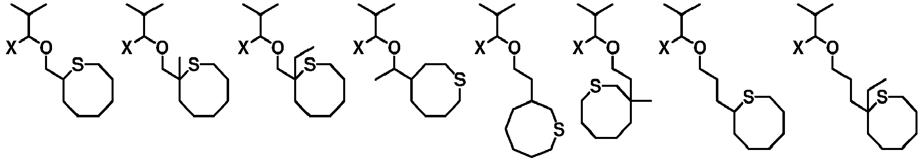

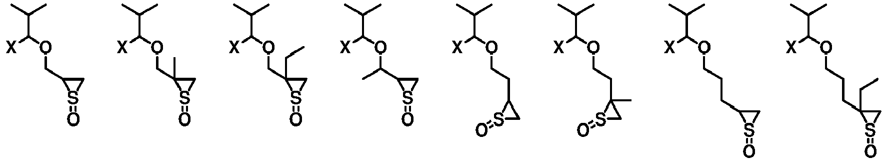

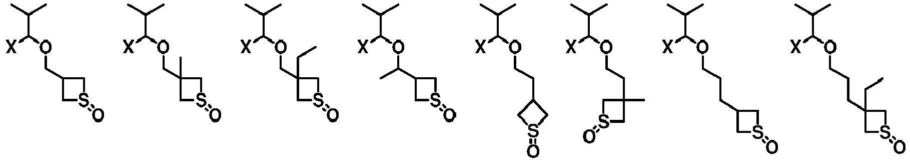

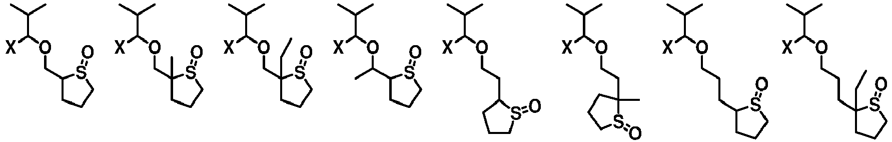

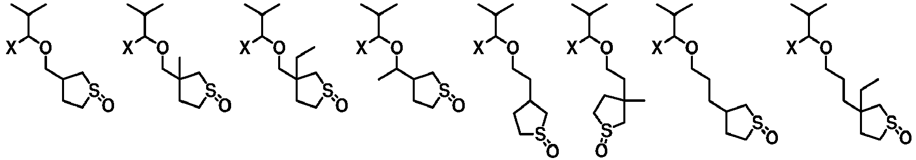

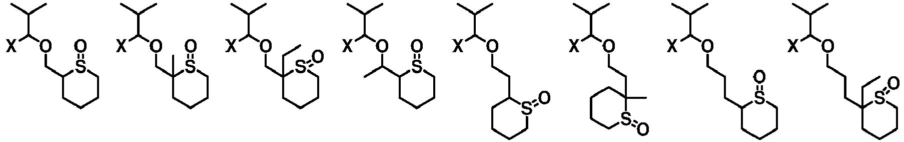

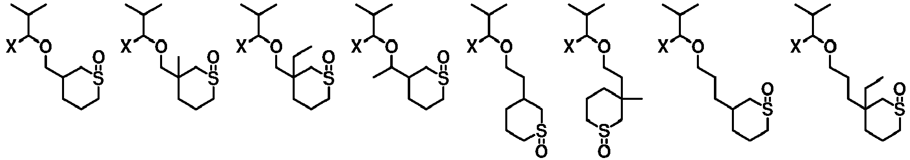

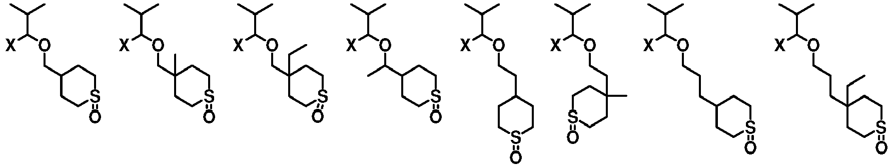

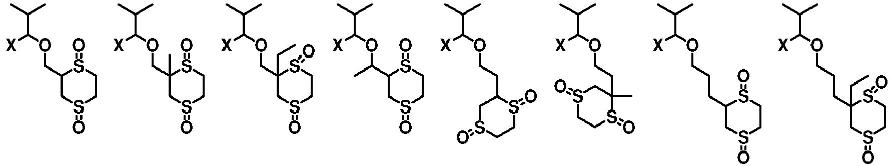

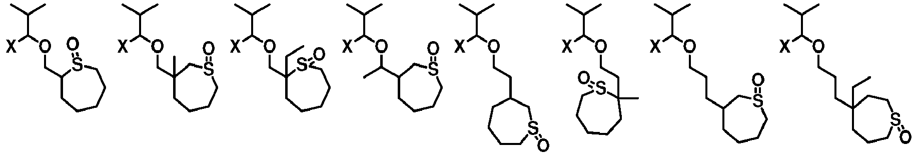

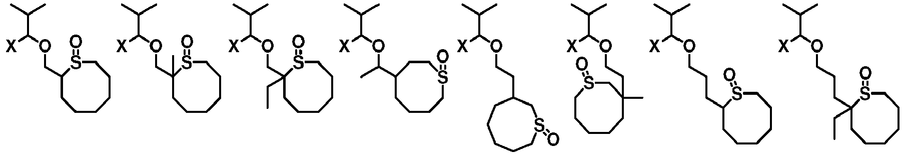

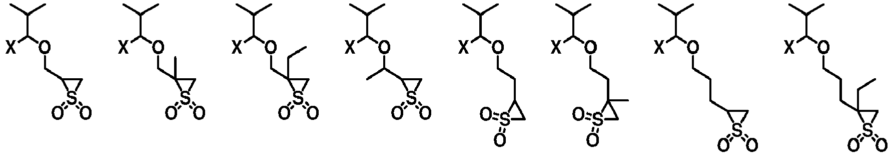

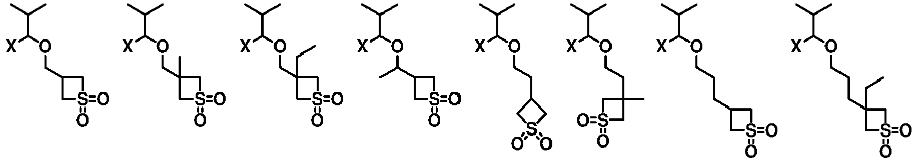

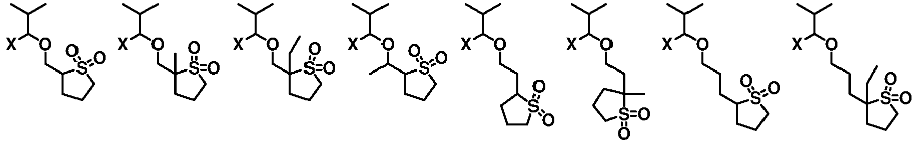

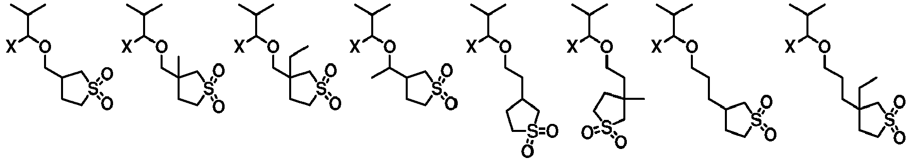

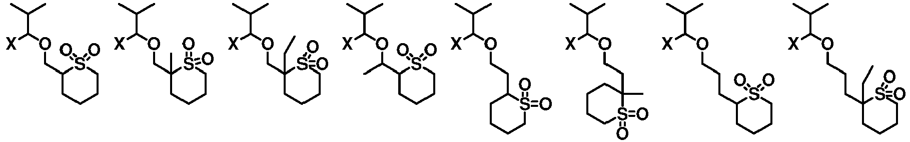

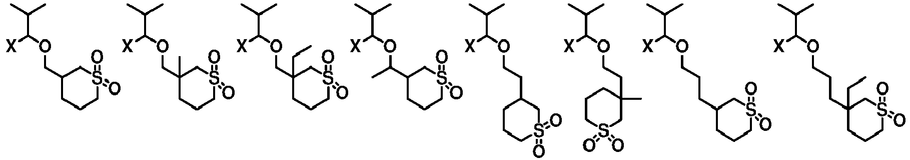

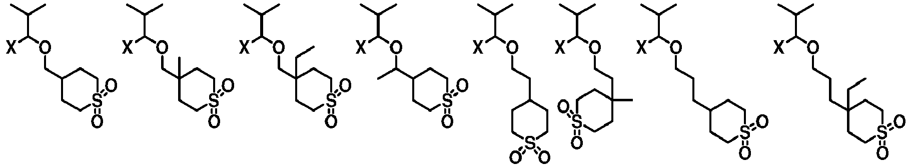

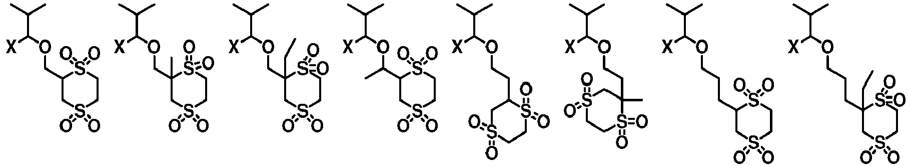

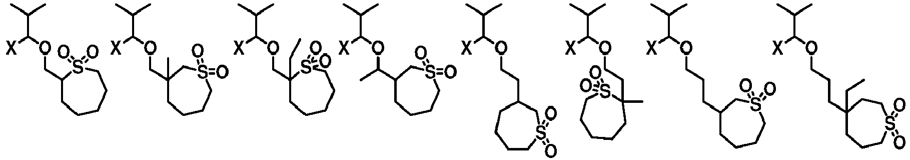

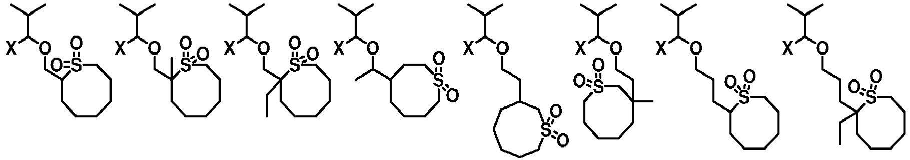

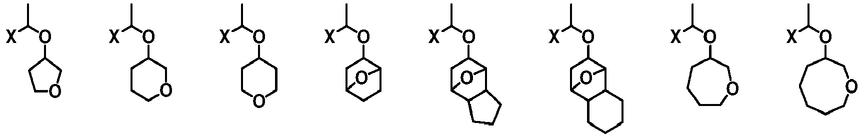

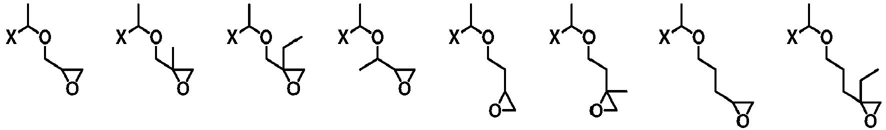

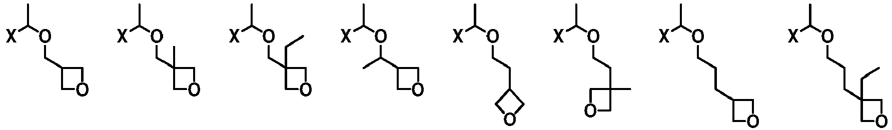

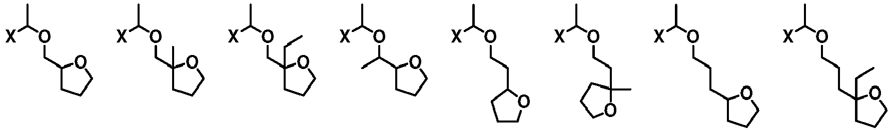

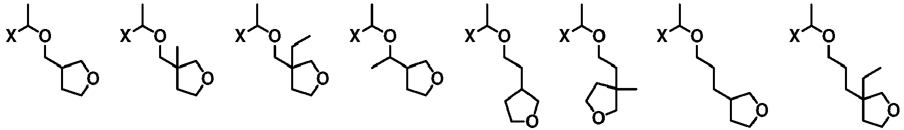

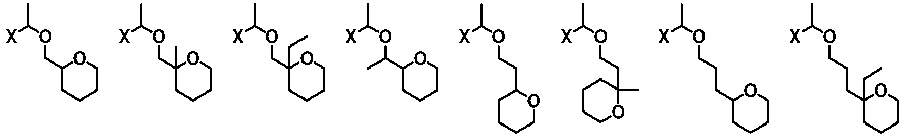

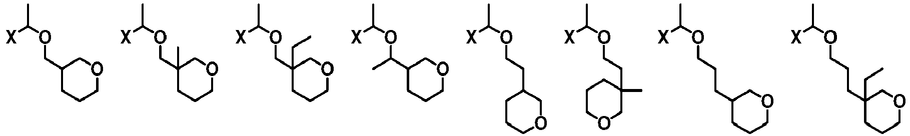

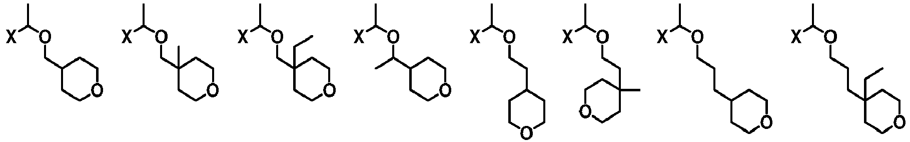

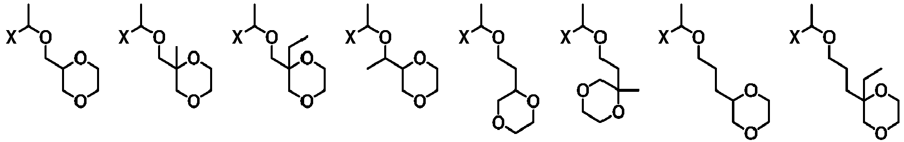

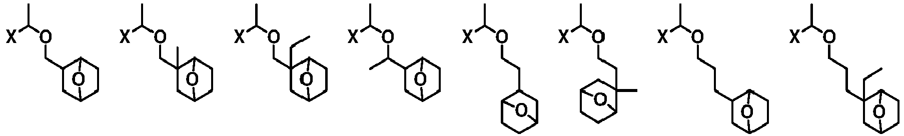

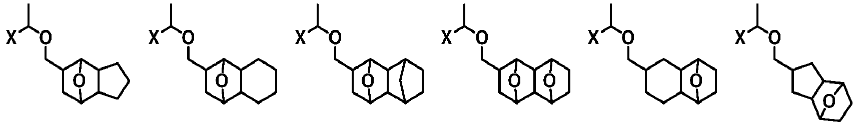

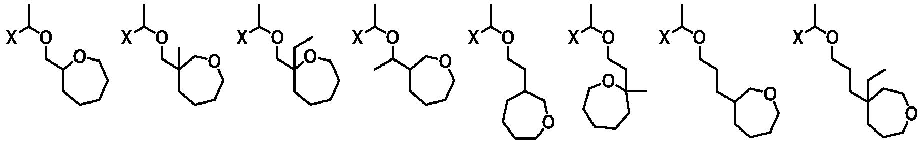

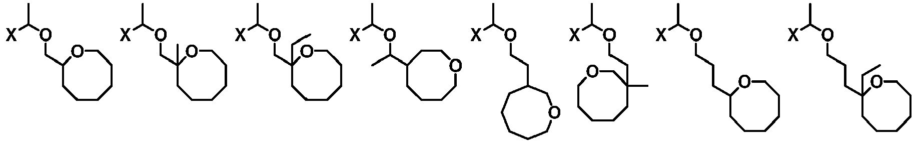

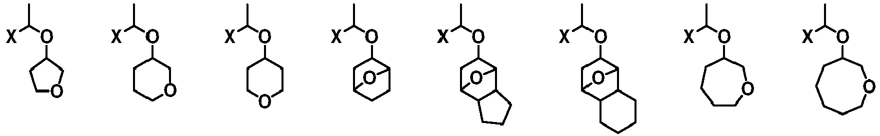

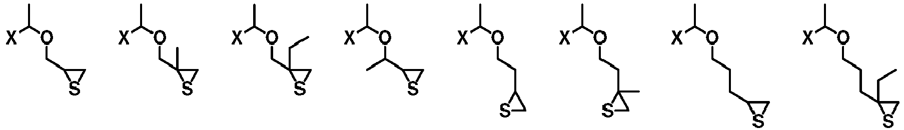

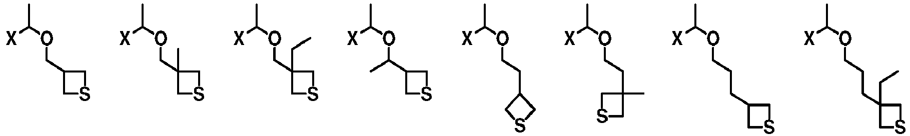

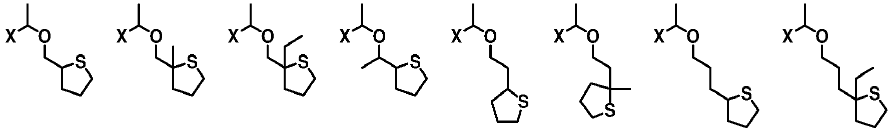

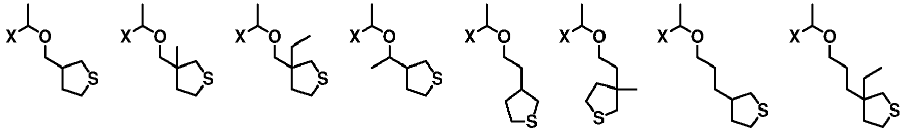

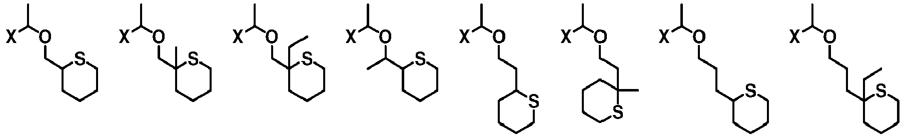

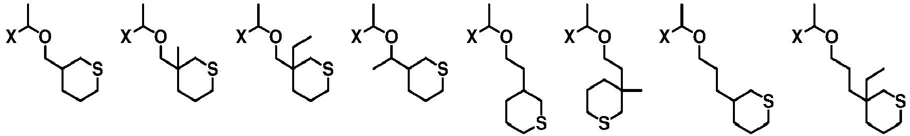

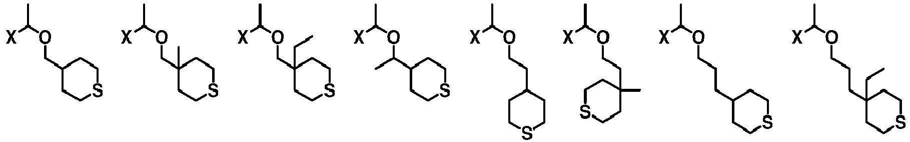

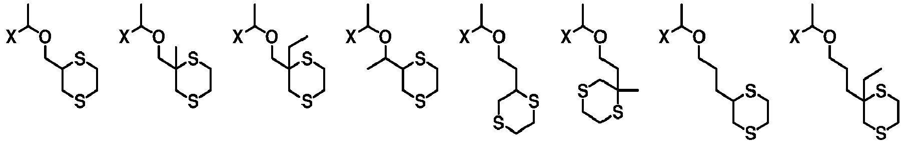

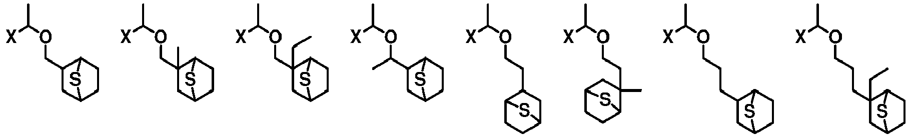

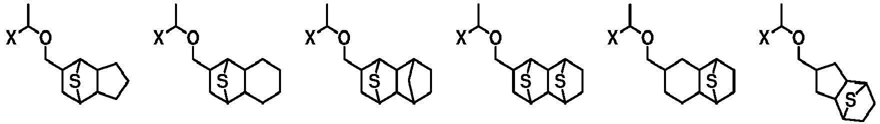

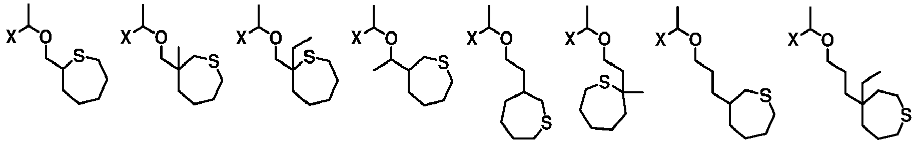

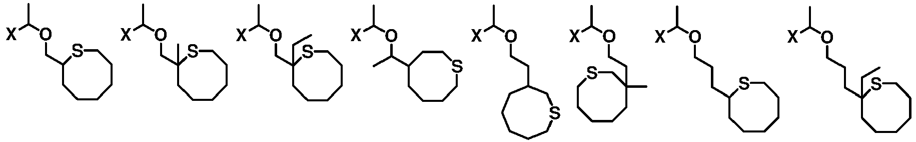

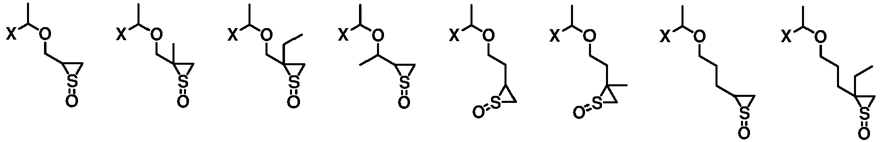

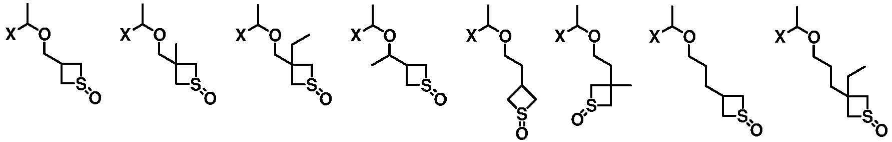

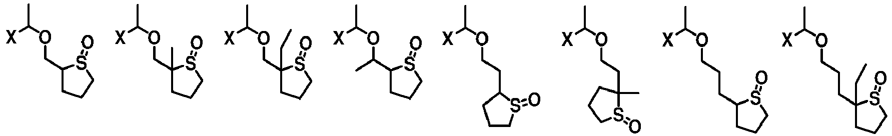

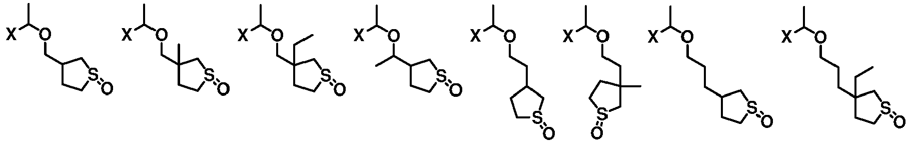

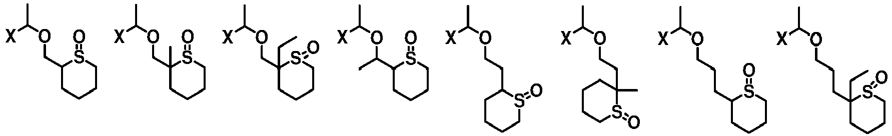

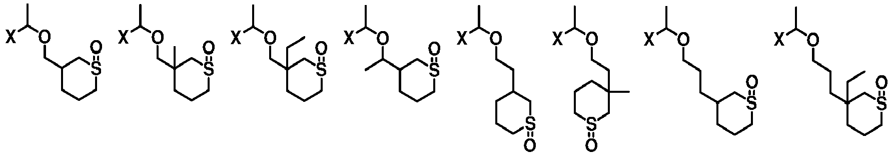

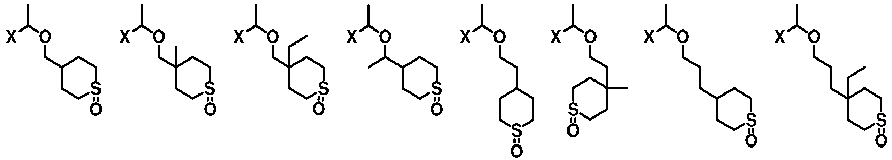

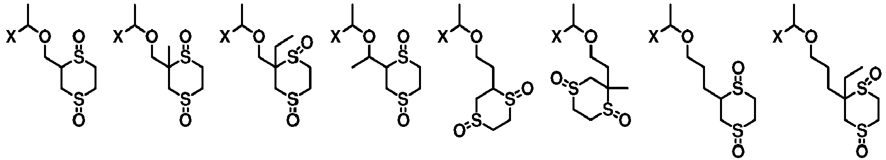

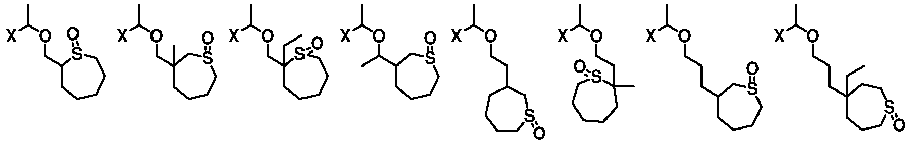

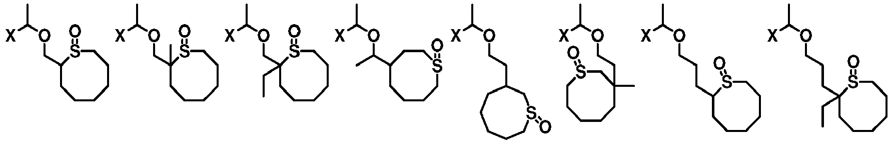

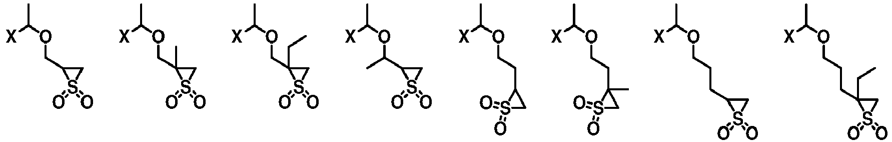

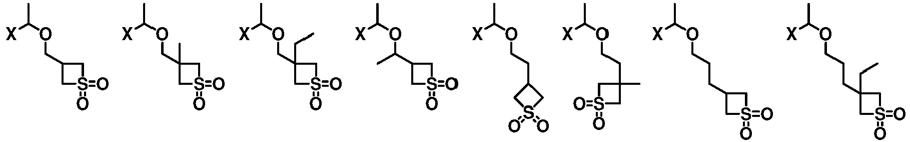

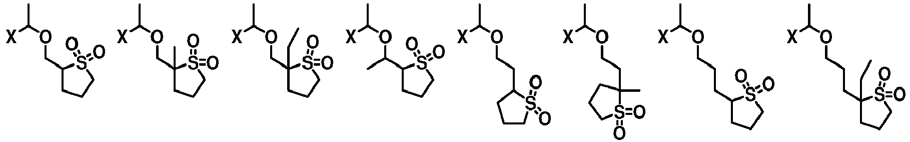

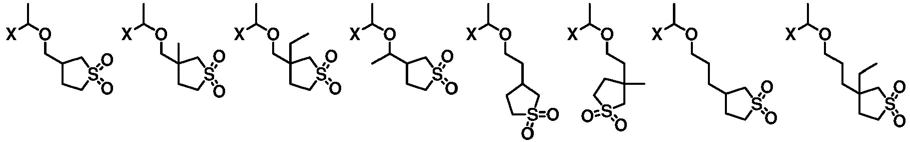

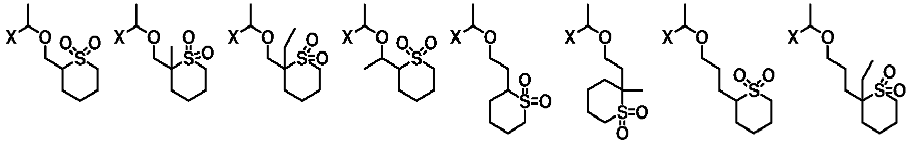

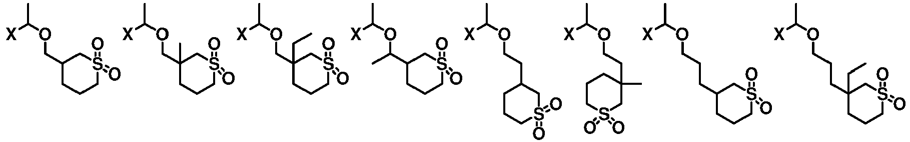

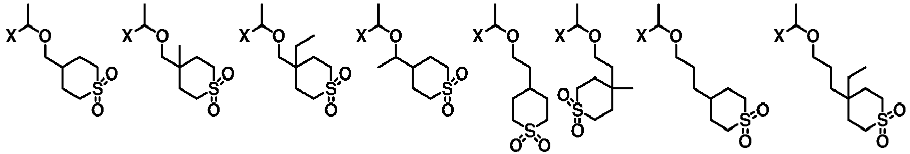

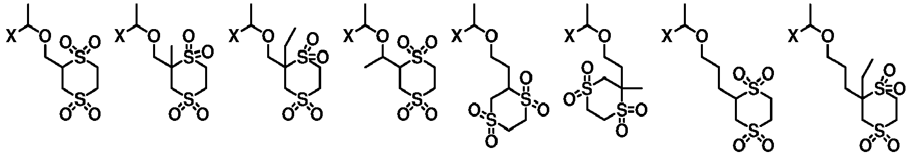

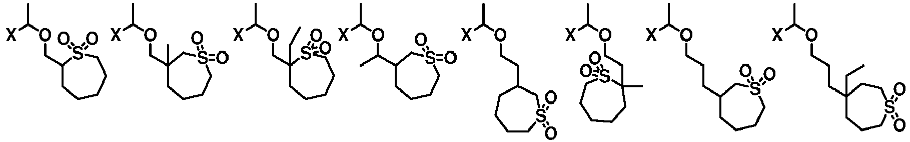

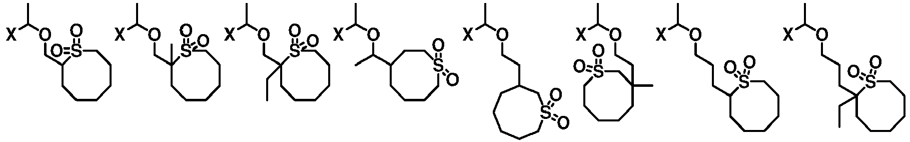

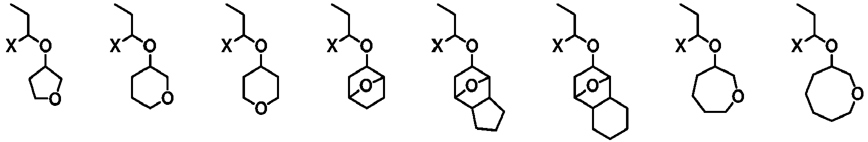

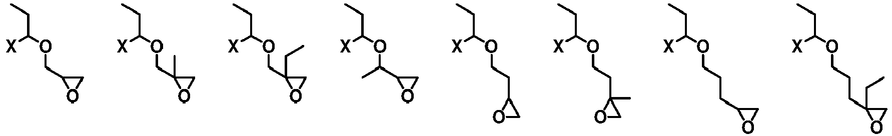

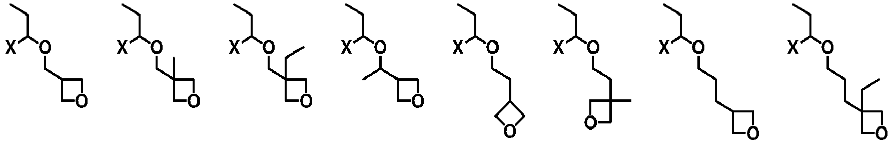

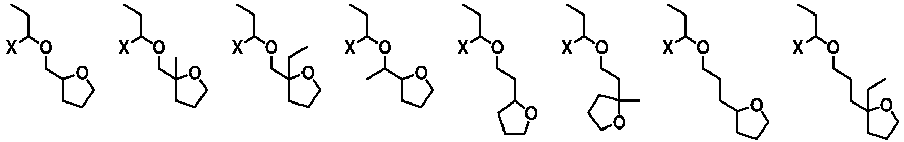

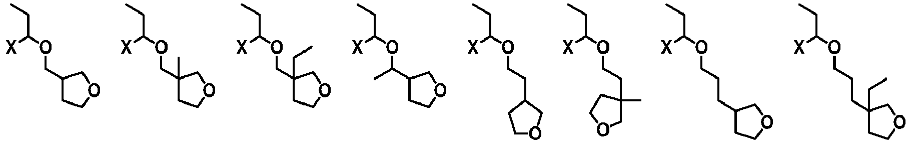

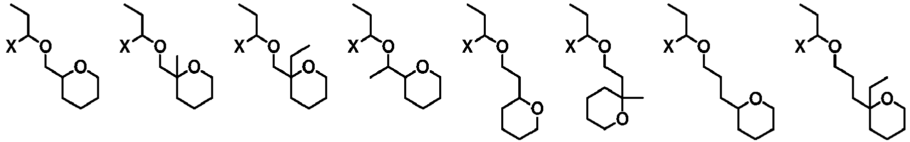

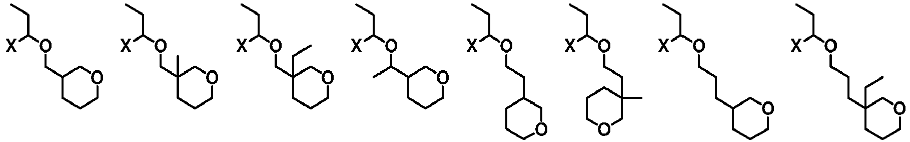

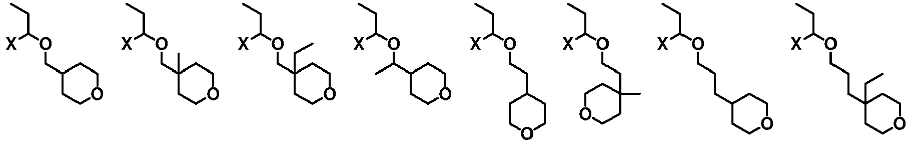

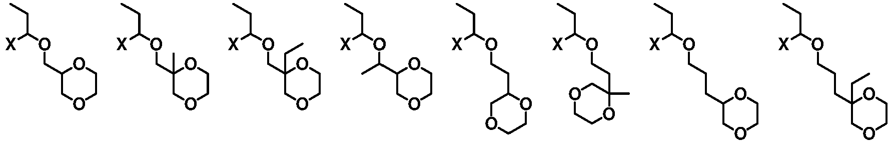

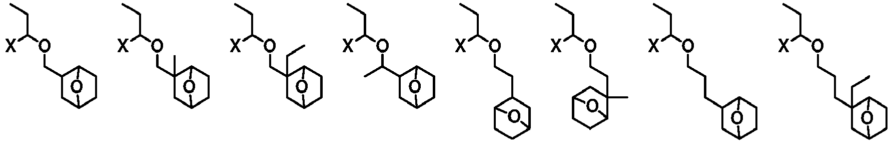

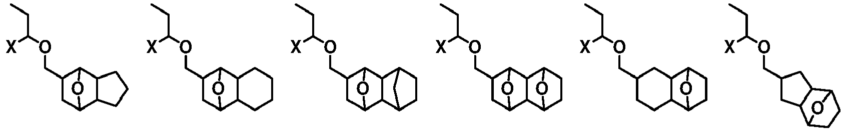

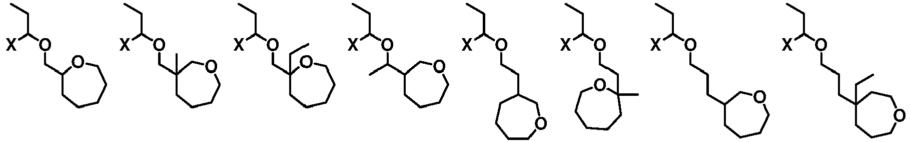

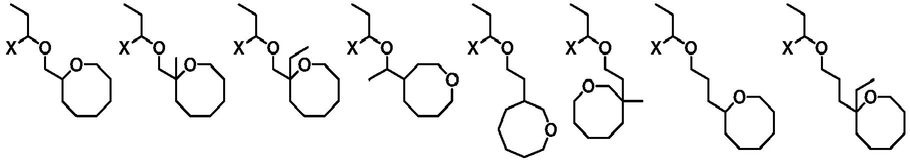

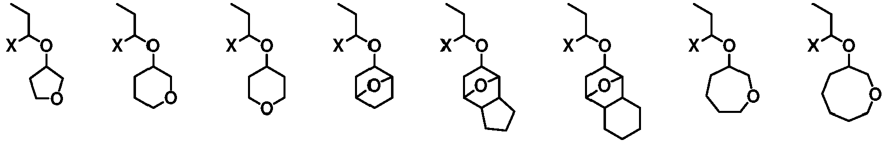

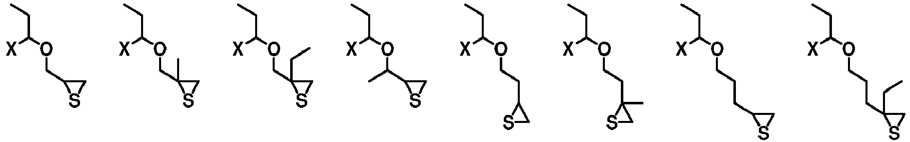

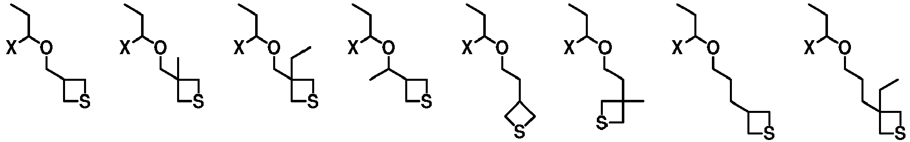

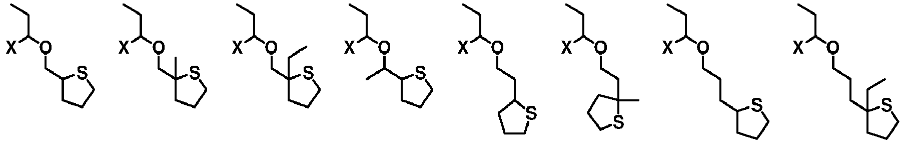

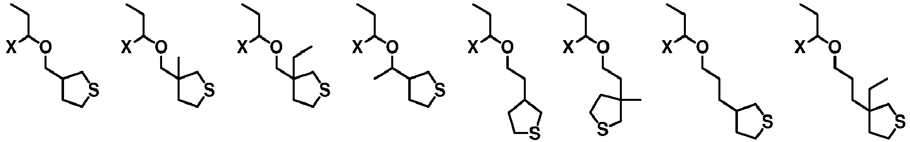

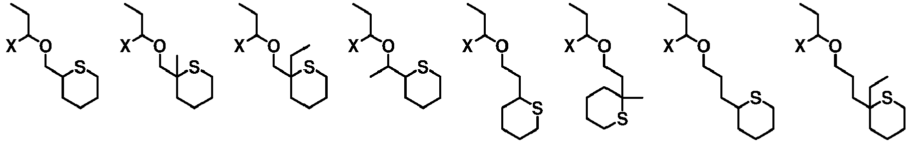

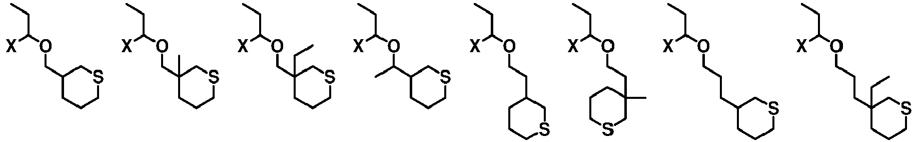

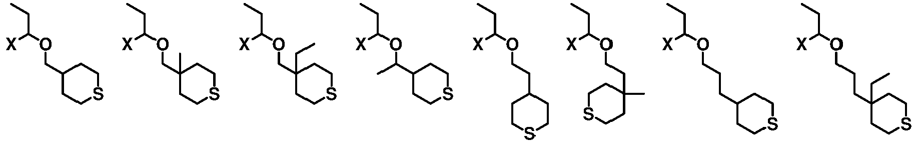

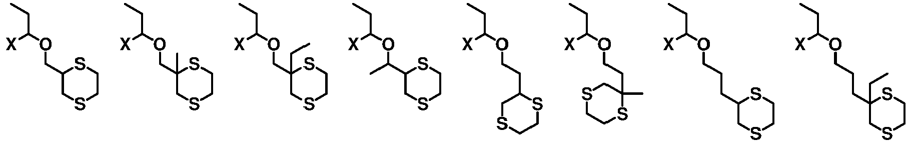

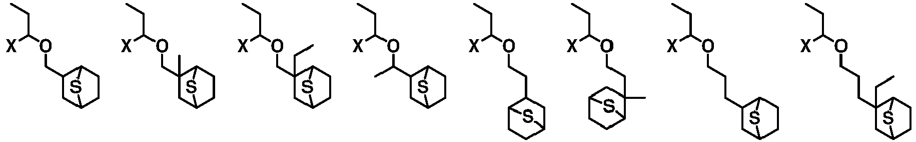

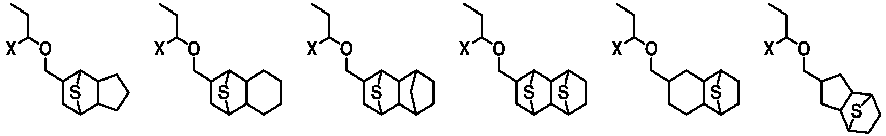

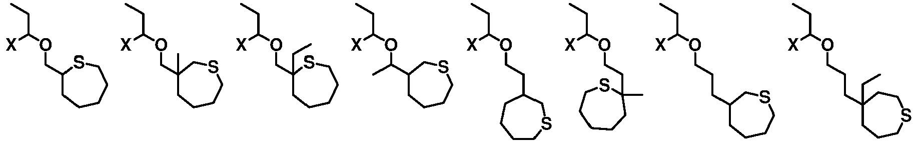

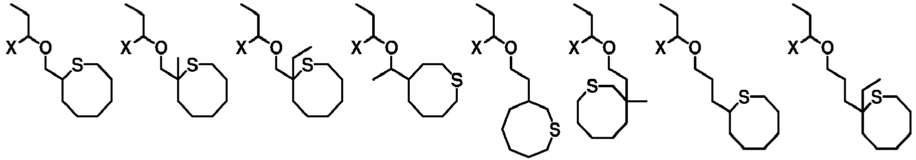

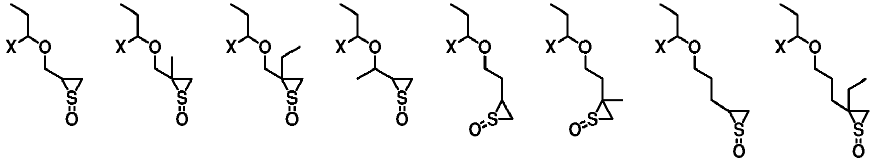

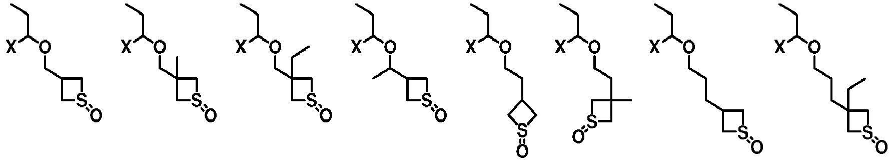

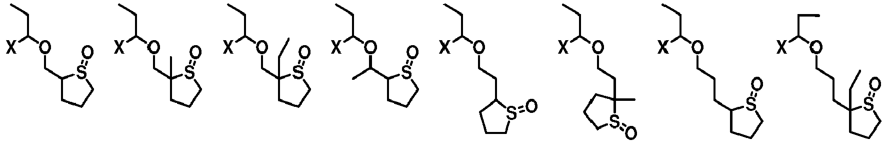

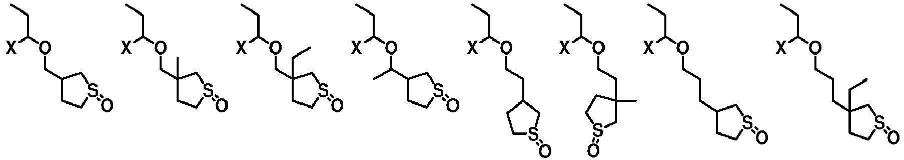

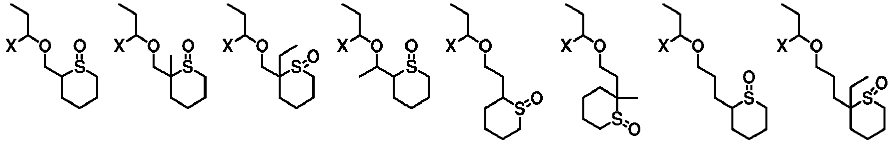

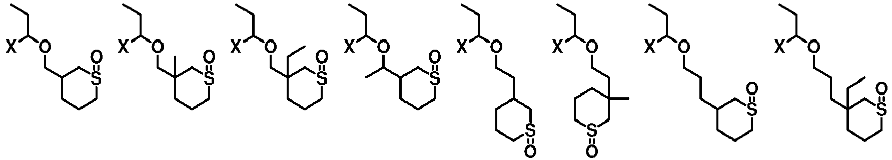

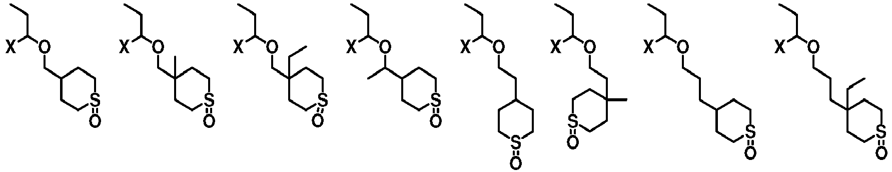

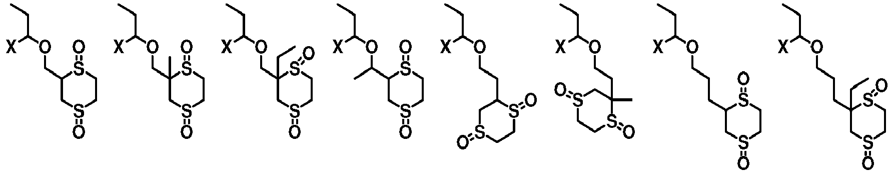

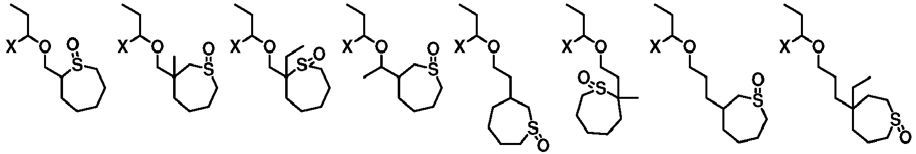

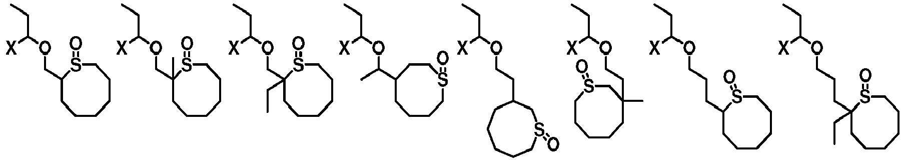

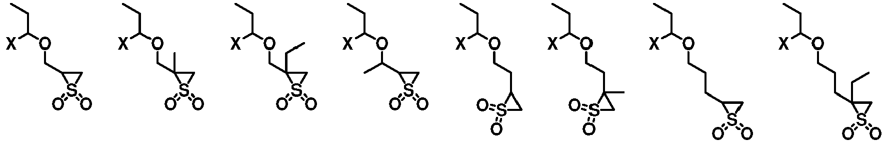

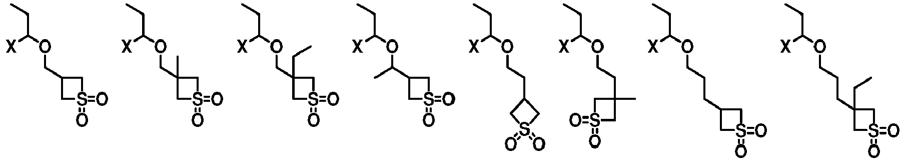

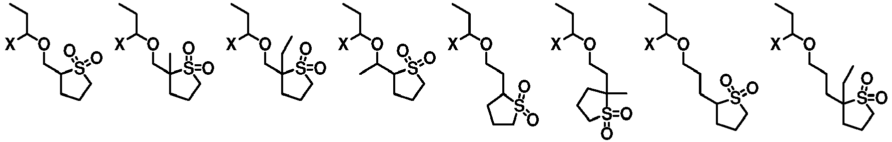

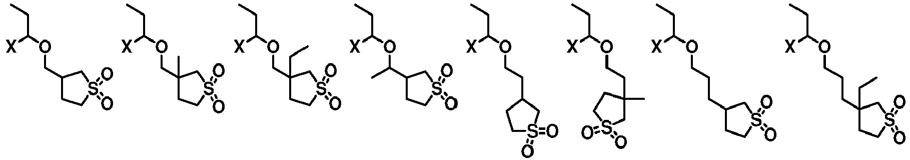

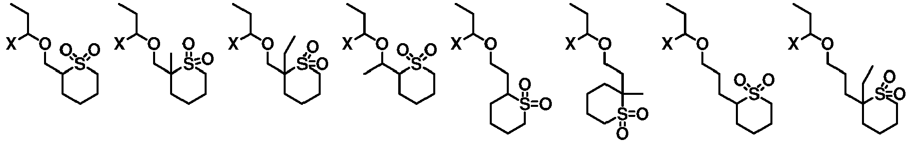

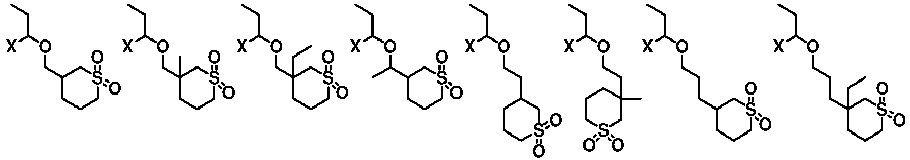

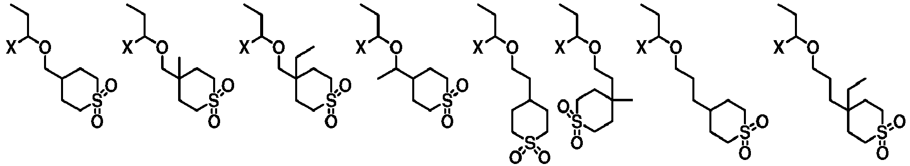

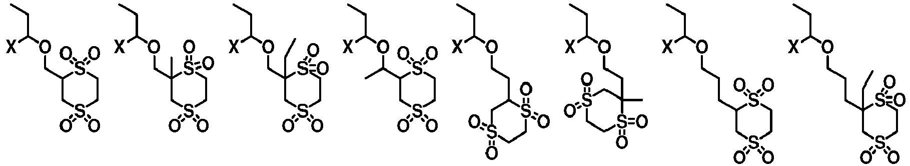

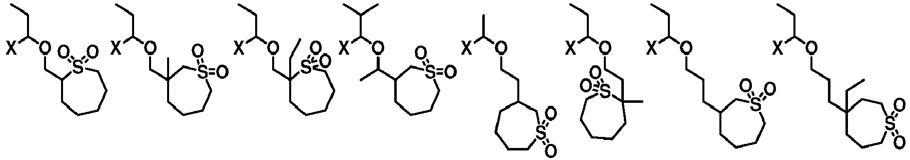

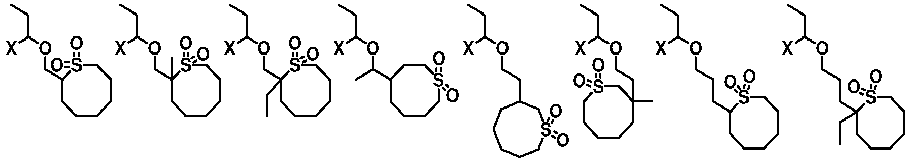

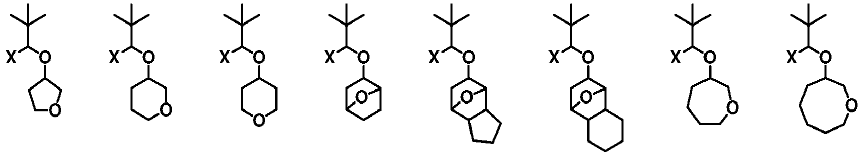

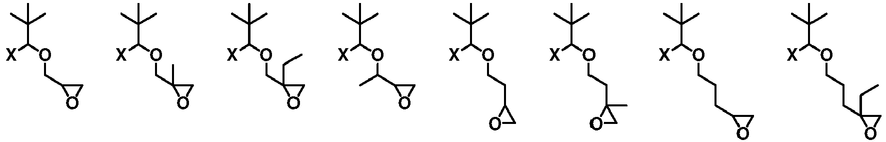

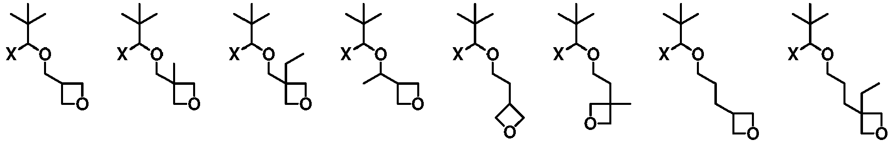

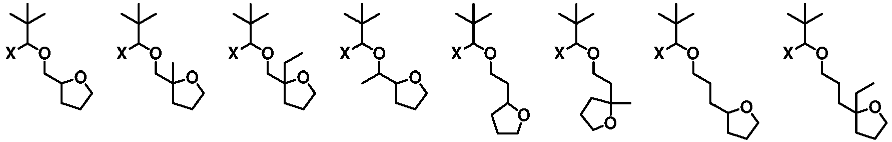

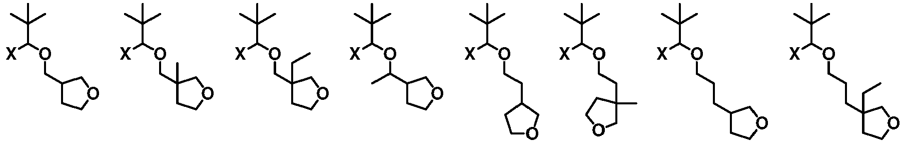

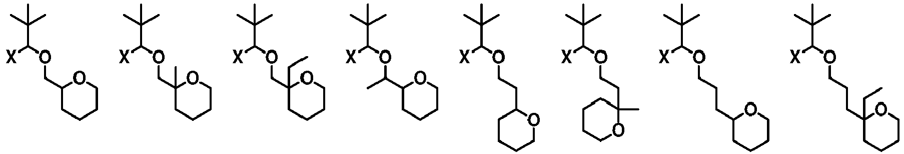

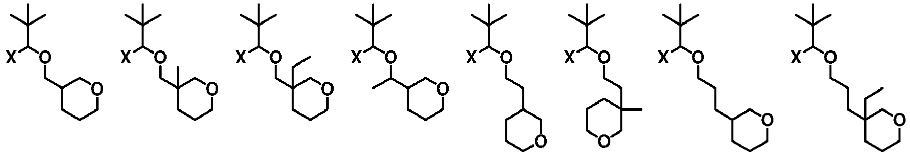

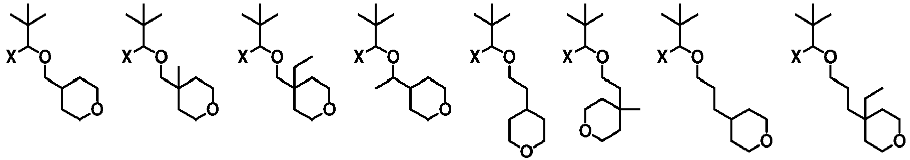

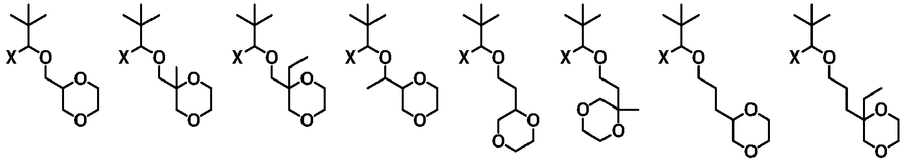

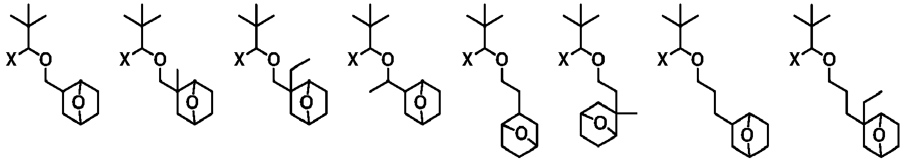

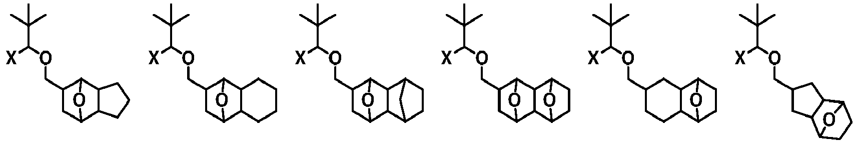

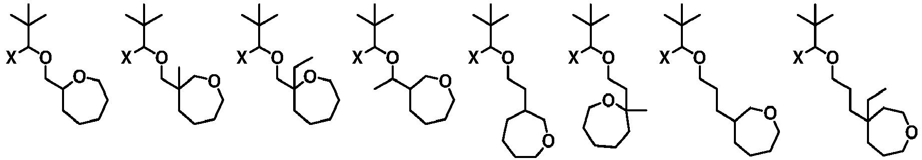

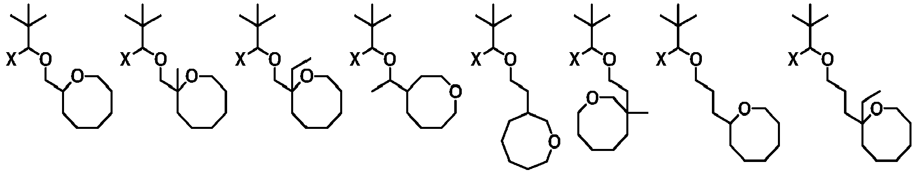

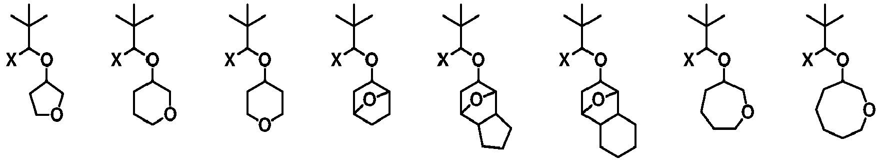

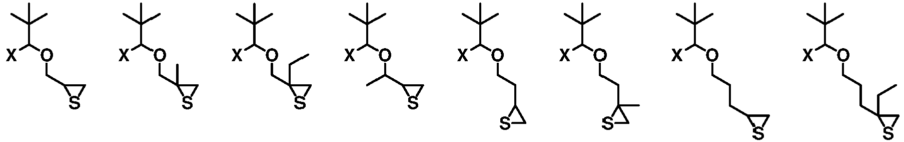

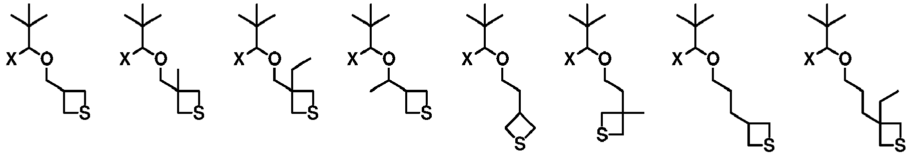

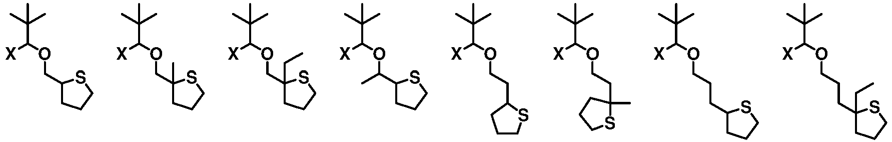

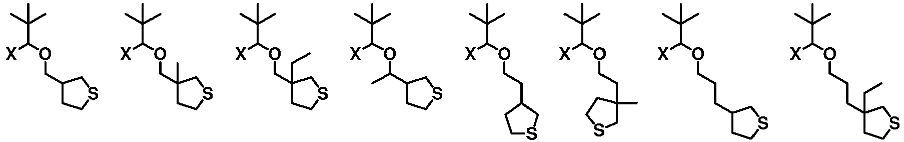

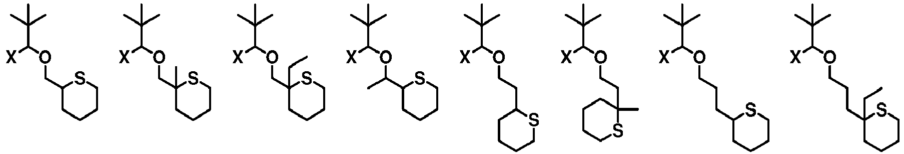

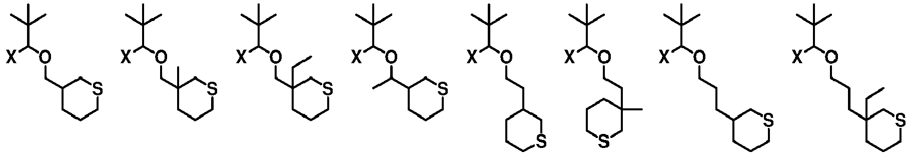

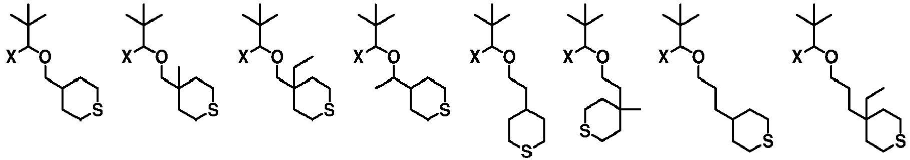

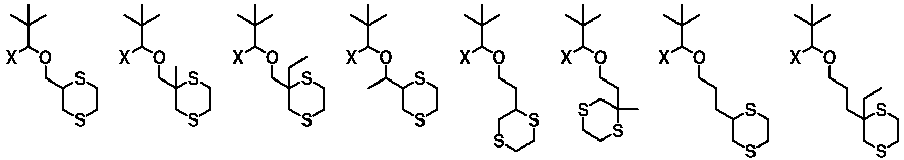

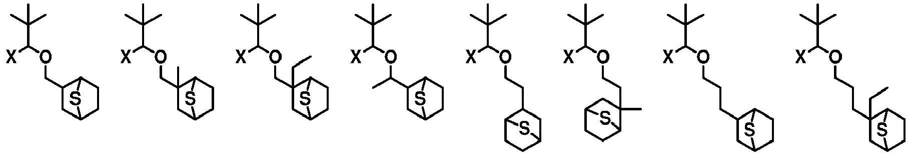

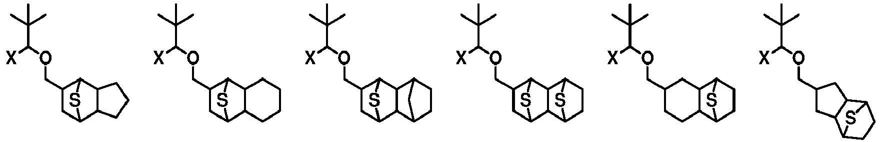

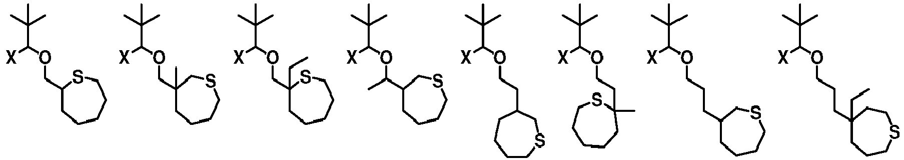

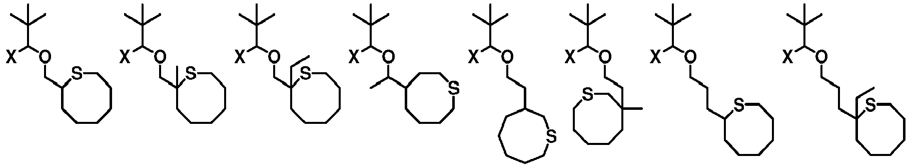

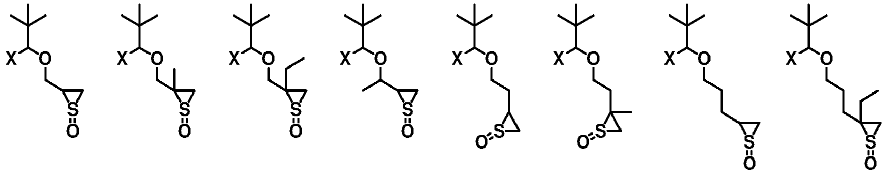

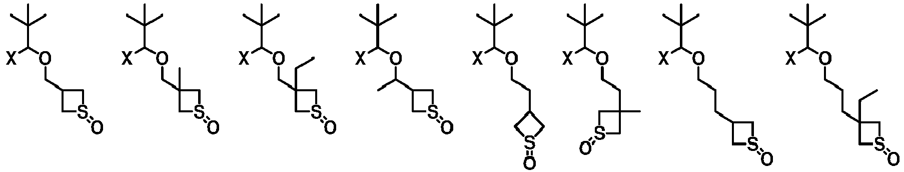

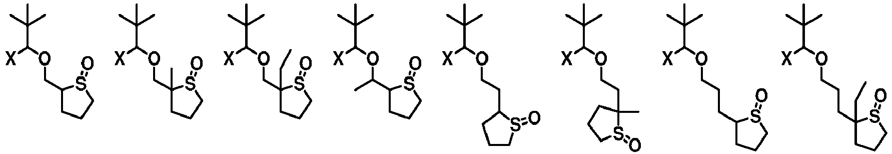

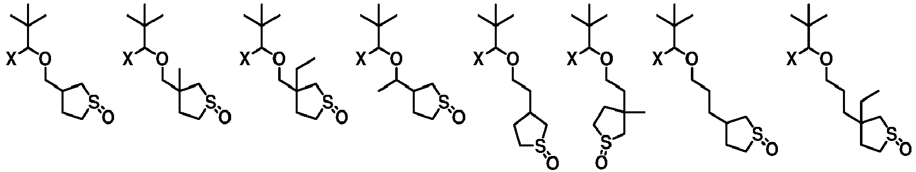

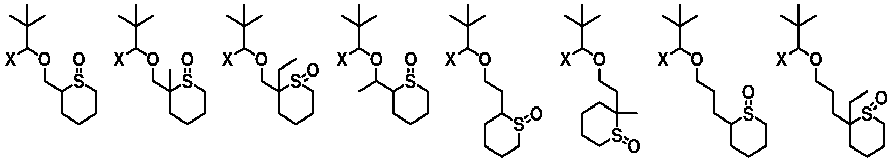

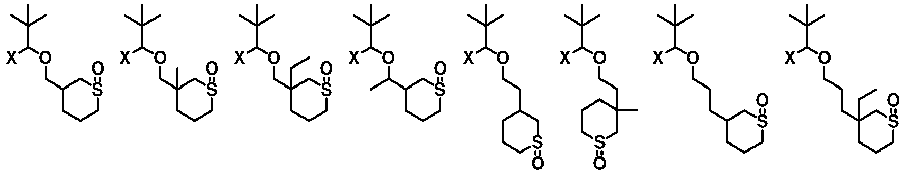

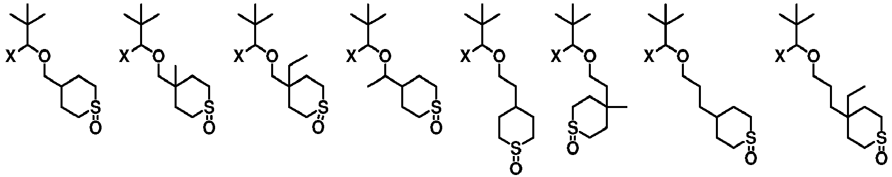

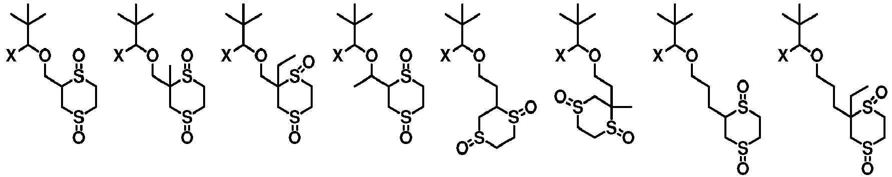

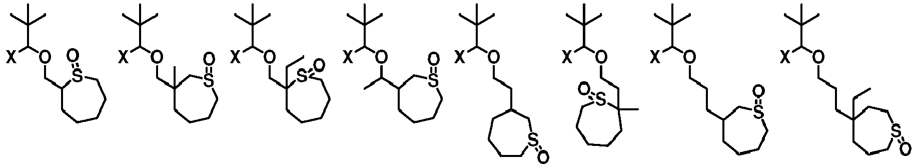

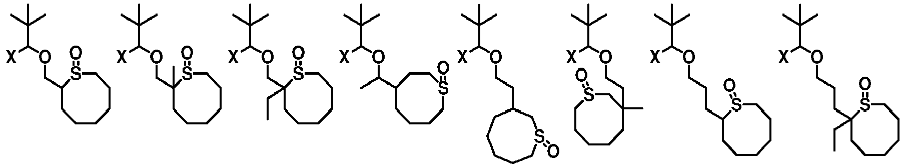

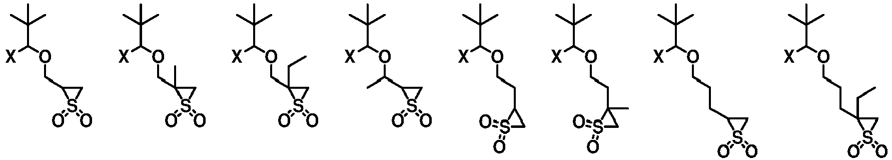

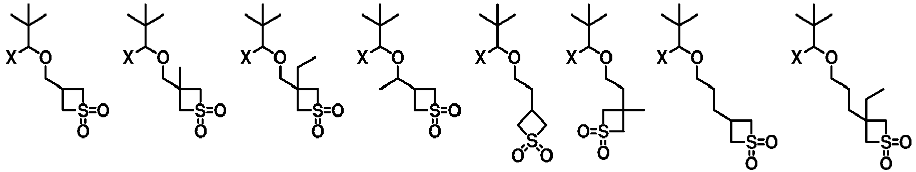

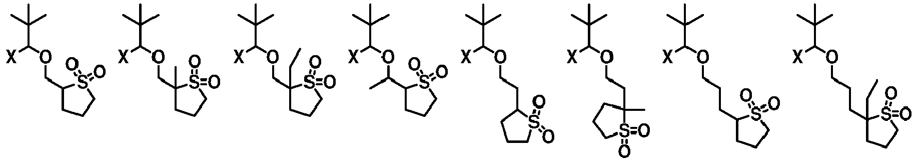

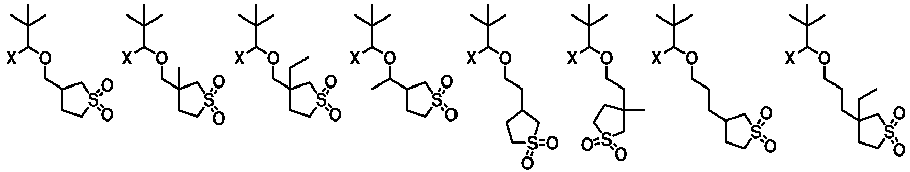

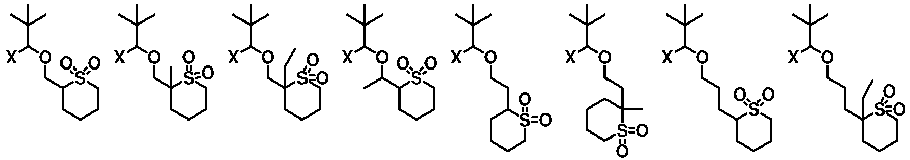

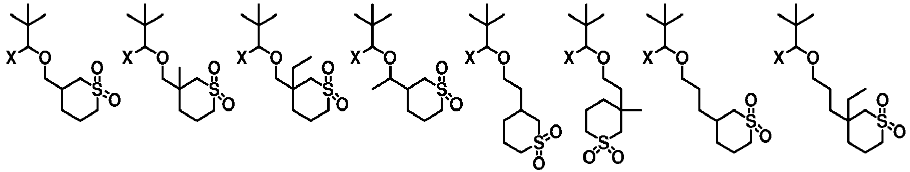

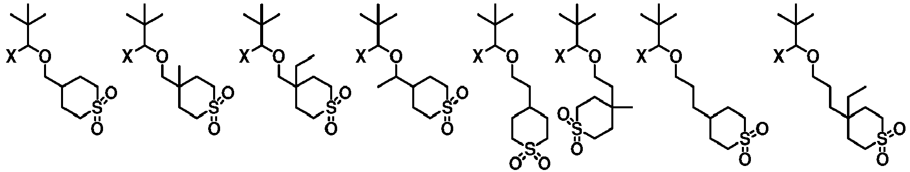

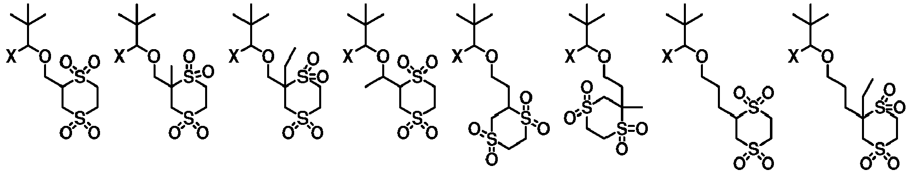

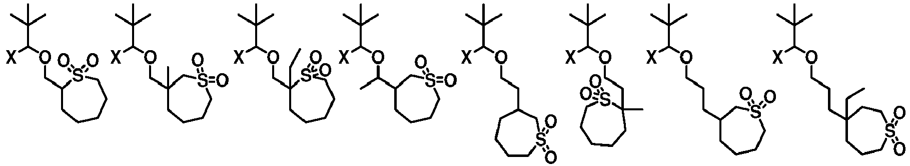

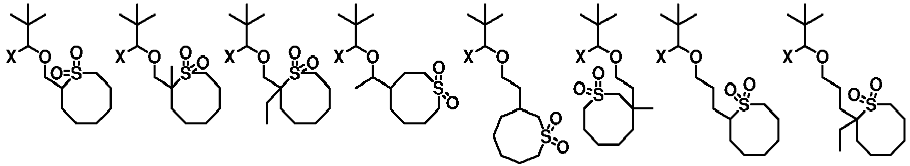

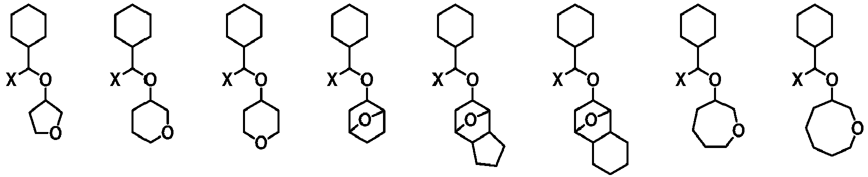

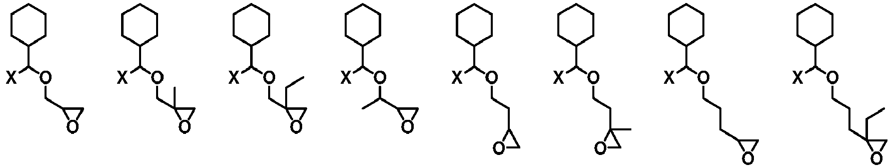

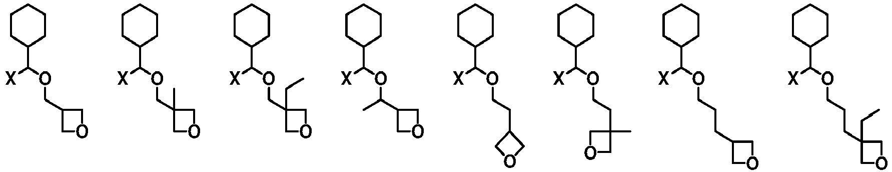

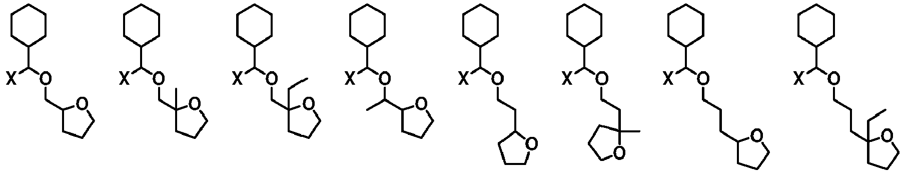

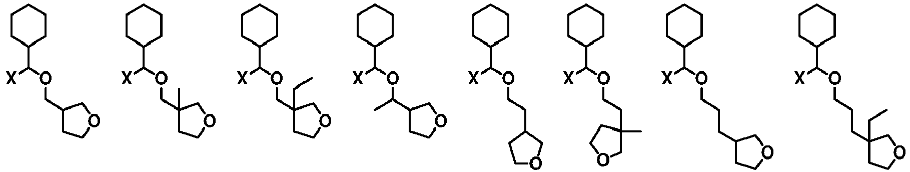

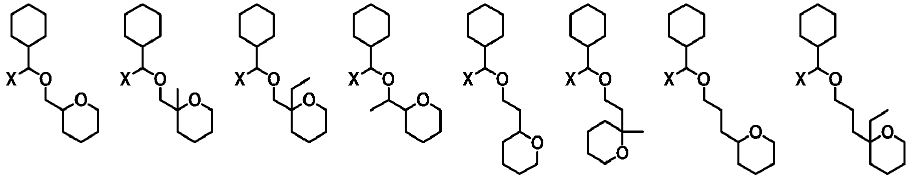

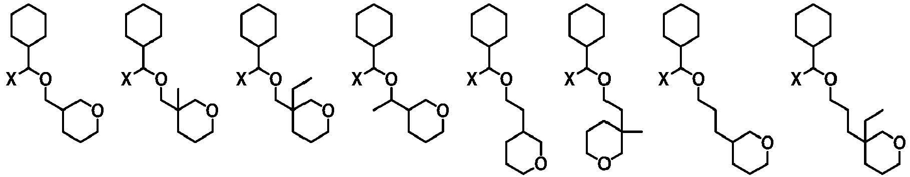

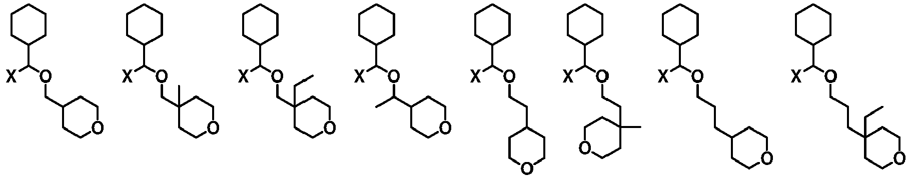

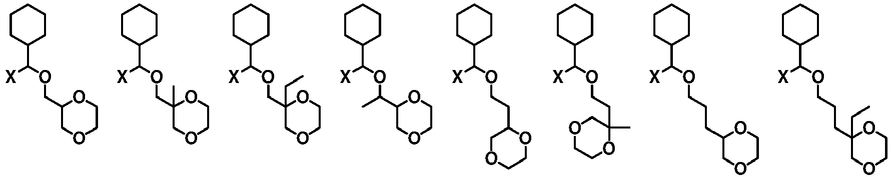

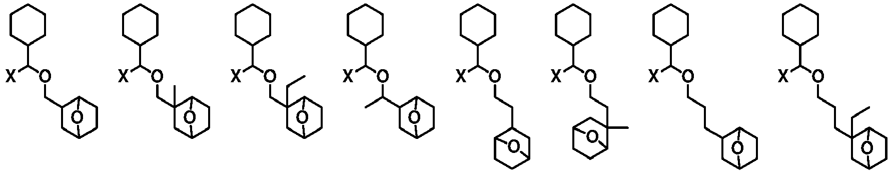

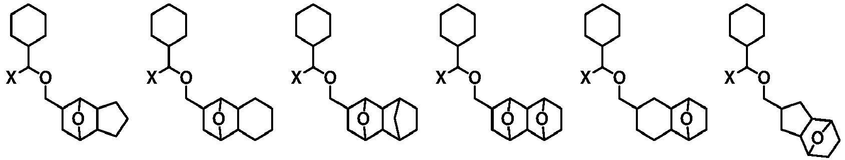

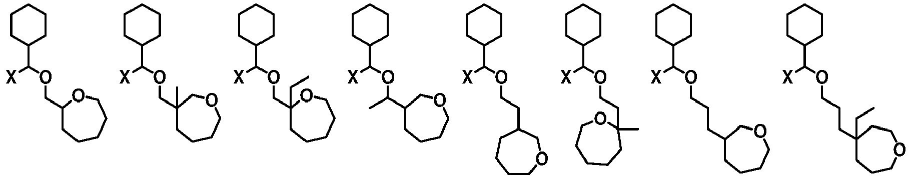

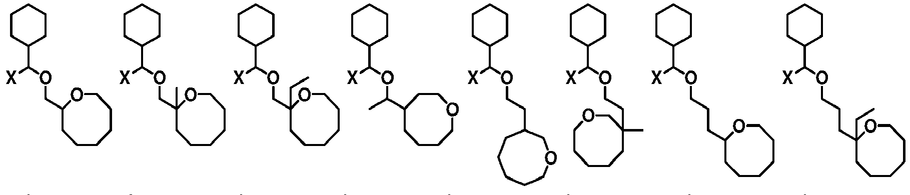

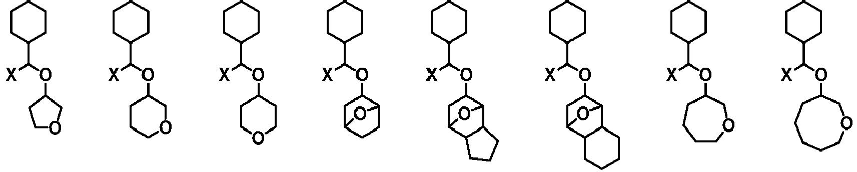

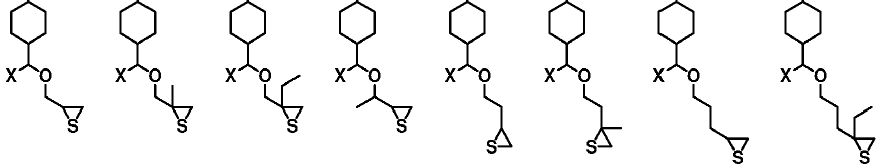

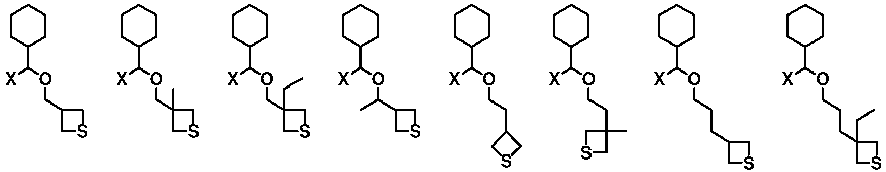

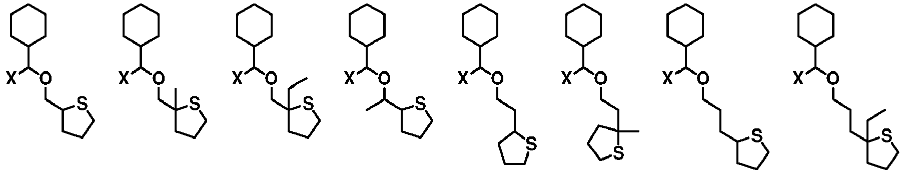

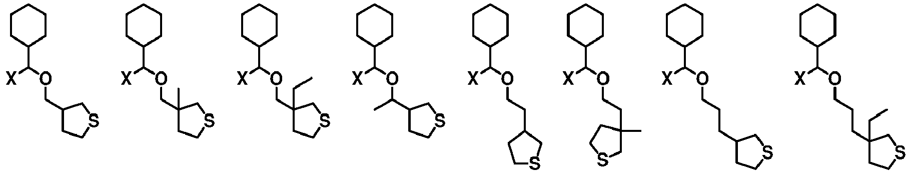

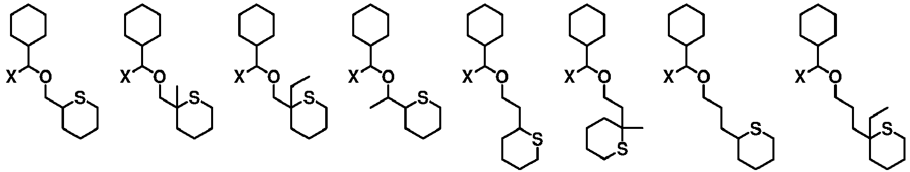

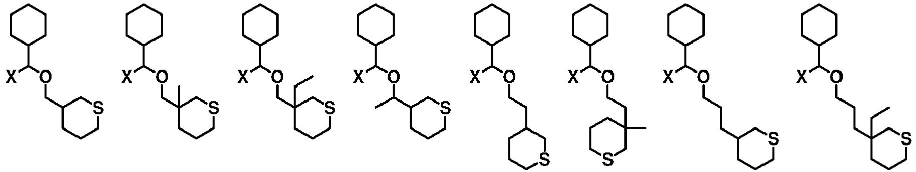

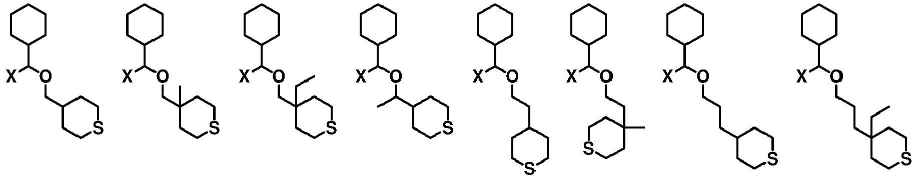

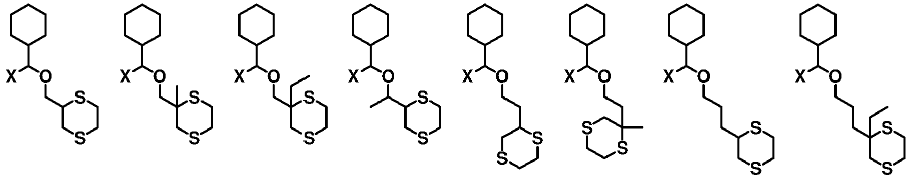

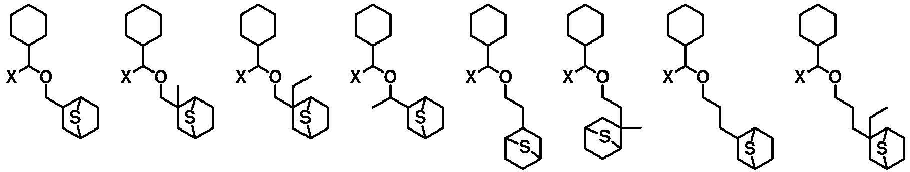

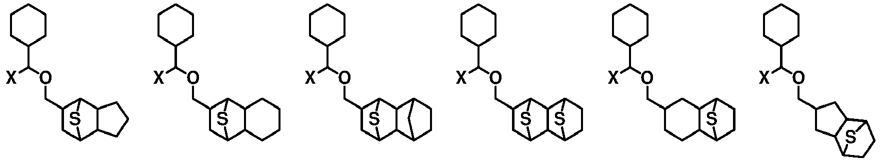

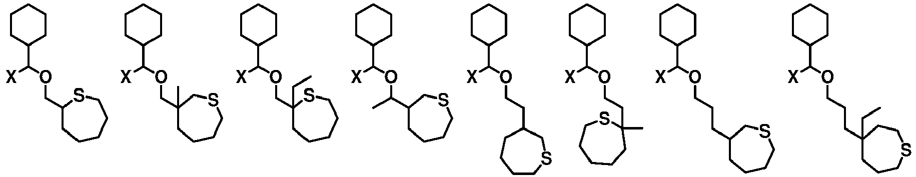

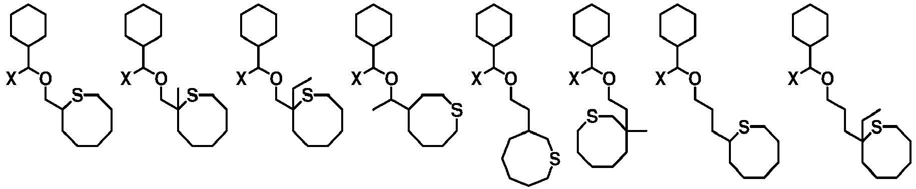

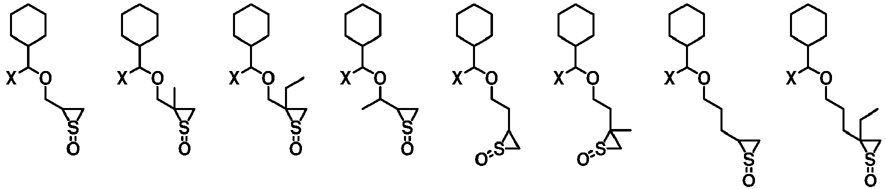

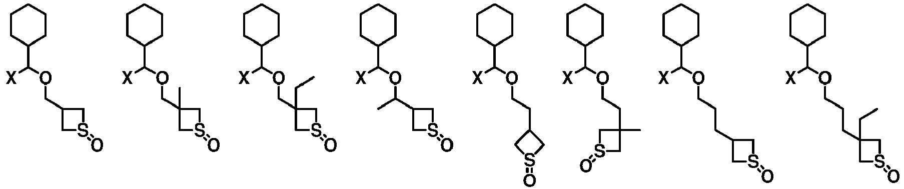

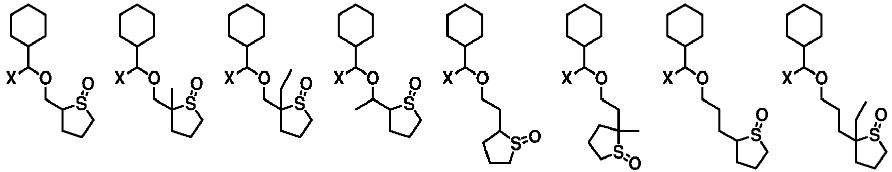

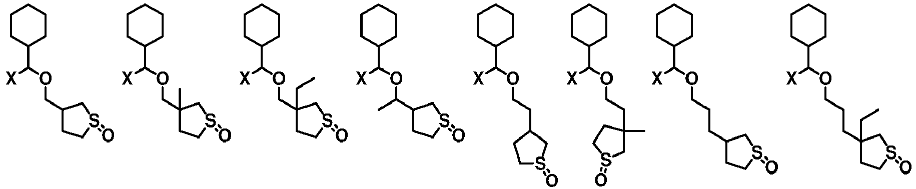

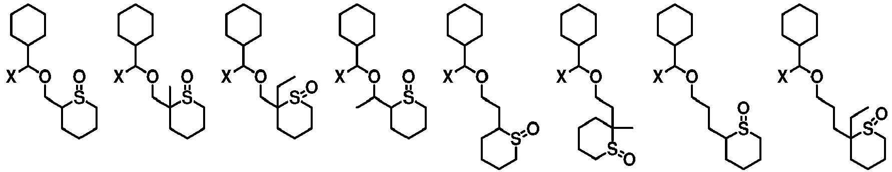

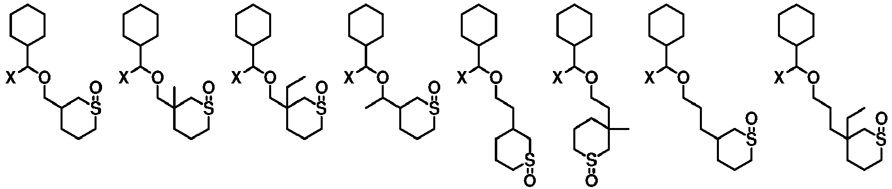

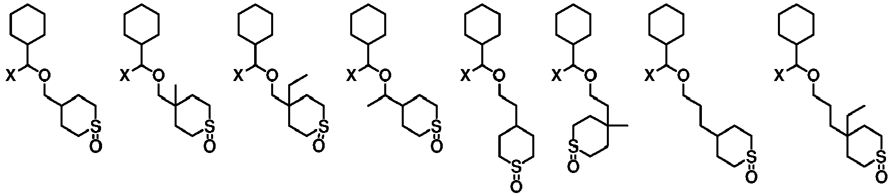

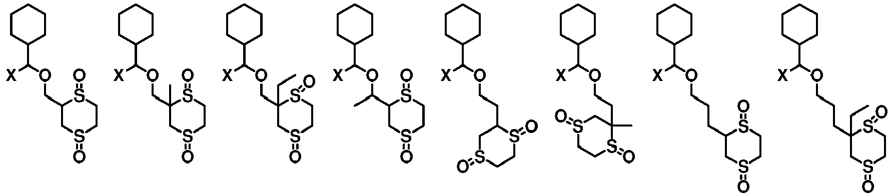

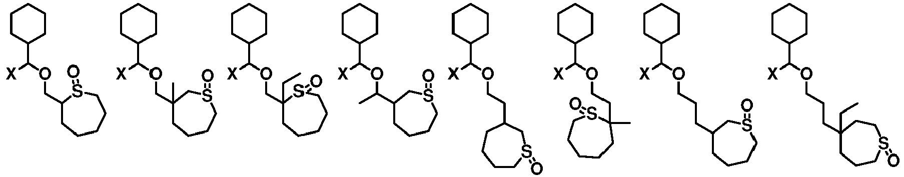

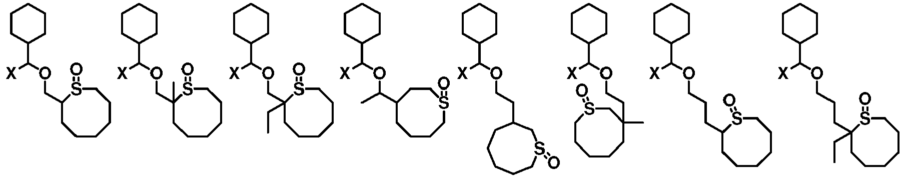

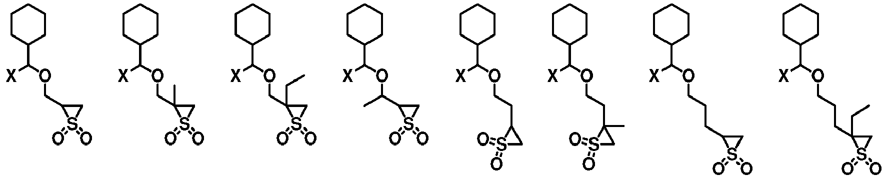

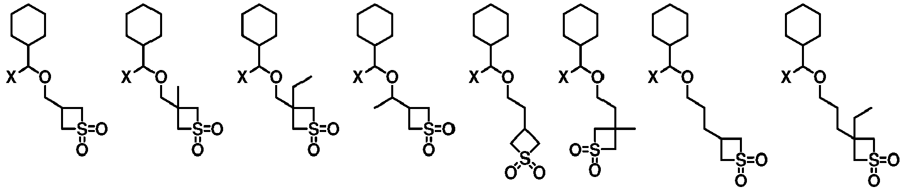

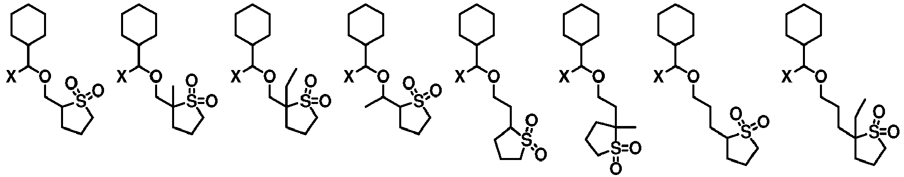

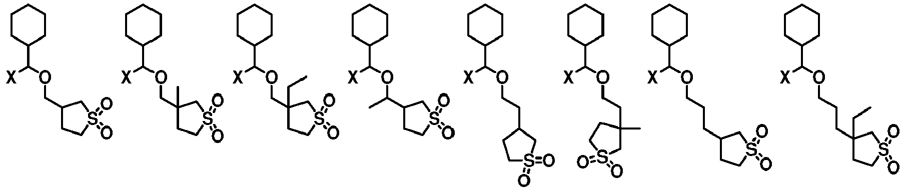

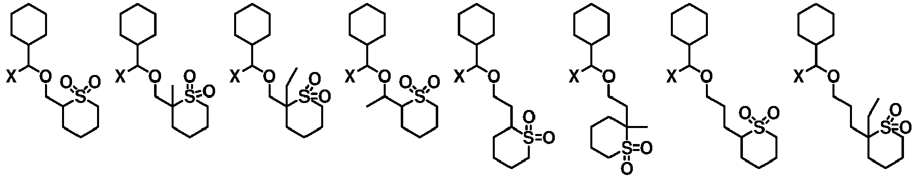

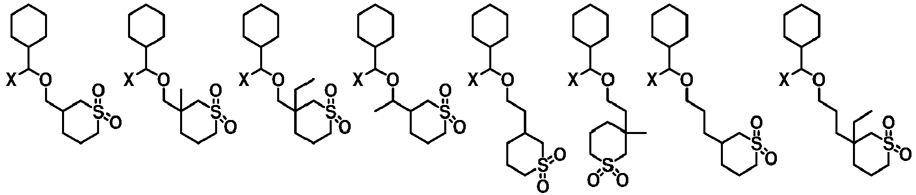

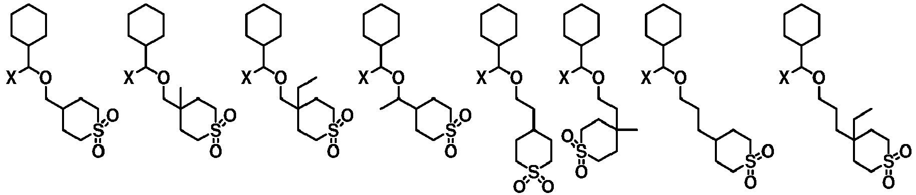

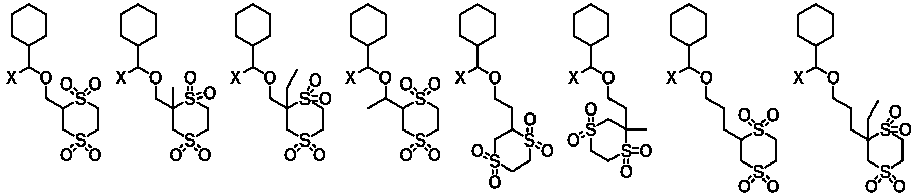

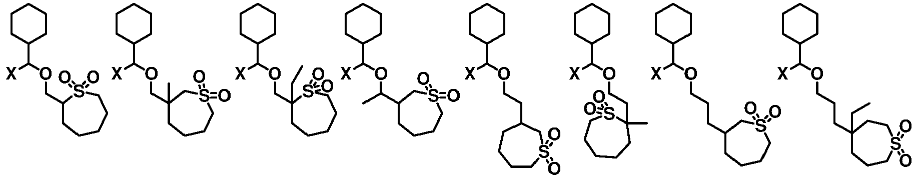

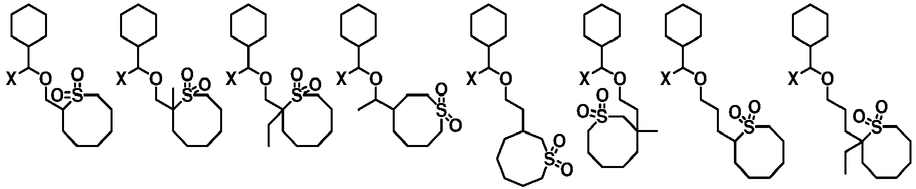

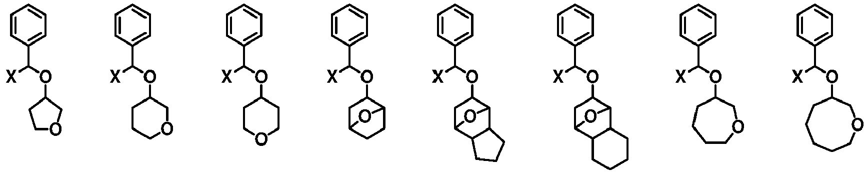

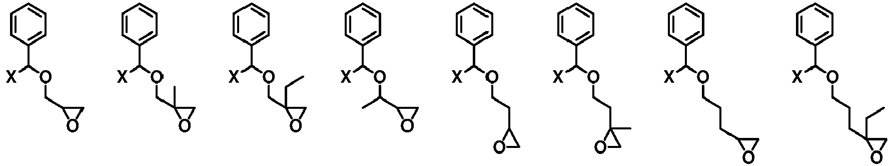

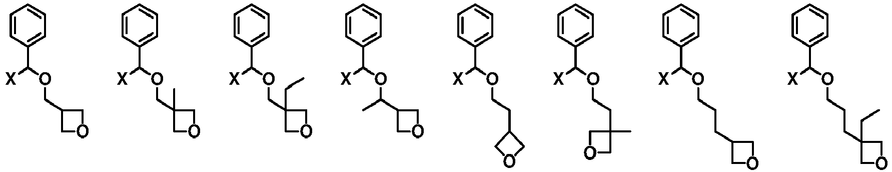

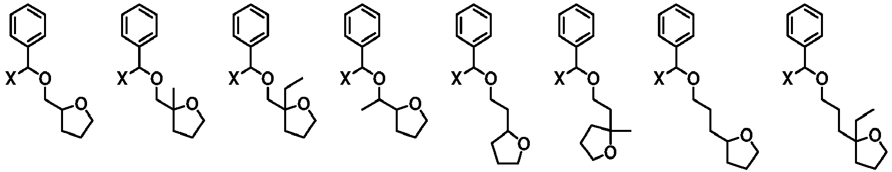

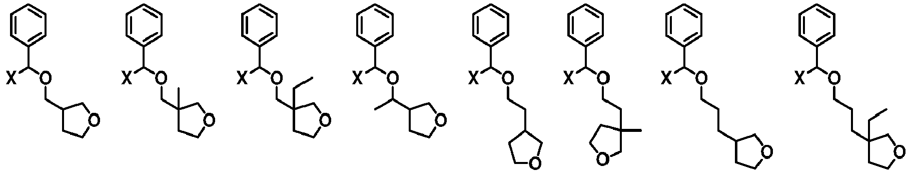

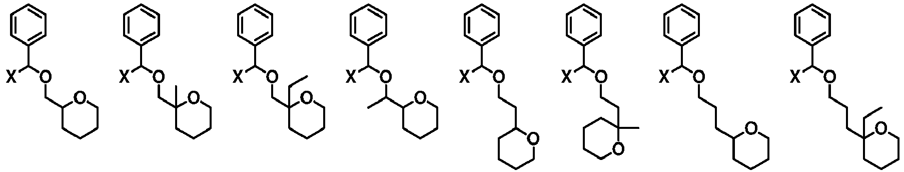

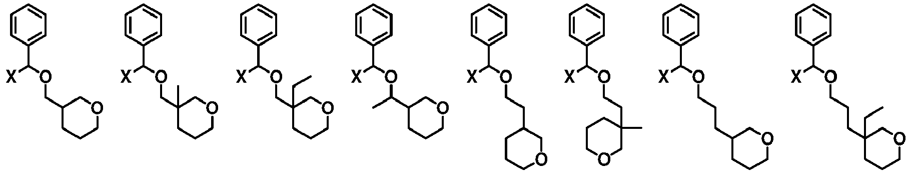

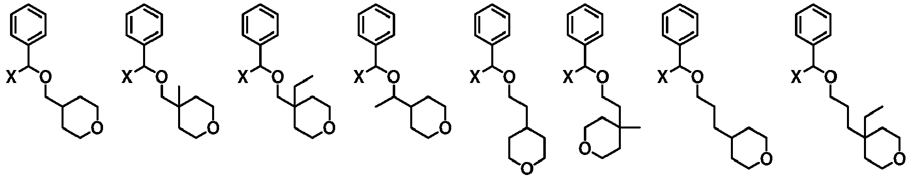

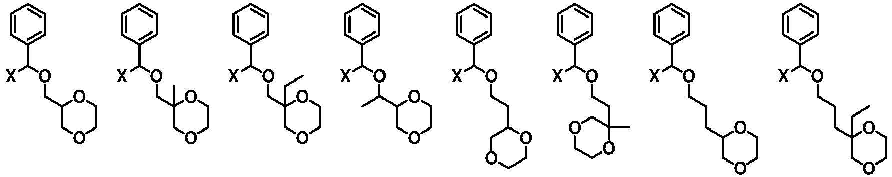

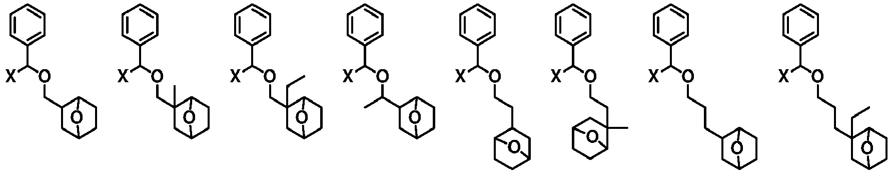

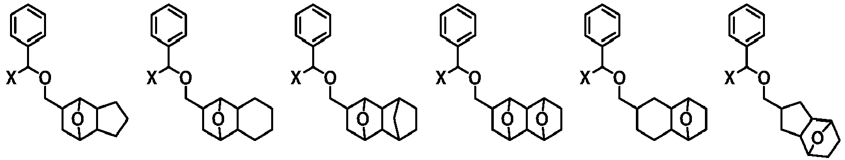

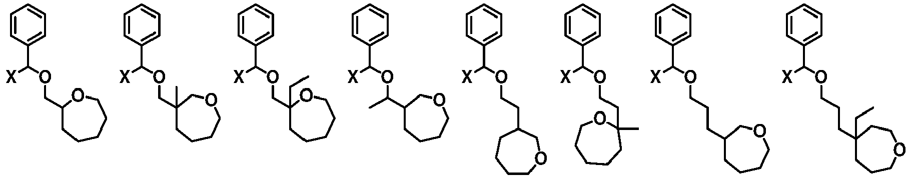

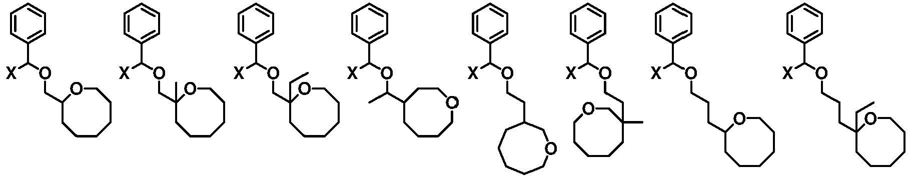

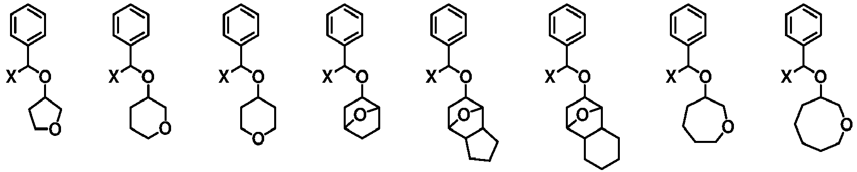

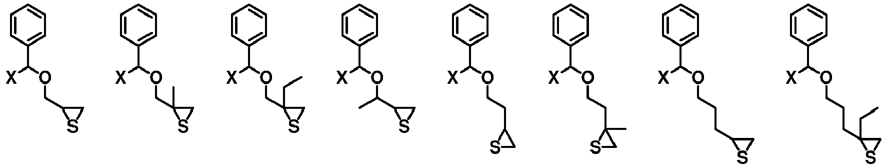

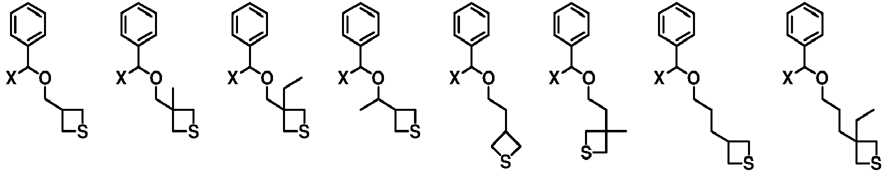

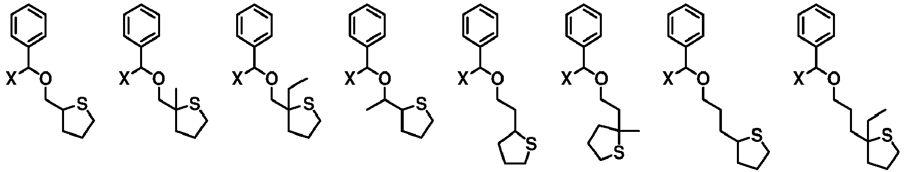

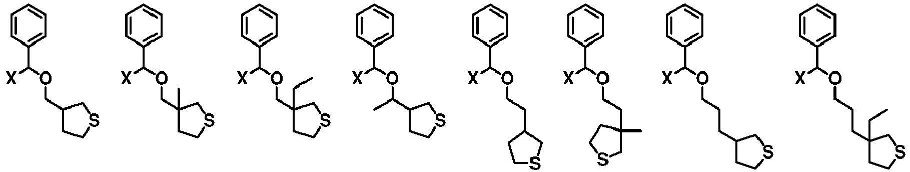

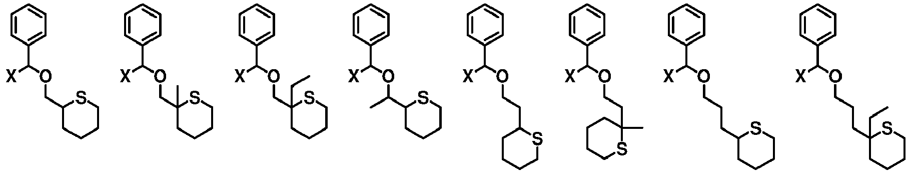

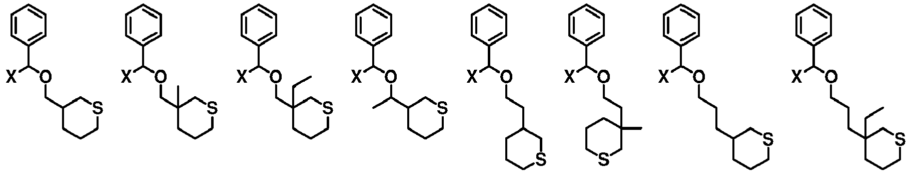

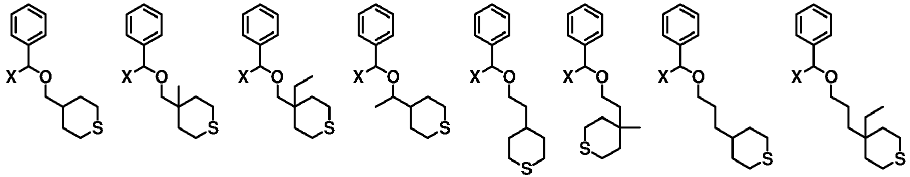

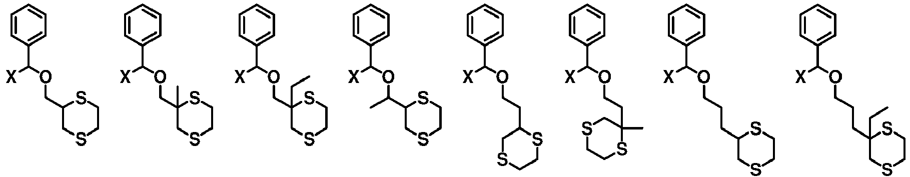

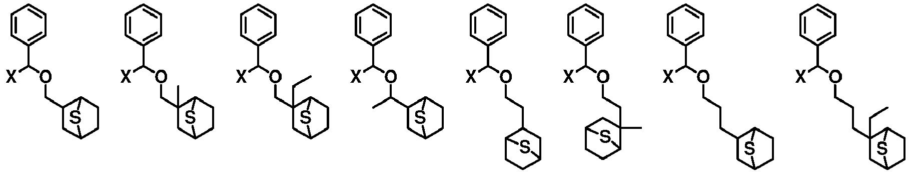

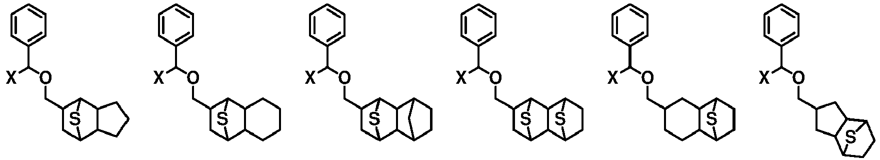

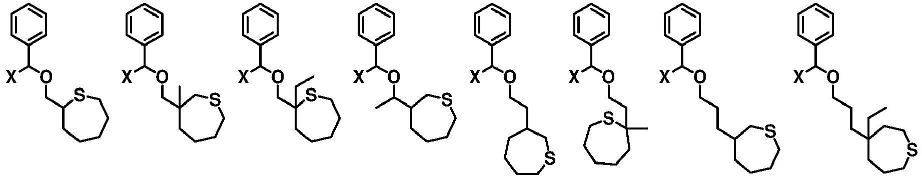

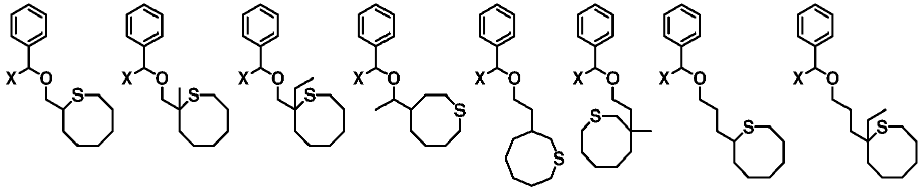

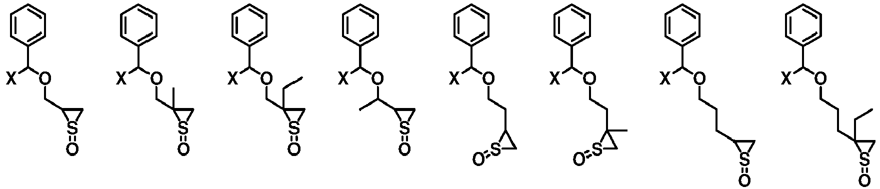

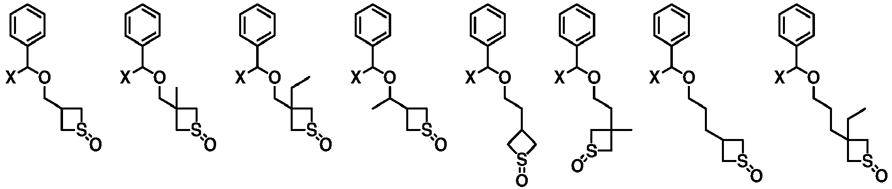

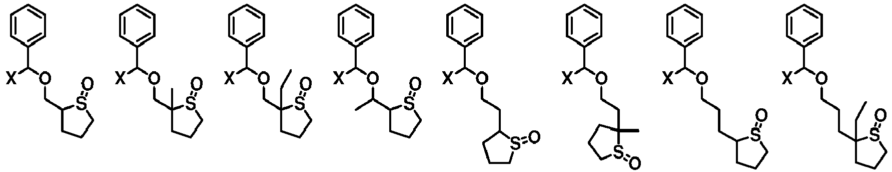

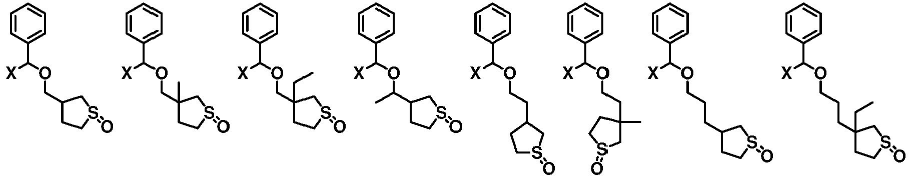

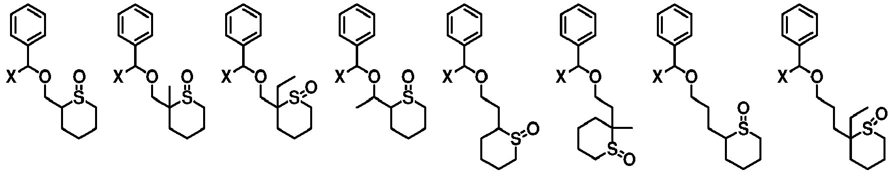

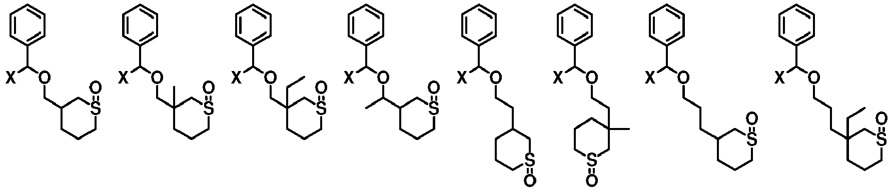

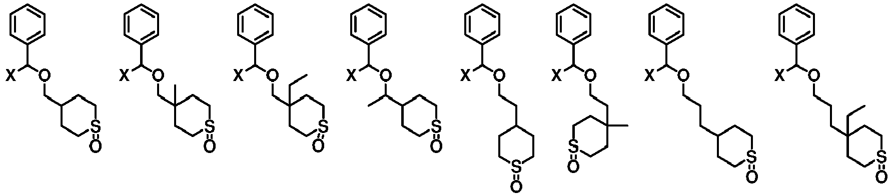

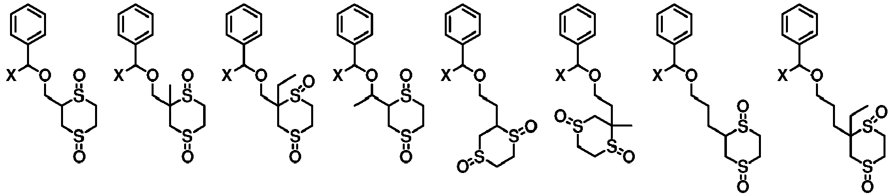

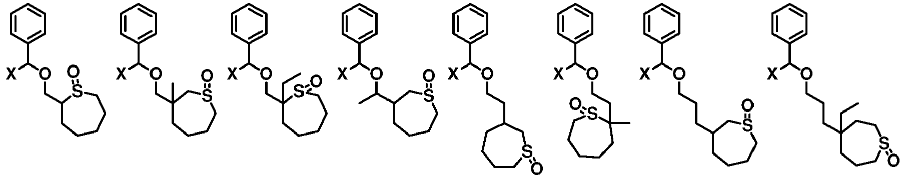

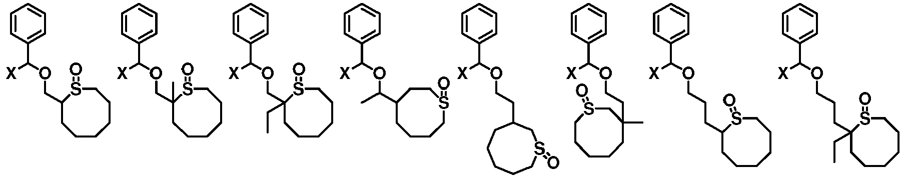

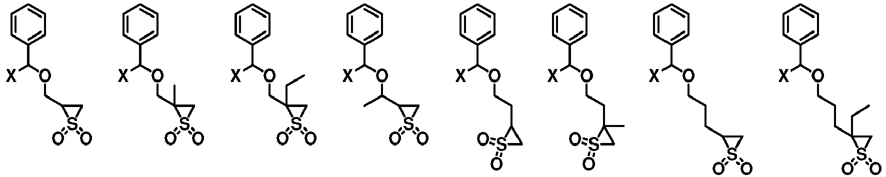

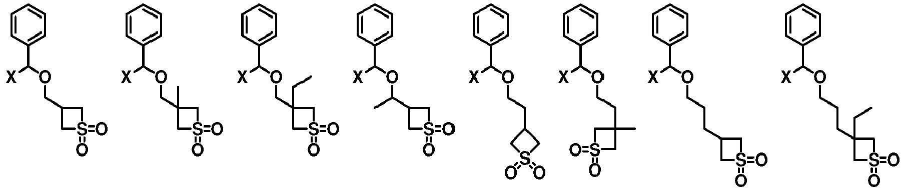

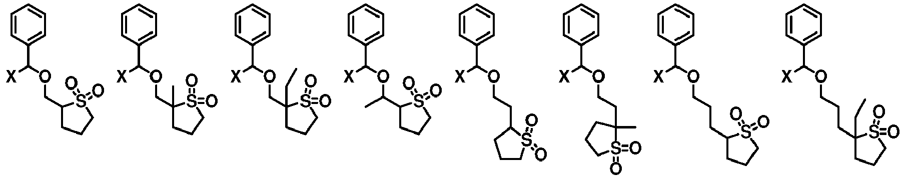

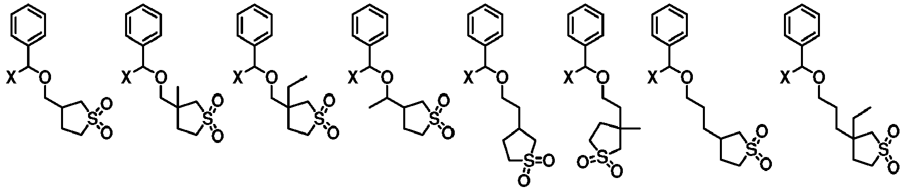

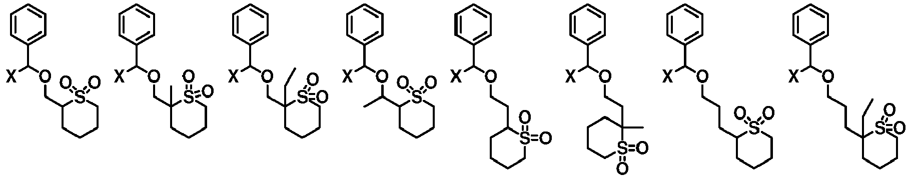

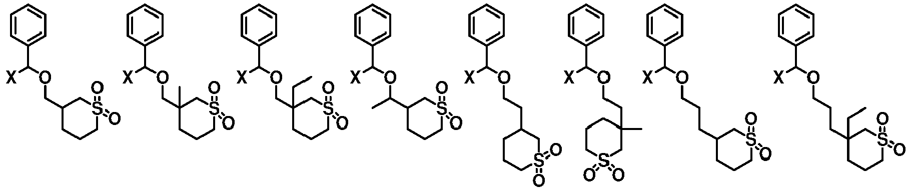

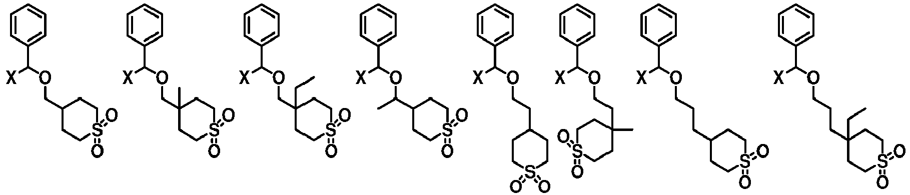

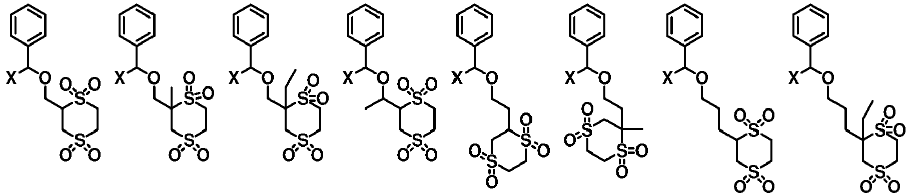

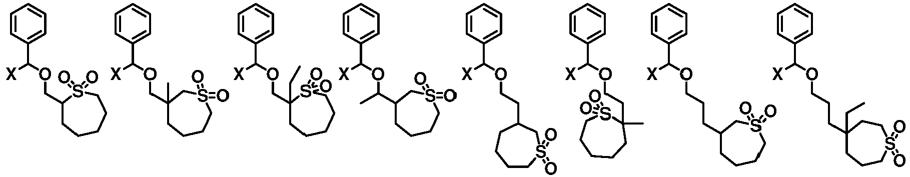

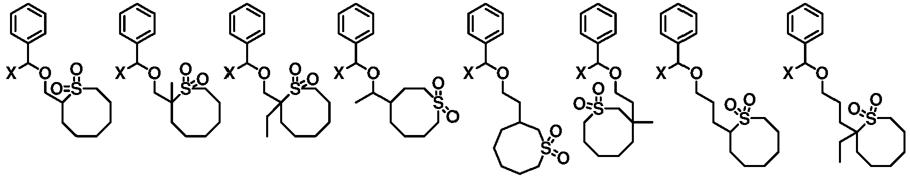

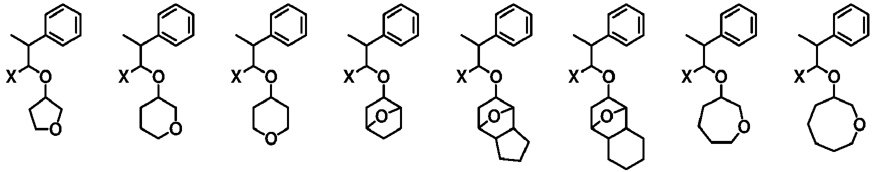

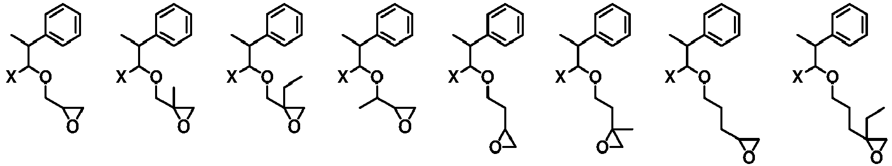

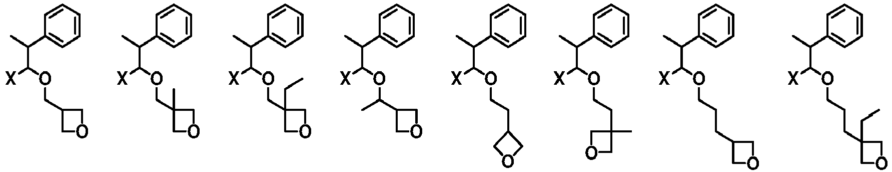

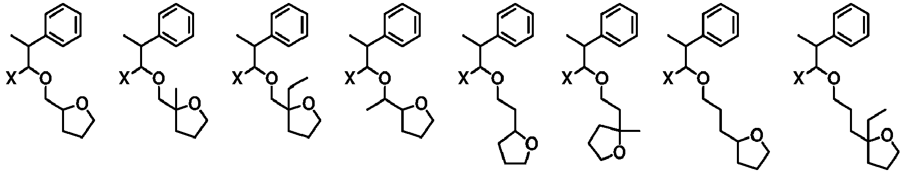

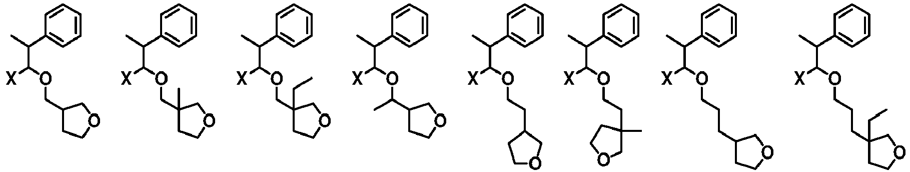

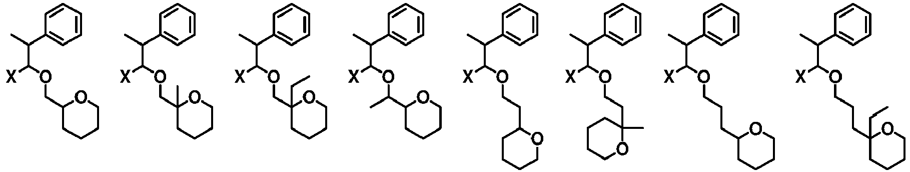

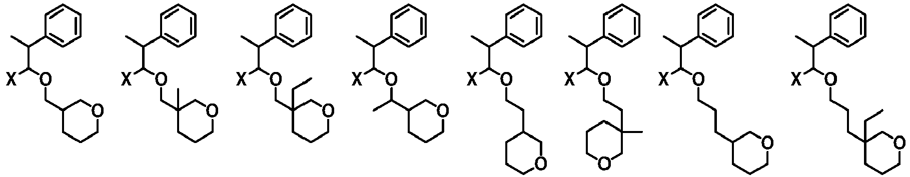

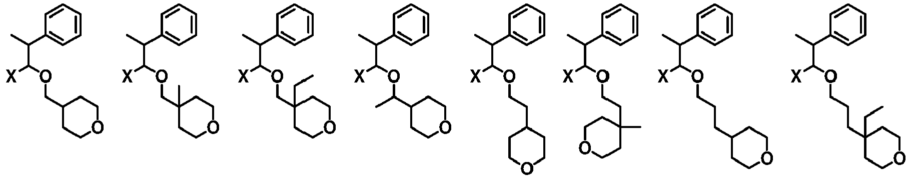

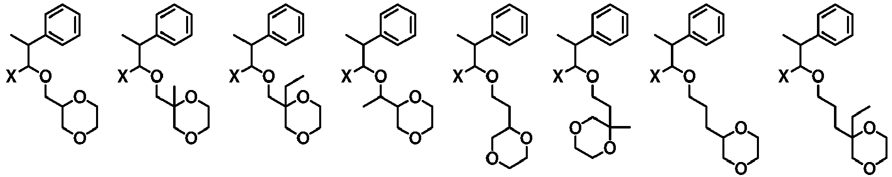

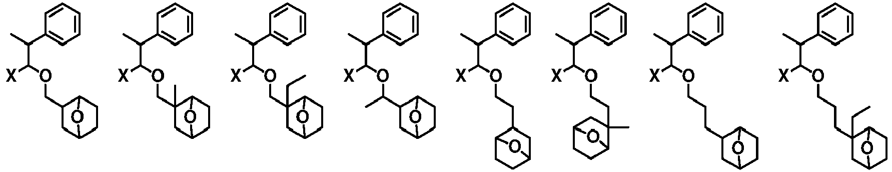

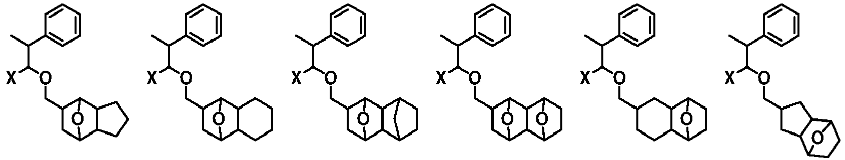

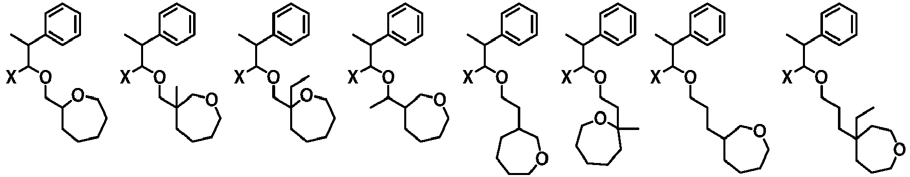

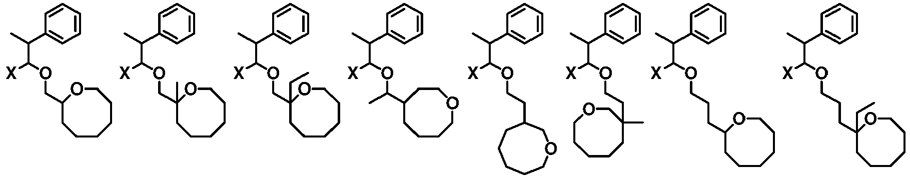

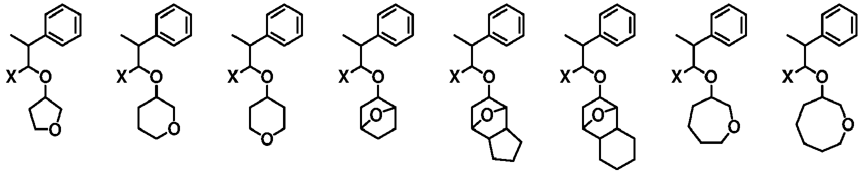

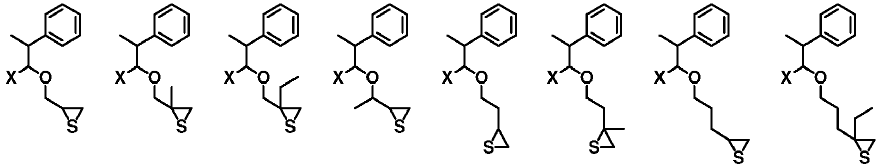

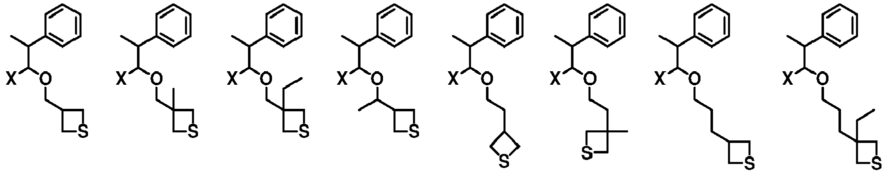

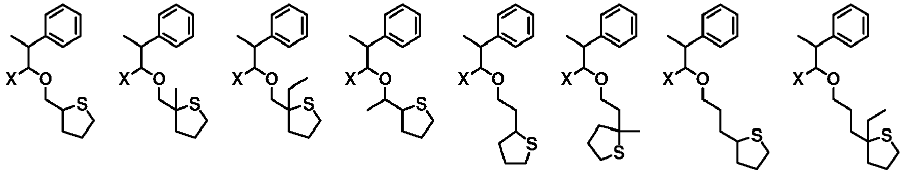

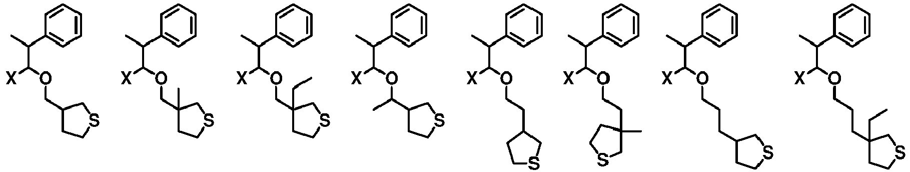

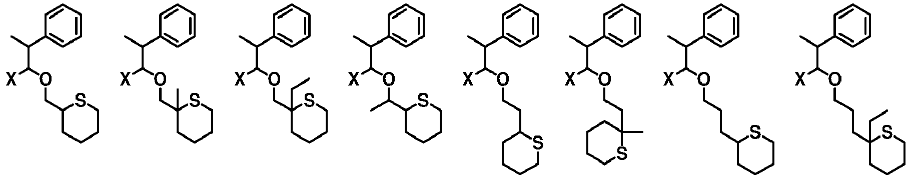

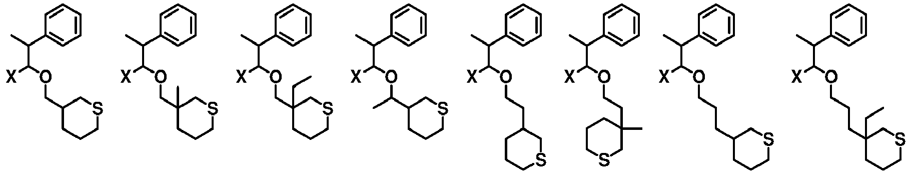

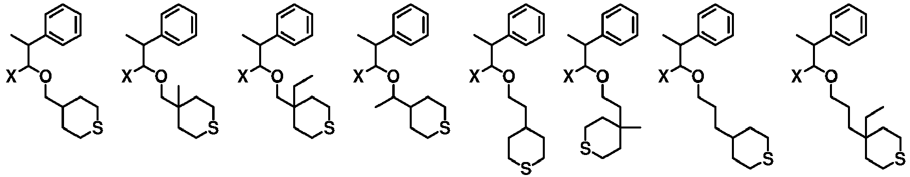

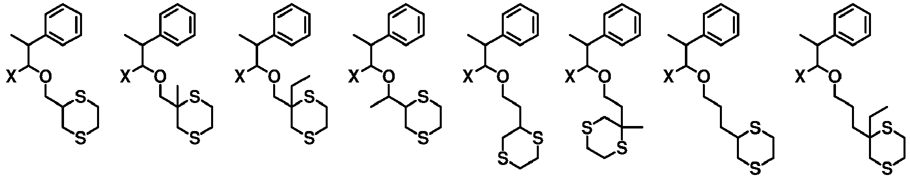

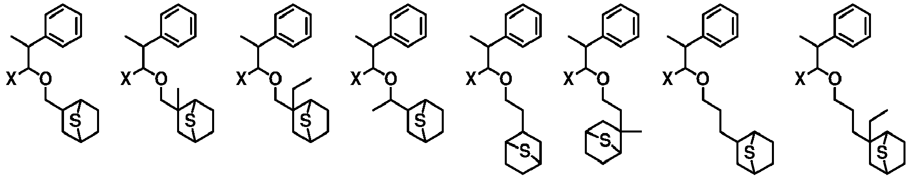

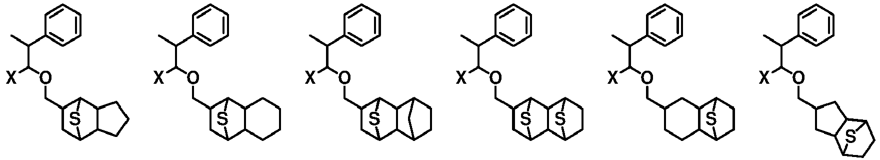

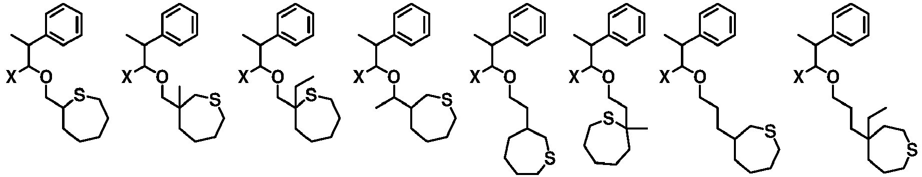

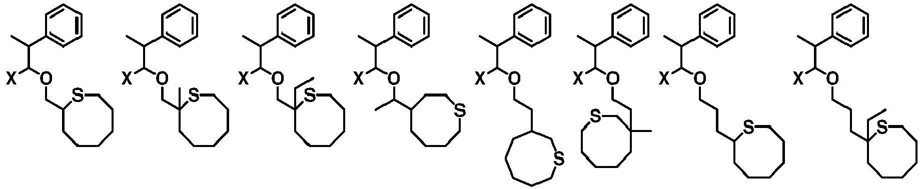

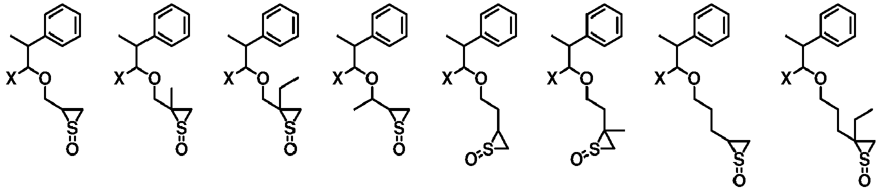

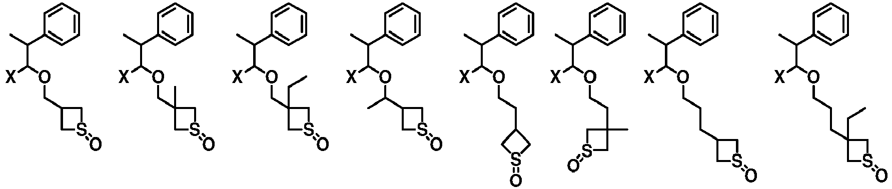

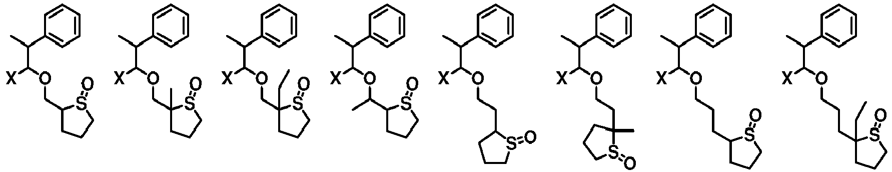

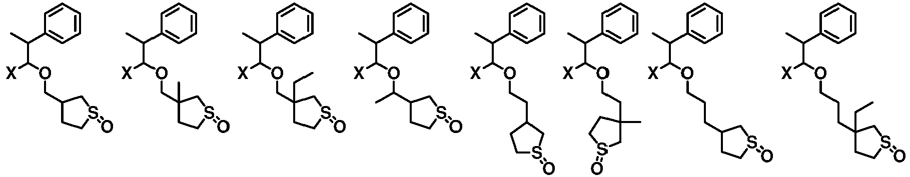

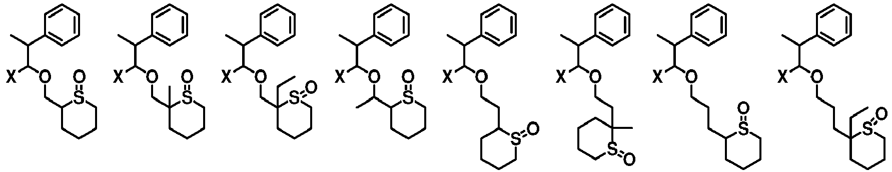

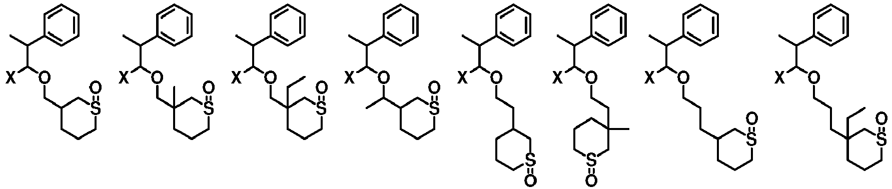

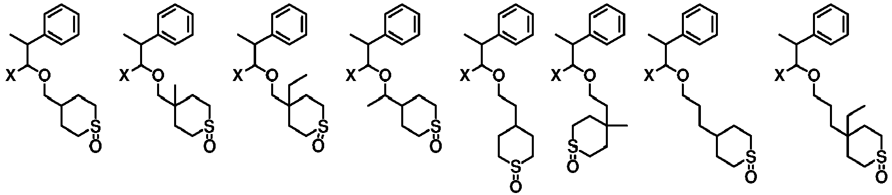

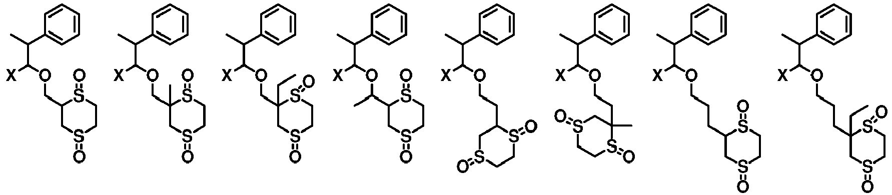

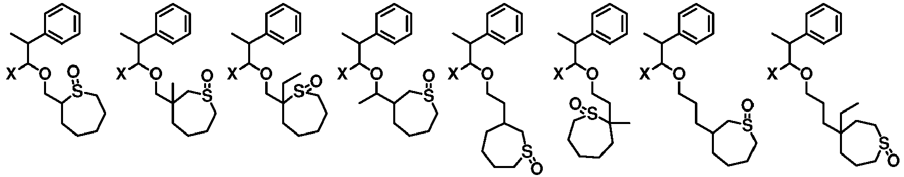

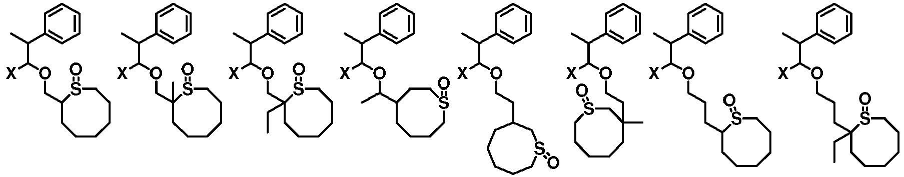

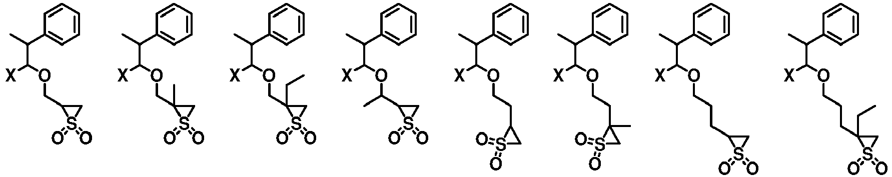

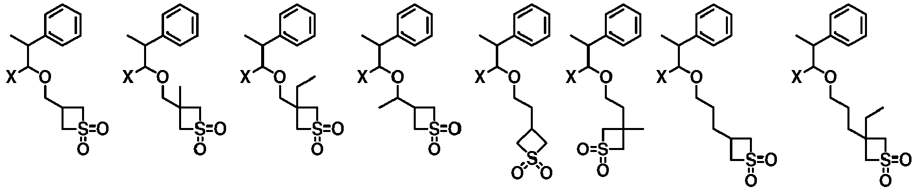

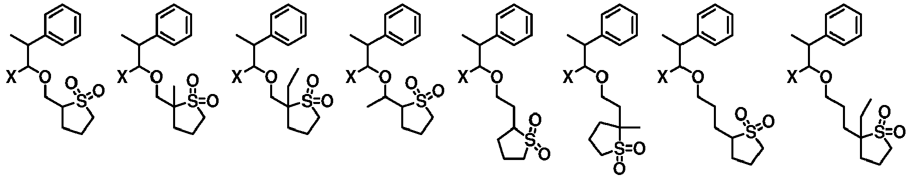

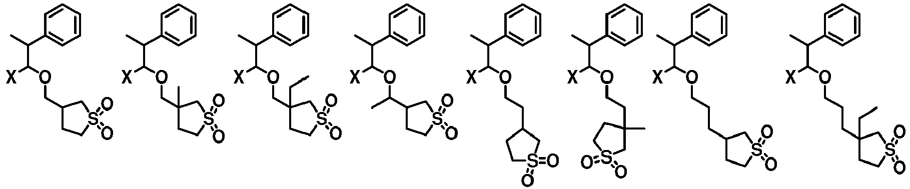

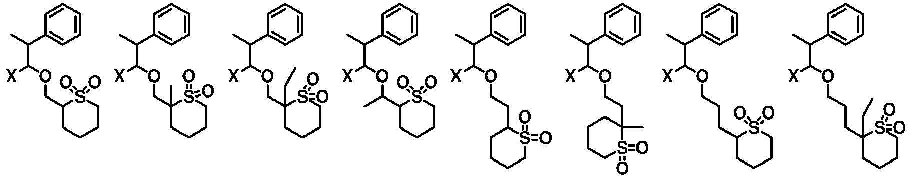

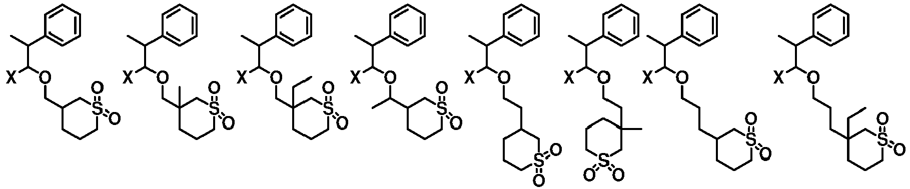

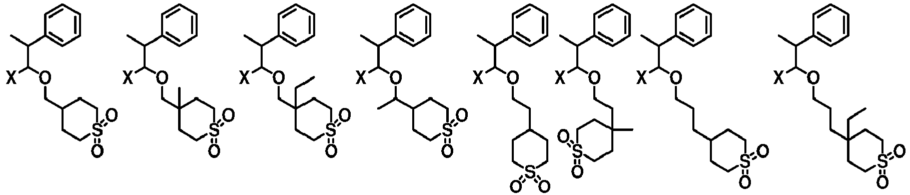

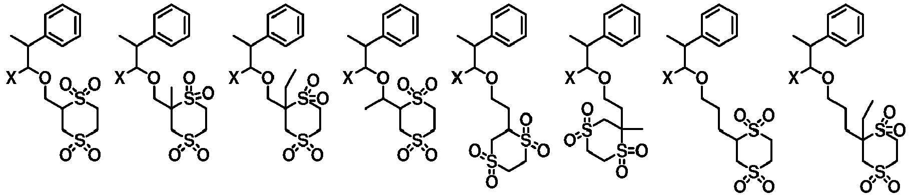

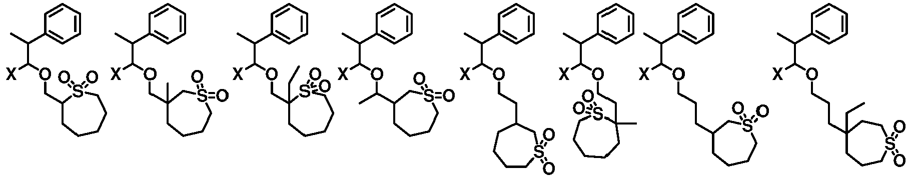

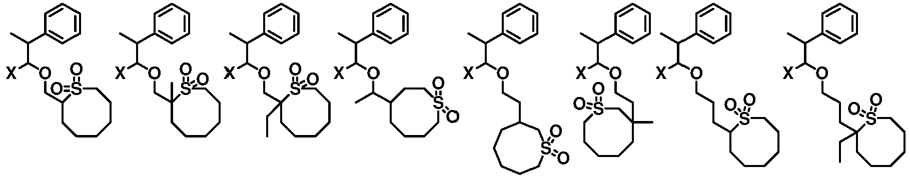

### Polymer

Another embodiment of the invention is a polymer adapted to turn alkali soluble as a result of deprotection under the action of acid, the polymer comprising repeat units A1 having on side chain a structure including an aromatic hydroxy group protected with an acid labile group having the formula (AL-1) or (AL-2). 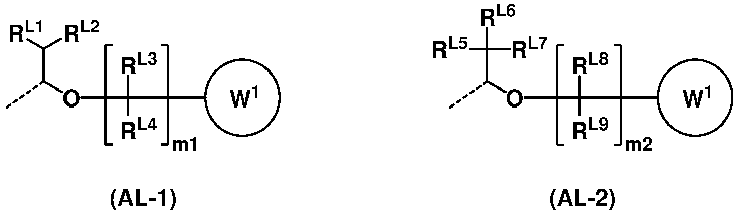 Herein R^{L1} to R^{L9}, m1, and m2 are as defined above. The broken line designates a point of attachment to the oxygen atom of the aromatic hydroxy group.

The repeat unit A1 preferably has the formula (A1).

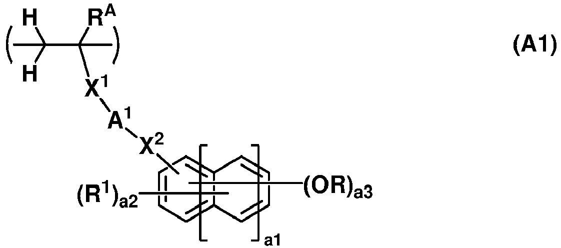

In formula (A1), a1 is 0 or 1, a2 is an integer of 0 to 4 in case of a1=0, and an integer of 0 to 6 in case of a1=1, a3 is an integer of 1 to 3, with the proviso that a2+a3 is from 1 to 5 in case of a1=0 and a2+a3 is from 1 to 7 in case of a1=1.

In formula (A1), R^{A} is hydrogen, fluorine, methyl or trifluoromethyl.

In formula (A1), X¹ is a single bond, *-C(=O)-O- or *-C(=O)-NH-, wherein * designates a point of attachment to the carbon atom in the backbone.

In formula (A1), X² is a single bond, ether bond, ester bond, carbonyl group, sulfonate ester bond, carbonate bond or carbamate bond. Inter alia, a single bond, ether bond or ester bond is preferred, with a single bond or ester bond being more preferred.

In formula (A1), A¹ is a single bond or a C₁-C₁₀ saturated hydrocarbylene group. In the saturated hydrocarbylene group, some constituent -CH₂- may be replaced by -O-. The saturated hydrocarbylene group may be straight, branched or cyclic. Examples thereof include C₁-C₁₀ alkanediyl groups such as methanediyl, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof.

In formula (A1), R¹ is halogen or a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl; C₂-C₂₀ alkenyl groups such as vinyl, allyl, propenyl, butenyl, and hexenyl; C₃-C₂₀ cyclic unsaturated hydrocarbyl groups such as cyclohexenyl; C₆-C₂₀ aryl groups such as phenyl and naphthyl; C₇-C₂₀ aralkyl groups such as benzyl, 1-phenylethyl, and 2-phenylethyl, and combinations thereof. Of these, aryl groups are preferred. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

In formula (A1), R is an acid labile group having formula (AL-1) or (AL-2).

Examples of repeat unit A1 are shown below, but not limited thereto. Herein R is as defined above.

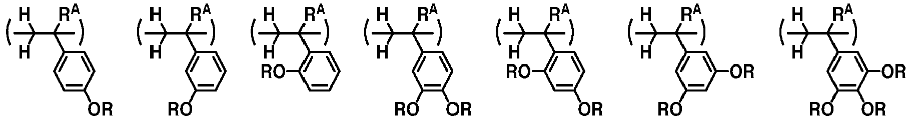

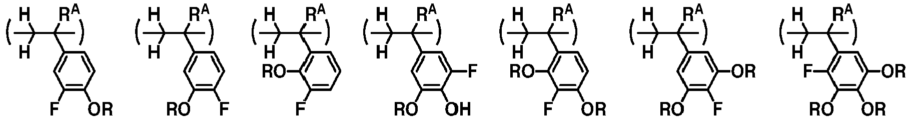

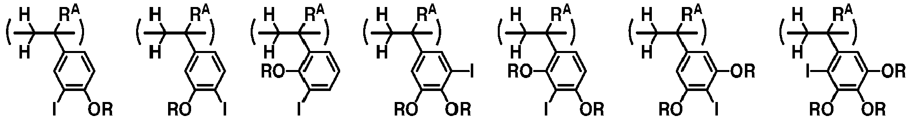

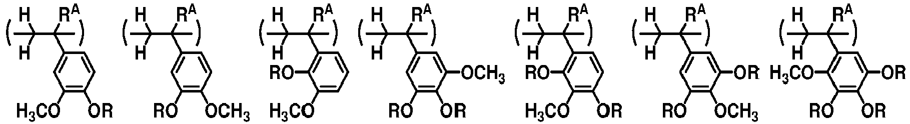

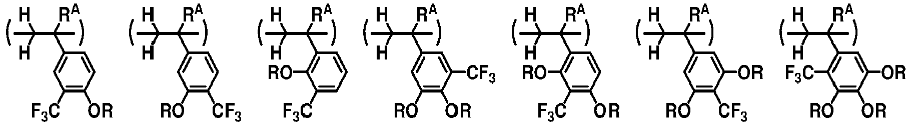

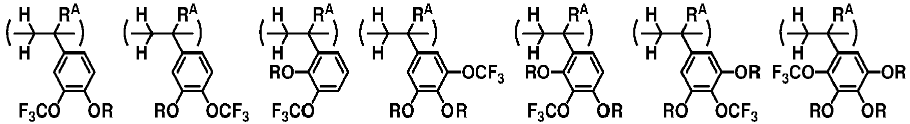

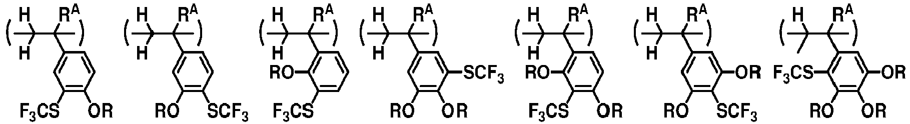

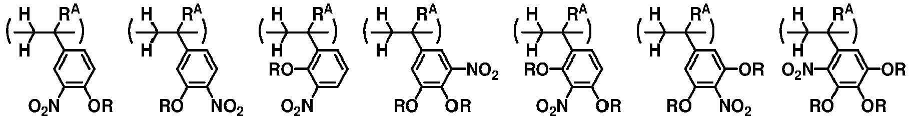

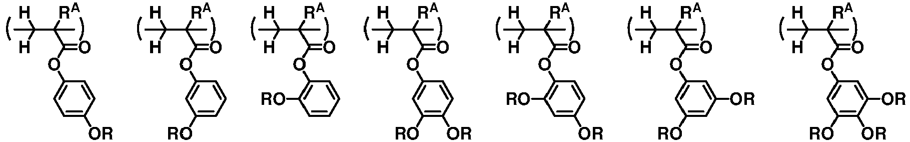

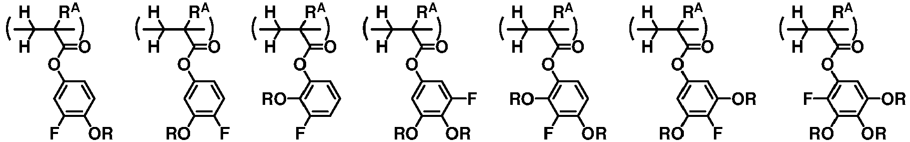

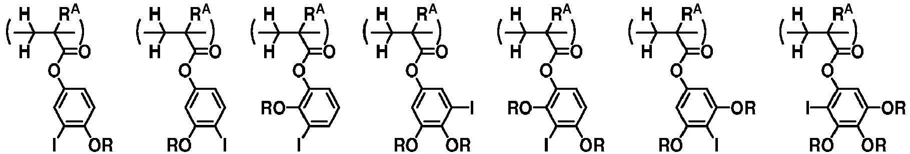

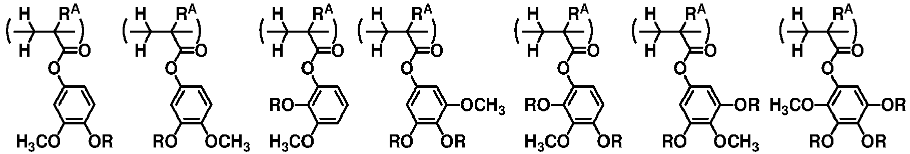

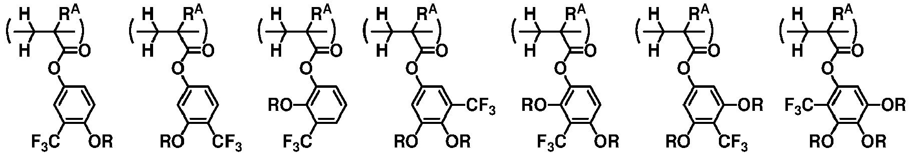

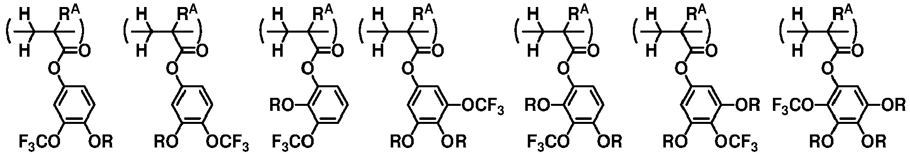

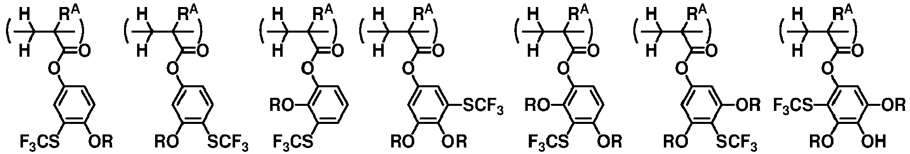

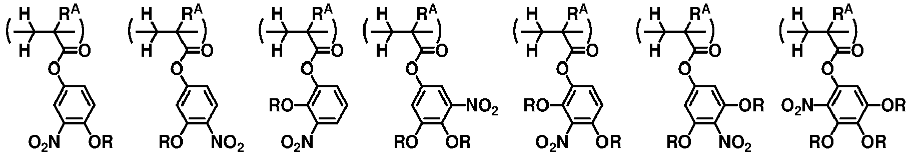

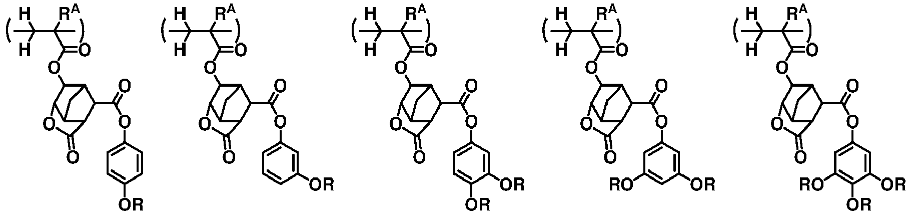

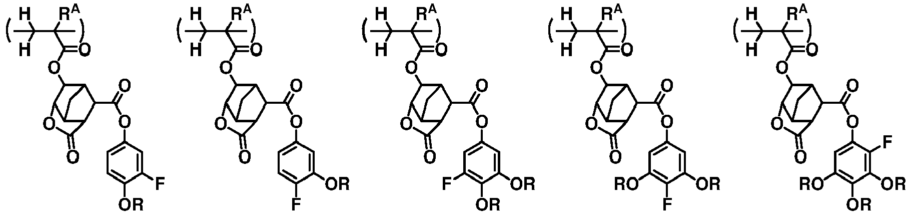

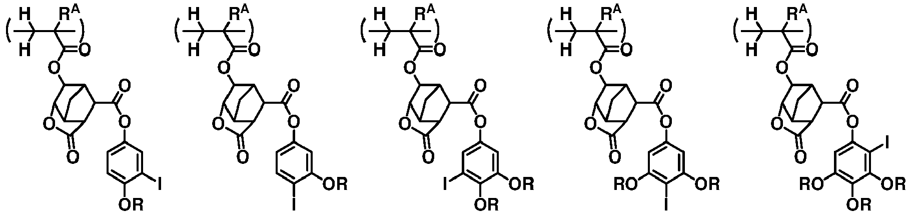

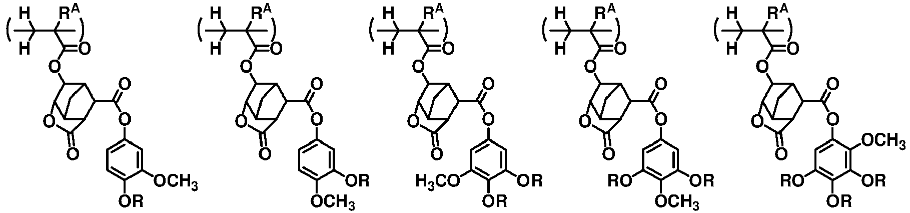

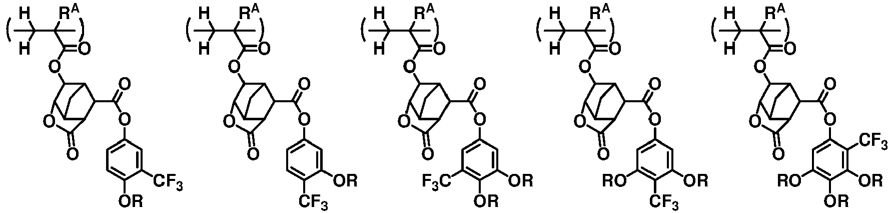

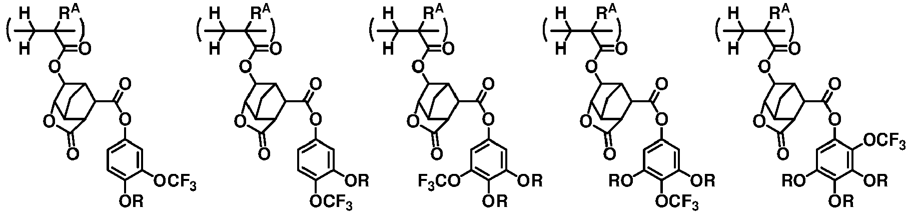

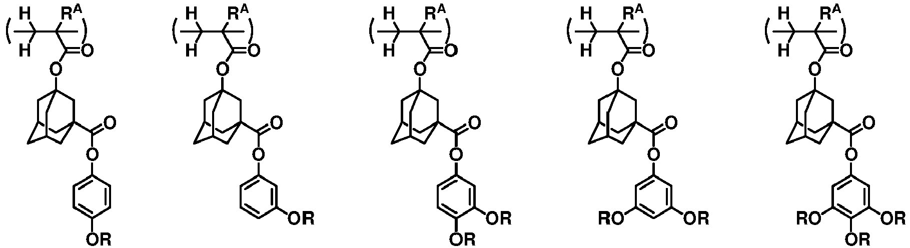

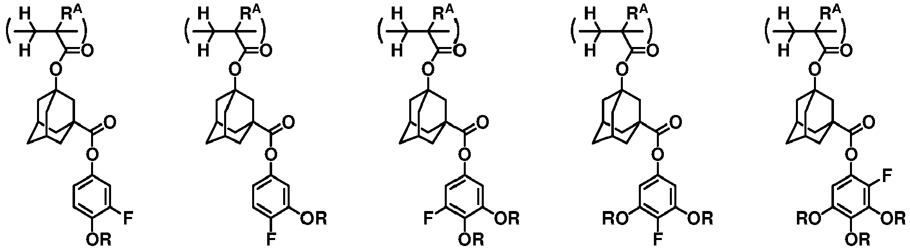

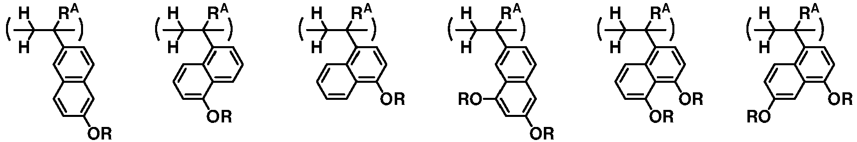

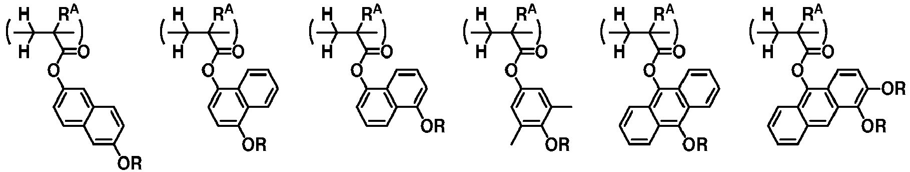

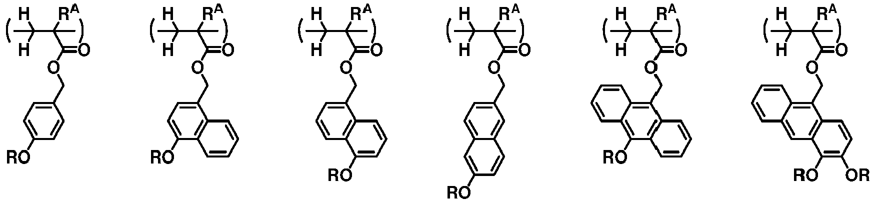

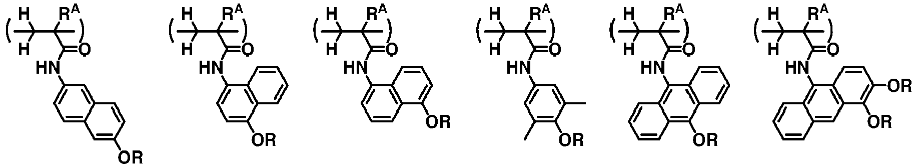

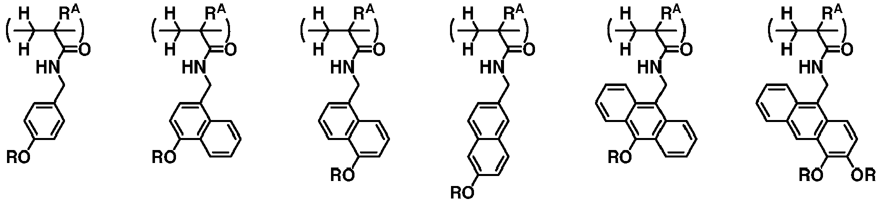

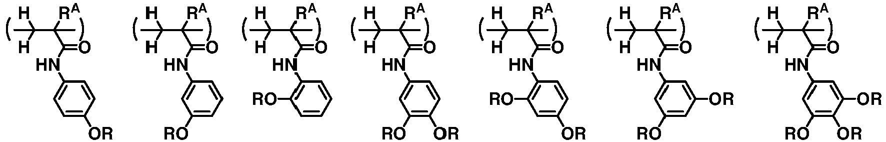

The content of repeat units A1 is preferably 10 to 40 mol%, more preferably 10 to 35 mol%, even more preferably 20 to 30 mol% based on the overall repeat units of the polymer. The repeat units A1 used herein may be of one type or a mixture of two or more types.

In a preferred embodiment, the polymer further comprises phenolic hydroxy group-containing repeat units A2 having the formula (A2).

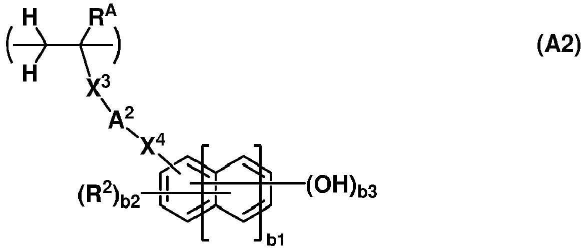

In formula (A2), b1 is 0 or 1, b2 is an integer of 0 to 4 in case of b1=0, and an integer of 0 to 6 in case of b1=1, and b3 is an integer of 1 to 3, with the proviso that b2+b3 is from 1 to 5 in case of b1=0 and b2+b3 is from 1 to 7 in case of b1=1.

In formula (A2), R^{A} is hydrogen, fluorine, methyl or trifluoromethyl.

In formula (A2), X³ is a single bond, *-C(=O)-O- or *-C(=O)-NH-, wherein * designates a point of attachment to the carbon atom in the backbone.

In formula (A2), X⁴ is a single bond, ether bond, ester bond, carbonyl group, sulfonate ester bond, carbonate bond or carbamate bond. Inter alia, a single bond, ether bond or ester bond is preferred, with a single bond or ester bond being more preferred.

In formula (A2), A² is a single bond or a C₁-C₁₀ saturated hydrocarbylene group in which some constituent -CH₂- may be replaced by -O-. The saturated hydrocarbylene group may be straight, branched or cyclic and examples thereof are as exemplified above for the saturated hydrocarbylene group A¹.

In formula (A2), R² is halogen or a C₁-C₂₀ hydrocarbyl group which may contain a heteroatom. The hydrocarbyl group may be saturated or unsaturated and straight, branched or cyclic and examples thereof are as exemplified above for the hydrocarbyl group R¹.

Preferred examples of repeat unit A2 wherein all X³, X⁴ and A² are a single bond include units derived from 3-hydroxystyrene, 4-hydroxystyrene, 5-hydroxy-2-vinylnaphthalene, and 6-hydroxy-2- vinylnaphthalene. Of these, repeat units having the following formula (A2-1) are more preferred. 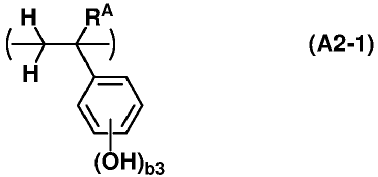 Herein R^{A} and b3 are as defined above.

Preferred examples of repeat unit A2 wherein X³ is other than a single bond include the above-mentioned examples of repeat unit A1 wherein R is replaced by hydrogen, but are not limited thereto.

When the polymer contains repeat units A2, the content of repeat units A2 is preferably 30 to 90 mol%, more preferably 40 to 85 mol% based on the overall repeat units of the polymer. In an embodiment wherein the polymer contains repeat units having formula (B1) and/or repeat units having formula (B2) which provide the polymer with higher etching resistance, and repeat units B1 and/or B2 contain a phenolic hydroxy group as the substituent, preferably the content of repeat units A2 plus repeat units B1 and/or B2 falls in the range. The repeat units A2 used herein may be of one type or a mixture of two or more types.

The polymer may further comprise repeat units of at least one type selected from repeat units having the formula (A3-1) and repeat units having the formula (A3-2). These units are also referred to as repeat units A3-1 and A3-2.

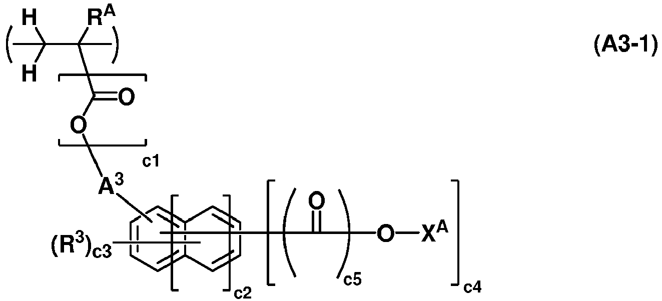

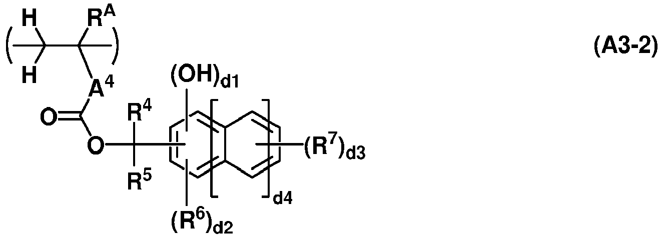

In formula (A3-1), c1 is 0 or 1. The subscript c2 is an integer of 0 to 2, and the structure represents a benzene skeleton when c2=0, a naphthalene skeleton when c2=1, and an anthracene skeleton when c2=2. The subscript c3 is an integer satisfying 0 ≤ c3 ≤ 5+2(c2)-c4; c4 is an integer of 1 to 3; and c5 is 0 or 1. In case of c2=0, preferably c3 is an integer of 0 to 3 and c4 is an integer of 1 to 3. In case of c2=1 or 2, preferably c3 is an integer of 0 to 4 and c4 is an integer of 1 to 3.

In formula (A3-2), d1 is an integer of 0 to 2, d2 is an integer of 0 to 2, d3 is an integer of 0 to 5, and d4 is an integer of 0 to 2.

In formulae (A3-1) and (A3-2), R^{A} is each independently hydrogen, fluorine, methyl or trifluoromethyl.

In formula (A3-1), A³ is a single bond or a C₁-C₁₀ saturated hydrocarbylene group. In the saturated hydrocarbylene group, some constituent -CH₂- may be replaced by -O-. The saturated hydrocarbylene group may be straight, branched or cyclic. Examples thereof include alkanediyl groups such as methylene, ethane-1,2-diyl, propane-1,3-diyl, butane-1,4-diyl, pentane-1,5-diyl, hexane-1,6-diyl, and structural isomers thereof; cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, when c1=1 in formula (A3-1), the ether bond may be incorporated at any position excluding the position between the α-carbon and β-carbon relative to the ester oxygen atom; and when c1=0 in formula (A3-1), the atom that bonds with the backbone becomes an ethereal oxygen atom, and a second ether bond may be incorporated at any position excluding the position between the α-carbon and β-carbon relative to the ethereal oxygen atom. Saturated hydrocarbylene groups having no more than 10 carbon atoms are desirable because of a sufficient solubility in alkaline developer.

In formula (A3-2), A⁴ is a single bond, phenylene group, naphthylene group or *-C(=O)-O-A⁴¹-. A⁴¹ is a C₁-C₂₀ aliphatic hydrocarbylene group which may contain hydroxy, ether bond, ester bond or lactone ring, or a phenylene group or naphthylene group, and * designates a point of attachment to the carbon atom in the backbone.

In formula (A3-1), R³ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched or cyclic. Examples thereof include C₁-C₆ alkyl groups such as methyl, ethyl, propyl, isopropyl, butyl, pentyl, and hexyl; C₃-C₆ cycloalkyl groups such as cyclopropyl, cyclobutyl, cyclopentyl, and cyclohexyl, and combinations thereof. A carbon count below the upper limit ensures a satisfactory solubility in alkaline developer. When c3 is 2 or more, a plurality of R³ may be the same or different.

In formula (A3-2), R⁴ and R⁵ are each independently a C₁-C₁₀ hydrocarbyl group which may contain a heteroatom. R⁴ and R⁵ may bond together to form a ring with the carbon atom to which they are attached.

In formula (A3-2), R⁶ is each independently fluorine or a C₁-C₅ fluorinated alkyl group or C₁-C₅ fluorinated alkoxy group.

In formula (A3-2), R⁷ is each independently a C₁-C₁₀ hydrocarbyl group which may contain a heteroatom.

In formula (A3-1), X^{A} is an acid labile group when c4=1, and hydrogen or an acid labile group, at least one X^{A} being an acid labile group, when c4=2 or 3. That is, repeat units A3-1 have phenolic hydroxy groups bonded to an aromatic ring, at least one of which is protected with an acid labile group, or repeat units A3-1 have a carboxy group bonded to an aromatic ring, which is protected with an acid labile group. The acid labile group used herein is not particularly limited as long as it is commonly used in a number of well-known chemically amplified positive resist compositions and eliminated under the action of acid to release an acidic group.

A choice of a tertiary saturated hydrocarbyl group as the acid labile group is preferred for the reason that even when a resist film is formed to a thickness of 10 to 100 nm and processed to form a fine pattern having a line width of up to 45 nm, the pattern has reduced LER. Of the tertiary saturated hydrocarbyl groups, tertiary alkyl groups of 4 to 18 carbon atoms are preferred because the corresponding monomer for use in polymerization is available through distillation. The group attached to the tertiary carbon atom in the tertiary saturated hydrocarbyl group is typically a C1-C15 saturated hydrocarbyl group which may contain an oxygen-containing functional group such as ether bond or carbonyl group. The groups attached to the tertiary carbon atom may bond together to form a ring.

Examples of the group bonded to the tertiary carbon atom include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, tetracyclo[4.4.0.1^{2,5}.1^{7,10}]dodecyl, and 3-oxo-1-cyclohexyl.

Examples of the tertiary saturated hydrocarbyl group having such a substituent include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-isopropylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl,3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (A3-1-1) is also suitable as the acid labile group. The group having formula (A3-1-1) is often used as the acid labile group. It is a good choice of the acid labile group that ensures to form a pattern having a relatively rectangular pattern-substrate interface in a consistent manner. An acetal structure is formed when X is a group having formula (A3-1-1).

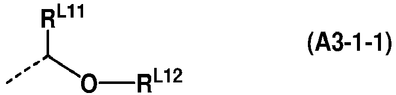

In formula (A3-1-1), R^{L11} is hydrogen or a C₁-C₁₀ saturated hydrocarbyl group. R^{L12} is a C₁-C₃₀ saturated hydrocarbyl group. The saturated hydrocarbyl group may be straight, branched or cyclic.

A choice of R^{L11} may depend on the designed sensitivity of decomposable group to acid. For example, hydrogen is selected when the acid decomposable group is designed to ensure relatively high stability and to be decomposed with strong acid. A straight alkyl group is selected when the acid decomposable group is designed to have relatively high reactivity and high sensitivity to pH changes. Although the choice varies with a particular combination of acid generator and basic compound in the resist composition, R^{L11} is preferably a group in which the carbon in bond with acetal carbon is secondary, when R^{L12} is a relatively large alkyl group substituted at the end and the acid decomposable group is designed to undergo a substantial change of solubility by decomposition. Examples of R^{L11} bonded to acetal carbon via secondary carbon include isopropyl, sec-butyl, cyclopentyl, and cyclohexyl.

In the acetal group, R^{L12} is preferably a C₇-C₃₀ polycyclic alkyl group for acquiring a higher resolution. When R^{L12} is a polycyclic alkyl group, a bond is preferably formed between secondary carbon on the polycyclic structure and acetal oxygen. The acetal oxygen bonded to secondary carbon on the cyclic structure, as compared with the acetal oxygen bonded to tertiary carbon on the cyclic structure, ensures that a corresponding polymer becomes a stable compound, suggesting that the resist composition has better shelf stability and is not degraded in resolution. Said acetal oxygen, as compared with R^{L12} bonded to primary carbon via a straight alkyl group of at least one carbon atom, ensures that a corresponding polymer has a higher glass transition temperature (Tg), suggesting that a resist pattern after development is not deformed by bake.

Preferred examples of the group having formula (A3-1-1) are given below, but not limited thereto. Herein R^{L11} is as defined above.

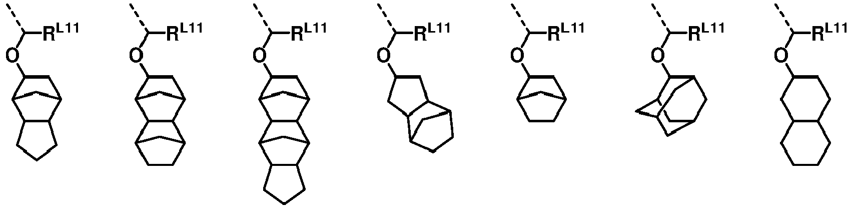

Preferred examples of repeat unit A3-2 are given below, but not limited thereto. Herein R^{A} is as defined above.

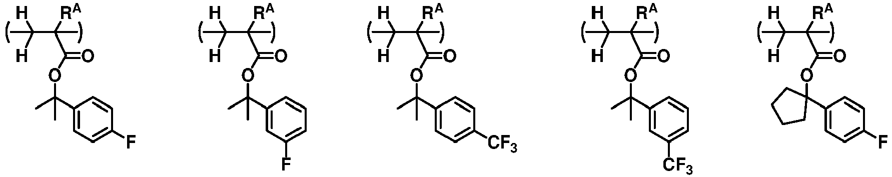

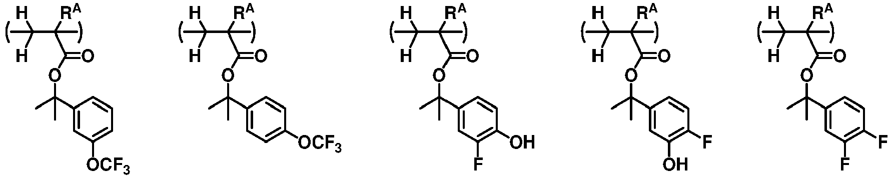

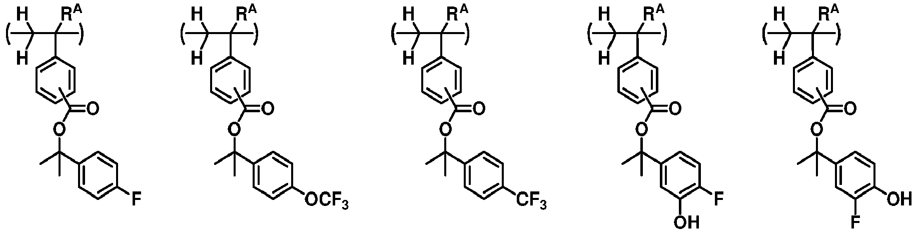

Another choice of acid labile group which can be used herein is a phenolic hydroxy group whose hydrogen is substituted by a tertiary saturated hydrocarbyl moiety: -CH₂COO-. Examples of the tertiary saturated hydrocarbyl moiety are as exemplified above for the tertiary saturated hydrocarbyl group used for the protection of phenolic hydroxy group.

Repeat units of at least one type selected from repeat units A3-1 and A3-2 are preferably incorporated in a range of 2 to 40 mol% of the overall repeat units of the polymer. The sum of repeat units A1, A3-1 and A3-2 is preferably incorporated in a range of 10 to 60 mol%, more preferably 10 to 50 mol%, even more preferably 10 to 40 mol% of the overall repeat units of the polymer. The repeat units A3-1 and A3-2 may be of one type or a mixture of two or more types.

The polymer may further comprise repeat units of at least one type selected from repeat units having the formula (B1), repeat units having the formula (B2), and repeat units having the formula (B3). These units are also referred to as repeat units B1, B2, and B3.

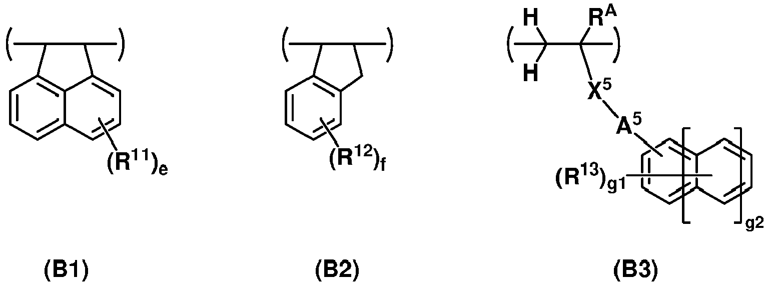

In formulae (B1) and (B2), e and f are each independently an integer of 0 to 4.

In formula (B3), R^{A} is hydrogen, fluorine, methyl or trifluoromethyl, g1 is an integer of 0 to 5, and g2 is an integer of 0 to 2.

In formula (B3), X⁵ is a single bond, *-C(=O)-O- or *-C(=O)-NH-, wherein * designates a point of attachment to the carbon atom in the backbone.

In formula (B3), A⁵ is a single bond or a C₁-C₁₀ saturated hydrocarbylene group. In the saturated hydrocarbylene group, some constituent -CH₂- may be replaced by -O-. The saturated hydrocarbylene group may be straight, branched or cyclic and examples thereof are as exemplified above for A¹ in formula (A1).

In formulae (B1) and (B2), R¹¹ and R¹² are each independently a hydroxy group, halogen, an optionally halogenated C₂-C₈ saturated hydrocarbylcarbonyloxy group, optionally halogenated C₁-C₈ saturated hydrocarbyl group, or optionally halogenated C₁-C₈ saturated hydrocarbyloxy group. The saturated hydrocarbylcarbonyloxy group, saturated hydrocarbyl group, and saturated hydrocarbyloxy group may be straight, branched or cyclic. When e is 2 or more, a plurality of R¹¹ may be the same or different. When f is 2 or more, a plurality of R¹² may be the same or different.

In formula (B3), R¹³ is an acetyl group, C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group or cyano group. R¹³ may also be a hydroxy group in case of g2=1 or 2. The saturated hydrocarbyl group, saturated hydrocarbyloxy group, saturated hydrocarbylcarbonyloxy group, saturated hydrocarbyloxyhydrocarbyl group and saturated hydrocarbylthiohydrocarbyl group may be straight, branched or cyclic. When g1 is 2 or more, a plurality of R¹³ may be the same or different.

When repeat units of at least one type selected from repeat units B1 to B3 are incorporated, better performance is obtained because not only the aromatic ring possesses etch resistance, but the cyclic structure incorporated into the main chain also exerts the effect of improving resistance to etching and EB irradiation during pattern inspection step.

The repeat units B1 to B3 are preferably incorporated in a range of at least 5 mol% based on the overall repeat units of the polymer for obtaining the effect of improving etch resistance. Also, the repeat units B1 to B3 are preferably incorporated in a range of up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B1 to B3 may be of one type or a combination of plural types.

The total content of repeat units A2 and repeat units of at least one type selected from repeat units B1 to B3 is preferably at least 50 mol%, more preferably at least 60 mol% based on the overall repeat units of the polymer.

The polymer may further comprise (meth)acrylate units protected with an acid labile group and/or (meth)acrylate units having an adhesive group such as a lactone structure or a hydroxy group other than phenolic hydroxy, as commonly used in the art. These repeat units are effective for fine adjustment of properties of a resist film, but not essential.

Examples of the (meth)acrylate unit having an adhesive group include repeat units having the following formula (B4), repeat units having the following formula (B5), and repeat units having the following formula (B6), which are also referred to as repeat units B4, B5, and B6, respectively. While these units do not exhibit acidity, they may be used as auxiliary units for providing adhesion to substrates or adjusting solubility.

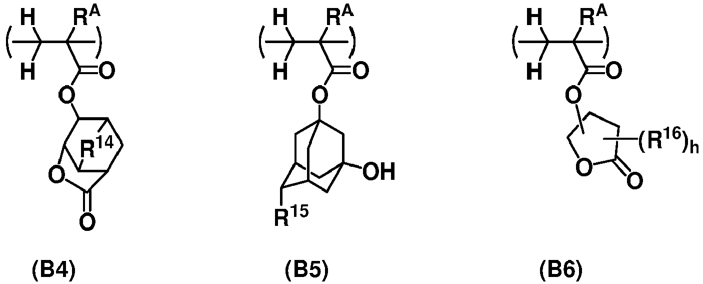

In formulae (B4) to (B6), R^{A} is each independently hydrogen, fluorine, methyl or trifluoromethyl. R¹⁴ is -O- or methylene. R¹⁵ is hydrogen or hydroxy. R¹⁶ is a C₁-C₄ saturated hydrocarbyl group, and h is an integer of 0 to 3.

When the repeat units B4 to B6 are included, their content is preferably 0 to 20 mol%, more preferably 0 to 10 mol% based on the overall repeat units of the polymer. Each of the repeat units B4 to B6 may be of one type or a combination of plural types.

The polymer may further comprise repeat units of at least one type selected from repeat units having the formula (C1), repeat units having the formula (C2), repeat units having the formula (C3), and repeat units having the formula (C4), which are also referred to as repeat units C1, C2, C3, and C4, respectively.

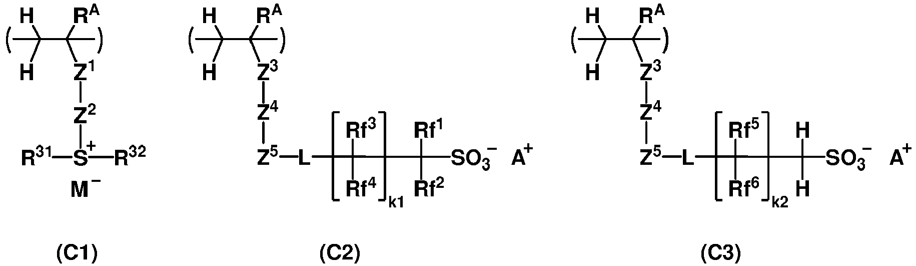

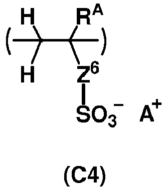

In formulae (C1) to (C4), R^{A} is each independently hydrogen, fluorine, methyl or trifluoromethyl, Z¹ is a single bond or phenylene group, Z² is *¹-C(=O)-O-Z²¹-, *¹-C(=O)-NH-Z²¹-, or *¹-O-Z²¹-, Z²¹ is a C₁-C₆ aliphatic hydrocarbylene group, phenylene group or divalent group obtained by combining the foregoing, which may contain a carbonyl moiety, ester bond, ether bond or hydroxy moiety, *¹ designates a point of attachment to Z¹, Z³ is each independently a single bond, phenylene group, naphthylene group or *²-C(=O)-O-Z³¹-, Z³¹ is a C₁-C₁₀ aliphatic hydrocarbylene group, phenylene group or naphthylene group, the aliphatic hydrocarbylene group may contain a hydroxy moiety, ether bond, ester bond or lactone ring, *² designates a point of attachment to the carbon atom in the backbone, Z⁴ is each independently a single bond, *³-Z⁴¹-C(=O)-O-, *³-C(=O)-NH-Z⁴¹-, or *³-O-Z⁴¹-, Z⁴¹ is a C₁-C₂₀ hydrocarbylene group which may contain a heteroatom, *³ designates a point of attachment to Z³, Z⁵ is each independently a single bond, *⁴-Z⁵¹-C(=O)-O-, *⁴-C(=O)-NH-Z⁵¹-, or *⁴-O-Z⁵¹-, Z⁵¹ is a C₁-C₂₀ hydrocarbylene group which may contain a heteroatom, *⁴ designates a point of attachment to Z⁴, Z⁶ is each independently a single bond, methylene, ethylene, phenylene, fluorinated phenylene, trifluoromethyl-substituted phenylene, *²-C(=O)-O-Z⁶¹-, *²-C(=O)-N(H)-Z⁶¹-, or *²-O-Z⁶¹-, Z⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, *² designates a point of attachment to the carbon atom in the backbone.

The aliphatic hydrocarbylene group represented by Z²¹, Z³¹ and Z⁶¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl, cyclohexanediyl; and combinations thereof.

The hydrocarbylene group represented by Z⁴¹ and Z⁵¹ may be saturated or unsaturated and straight, branched or cyclic. Examples thereof are shown below, but not limited thereto.

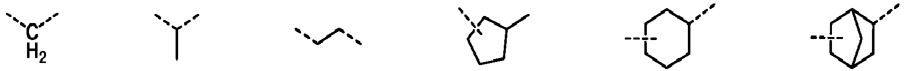

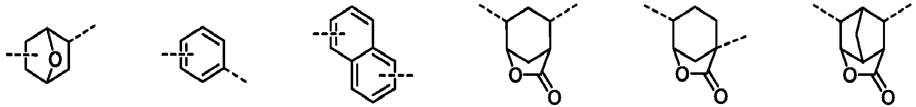

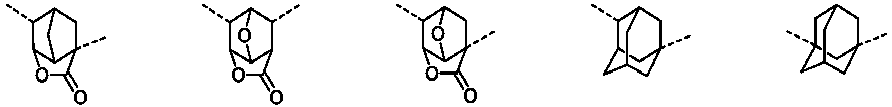

In formula (C1), R³¹ and R³² are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl; C₂-C₂₀ alkenyl groups such as vinyl, allyl, propenyl, butenyl, and hexenyl; C₃-C₂₀ cyclic unsaturated hydrocarbyl groups such as cyclohexenyl; C₆-C₂₀ aryl groups such as phenyl, naphthyl and thienyl; C₇-C₂₀ aralkyl groups such as benzyl, 1-phenylethyl, and 2-phenylethyl, and combinations thereof. Of these, aryl groups are preferred. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, fluorine, chlorine, bromine, iodine, cyano moiety, carbonyl moiety, ether bond, ester bond, sulfonate ester bond, carbonate bond, lactone ring, sultone ring, carboxylic anhydride (-C(=O)-O-C(=O)-) or haloalkyl moiety.

R³¹ and R³² may bond together to form a ring with the sulfur atom to which they are attached. Examples of the ring are as will be exemplified later for the ring that R^{ct1} and R^{ct2} in formula (cation-1), taken together, form with the sulfur atom to which they are attached.

Examples of the cation in repeat units C1 are shown below, but not limited thereto. Herein R^{A} is as defined above.

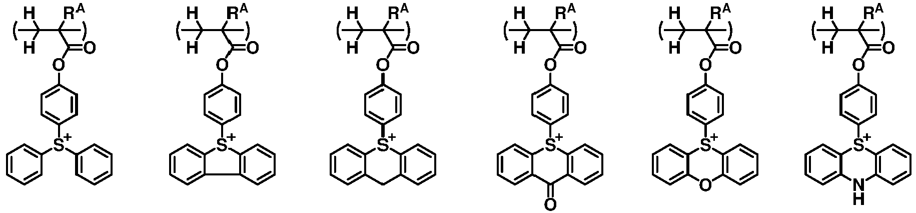

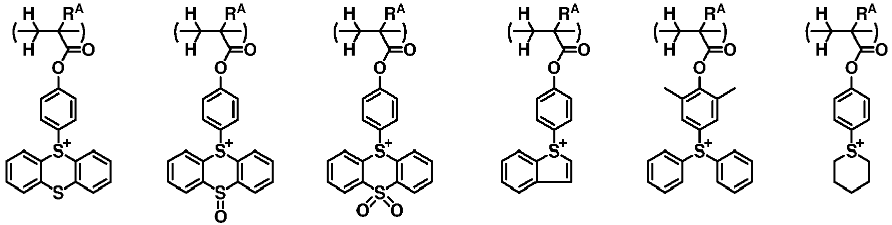

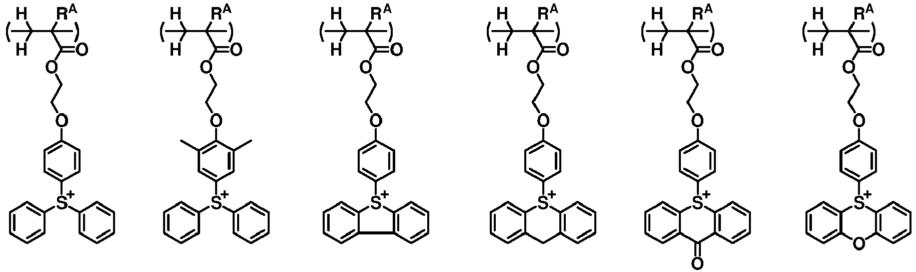

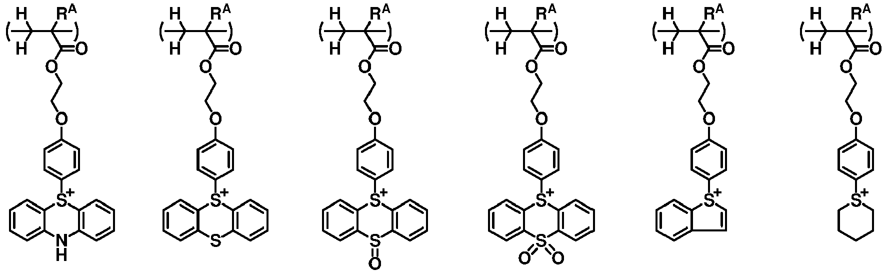

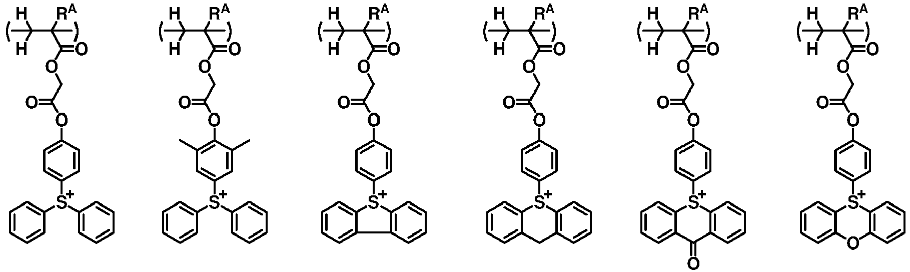

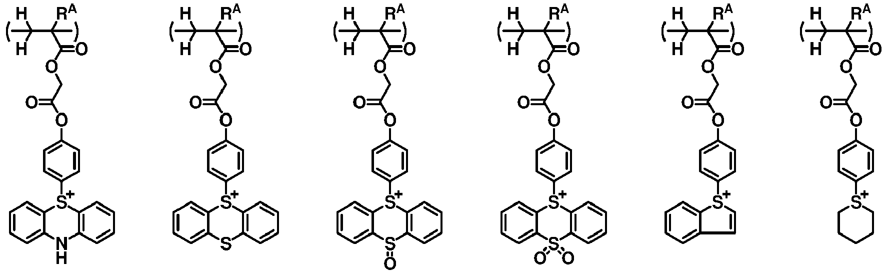

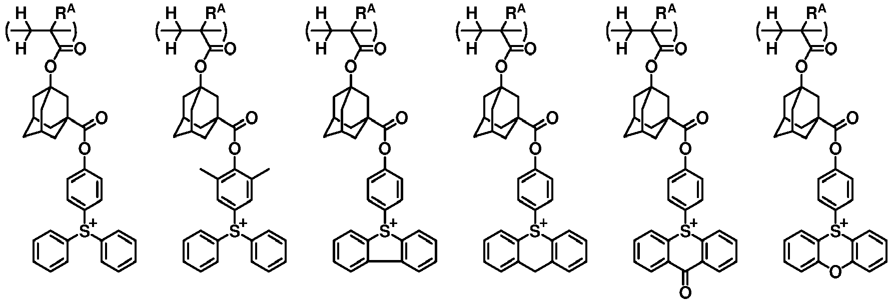

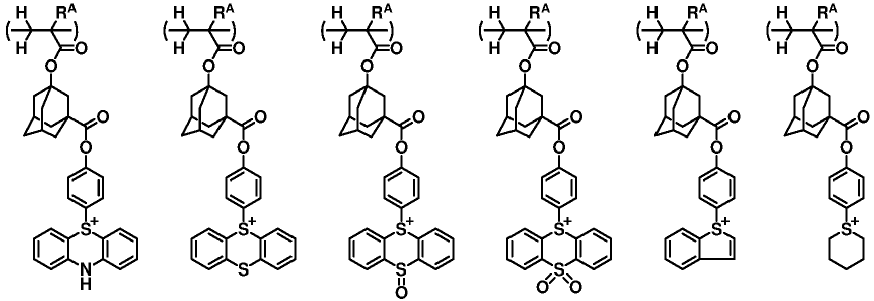

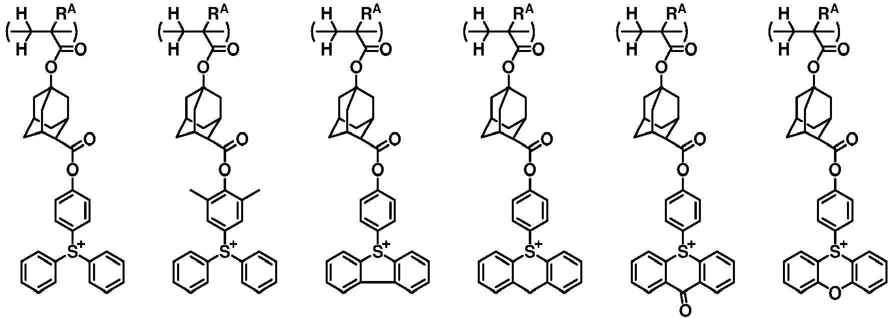

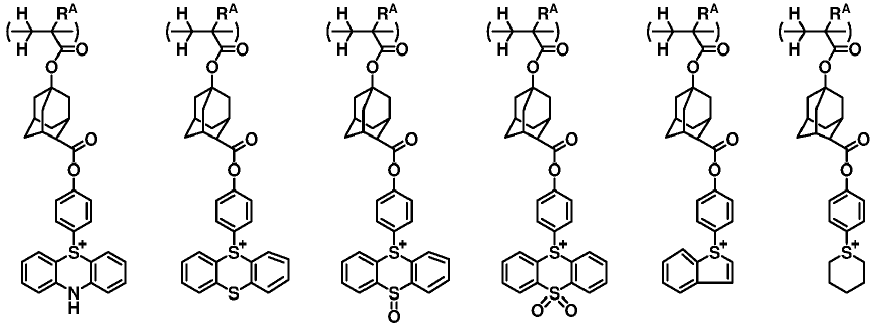

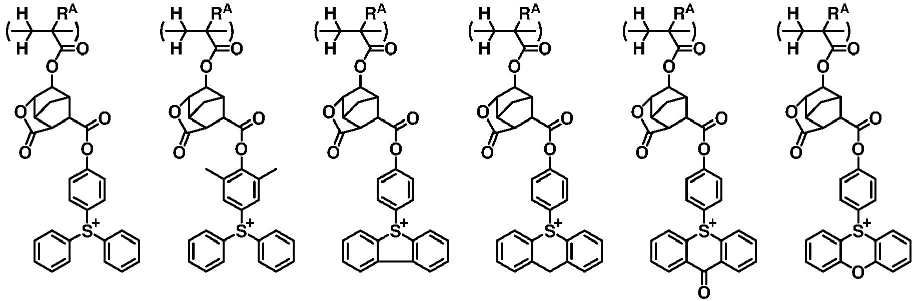

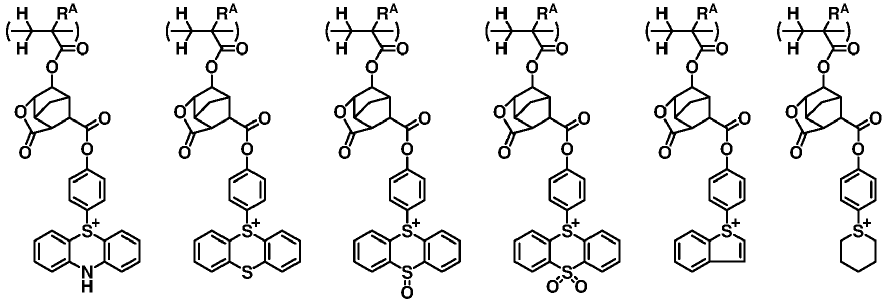

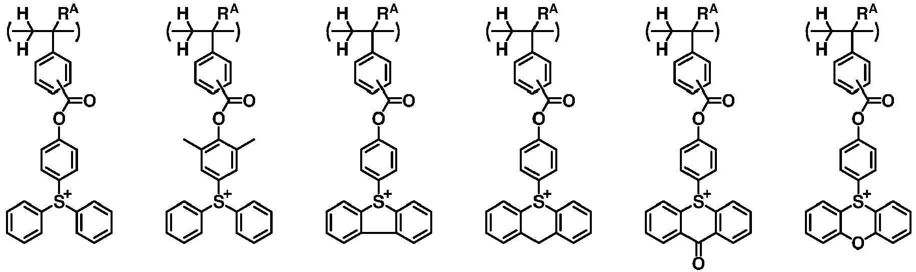

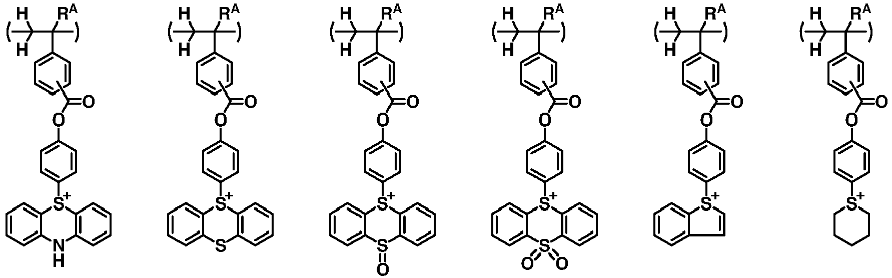

In formula (C1), M⁻ is a non-nucleophilic counter ion. Halide, sulfonate, imide and methide anions are preferred. Examples of the non-nucleophilic counter ion include halide ions such as chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylsulfonyl)imide and bis(perfluorobutylsulfonyl)imide; and methide ions such as tris(trifluoromethylsulfonyl)methide and tris(perfluoroethylsulfonyl)methide.

Anions having the following formulae (C1-1) to (C1-4) are also useful as the non-nucleophilic counter ion.

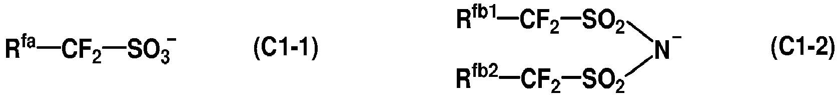

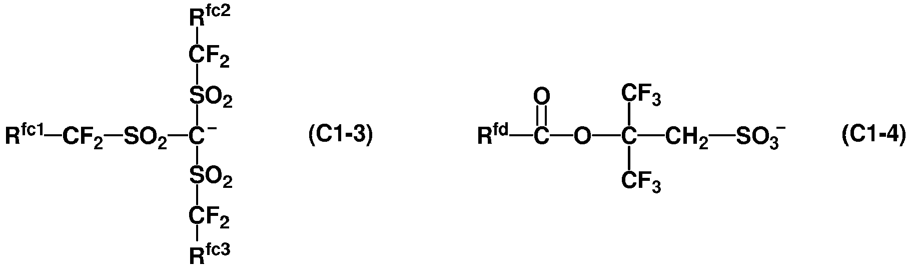

In formula (C1-1), R^{fa} is fluorine or a C₁-C₄₀ hydrocarbyl group which may contain a heteroatom. The hydrocarbyl group may be saturated or unsaturated and straight, branched or cyclic. Examples thereof are as will be exemplified below for the hydrocarbyl group R^{fa1} in formula (C1-1-1).

Of the anions of formula (C1-1), an anion having the formula (C1-1-1) is preferred.

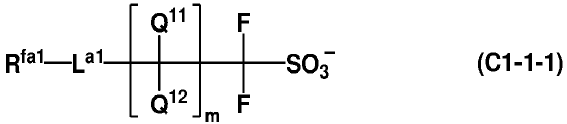

In formula (C1-1-1), Q¹¹ and Q¹² are each independently hydrogen, fluorine or a C₁-C₆ fluorinated saturated hydrocarbyl group. It is preferred for solvent solubility that at least one of Q¹¹ and Q¹² be trifluoromethyl. The subscript m is an integer of 0 to 4, preferably 1.

R^{fa1} is a C₁-C₃₅ hydrocarbyl group which may contain a heteroatom. As the heteroatom, oxygen, nitrogen, sulfur and halogen atoms are preferred, with oxygen being most preferred. Of the hydrocarbyl groups, those groups of 6 to 30 carbon atoms are preferred from the aspect of achieving a high resolution in forming patterns of small feature size. The hydrocarbyl group may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl, and icosyl; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclododecyl, tetracyclododecylmethyl, and dicyclohexylmethyl; C₂-C₃₅ unsaturated aliphatic hydrocarbyl groups such as allyl and 3-cyclohexenyl; C₆-C₃₅ aryl groups such as phenyl, 1-naphthyl, 2-naphthyl and 9-fluorenyl; and C₇-C₃₅ aralkyl groups such as benzyl and diphenylmethyl, and combinations thereof.

In the foregoing hydrocarbyl groups, some or all hydrogen may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl.

In formula (C1-1-1), L^{a1} is a single bond, ether bond, ester bond, sulfonate ester bond, carbonate bond or carbamate bond. From the aspect of synthesis, an ether bond or ester bond is preferred, with the ester bond being more preferred.

Examples of the anion having formula (C1-1) are shown below, but not limited thereto. Herein Q¹¹ is as defined above.

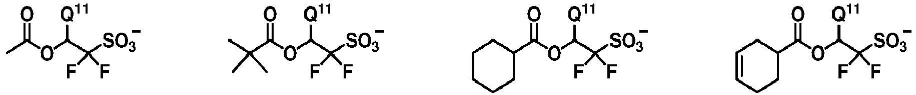

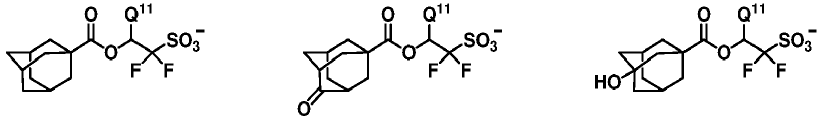

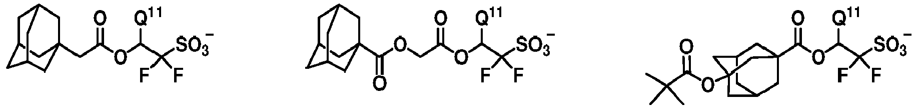

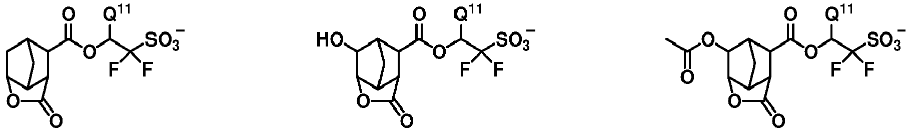

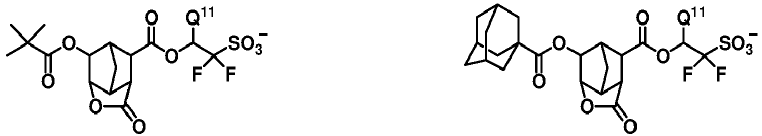

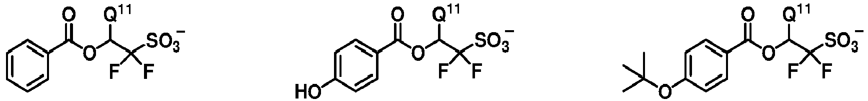

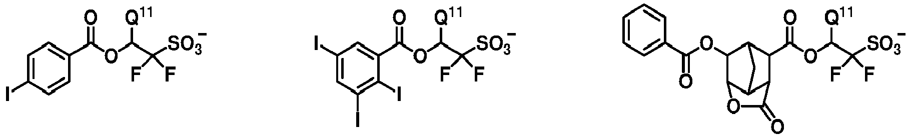

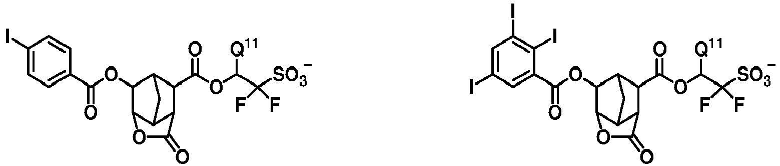

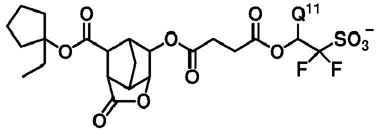

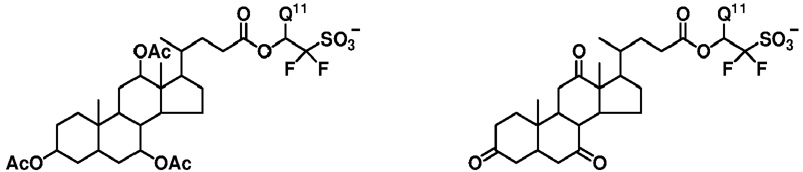

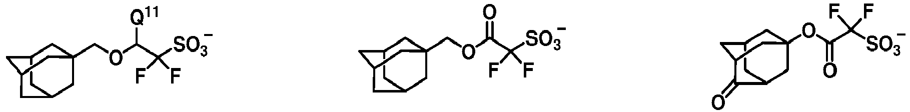

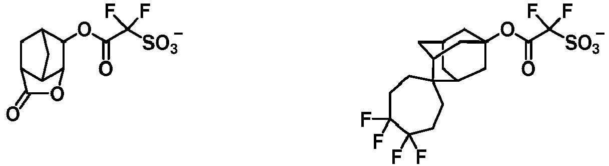

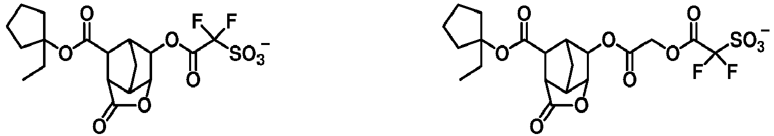

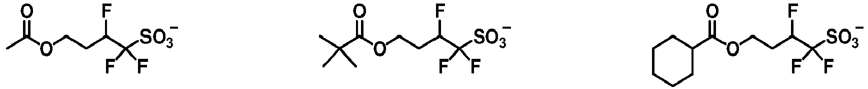

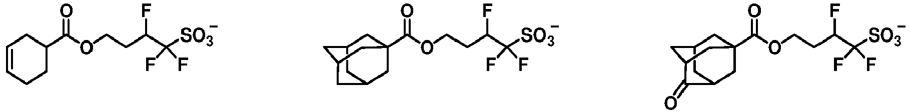

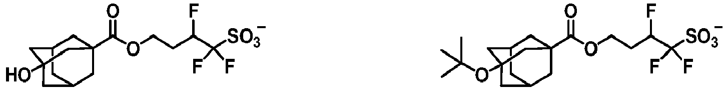

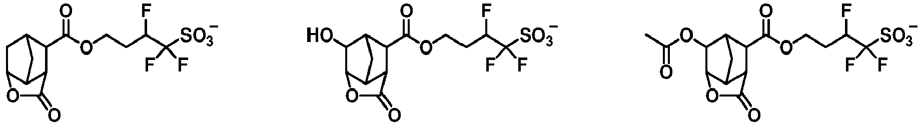

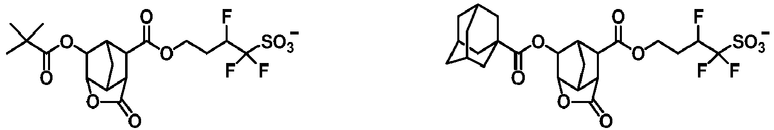

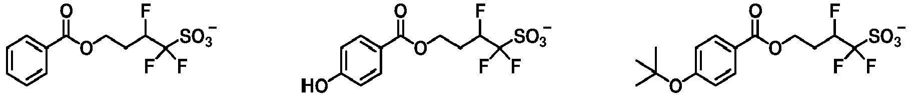

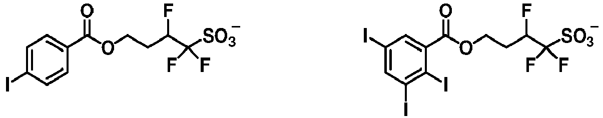

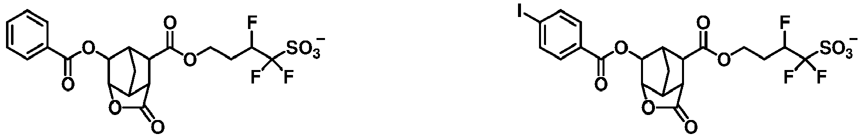

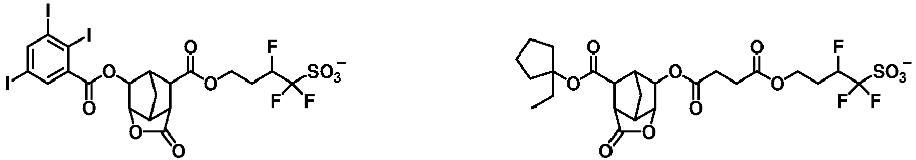

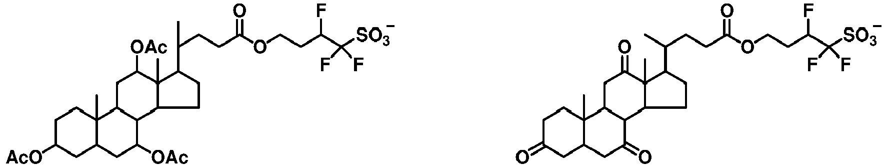

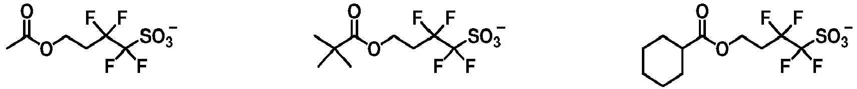

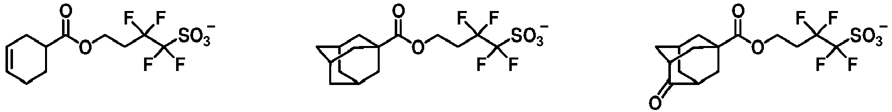

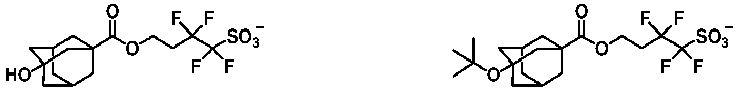

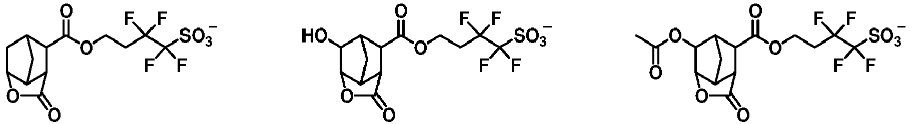

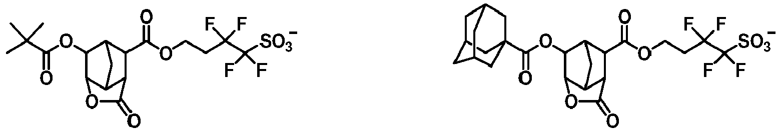

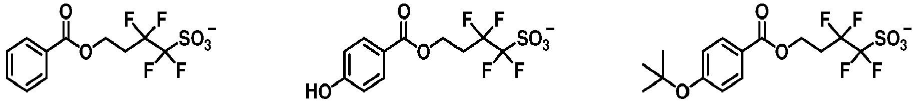

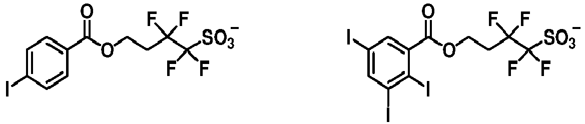

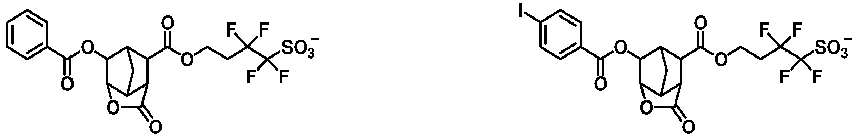

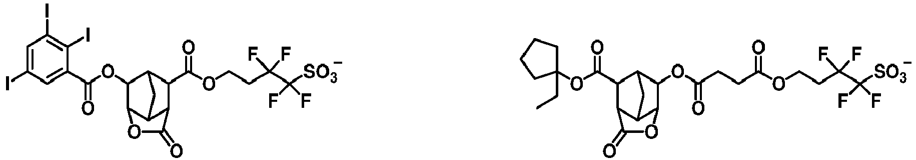

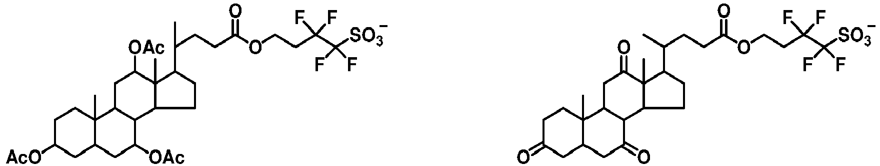

In formula (C1-2), R^{fb1} and R^{fb2} are each independently fluorine or a C₁-C₄₀ hydrocarbyl group which may contain a heteroatom. The hydrocarbyl group may be saturated or unsaturated and straight, branched or cyclic. Examples thereof are as exemplified above for R^{fa1} in formula (C1-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (C1-3), R^{fc1}, R^{fc2} and R^{fc3} are each independently fluorine or a C₁-C₄₀ hydrocarbyl group which may contain a heteroatom. The hydrocarbyl group may be saturated or unsaturated and straight, branched or cyclic. Examples thereof are as exemplified for R^{fa1} in formula (C1-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C⁻-SO₂-CF₂- to which they are attached. It is preferred that a combination of R^{fc1} and R^{fc2} be a fluorinated ethylene or fluorinated propylene group.

In formula (C1-4), R^{fd} is a C₁-C₄₀ hydrocarbyl group which may contain a heteroatom. The hydrocarbyl group may be saturated or unsaturated and straight, branched or cyclic. Examples thereof are as exemplified above for R^{fa1}.

Examples of the anion having formula (C1-4) are shown below, but not limited thereto.

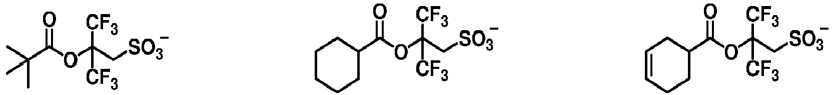

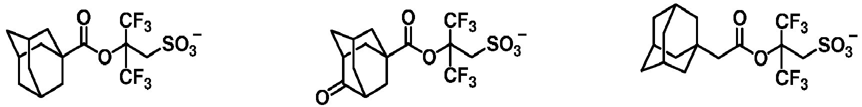

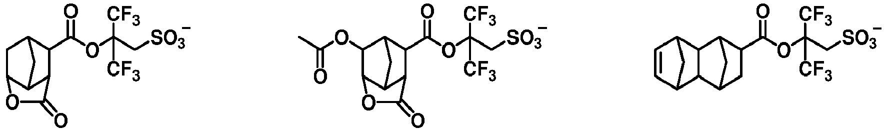

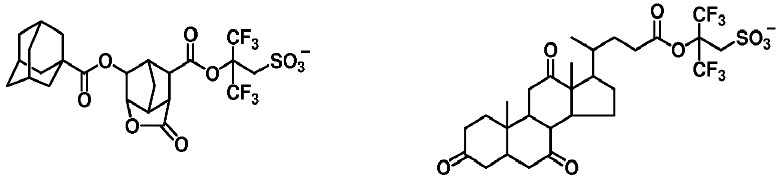

Anions having an iodized or brominated aromatic ring are also useful as the non-nucleophilic counter ion. These anions have the formula (C1-5).

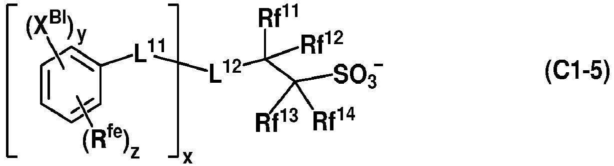

In formula (C1-5), x is an integer of 1 to 3, y is an integer of 1 to 5, z is an integer of 0 to 3, and y+z is from 1 to 5; preferably, y is an integer of 1 to 3, more preferably 2 or 3, and z is an integer of 0 to 2.

X^{BI} is iodine or bromine. A plurality of X^{BI} may be identical or different when x and/or y is 2 or more.

L¹¹ is a single bond, ether bond, ester bond, or a C₁-C₆ saturated hydrocarbylene group which may contain an ether bond or ester bond. The saturated hydrocarbylene group may be straight, branched or cyclic.

L¹² is a single bond or a C₁-C₂₀ divalent linking group when x=1, or a C₁-C₂₀ (x+1)-valent linking group when x=2 or 3. The linking group may contain an oxygen, sulfur or nitrogen atom.

R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. R^{feD} is a C₁-C₁₆ aliphatic hydrocarbyl group, C₆-C₁₂ aryl group or C₇-C₁₅ aralkyl group, which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy, and hydrocarbylsulfonyloxy groups may be straight, branched or cyclic. A plurality of R^{fe} may be identical or different when x and/or z is 2 or more.

Of these, R^{fe} is preferably hydroxy, -N(R^{feC})-C(=O)-R^{feD}, -N(R^{feC})-C(=O)-O-R^{feD}, fluorine, chlorine, bromine, methyl or methoxy.

Rf¹¹ to Rf¹⁴ are each independently hydrogen, fluorine or trifluoromethyl, at least one of Rf¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (C1-5) are shown below, but not limited thereto. X^{BI} is as defined above.

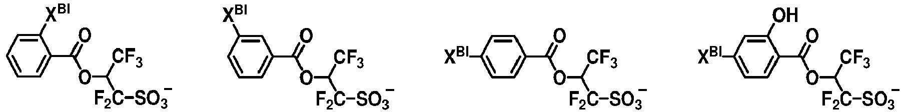

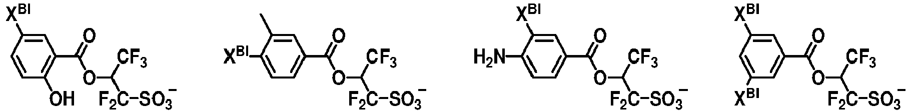

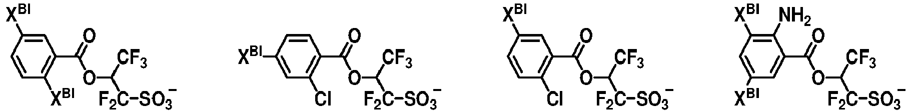

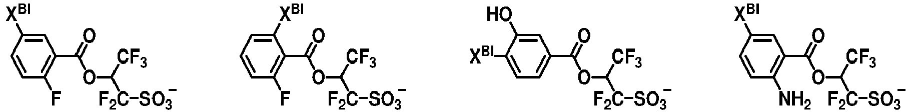

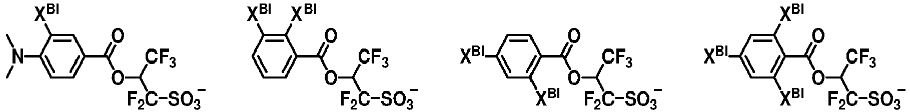

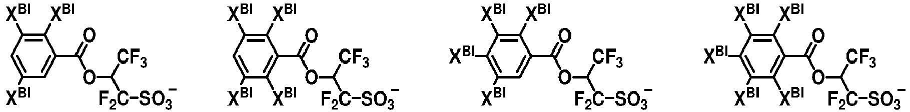

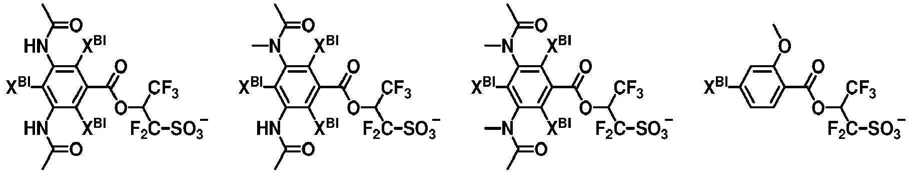

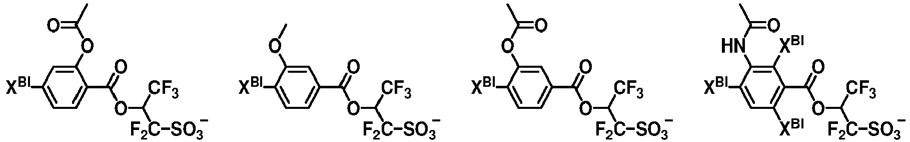

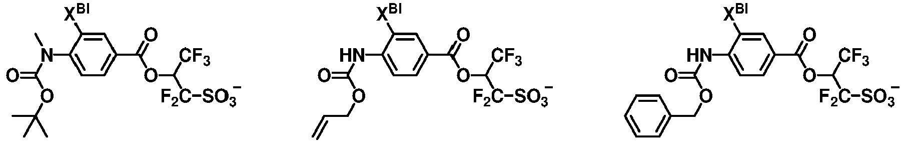

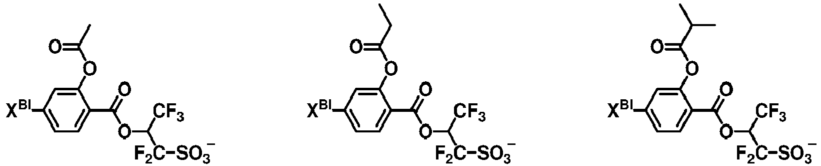

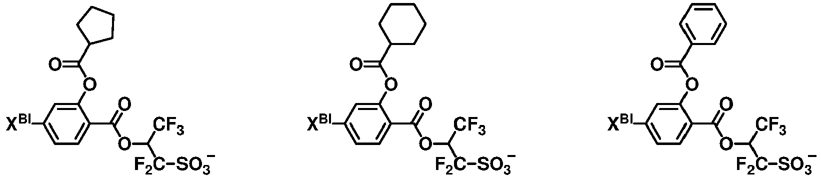

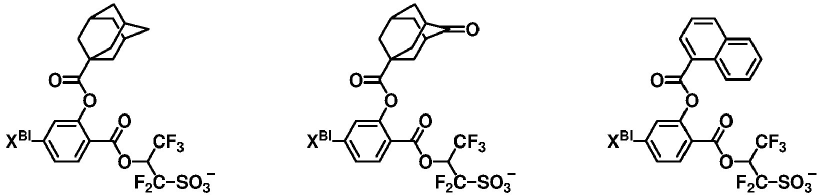

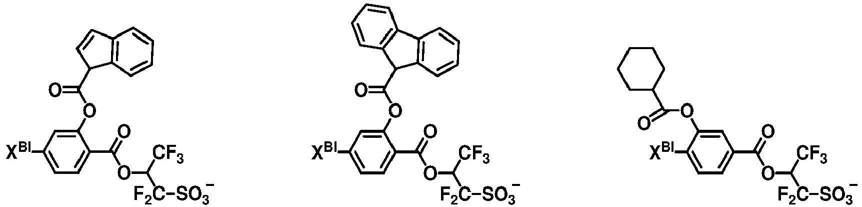

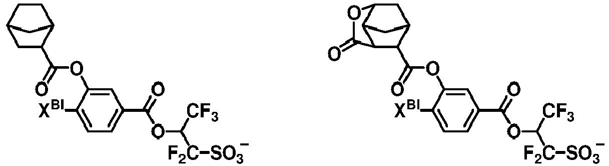

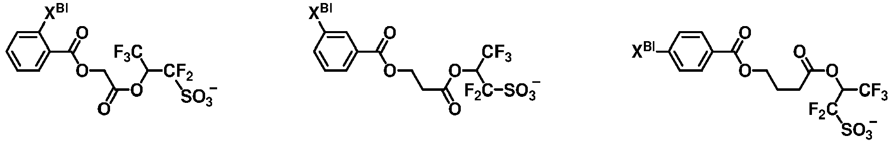

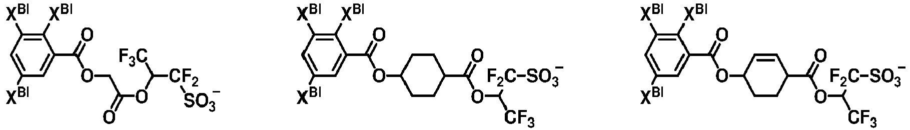

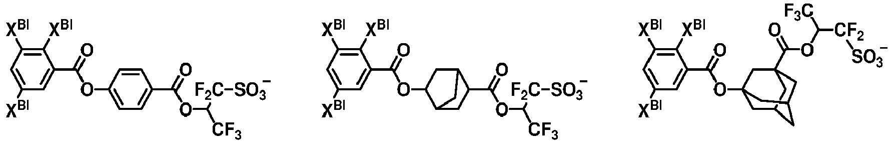

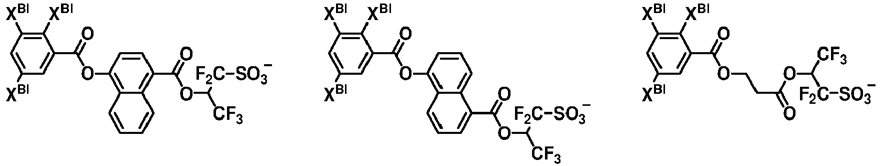

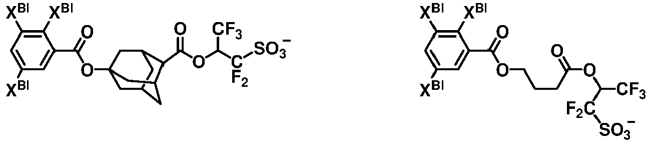

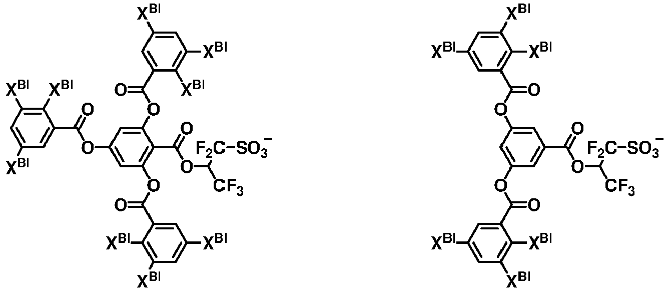

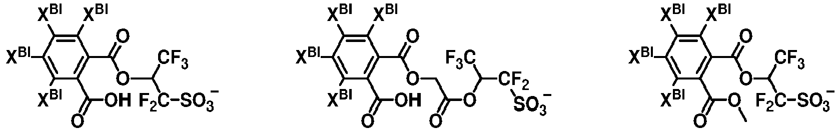

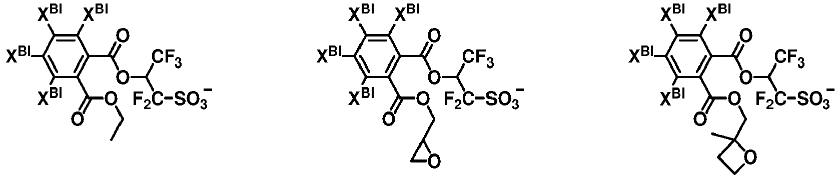

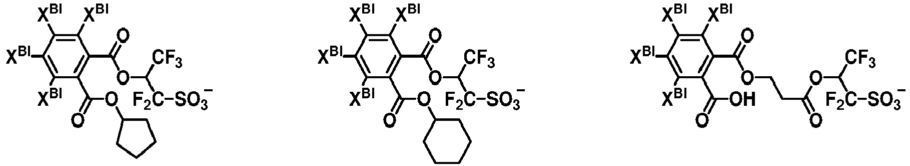

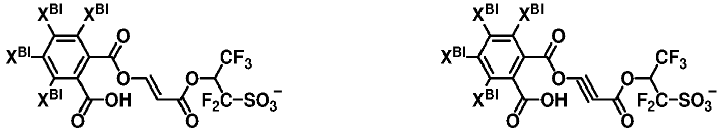

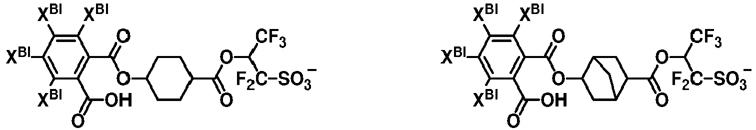

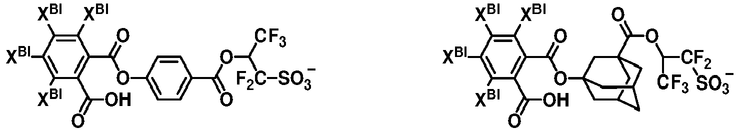

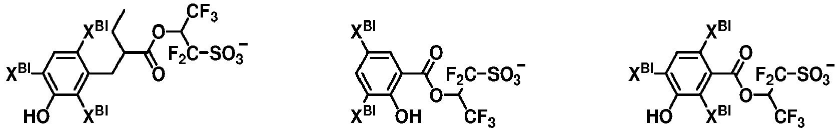

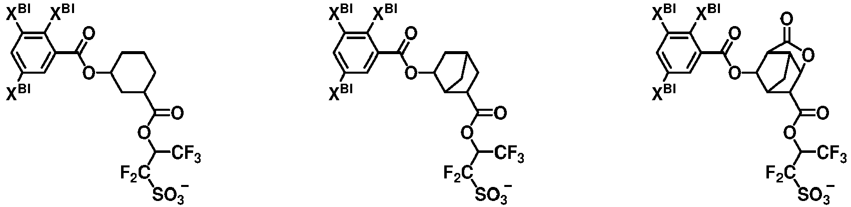

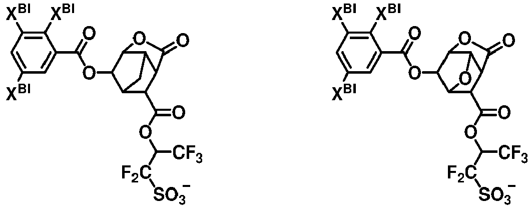

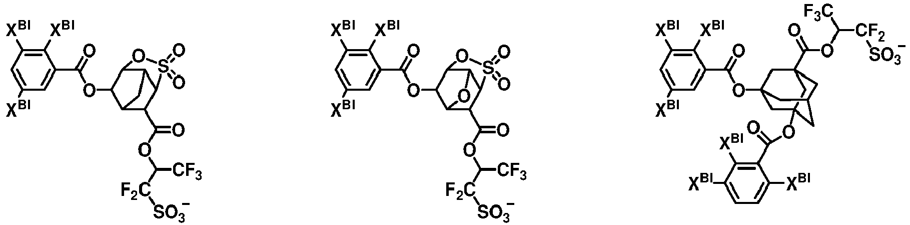

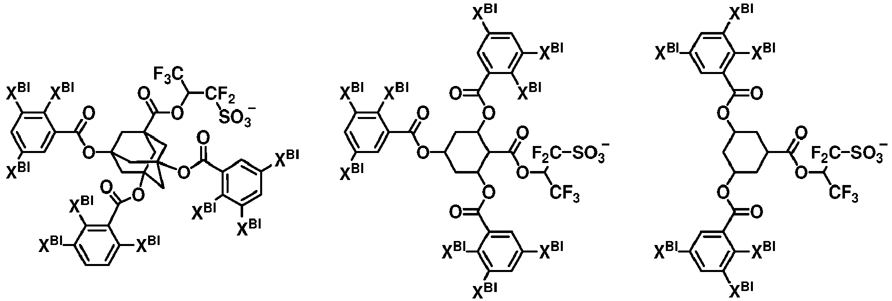

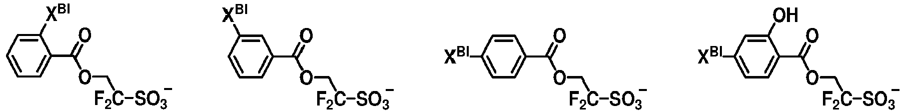

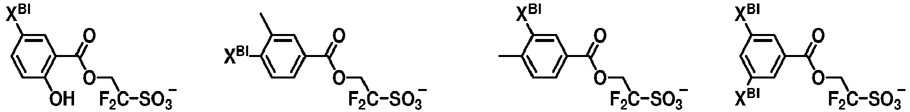

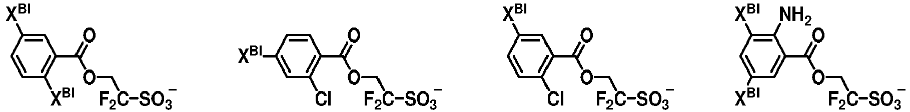

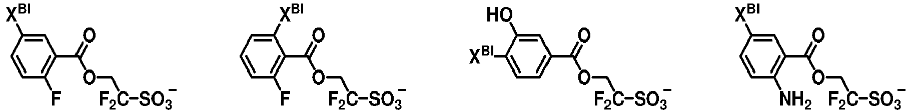

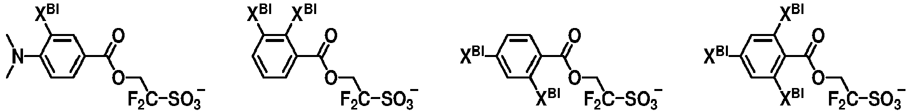

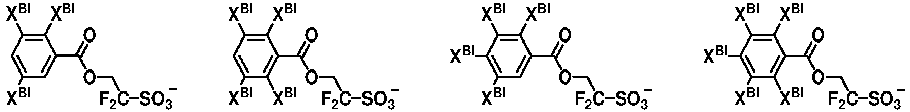

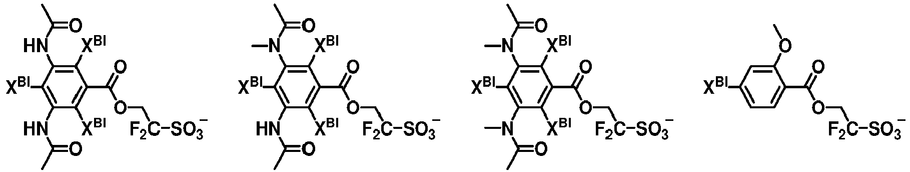

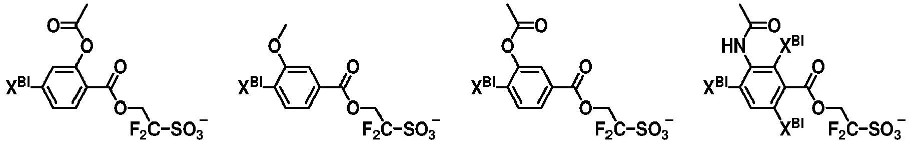

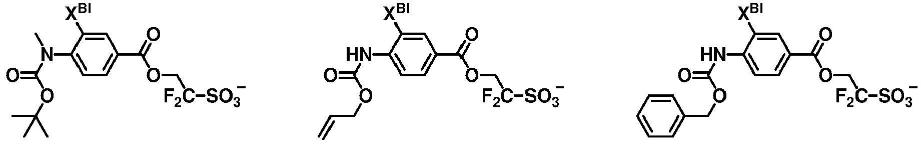

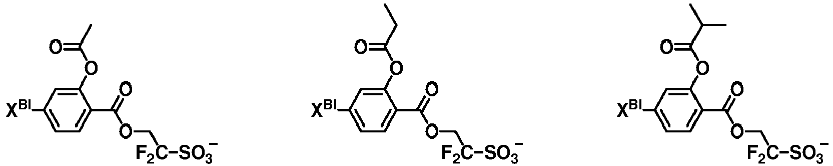

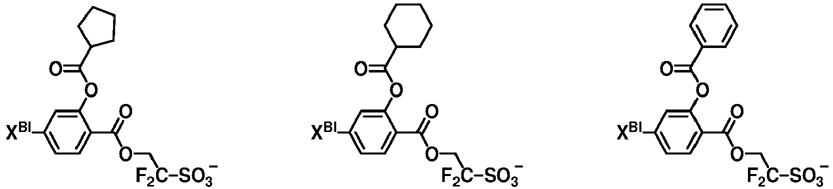

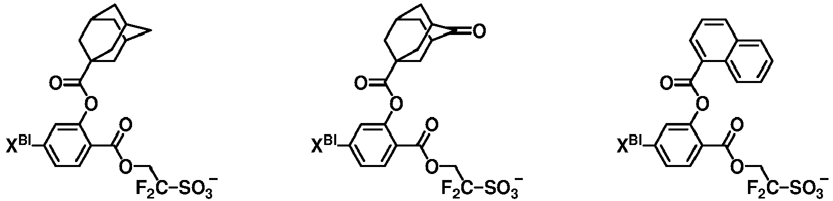

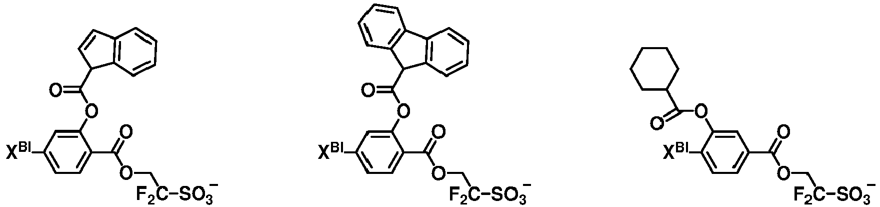

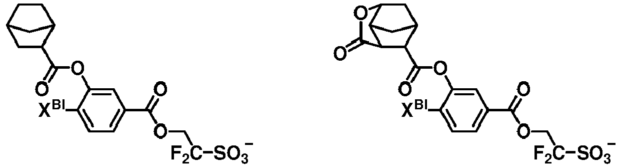

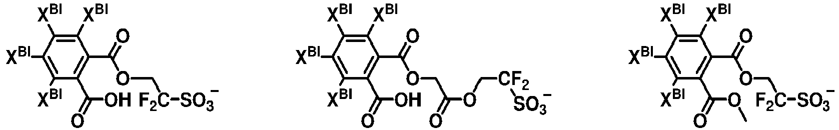

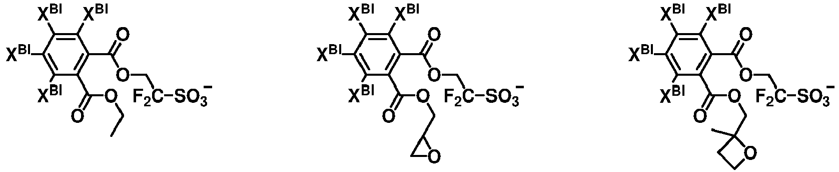

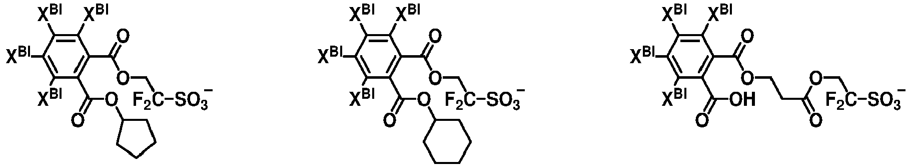

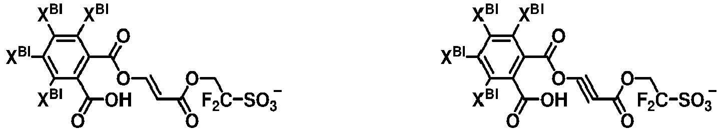

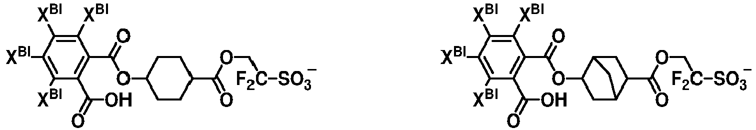

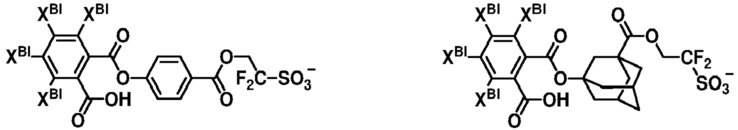

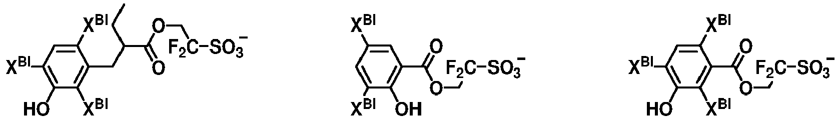

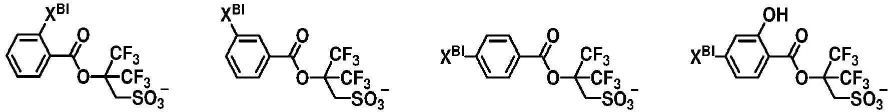

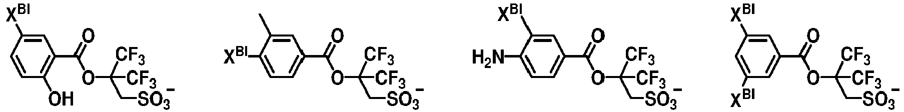

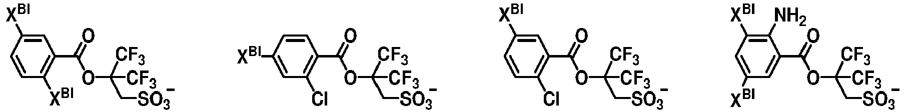

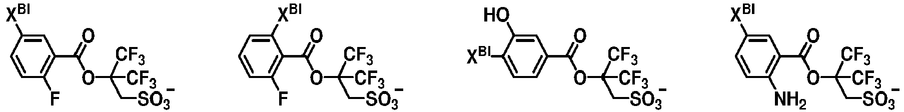

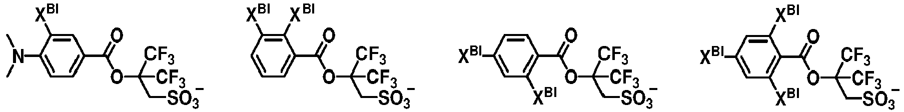

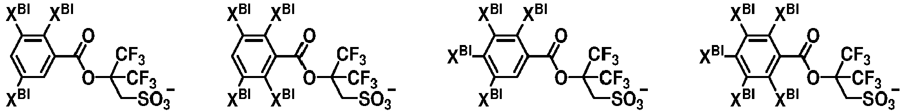

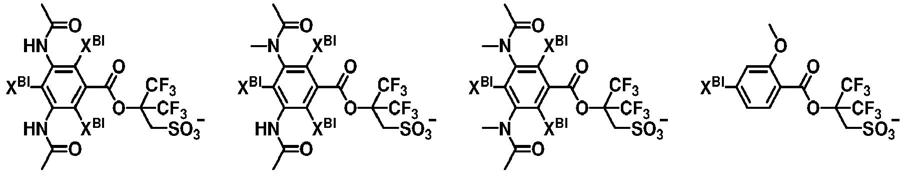

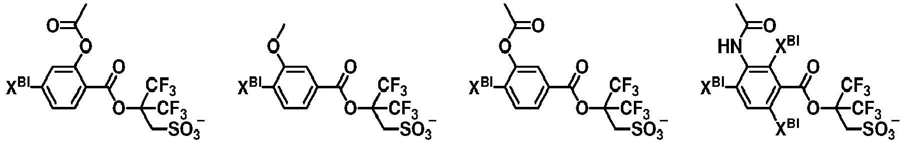

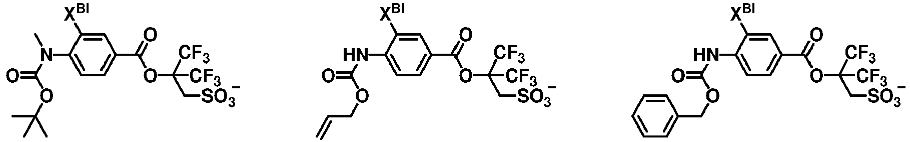

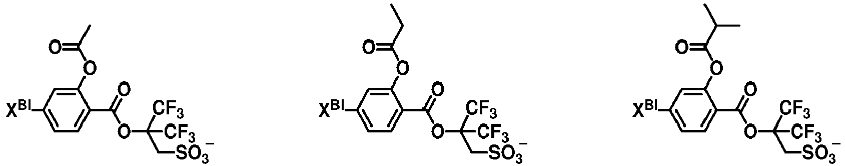

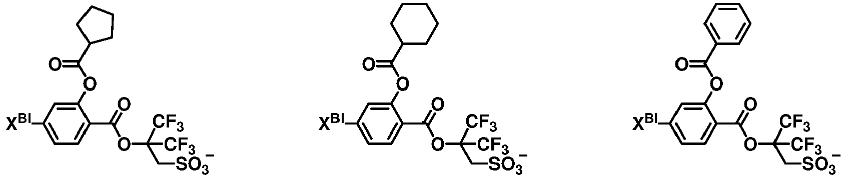

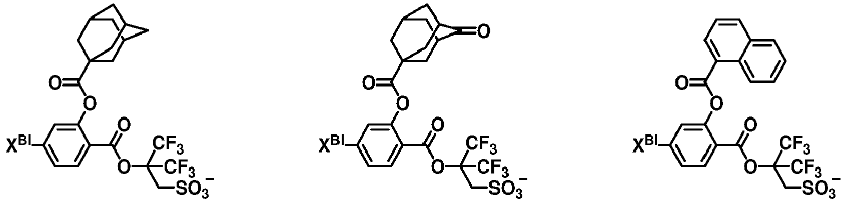

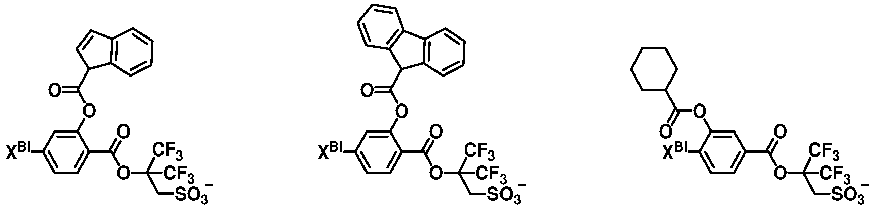

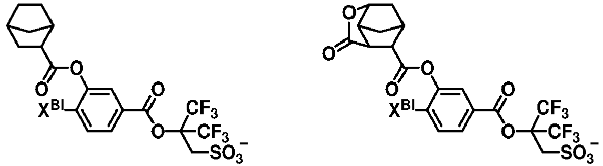

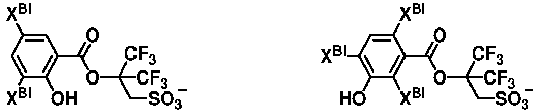

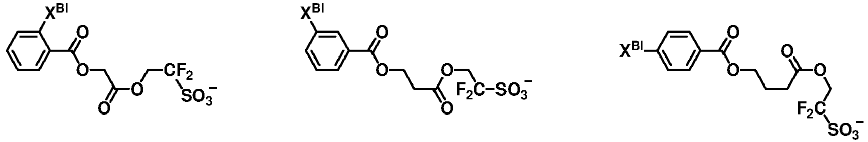

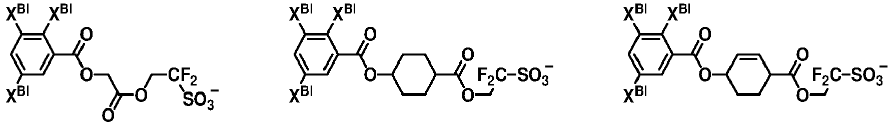

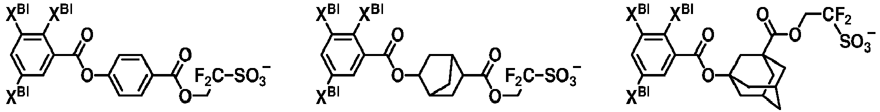

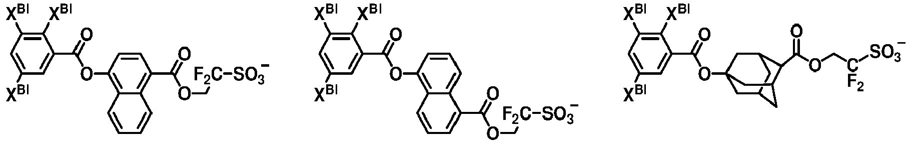

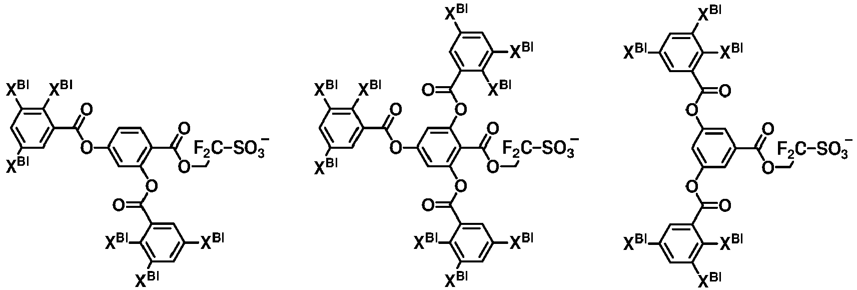

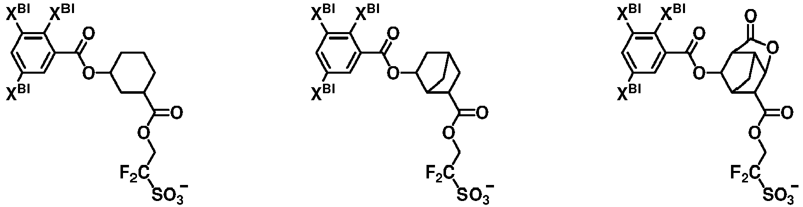

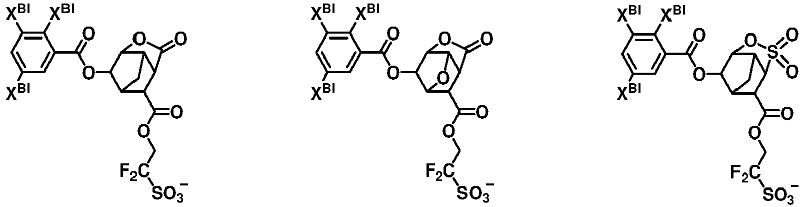

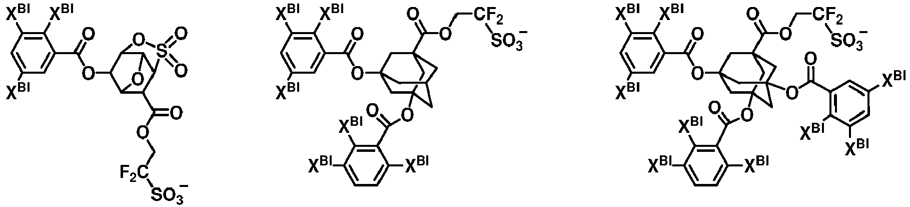

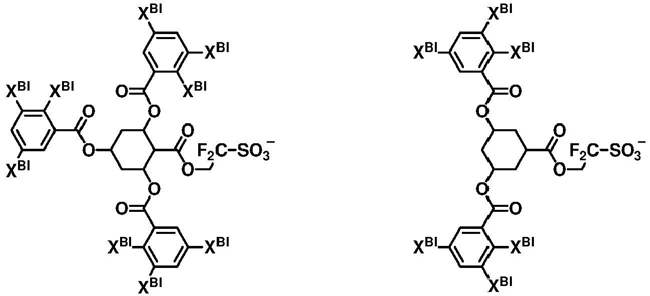

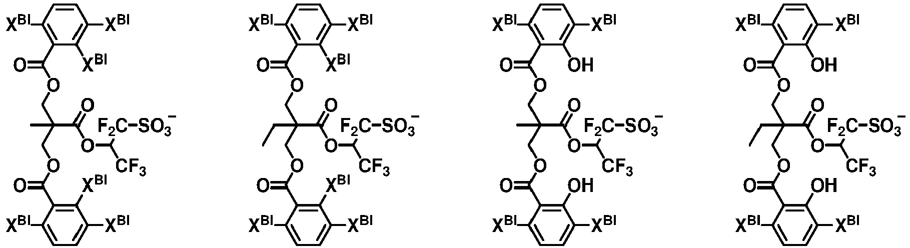

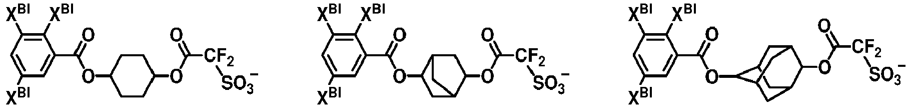

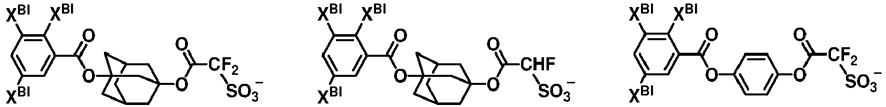

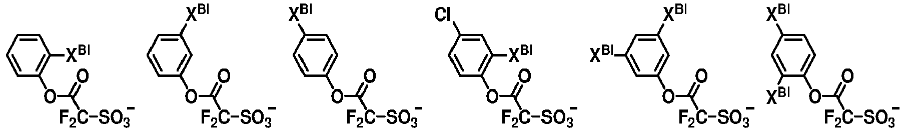

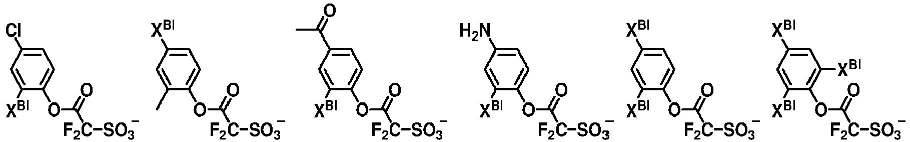

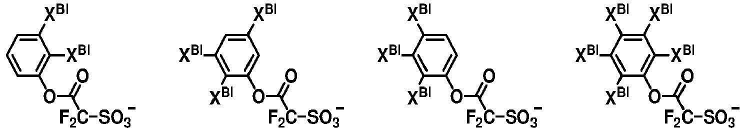

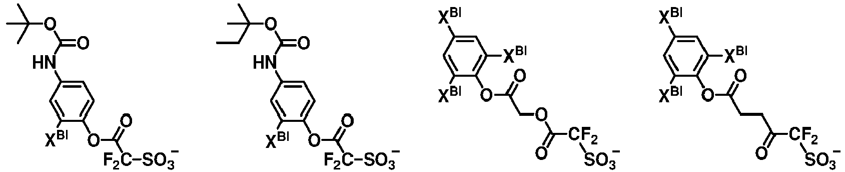

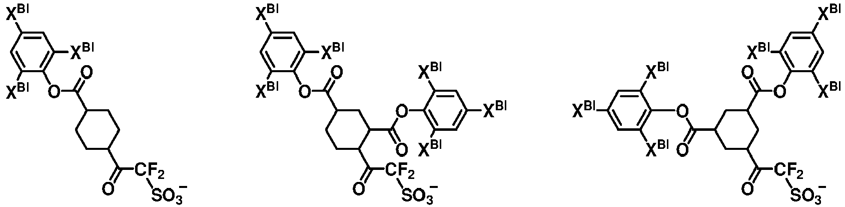

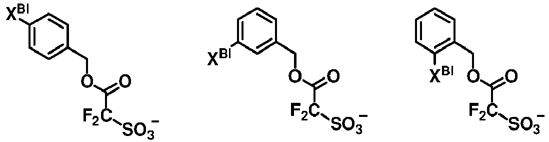

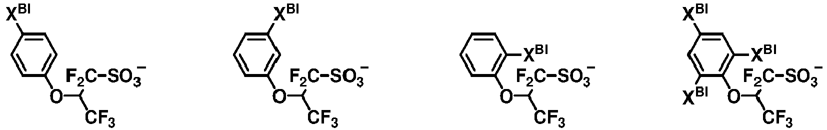

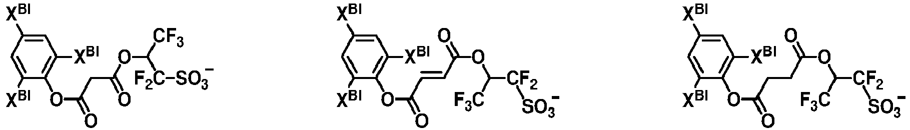

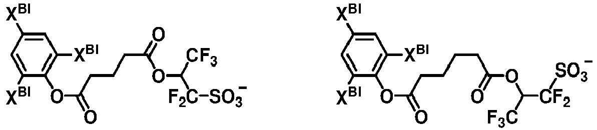

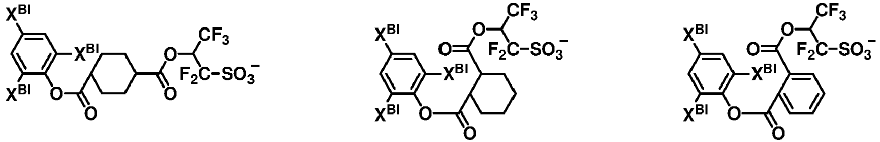

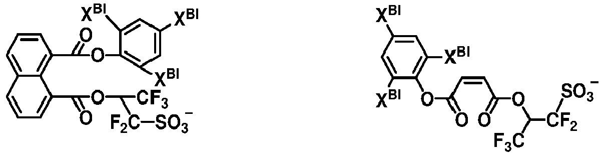

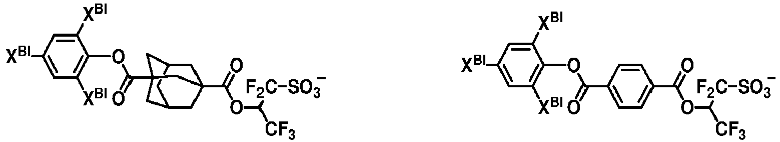

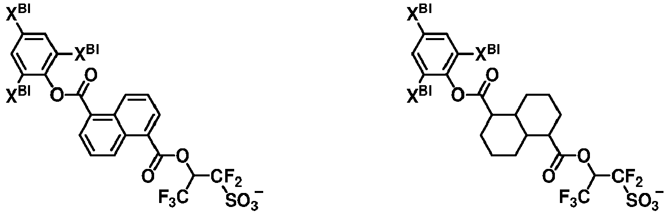

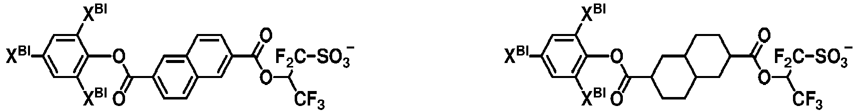

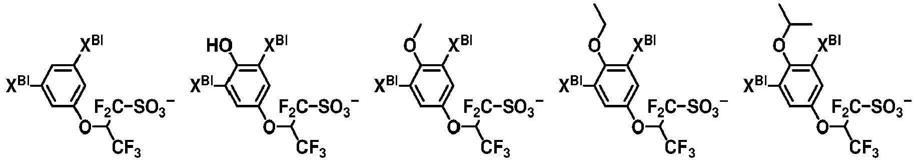

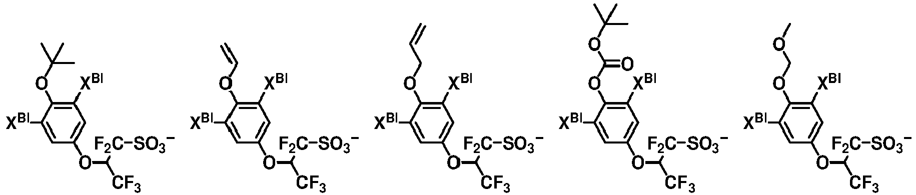

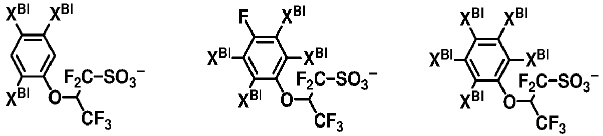

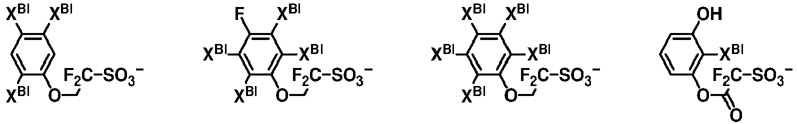

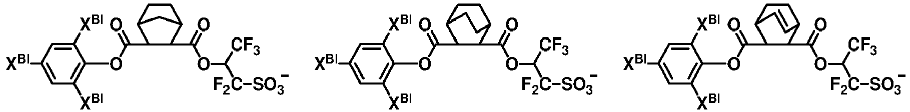

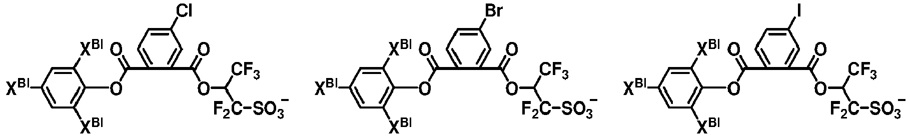

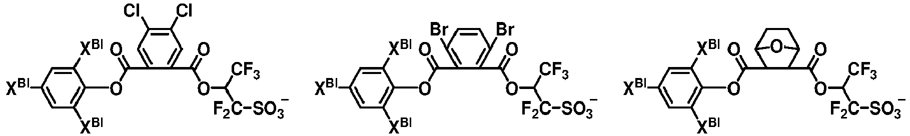

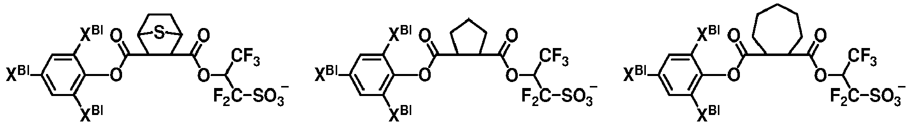

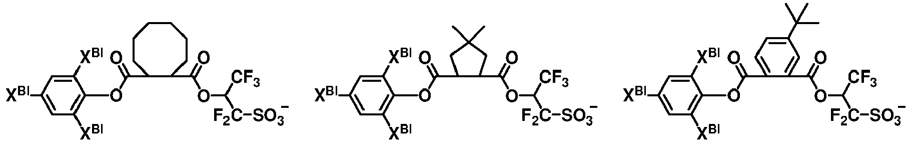

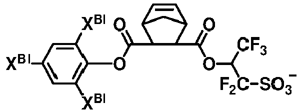

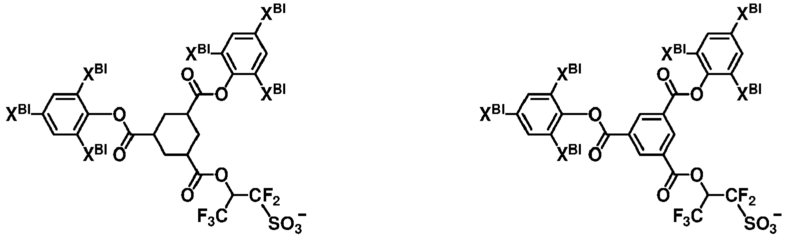

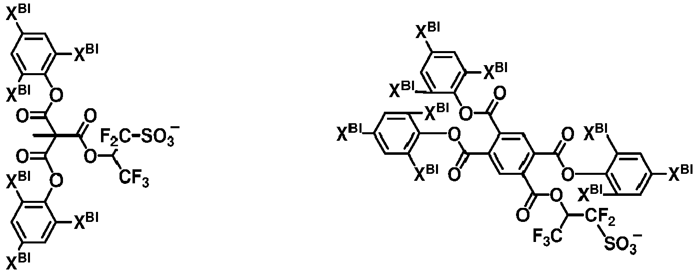

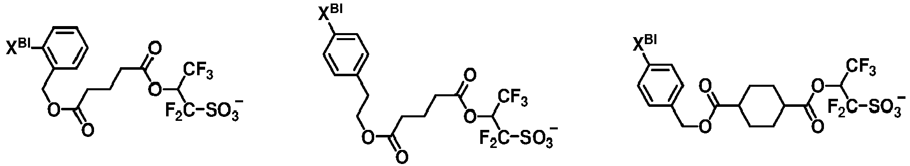

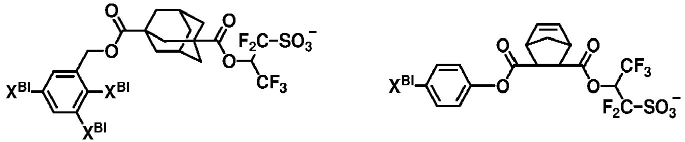

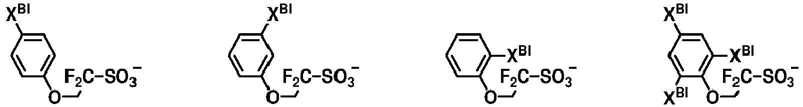

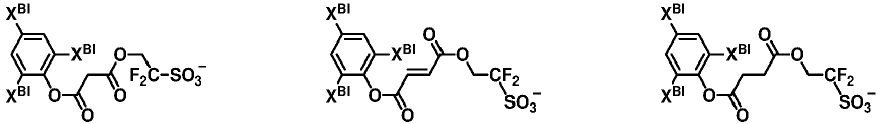

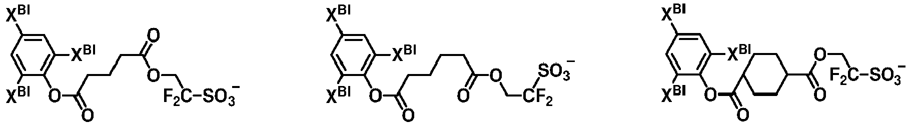

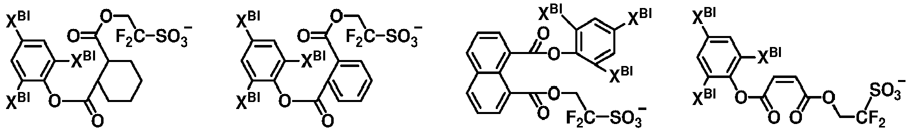

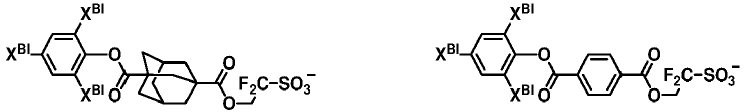

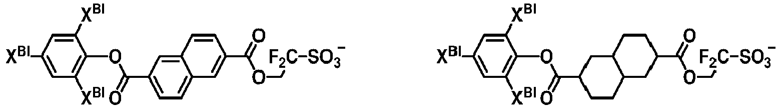

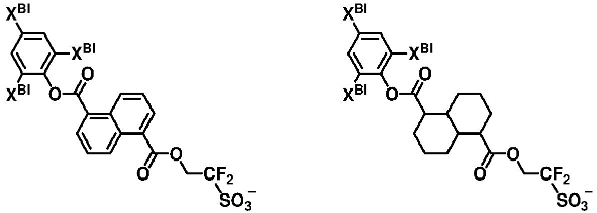

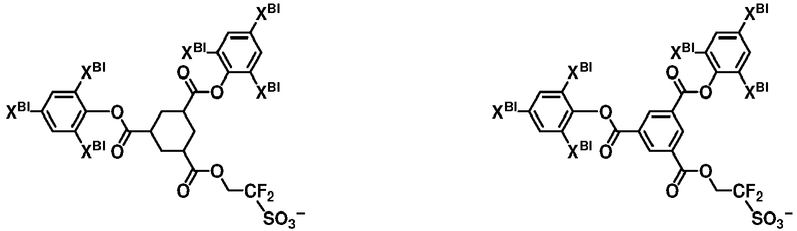

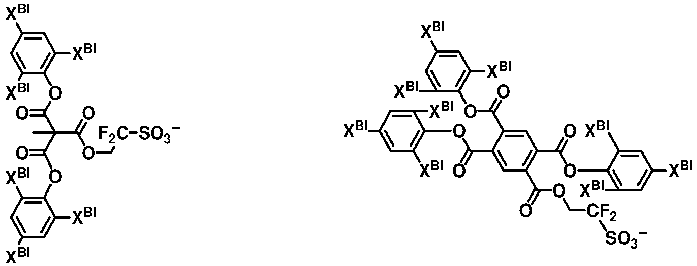

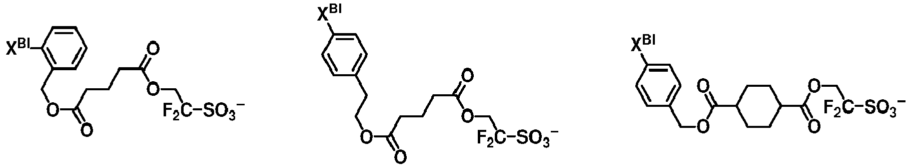

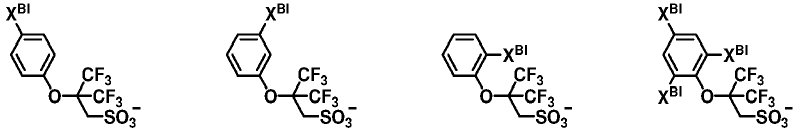

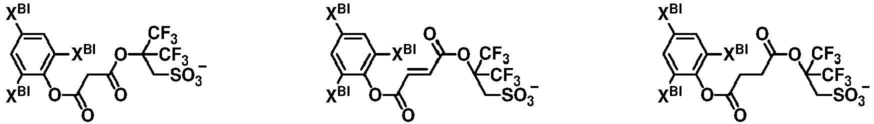

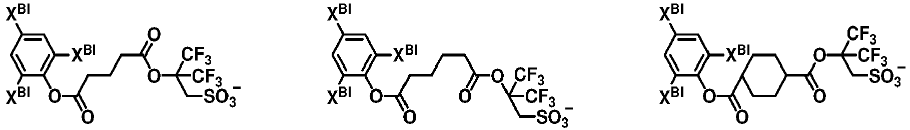

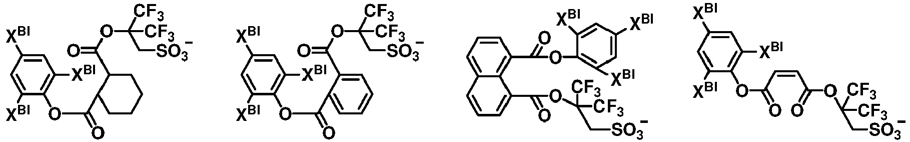

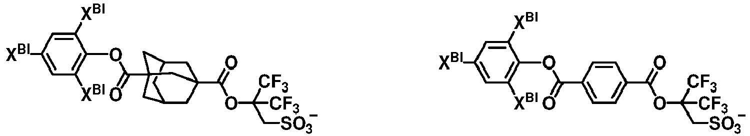

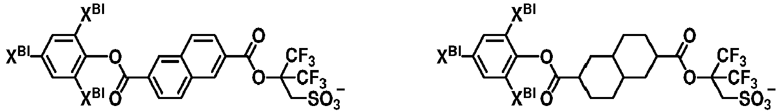

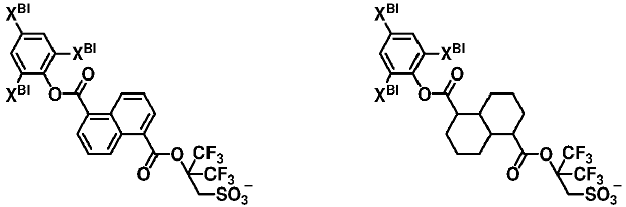

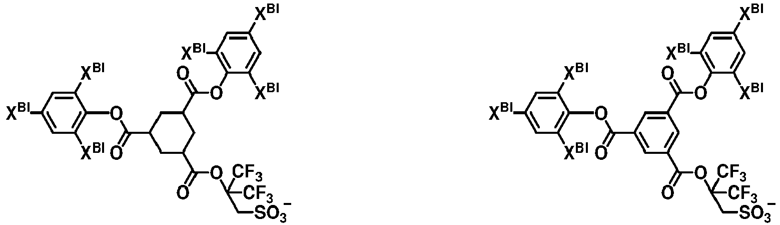

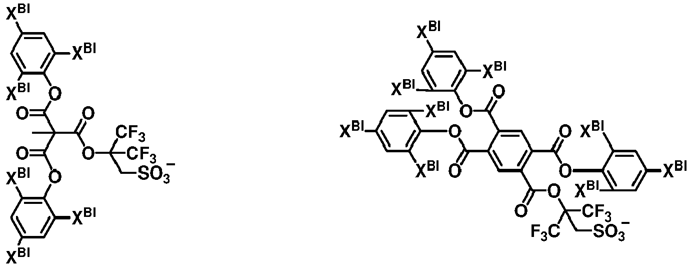

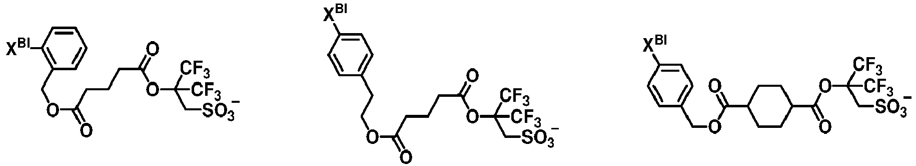

Other useful examples of the non-nucleophilic counter ion include fluorobenzenesulfonic acid anions having an iodized aromatic ring bonded thereto as described in JP 6648726, anions having an acid-catalyzed decomposition mechanism as described in WO 2021/200056 and JP-A 2021-070692, anions having a cyclic ether group as described in JP-A 2018-180525 and JP-A 2021-035935, and anions as described in JP-A 2018-092159.

Further useful examples of the non-nucleophilic counter ion include fluorine-free bulky benzenesulfonic acid anions as described in JP-A 2006-276759, JP-A 2015-117200, JP-A 2016-065016, and JP-A 2019-202974; fluorine-free benzenesulfonic acid or alkylsulfonic acid anions having an iodized aromatic group bonded thereto as described in JP 6645464.

Also useful are bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-024989.

In formulae (C2) and (C3), L is a single bond, ether bond, ester bond, carbonyl, sulfonate ester bond, carbonate bond or carbamate bond. From the aspect of synthesis, an ether bond, ester bond or carbonyl is preferred, with the ester bond or carbonyl being more preferred.

In formula (C2), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine or a C₁-C₆ fluorinated saturated hydrocarbyl group. It is preferred for solvent solubility that at least one of Rf³ and Rf⁴ be trifluoromethyl.

In formula (C3), Rf⁵ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for solvent solubility that at least one of Rf⁵ and Rf⁶ be trifluoromethyl.

In formulae (C2) and (C3), k1 and k2 are each independently an integer of 0 to 3, preferably 1.

Examples of the anion in repeat unit C2 are shown below, but not limited thereto. R^{A} is as defined above.

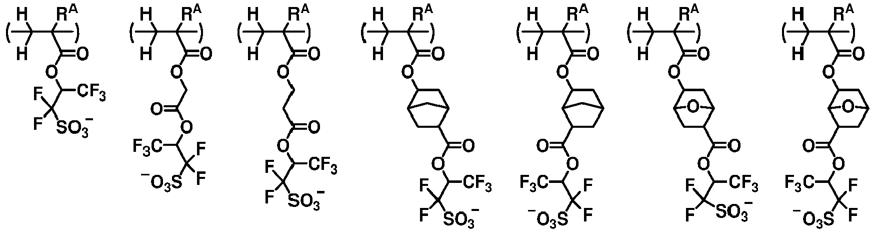

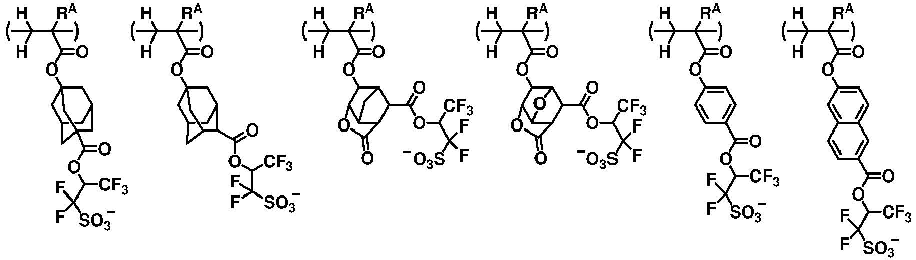

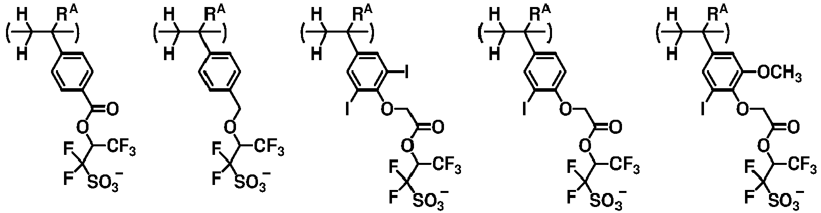

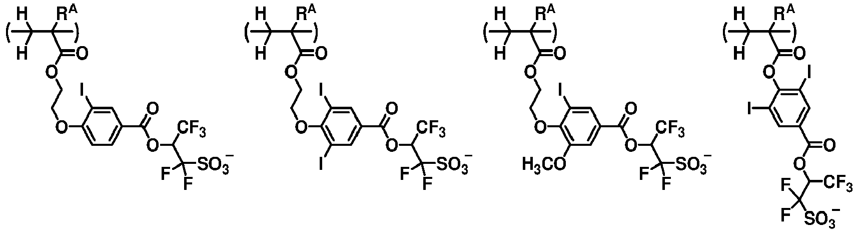

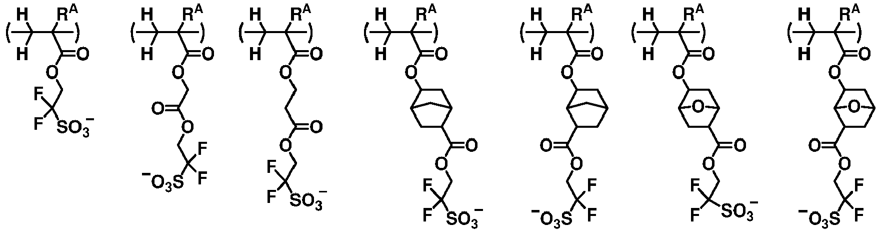

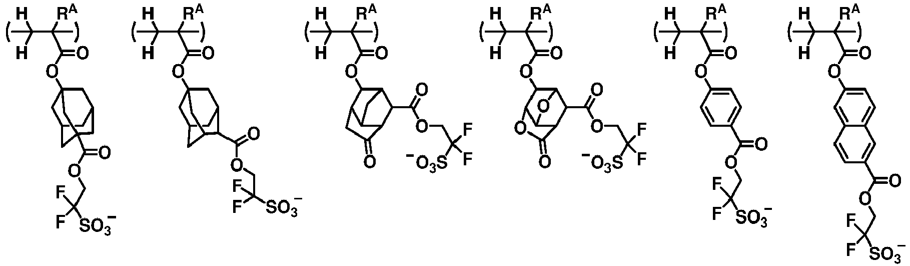

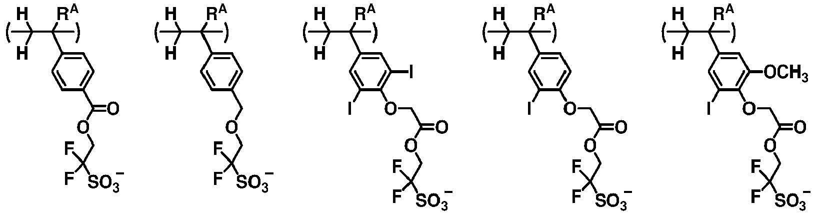

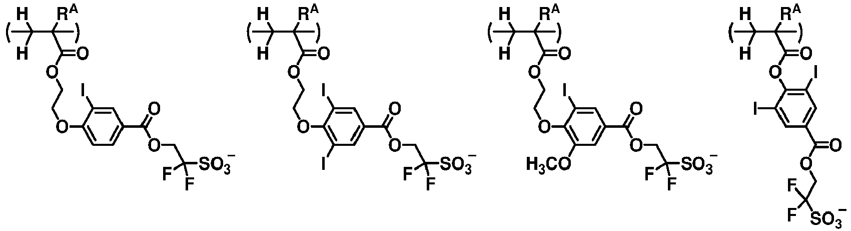

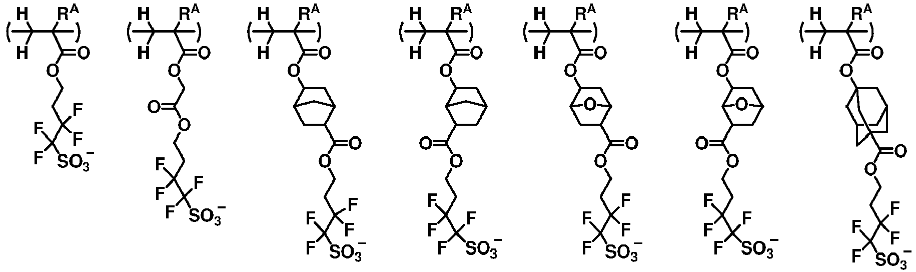

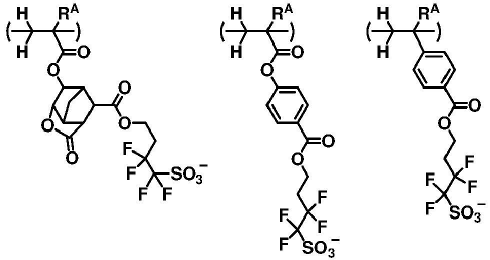

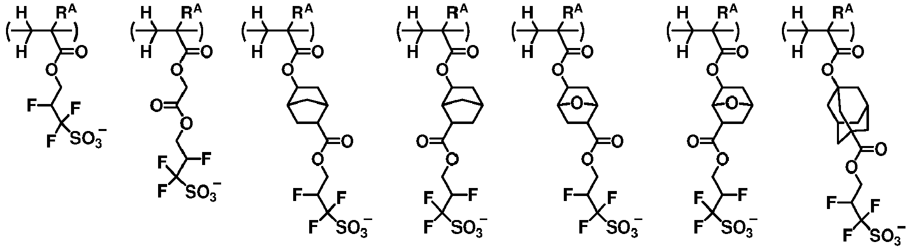

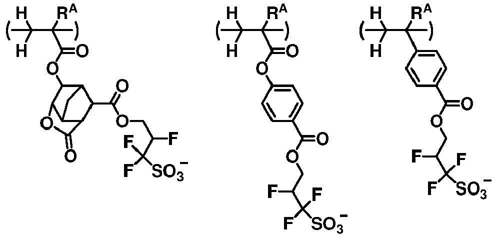

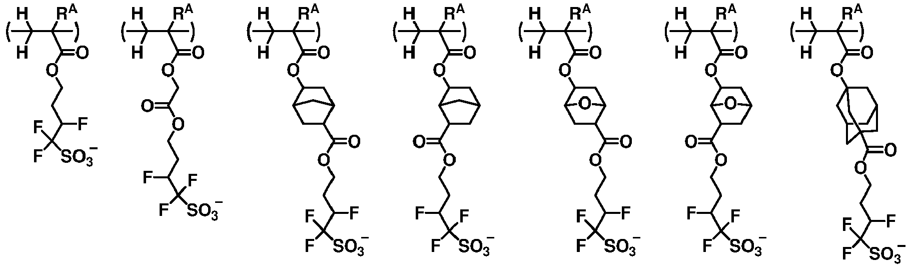

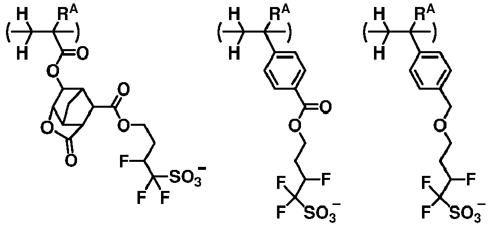

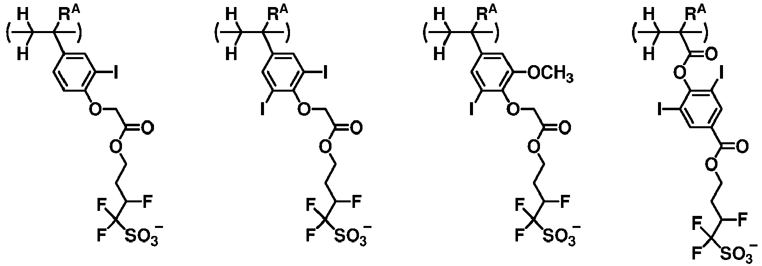

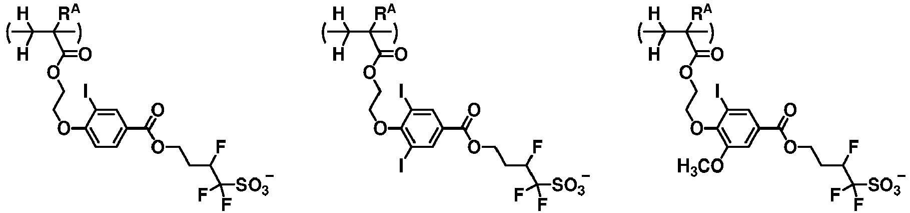

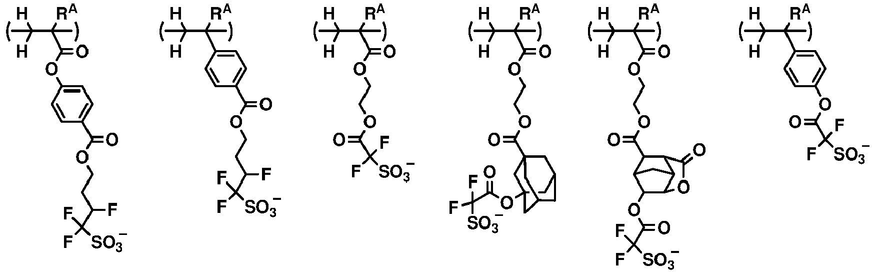

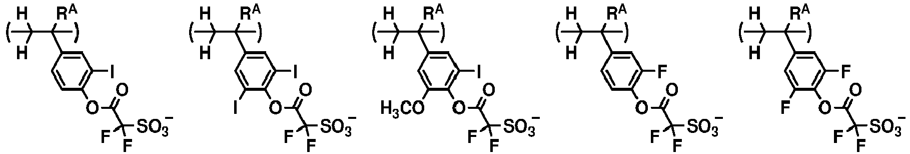

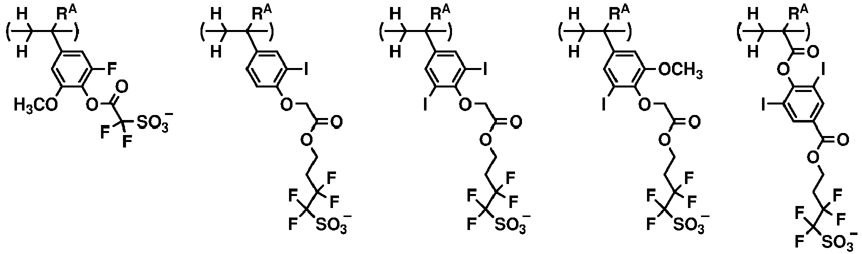

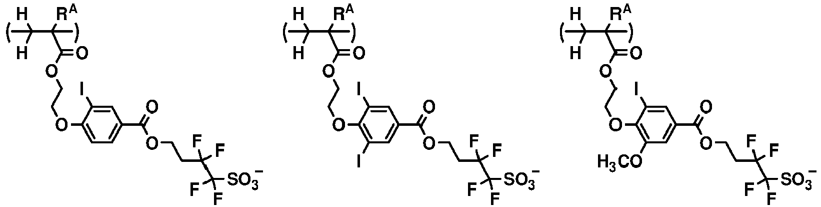

Examples of the anion in repeat unit C3 are shown below, but not limited thereto. R^{A} is as defined above.

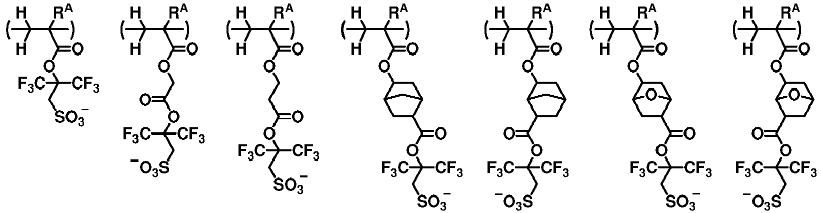

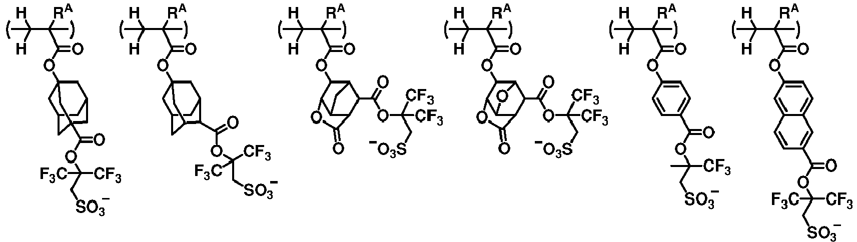

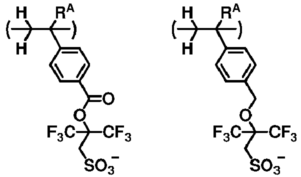

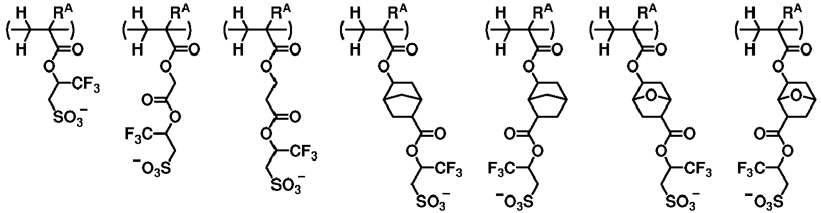

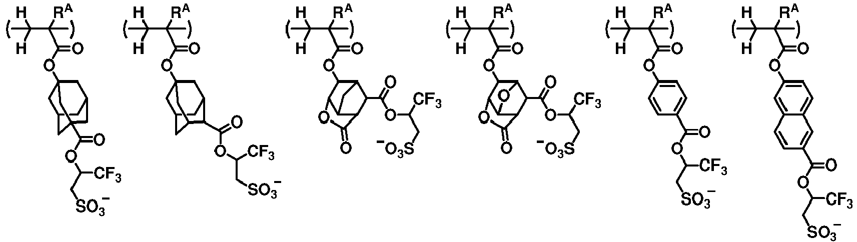

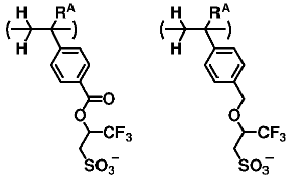

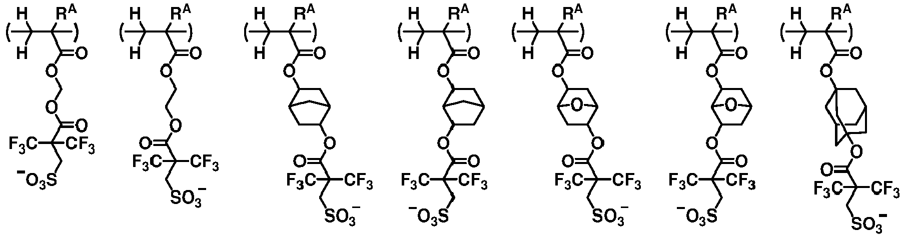

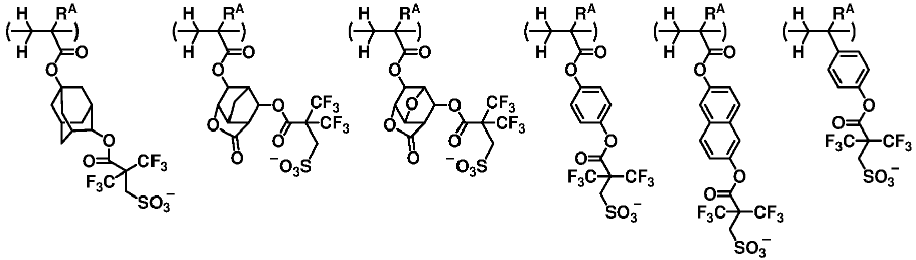

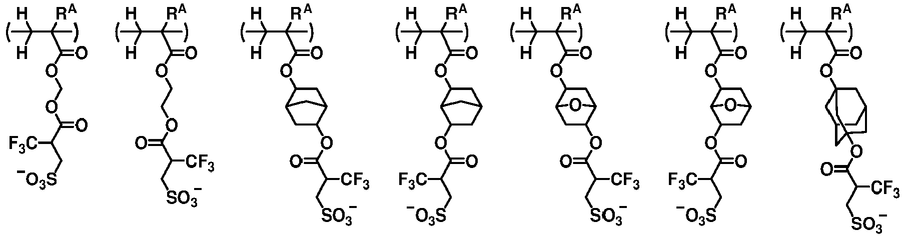

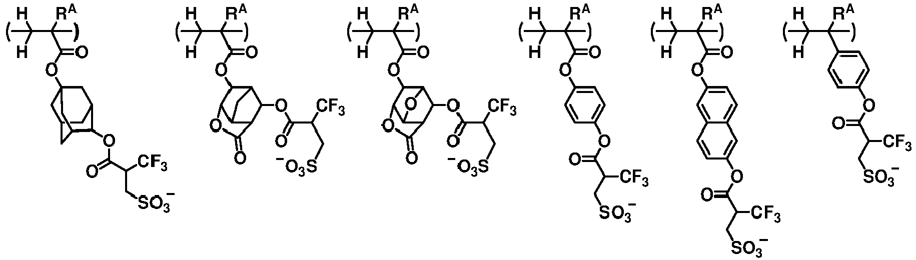

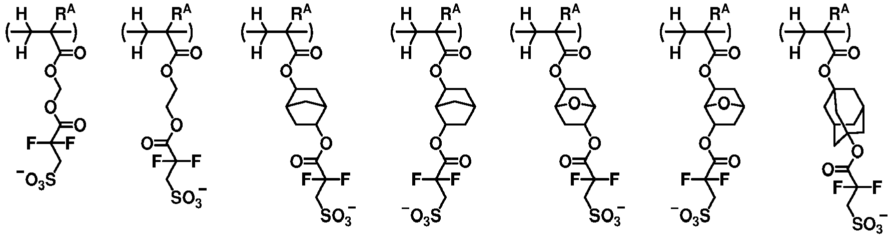

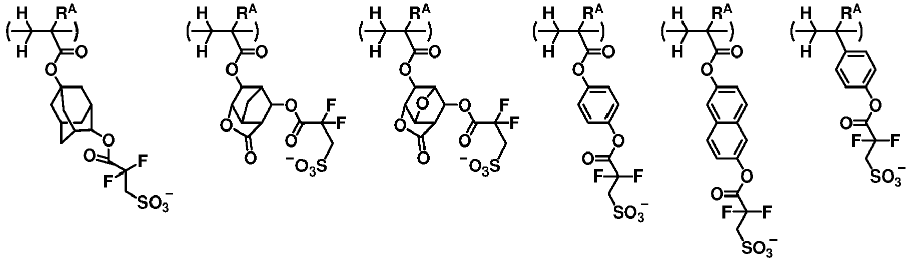

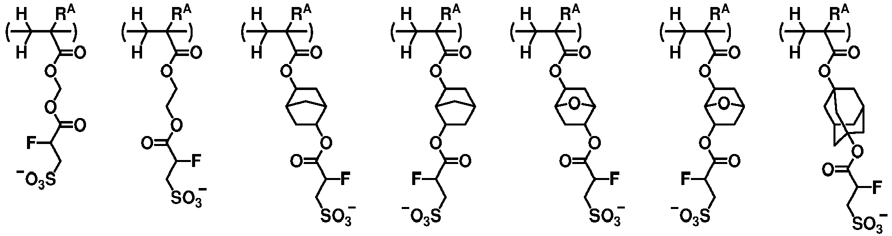

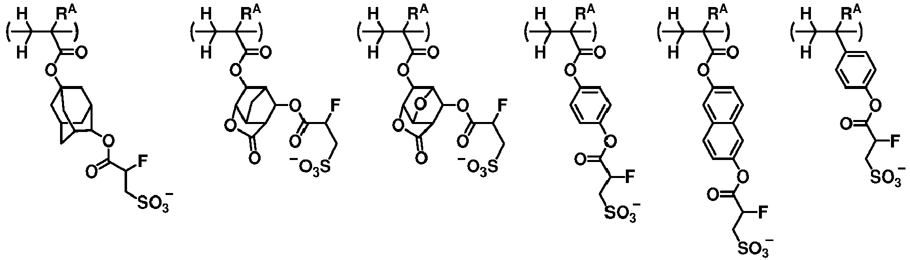

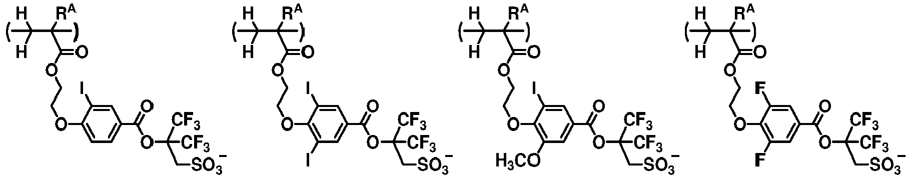

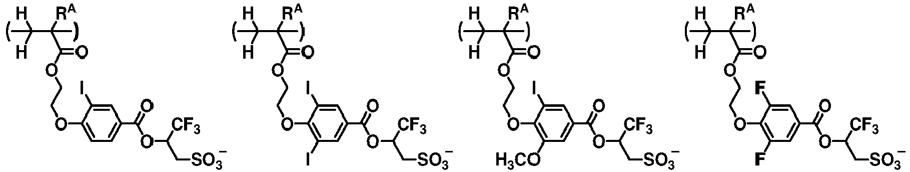

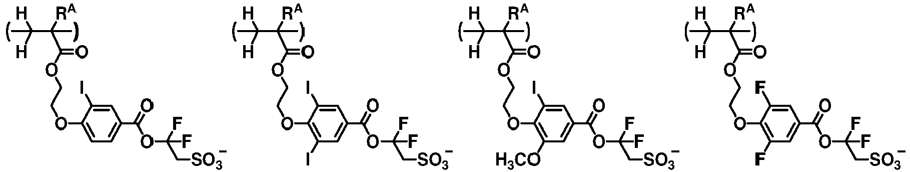

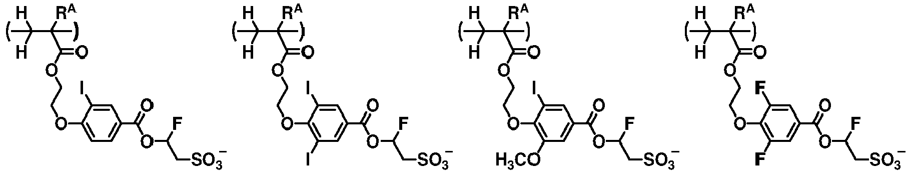

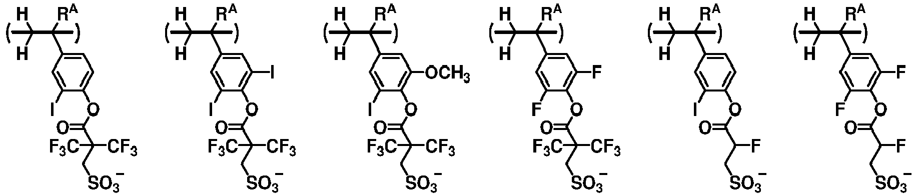

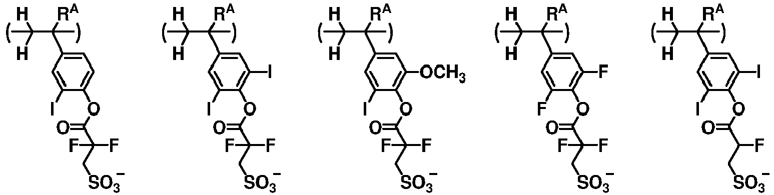

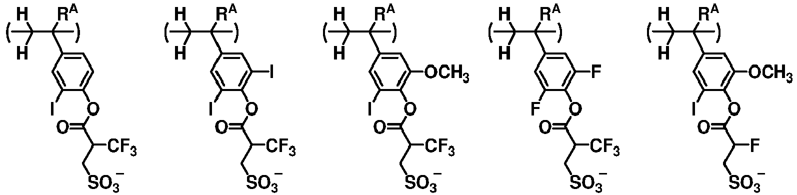

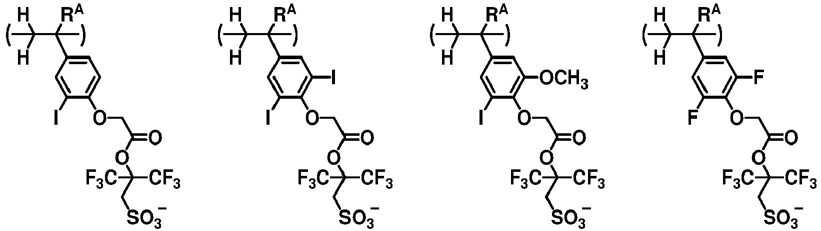

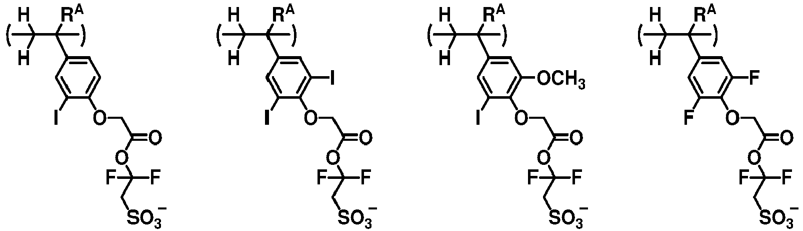

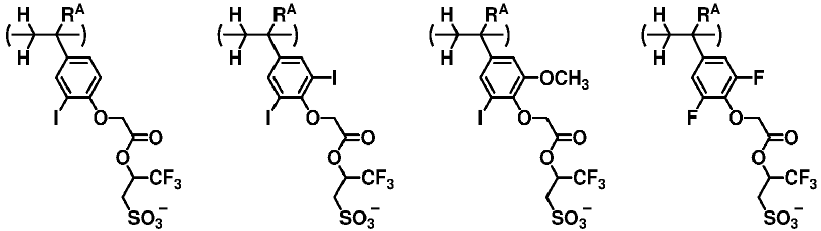

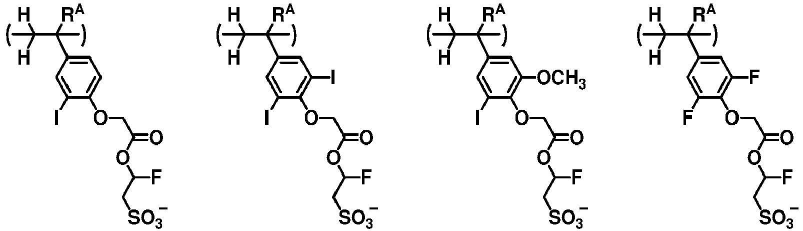

Examples of the anion in repeat unit C4 are shown below, but not limited thereto. R^{A} is as defined above.

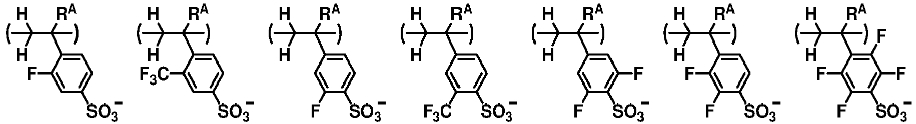

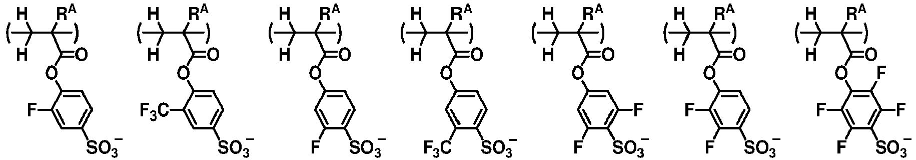

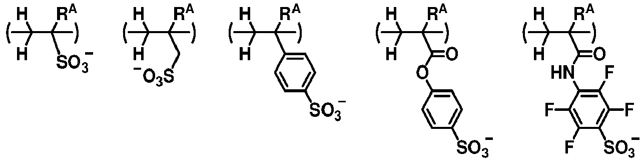

In formulae (C2) to (C4), A⁺ is an onium cation, preferably a sulfonium cation having the formula (cation-1) or an iodonium cation having the formula (cation-2).

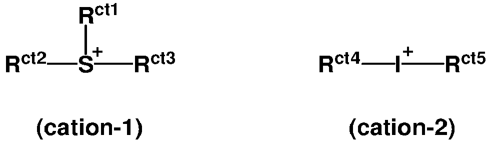

In formulae (cation-1) and (cation-2), R^{ct1} to R^{ct5} are each independently halogen or a C₁-C₃₀ hydrocarbyl group which may contain a heteroatom. Suitable halogen atoms include fluorine, chlorine, bromine, and iodine. The hydrocarbyl group may be saturated or unsaturated and straight, branched or cyclic. Examples thereof include C₁-C₃₀ alkyl groups such as methyl, ethyl, n-propyl, isopropyl, n-butyl, isobutyl, sec-butyl, and tert-butyl; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₃₀ alkenyl groups such as vinyl, allyl, propenyl, butenyl, and hexenyl; C₃-C₃₀ cyclic unsaturated hydrocarbyl groups such as cyclohexenyl; C₆-C₃₀ aryl groups such as phenyl, naphthyl and thienyl; C₇-C₃₀ aralkyl groups such as benzyl, 1-phenylethyl and 2-phenylethyl, and combinations thereof. Inter alia, aryl groups are preferred. In the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-), or haloalkyl moiety.

Also, R^{ct1} and R^{ct2} may bond together to form a ring with the sulfur atom to which they are attached. Exemplary structures of the ring are shown below.

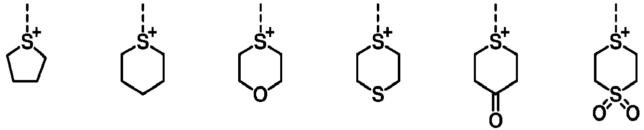

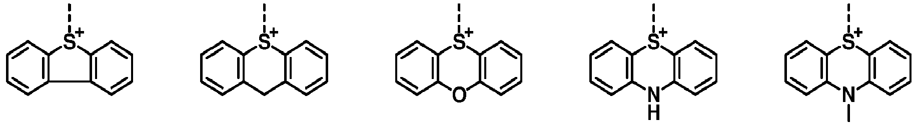

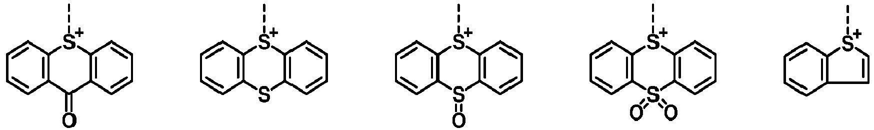

Herein the broken line designates a point of attachment to R^{ct3}.

Examples of the sulfonium cation having formula (cation-1) are shown below, but not limited thereto.

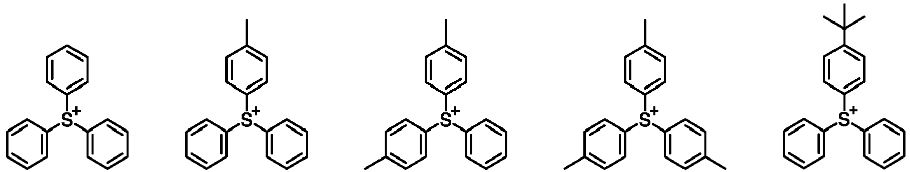

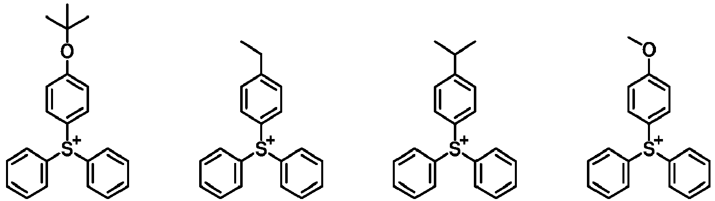

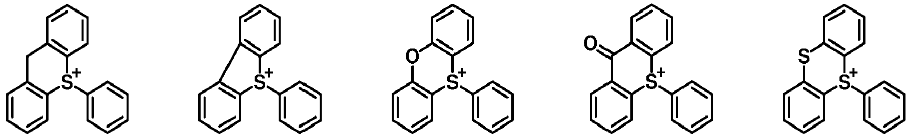

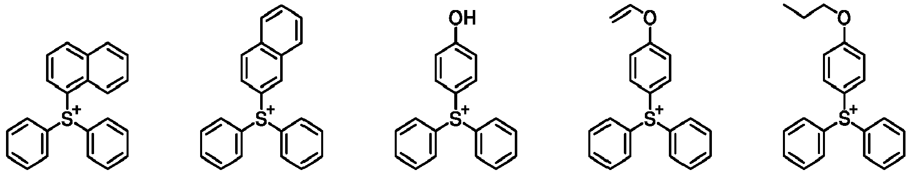

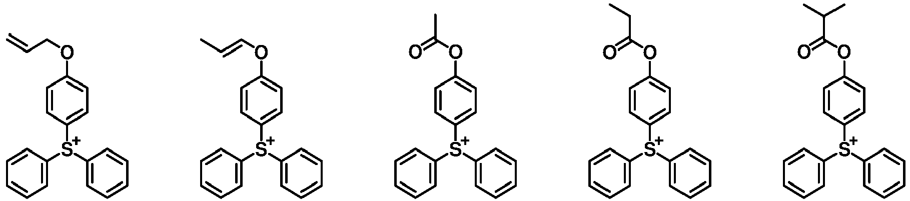

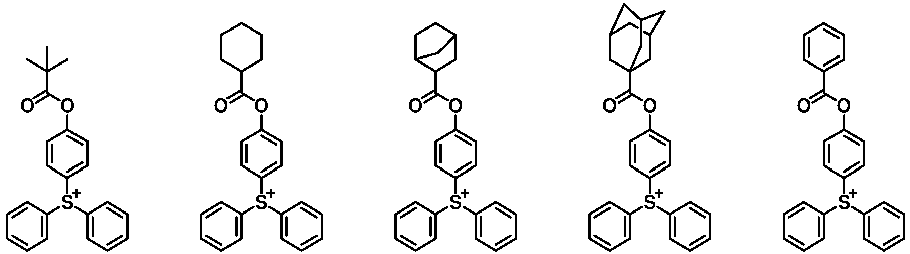

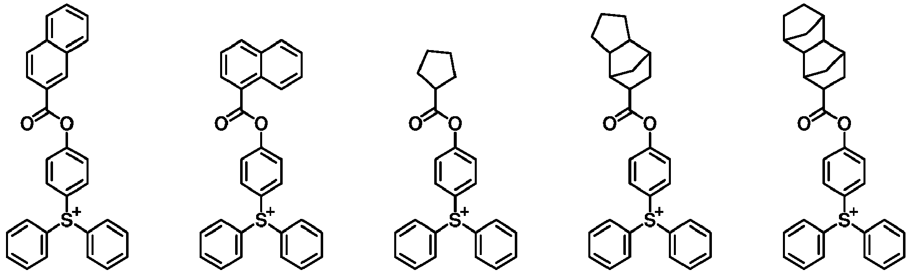

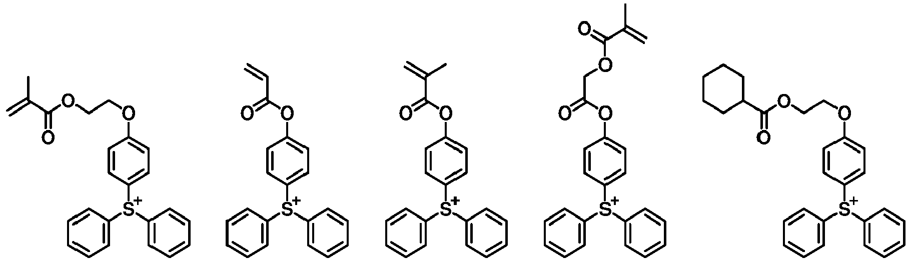

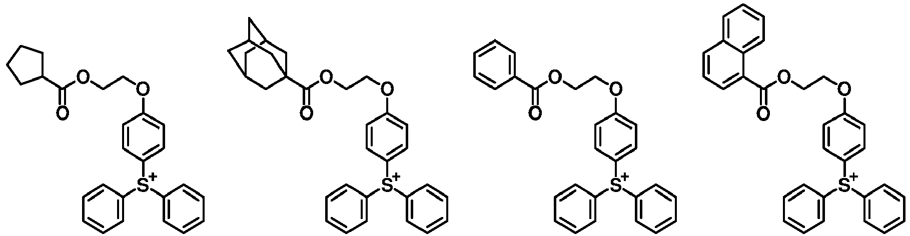

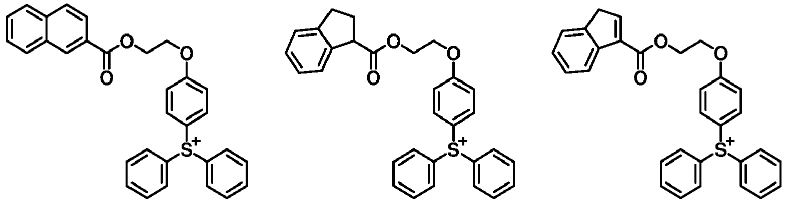

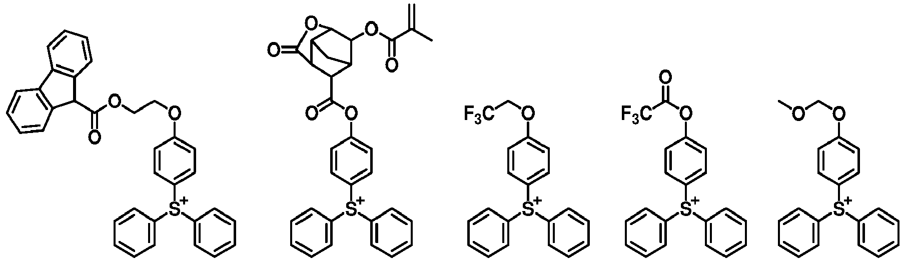

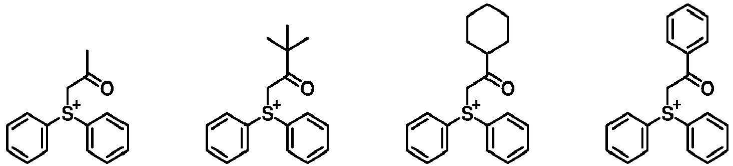

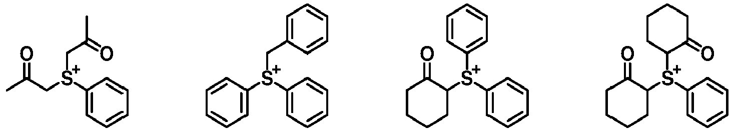

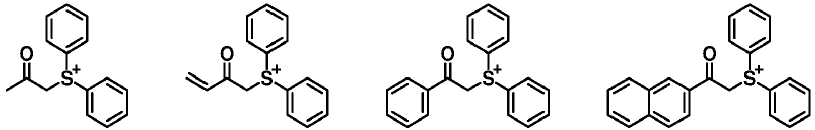

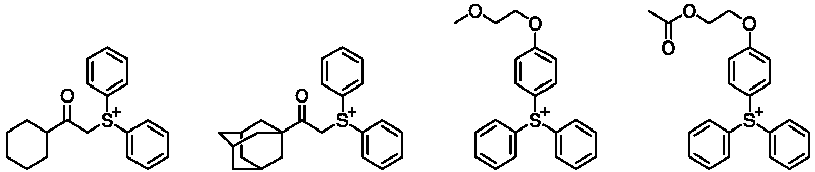

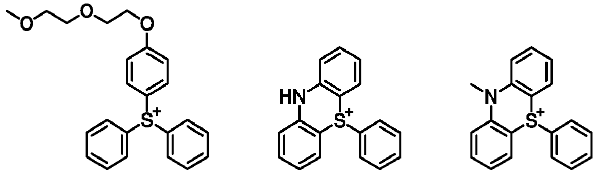

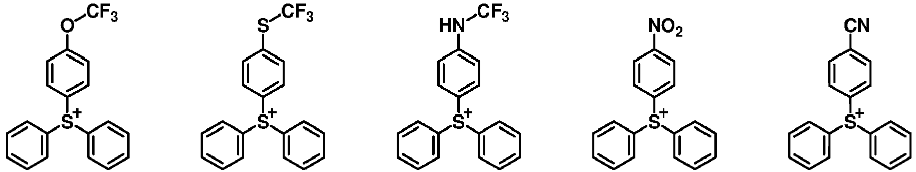

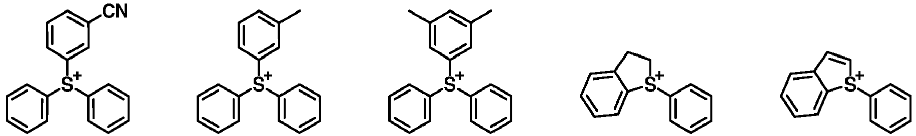

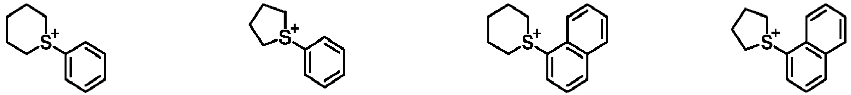

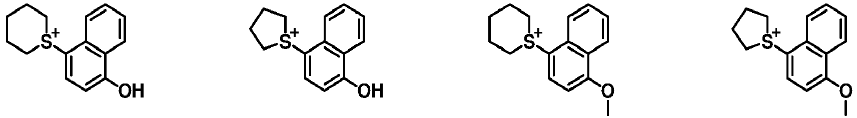

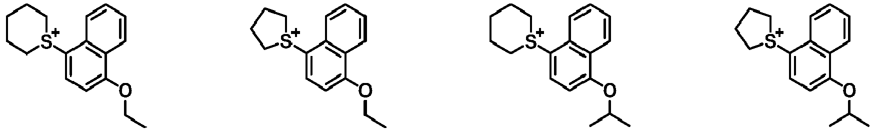

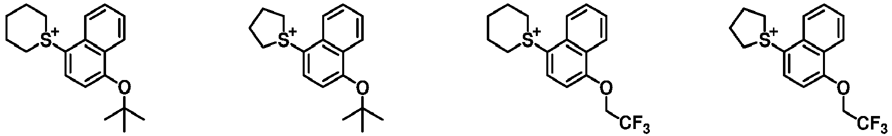

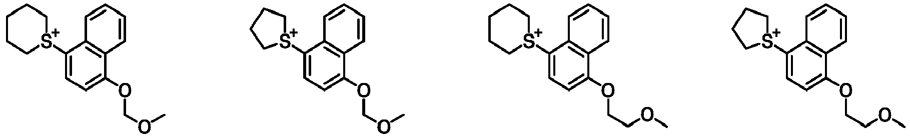

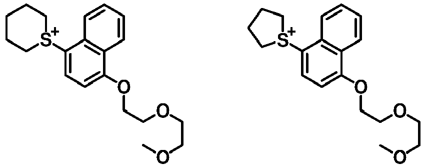

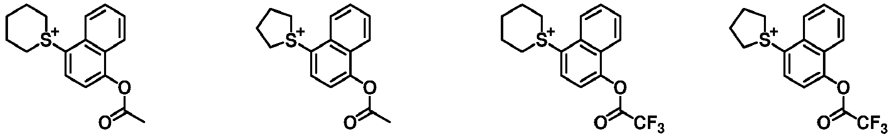

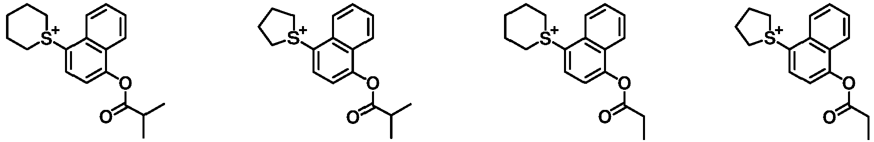

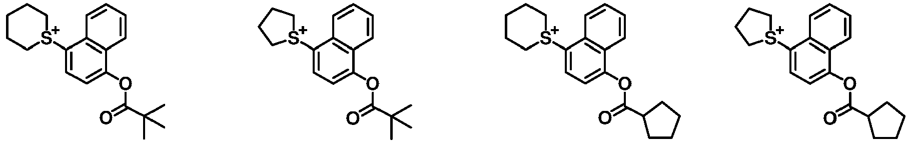

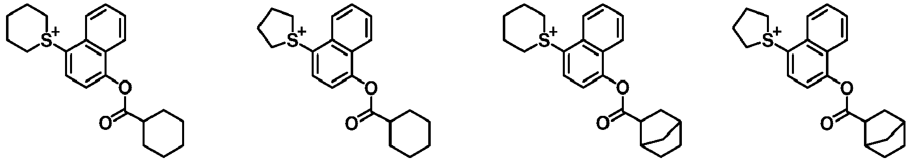

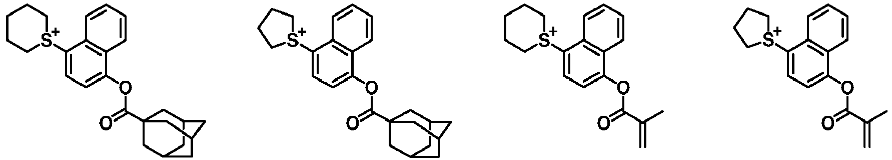

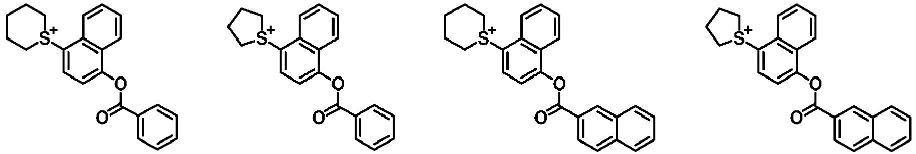

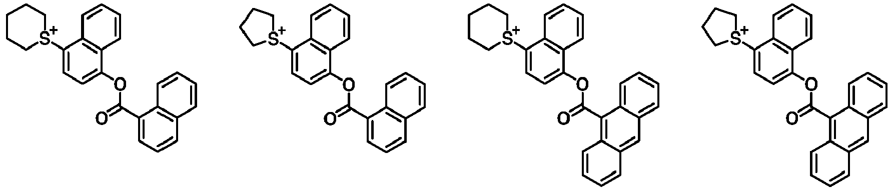

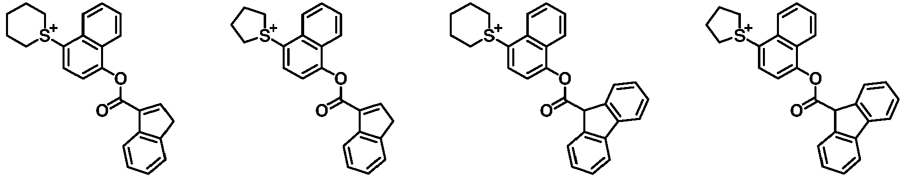

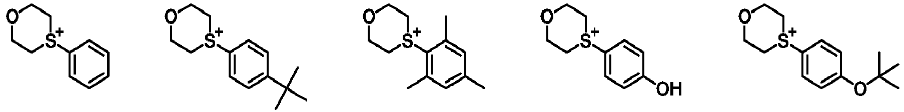

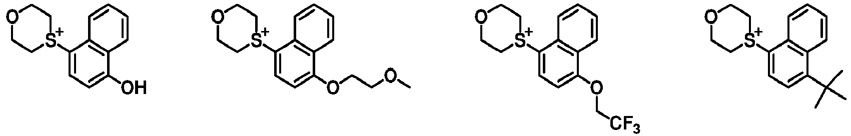

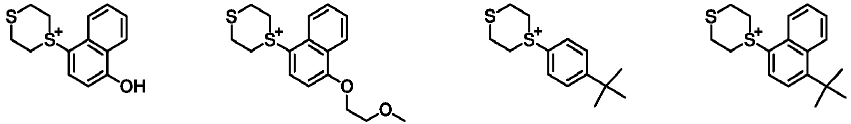

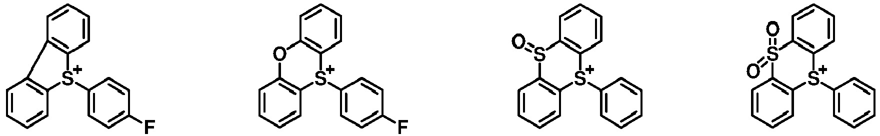

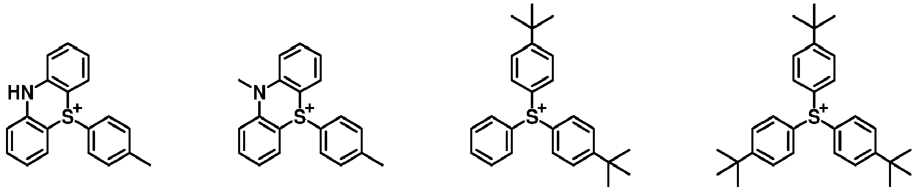

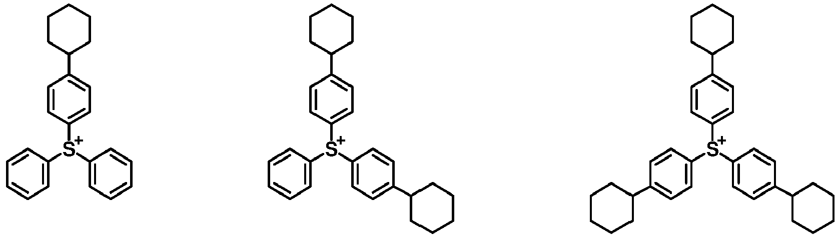

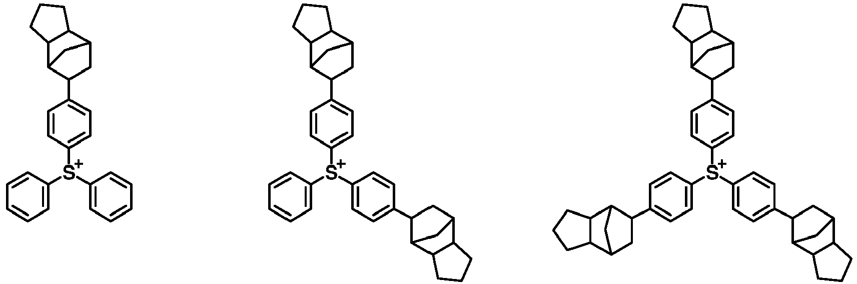

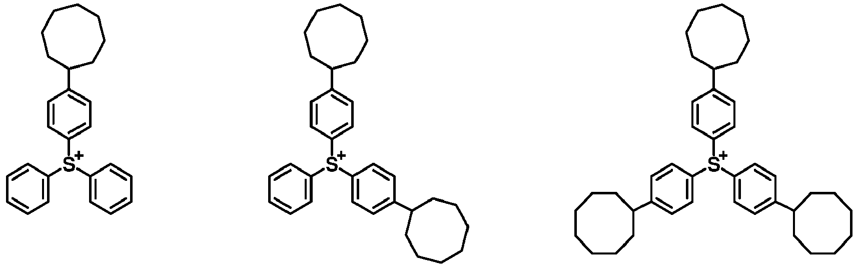

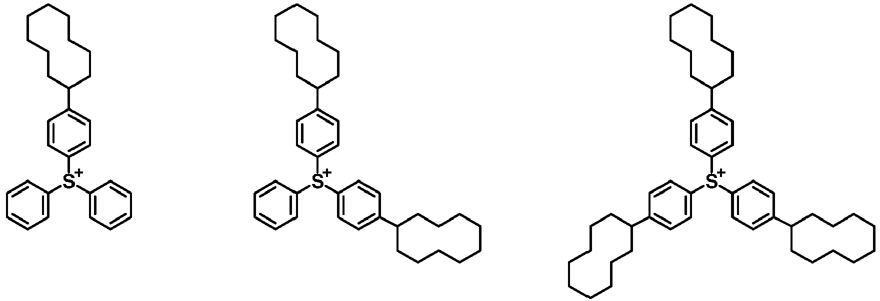

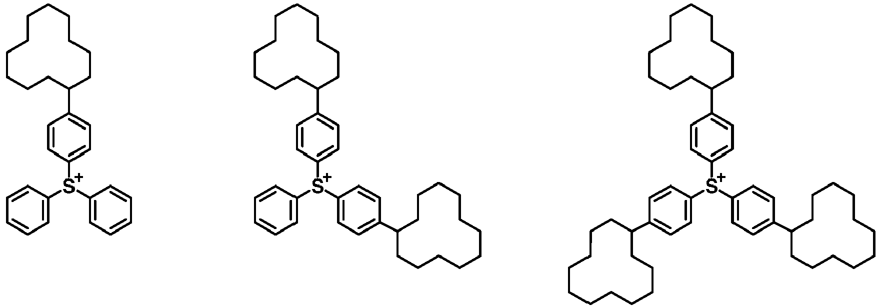

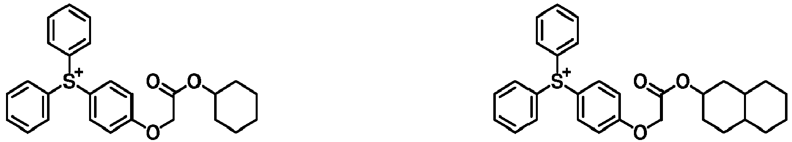

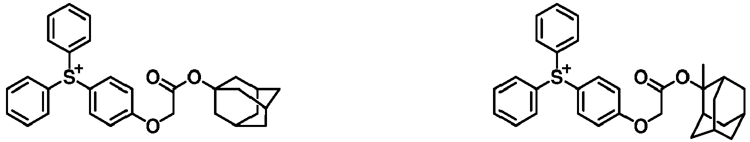

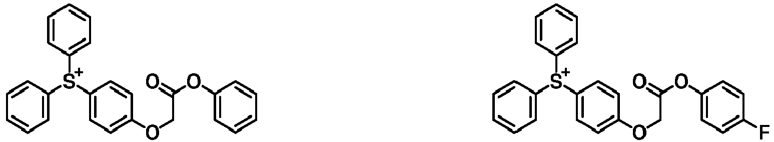

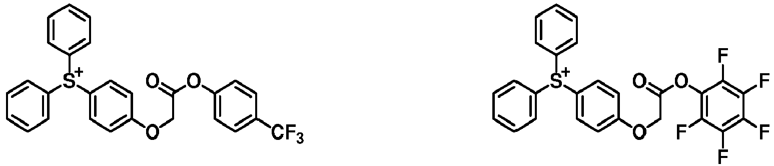

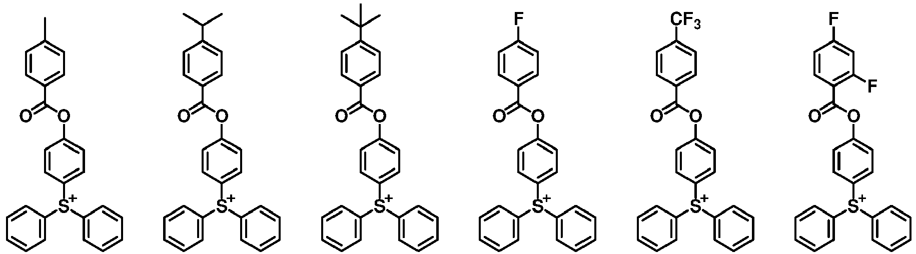

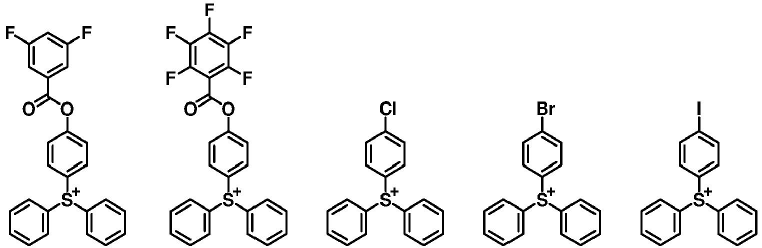

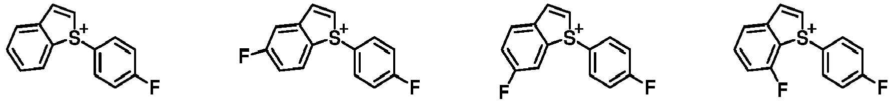

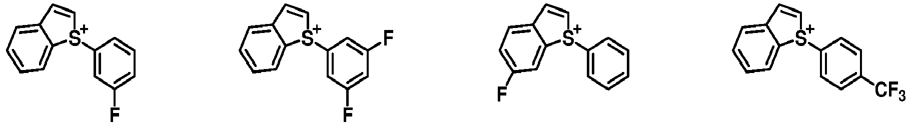

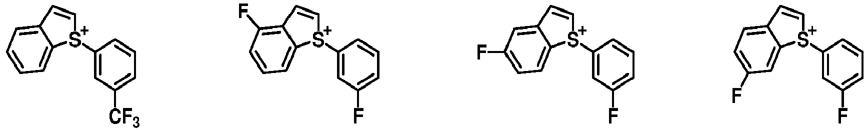

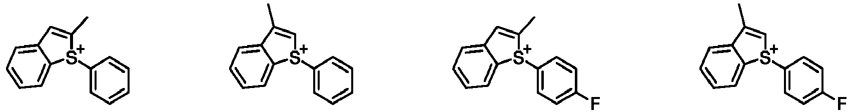

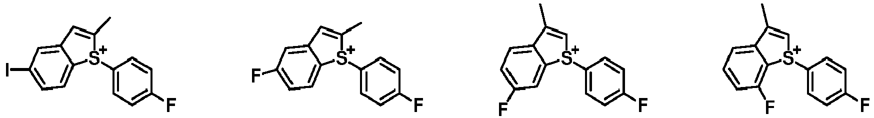

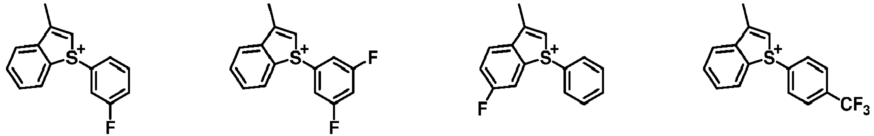

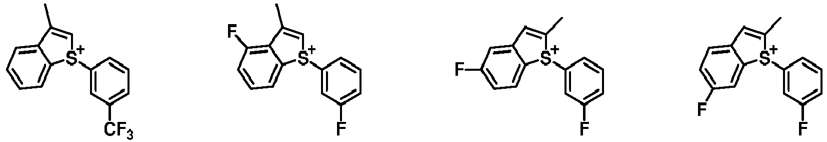

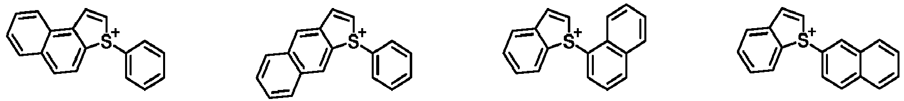

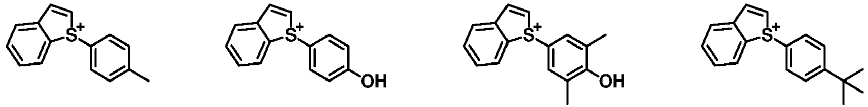

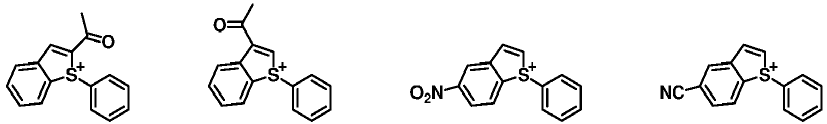

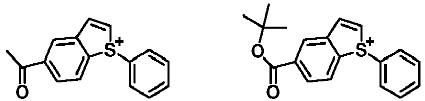

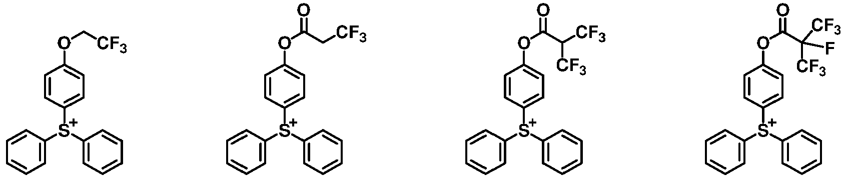

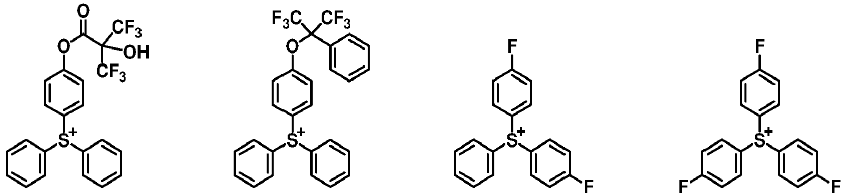

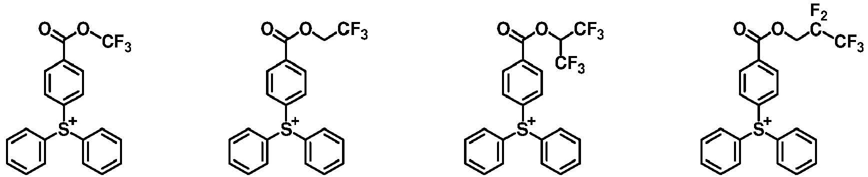

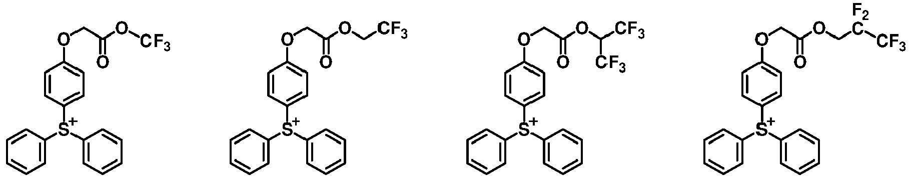

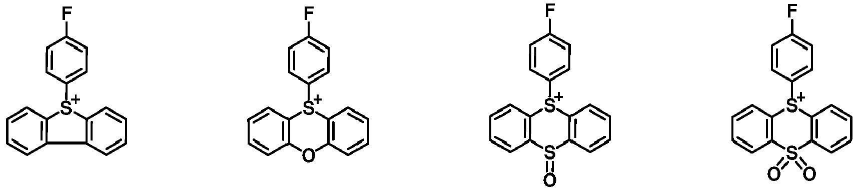

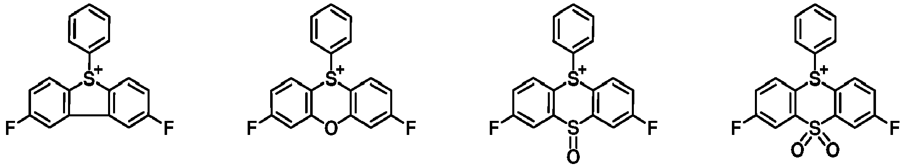

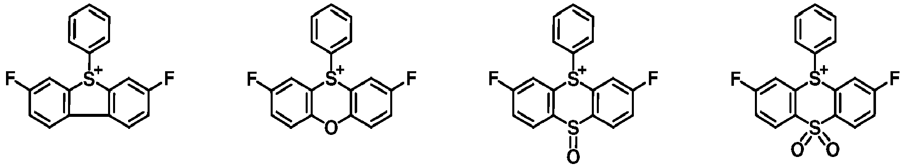

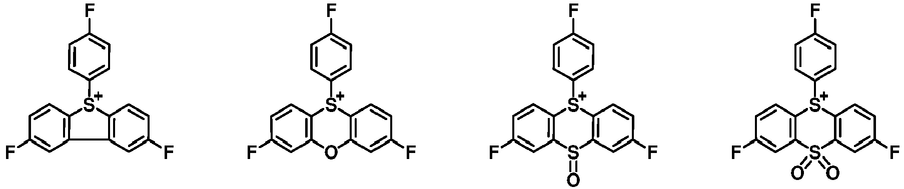

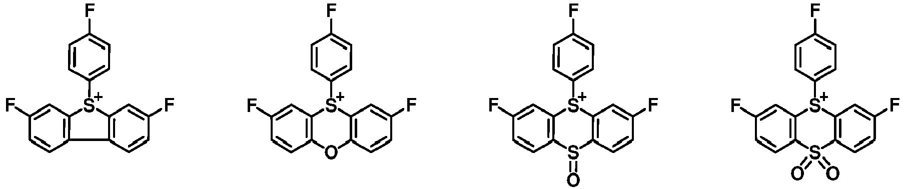

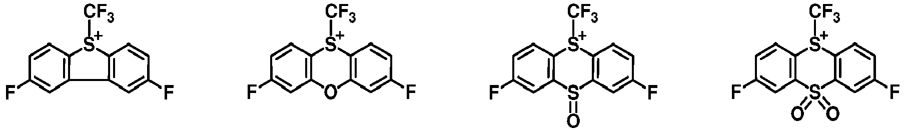

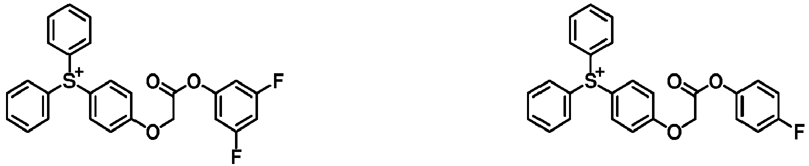

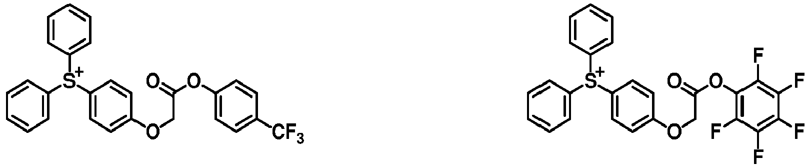

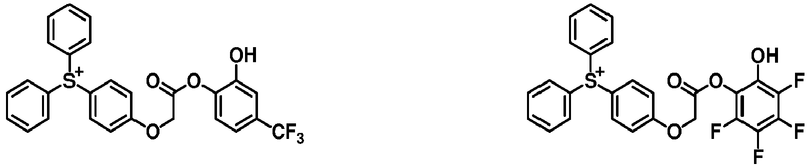

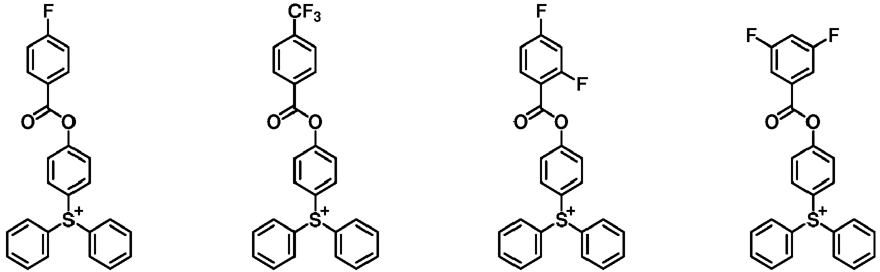

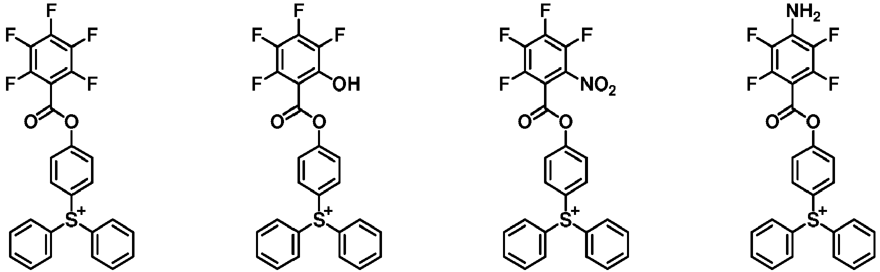

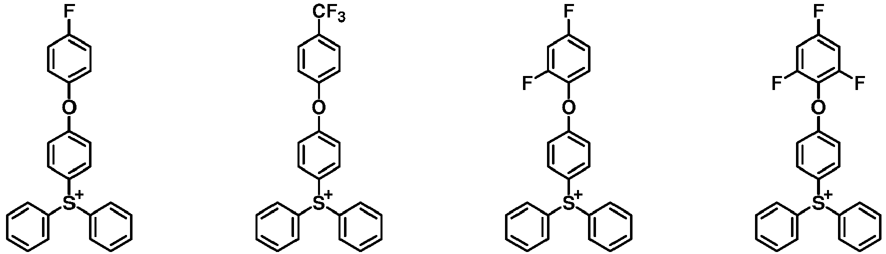

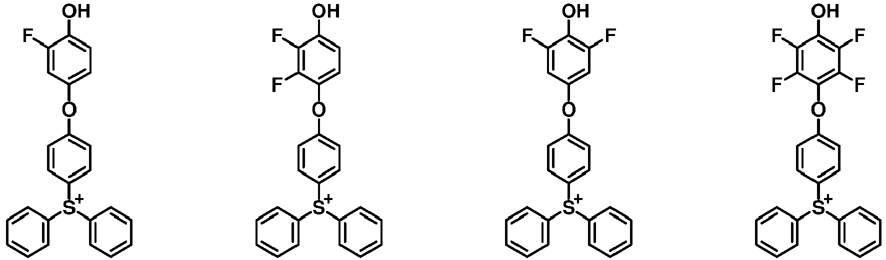

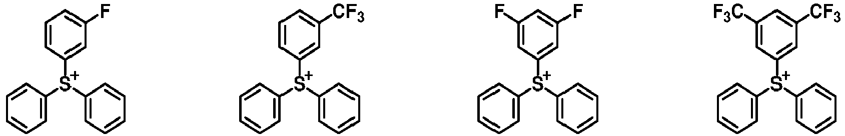

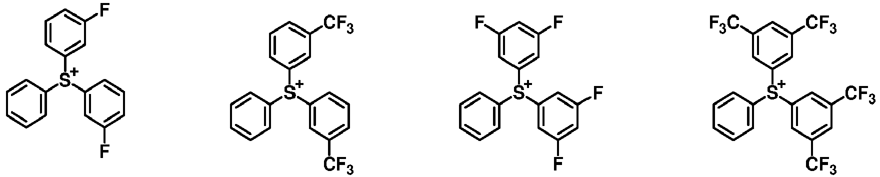

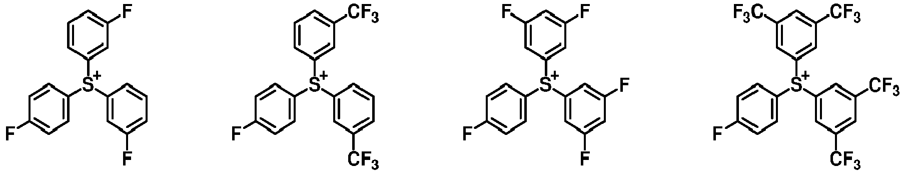

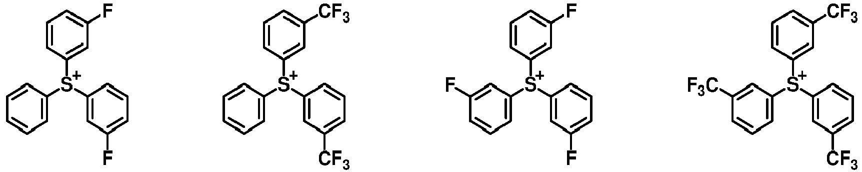

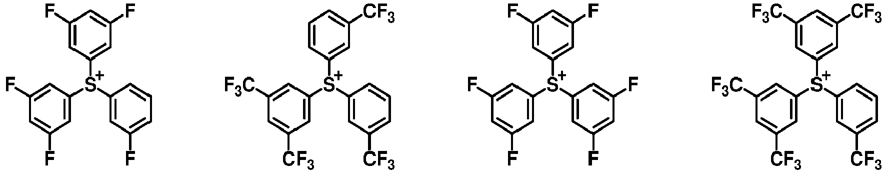

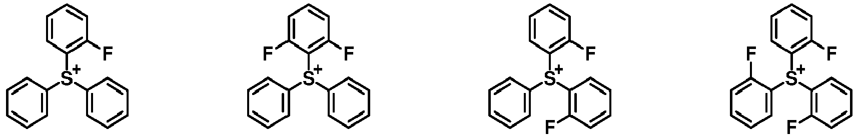

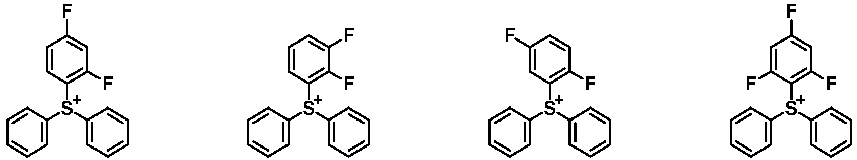

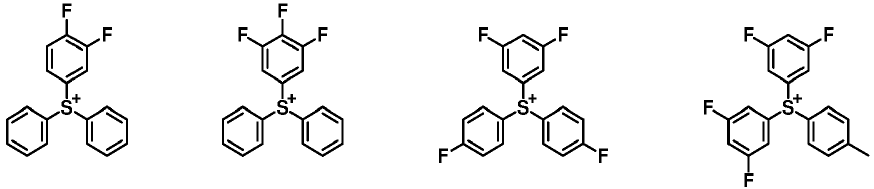

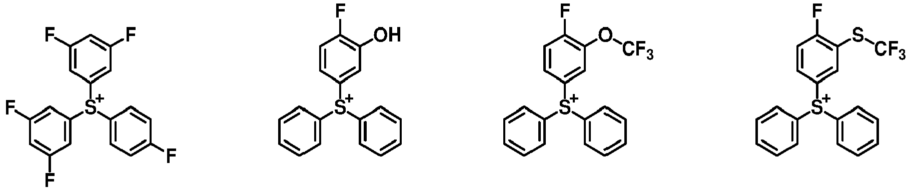

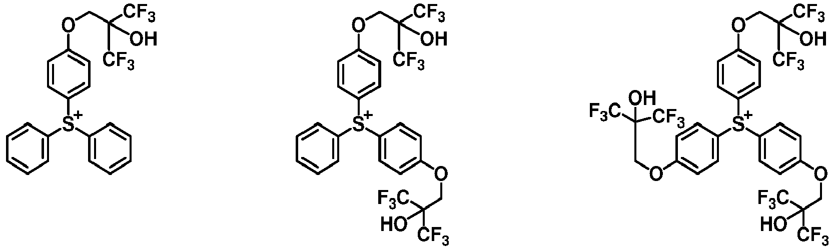

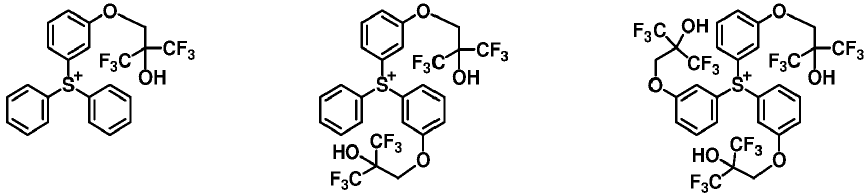

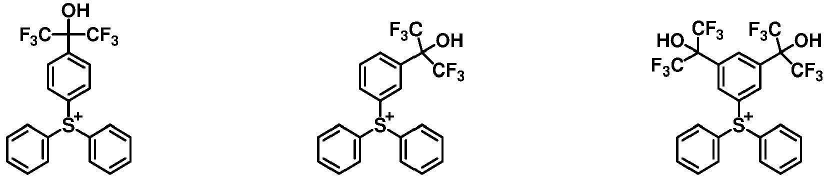

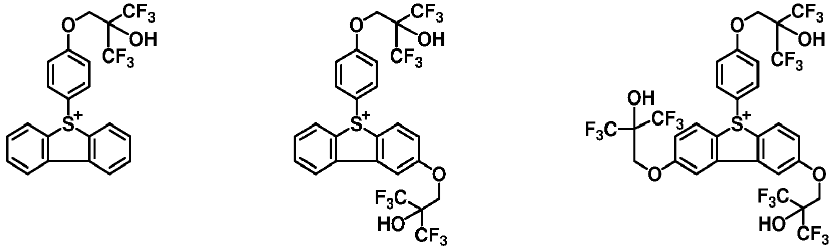

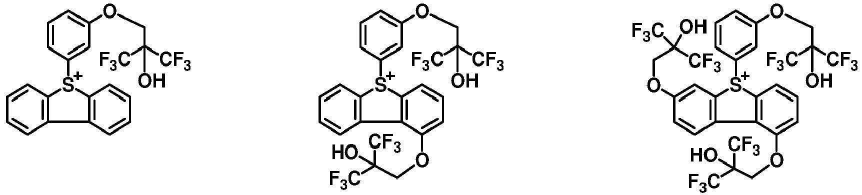

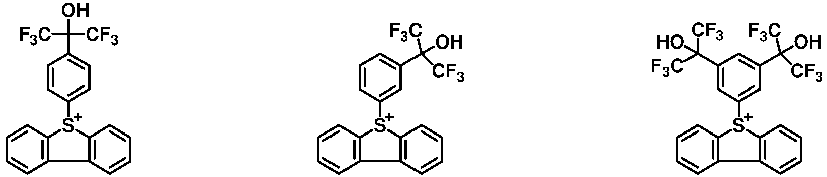

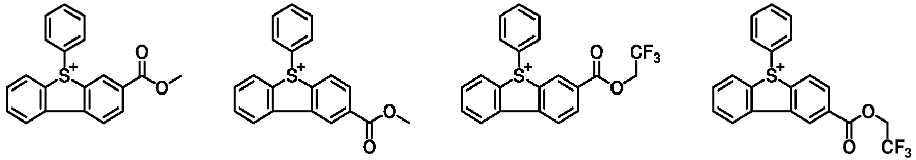

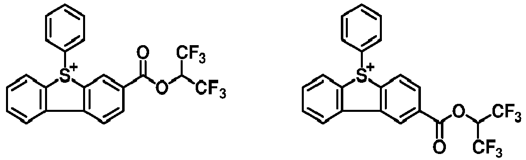

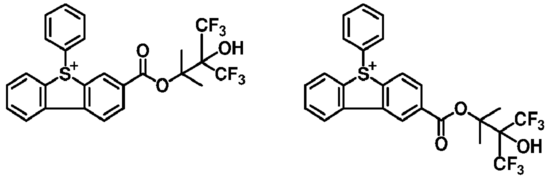

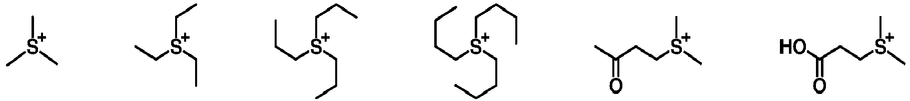

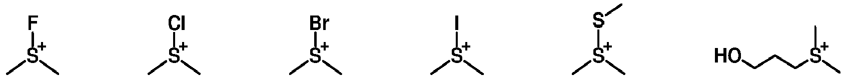

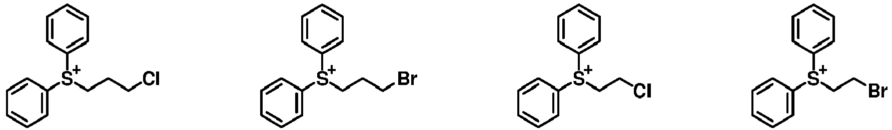

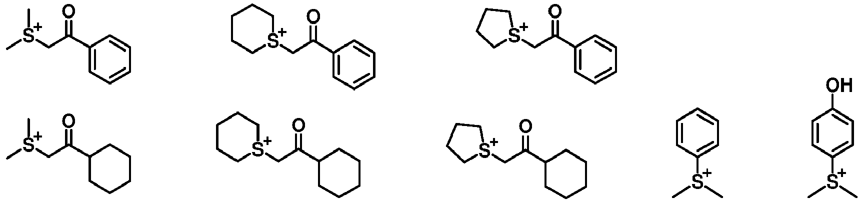

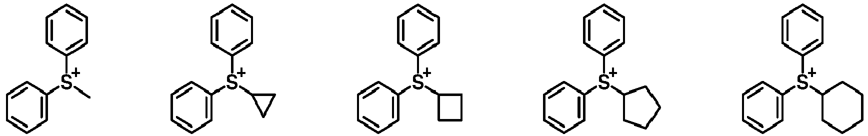

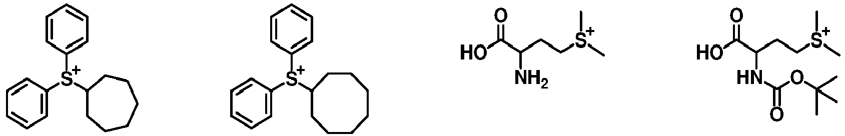

Examples of the iodonium cation having formula (cation-2) are shown below, but not limited thereto.

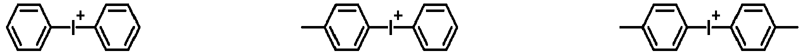

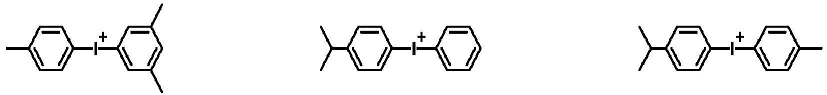

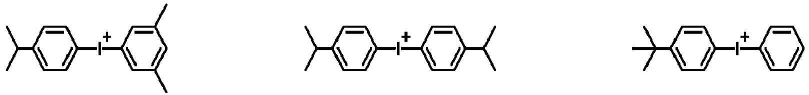

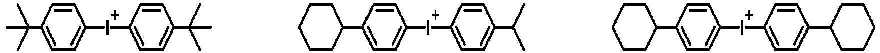

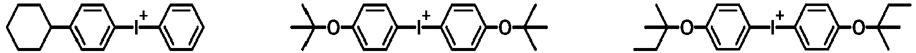

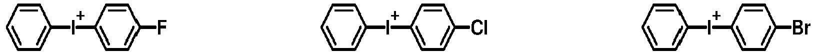

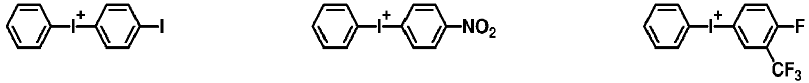

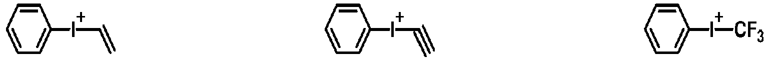

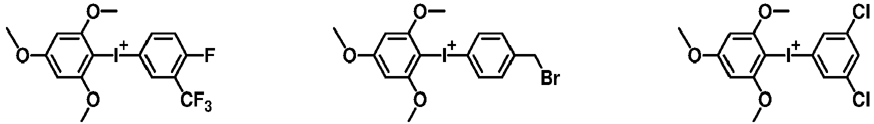

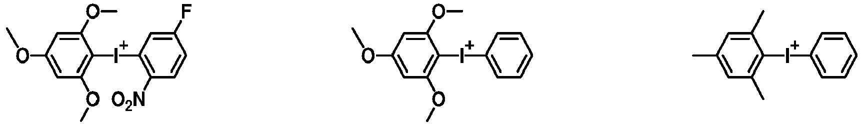

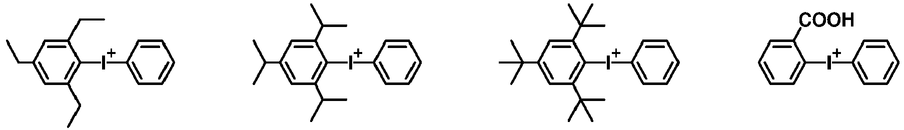

Exemplary structures of the repeat units C1 to C4 include arbitrary combinations of anions with cations, both as exemplified above.

Of the repeat units C1 to C4, repeat units C2 and C3 are preferred for the processing of photomask blanks because the acid strength is optimum in designing the acid labile group on a polymer.

The repeat units C1 to C4 are capable of generating an acid upon receipt of high-energy radiation. With the relevant units bound into a polymer, an appropriate control of acid diffusion becomes possible, and a pattern with reduced LER can be formed. Since the acid-generating unit is bound to a polymer, the phenomenon that acid volatilizes from the exposed region and re-deposits on the unexposed region during bake in vacuum is suppressed. This is effective for reducing LER and preventing profile degradation due to unwanted film thickness loss in the unexposed region.

When the polymer contains repeat units C1 to C4, their content is preferably 0.1 to 30 mol%, more preferably 0.5 to 20 mol% of the overall repeat units of the polymer.

The repeat units having an aromatic ring structure are preferably incorporated in a range of at least 65 mol%, more preferably at least 75 mol%, even more preferably at least 85 mol% based on the overall repeat units of the polymer. In the case of a polymer not containing repeat units C1 to C4, it is preferred that all the repeat units have an aromatic ring structure.

The total content of repeat units A1, repeat units A2, repeat units A3-1, repeat units A3-2, and repeat units of at least one type selected from repeat units B1 to B3 is preferably at least 70 mol%, more preferably at least 80 mol%, even more preferably at least 90 mol% based on the overall repeat units of the polymer.

The polymer may be synthesized by copolymerizing suitable monomers corresponding to the desired repeat units in the standard way. The monomer may have been protected with a protective group, and polymerization be followed by deprotection reaction. The copolymerization reaction is preferably radical or anionic polymerization though not limited thereto. For the polymerization reaction, reference may be made to JP-A 2004-115630, for example.

The polymer may also be synthesized by copolymerizing a monomer mixture which contains a monomer providing repeat unit A2, but not a monomer providing repeat unit A1 to synthesize a polymer, and protecting an aromatic hydroxy group on the polymer with an acetal group. For this synthesis, a method using a vinyl ether and an acid catalyst and a method using an acetalizing agent having a haloalkoxy group and a base are known, and either of these methods may be utilized.

For example, in the former method using an acetal modifier having formula (AC-1), i.e., vinyl ether and an acid catalyst, examples of the acid catalyst include hydrochloric acid, sulfuric acid, nitric acid, methanesulfonic acid, p-toluenesulfonic acid, trifluoromethanesulfonic acid, trifluoroacetic acid, oxalic acid, and methanesulfonic acid pyridine salt. The reaction temperature is preferably 5 to 30°C. The reaction time is preferably 0.2 to 10 hours, more preferably 0.5 to 6 hours.

In the latter method using an acetal modifier having formula (AC-2), i.e., acetalizing agent having a haloalkoxy group and a base, the acetalizing agent having a haloalkoxy group is added dropwise in the presence of a basic compound such as triethylamine, diisopropylamine, pyridine, 2,6-lutidine, sodium hydroxide, potassium hydroxide, lithium hydroxide, sodium carbonate, potassium carbonate, and cesium carbonate. The reaction temperature is preferably -20°C to 50°C. The reaction time is preferably 0.2 to 10 hours, more preferably 0.5 to 6 hours.

It is noted that the latter method using an acetalizing agent having a haloalkoxy group and a base has the risk of generating a corrosive strong acid such as hydrochloric acid which can corrode metallic reactors and piping, suggesting that the polymer can be contaminated with metal impurities which cause defects to semiconductor products. Since the lithography of advanced generation requires that raw materials for use in resist compositions have a metal impurity content of less than 10 ppb, the former method using a vinyl ether and an acid catalyst is recommended.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9, even more preferably 1.0 to 1.8. A polymer with such a narrow dispersity eliminates the risk that foreign particles are left on the pattern after development and the pattern profile is aggravated.

### Chemically amplified positive resist composition

A further embodiment of the invention is a chemically amplified positive resist composition comprising a base polymer containing the polymer defined above. The polymer undergoes deprotection reaction of acid labile groups under the action of acid to generate an aromatic hydroxy group which has a higher acidity than an aliphatic hydroxy group. This ensures that when a resist film is formed by applying the positive resist composition, exposed, and developed in an alkaline developer, the dissolution contrast of the resist film between exposed and unexposed regions is increased. A higher resolution is thus obtained.

The base polymer may either consist of the above-defined polymer or contain another polymer in addition to the above-defined polymer. Examples of the other polymer include polymers comprising repeat units A3-1 and/or A3-2, and repeat units of at least one type selected from repeat units A2, B1 to B3, and C1 to C4, and polymers comprising repeat units having a well-known acid labile group such as tertiary alkyl group or tert-butoxycarbonyl group, and repeat units of at least one type selected from repeat units A2, B1 to B3, and C1 to C4. The benefits of the invention are obtained particularly when the base polymer contains at least 30% by weight of a polymer comprising repeat units A1.

In the embodiment wherein the base polymer contains another polymer in addition to the above-defined polymer, the content of repeat units A1 is preferably 10 to 40 mol%, more preferably 10 to 35 mol%, even more preferably 20 to 30 mol% of the overall repeat units of the polymer in the base polymer. The content of repeat units A2 is preferably 30 to 90 mol%, more preferably 40 to 85 mol% of the overall repeat units of the polymer in the base polymer. It is noted that when the polymer further contains repeat units of at least one type selected from repeat units having formulae (B1) and (B2), preferably the repeat units of at least one type containing a phenolic hydroxy group as a substituent, the content of repeat units A1 or A2 plus repeat units B1 and/or B2 falls in the above range. The content of repeat units B1 to B3 is preferably at least 5 mol% of the overall repeat units of the polymer in the base polymer while its upper limit is preferably up to 25 mol%, more preferably up to 20 mol%. The content of repeat units B4 to B6 is preferably 0 to 20 mol%, more preferably 0 to 10 mol% of the overall repeat units of the polymer in the base polymer. The content of repeat units C1 to C4 is preferably 0.1 to 30 mol%, more preferably 0.5 to 20 mol% of the overall repeat units of the polymer in the base polymer.

The content of repeat units having an aromatic ring skeleton is preferably at least 65 mol%, more preferably at least 75 mol%, even more preferably at least 85 mol% of the overall repeat units of the polymer in the base polymer. When repeat units C1 to C4 are not contained, preferably all the repeat units have an aromatic ring skeleton.

The content of repeat units A1 and repeat units of at least one type selected from repeat units A2, repeat units A3-1, repeat units A3-2, and repeat units B1 to B3 is preferably at least 70 mol%, more preferably at least 80 mol%, even more preferably at least 90 mol% of the overall repeat units of the polymer in the base polymer.

The base polymer is designed such that the dissolution rate in alkaline developer is preferably up to 10 nm/min, more preferably up to 7 nm/min, even more preferably up to 5 nm/min. In the lithography of advanced generation wherein the coating film on the substrate is in a thin film range of up to 100 nm, the influence of pattern film thickness loss during alkaline development becomes strong. When the polymer has an alkaline dissolution rate in excess of 10 nm/min, pattern collapse occurs, meaning a failure to form small-size patterns. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

The reason why a higher resolution is available from the acid labile group derived from the acetal modifier of the invention bearing an oxygen or sulfur-containing saturated heterocycle is described below. In the unexposed region, the saturated heterocycle functions as an acid diffusion suppressing group because the heteroatom, oxygen or sulfur has an adequate affinity to the proton of the generated acid, and its cyclic structure contributes to an improvement in etch resistance after pattern formation. In the exposed region, acid elimination reactivity is high because the acetal structure shows a sharp reactivity to acid, the alcohol having a cyclic saturated heterocycle formed after the acid elimination reaction is so hydrophilic that alkaline development is carried out with no residues being left in the exposed region. Accordingly, the contrast between exposed and unexposed regions is enhanced. By virtue of the synergy of these effects, a satisfactory resolution is achieved in forming positive small-size patterns through alkaline development, which is advantageous especially for photomask processing of the sub-10 nm node generation.

### Organic solvent

The chemically amplified positive resist composition may comprise an organic solvent. The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone and methyl-2-n-pentyl ketone; alcohols such as 3-methoxybutanol, 3-methyl-3-methoxybutanol, 1-methoxy-2-propanol, 1-ethoxy-2-propanol, and diacetone alcohol; ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof. Where an acid labile group of acetal form is used, a high-boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, and mixtures thereof.

In the resist composition, the organic solvent is preferably used in an amount of 200 to 10,000 parts, more preferably 400 to 5,000 parts by weight per 80 parts by weight of the base polymer. The organic solvent may be used alone or in admixture.

### Photoacid generator

The chemically amplified positive resist composition may further comprise a photoacid generator (PAG). The PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to deprotect the acid labile group in repeat unit A1.

The preferred PAGs are salt compounds having a sulfonium anion of the structure shown below.

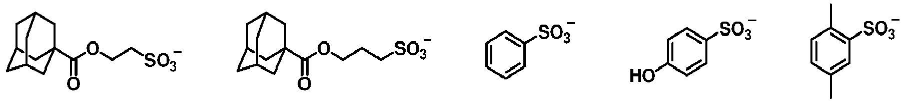

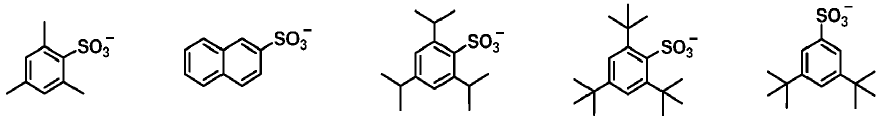

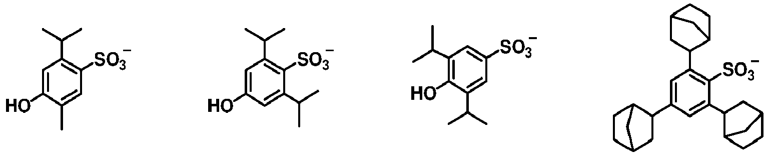

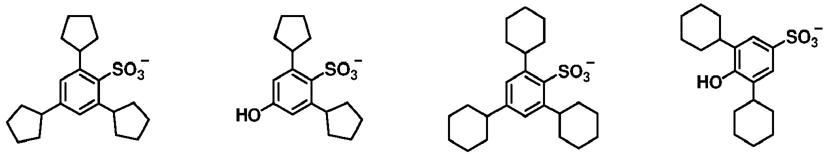

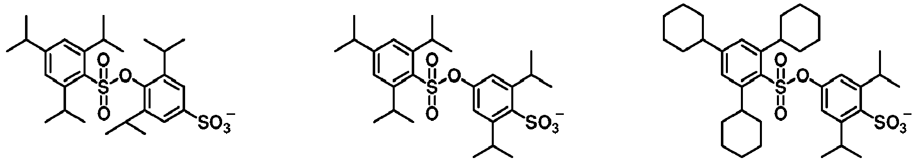

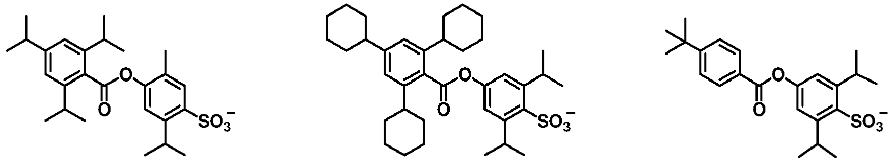

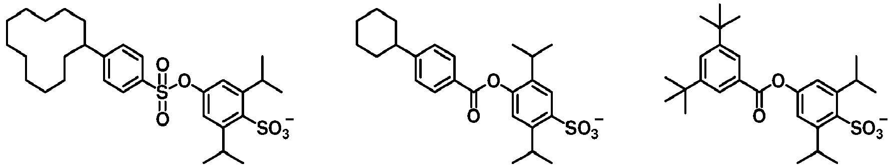

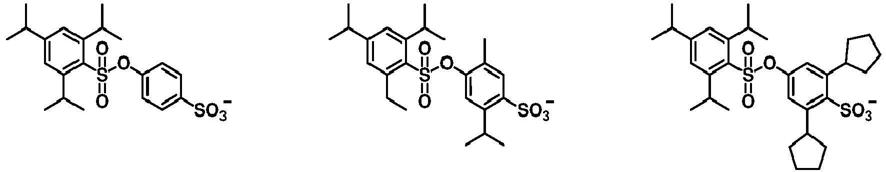

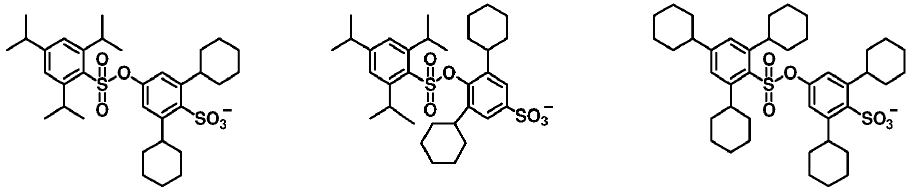

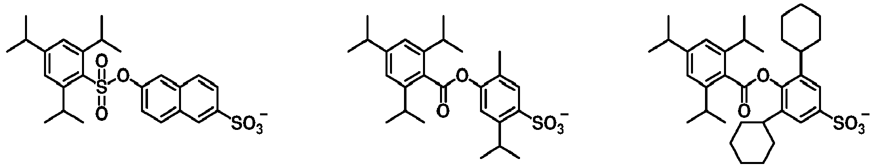

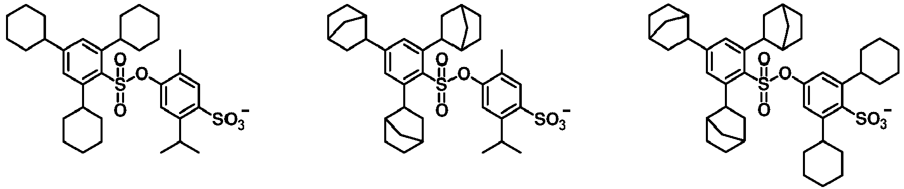

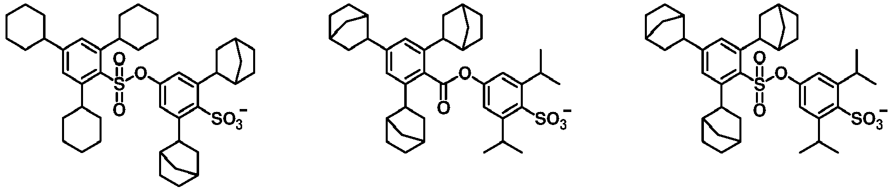

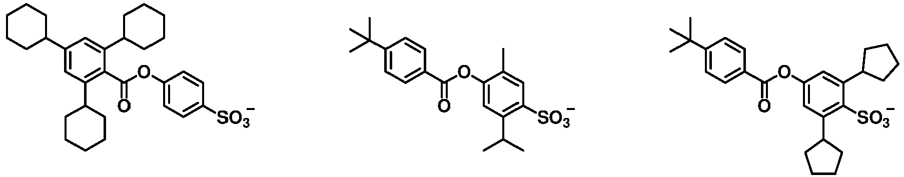

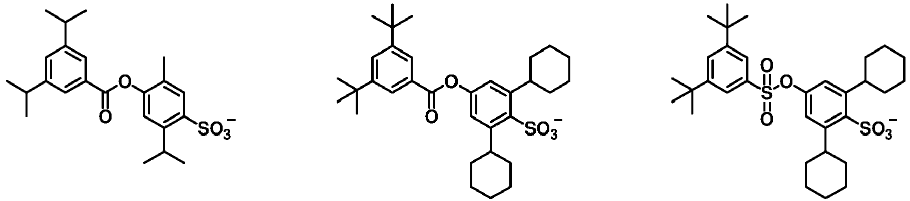

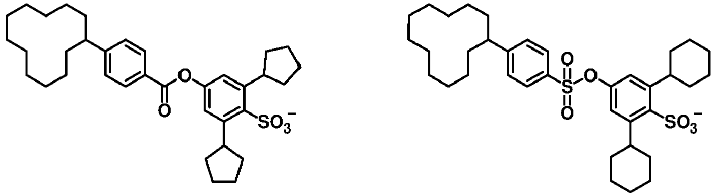

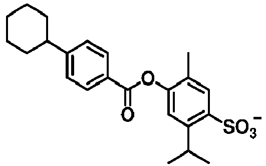

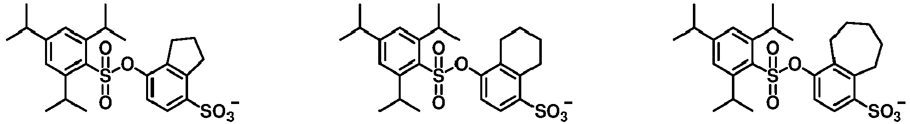

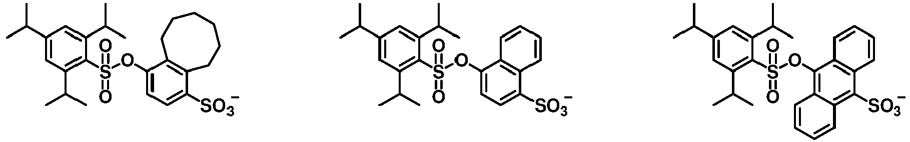

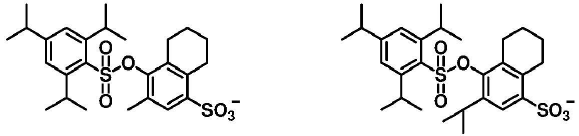

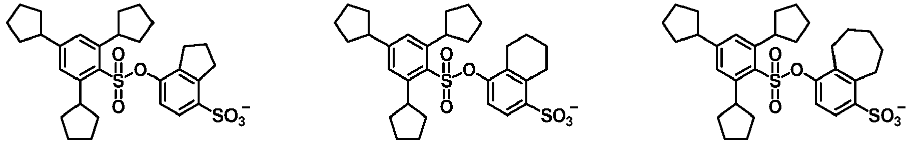

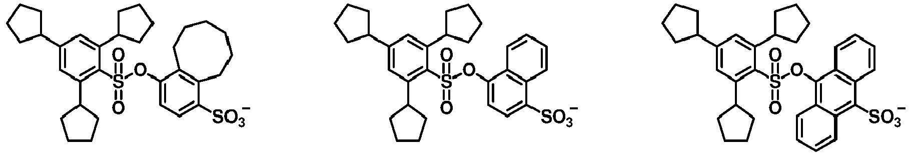

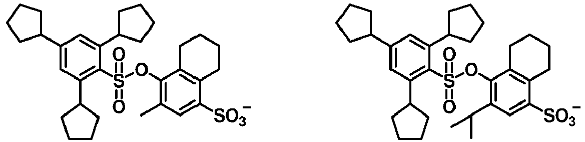

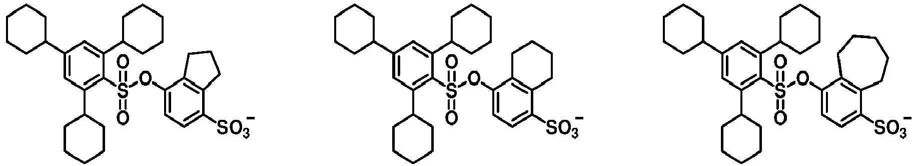

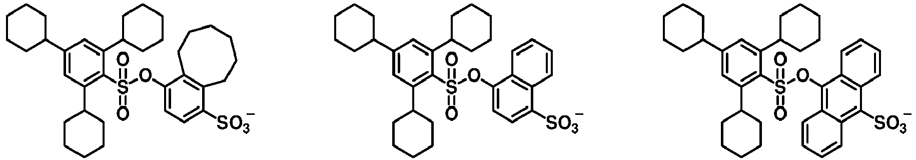

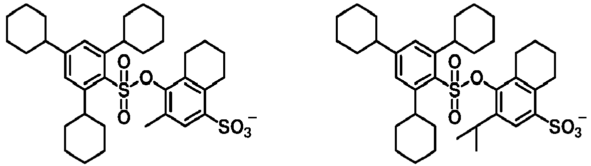

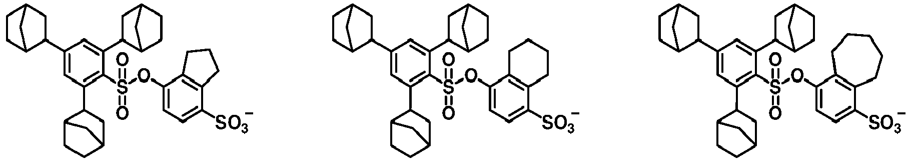

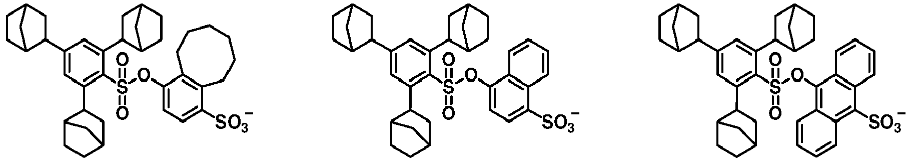

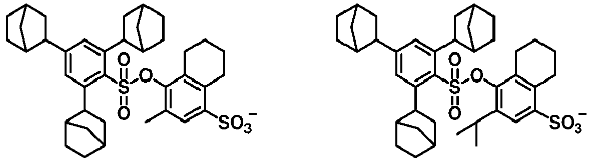

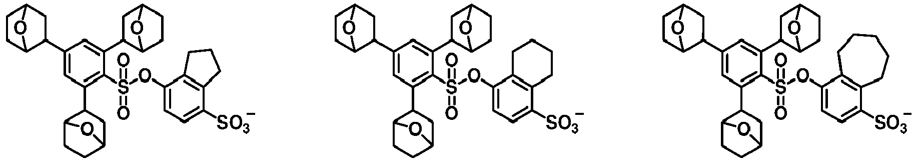

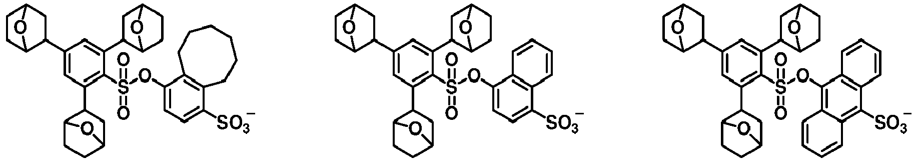

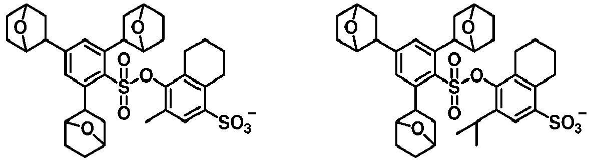

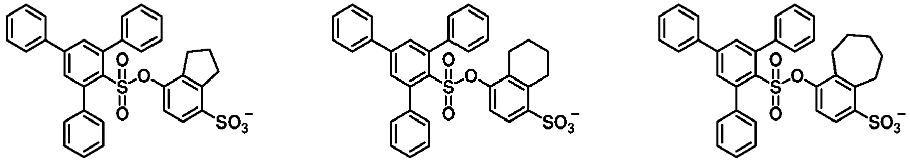

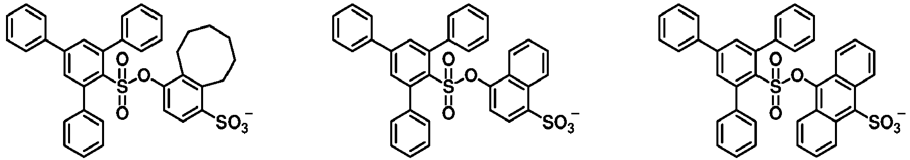

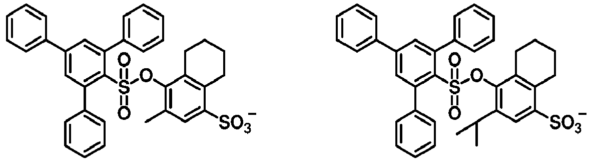

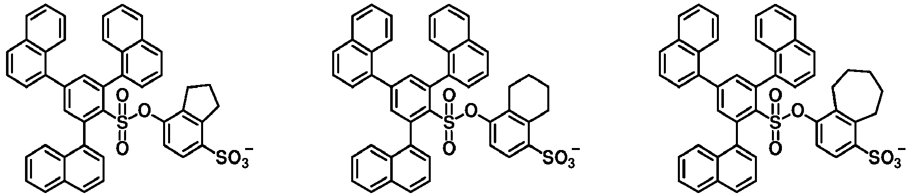

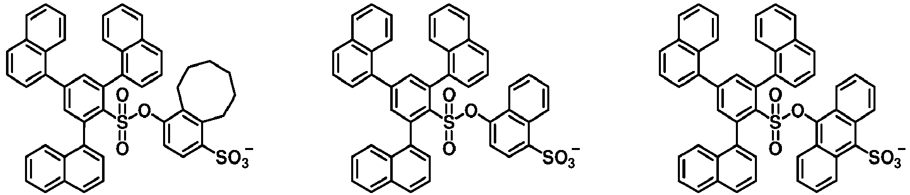

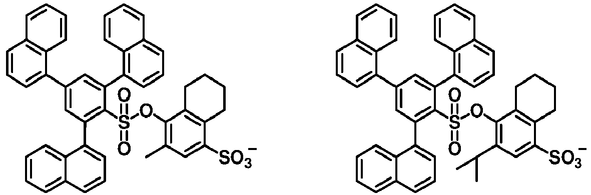

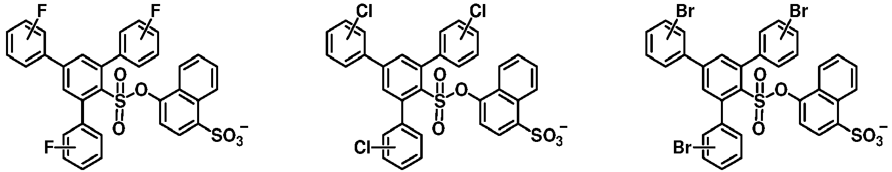

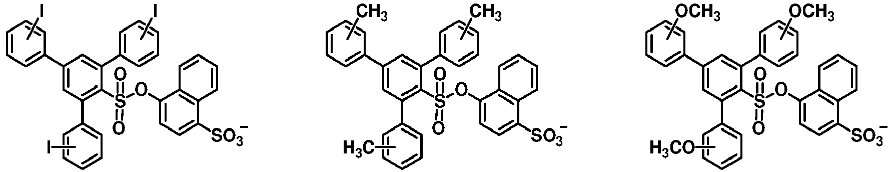

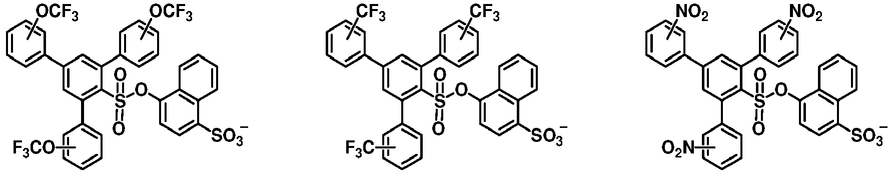

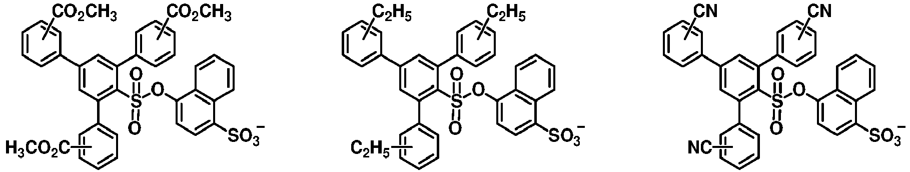

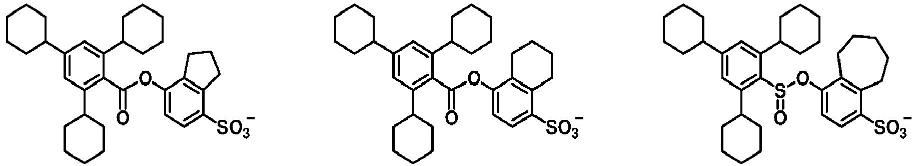

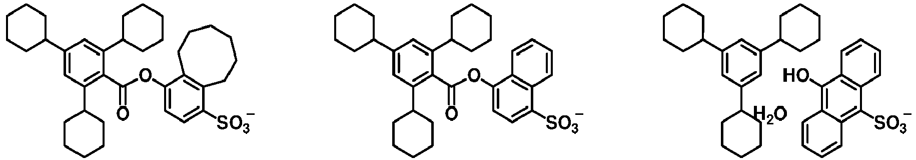

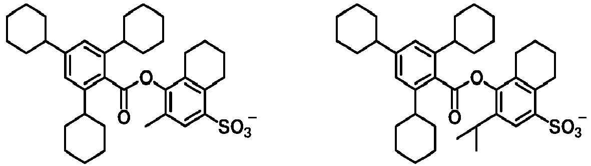

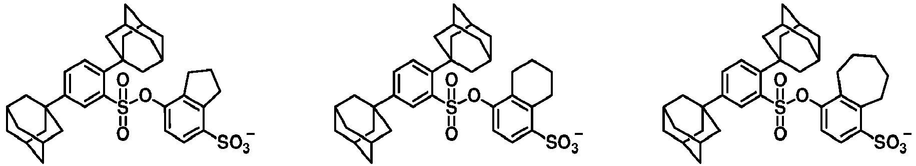

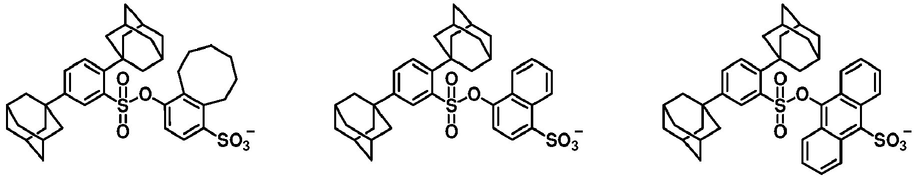

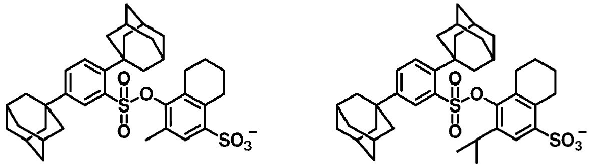

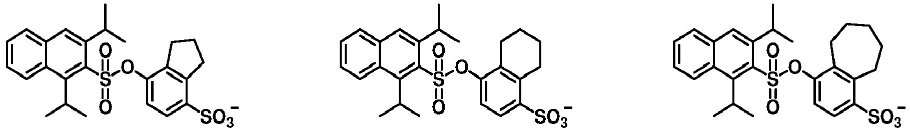

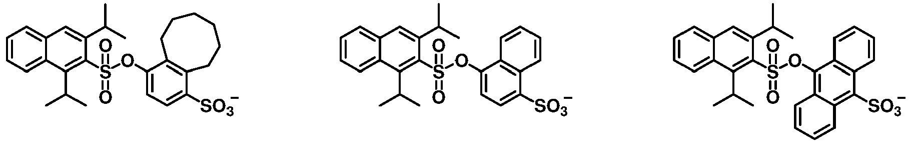

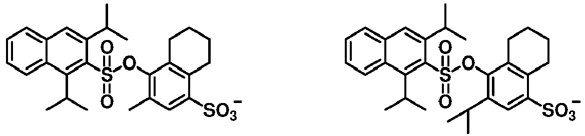

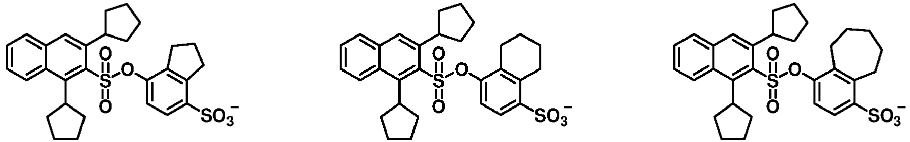

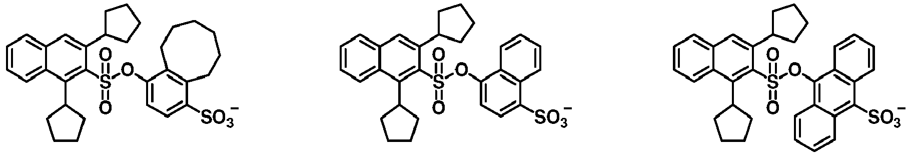

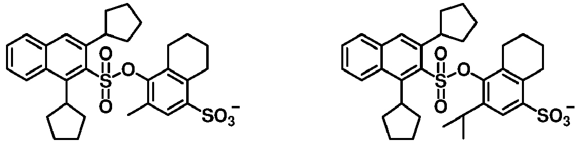

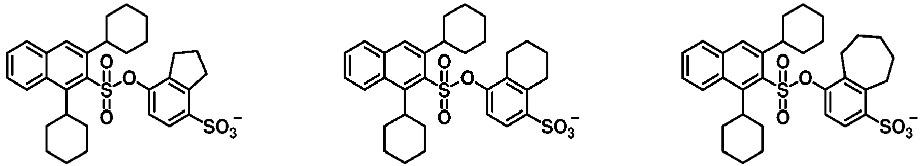

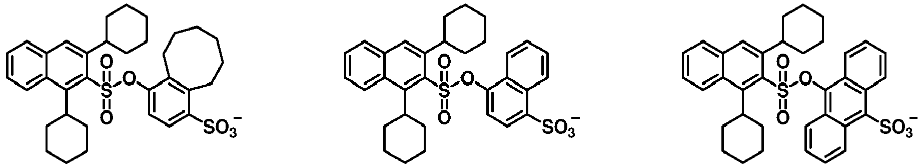

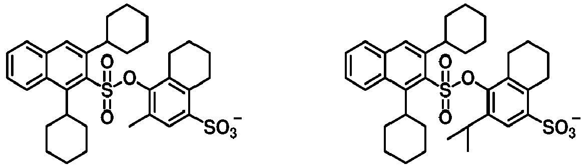

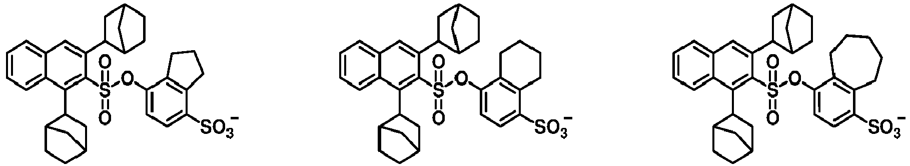

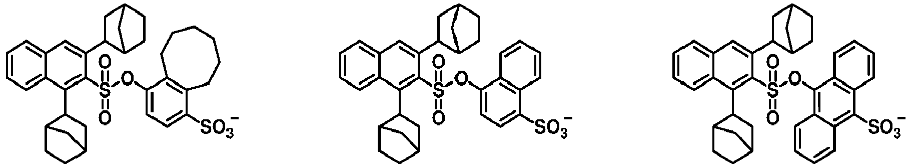

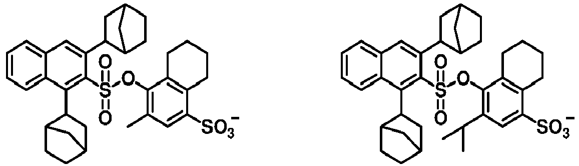

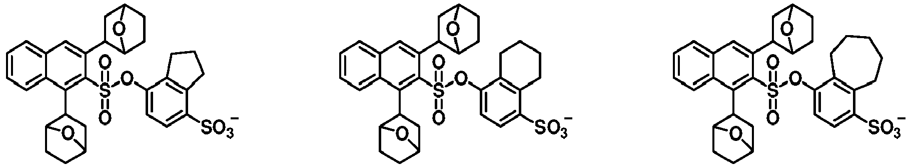

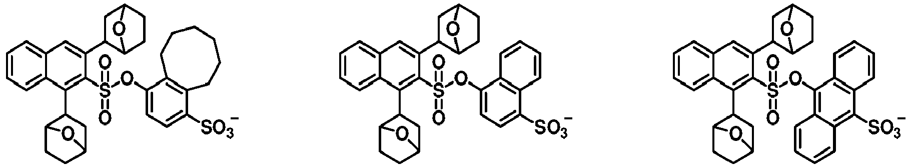

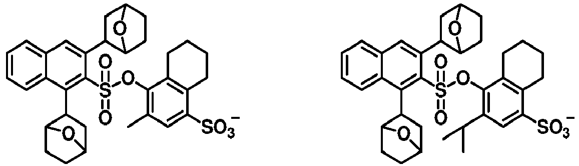

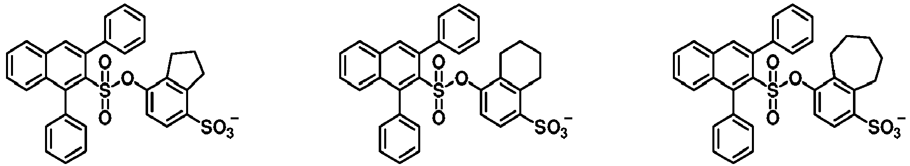

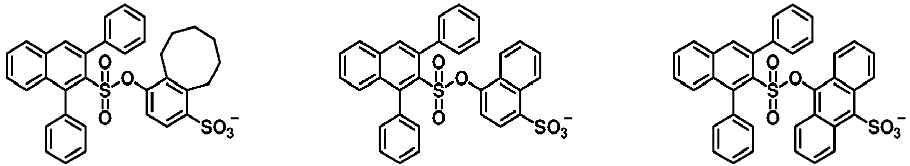

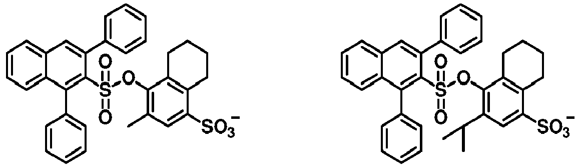

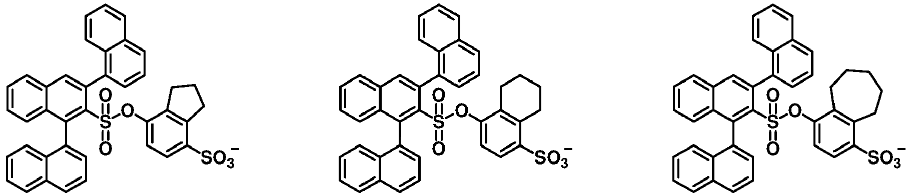

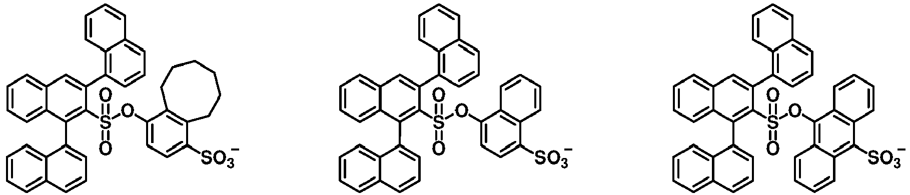

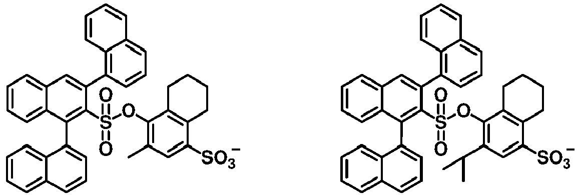

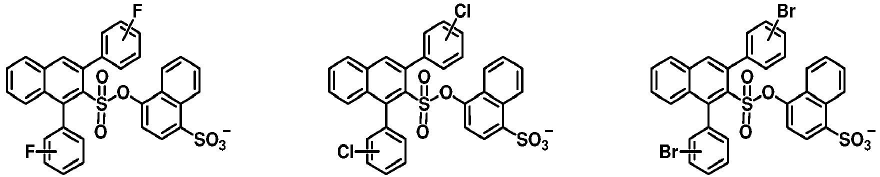

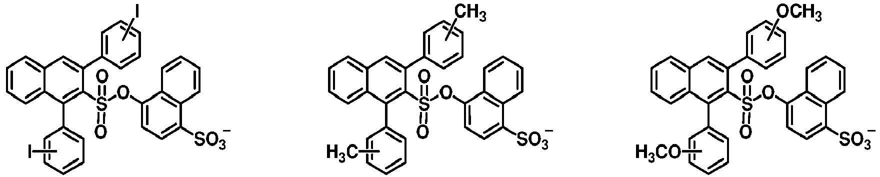

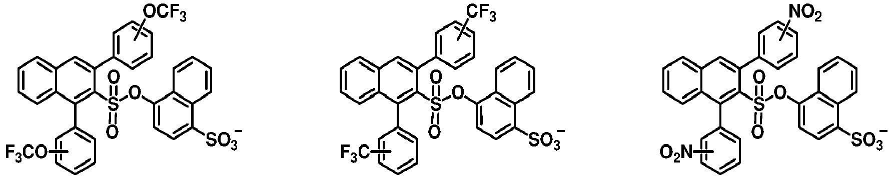

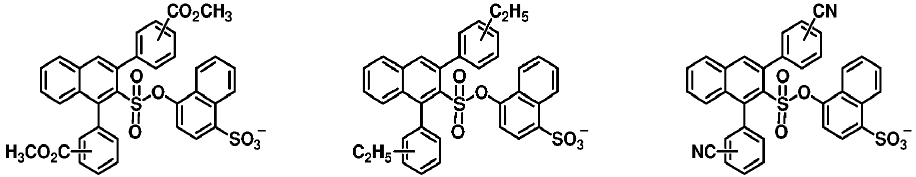

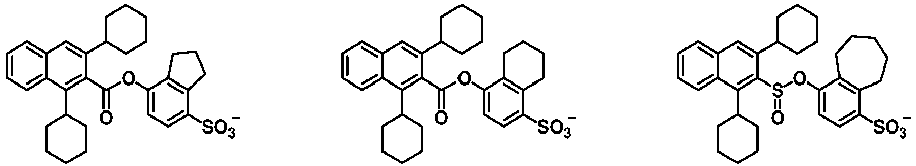

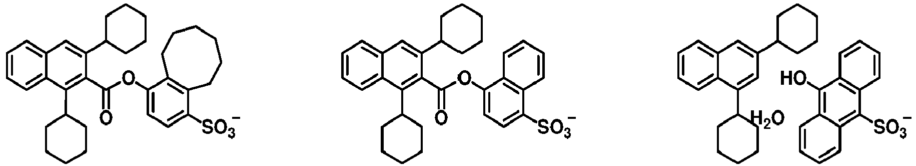

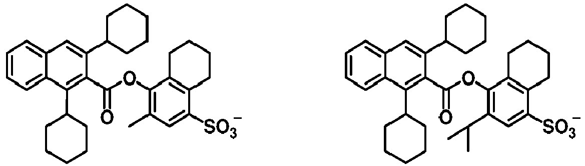

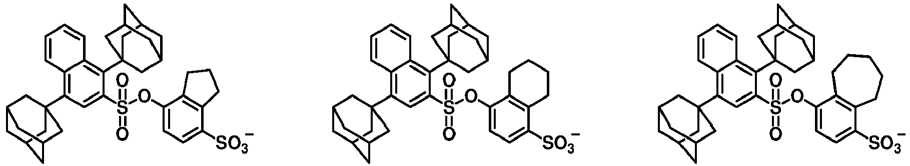

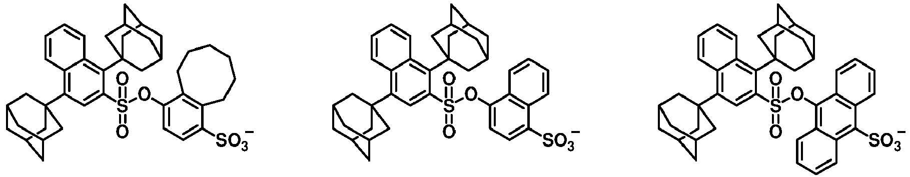

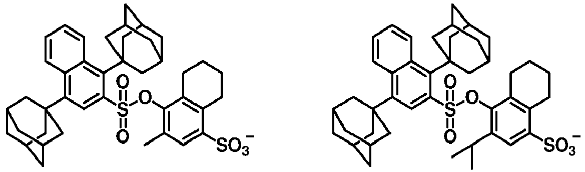

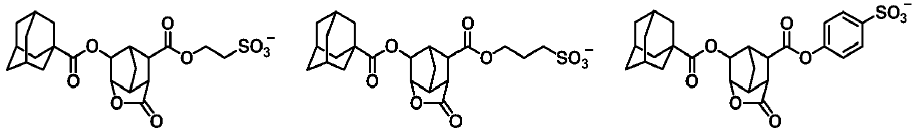

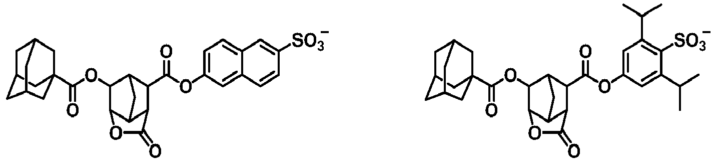

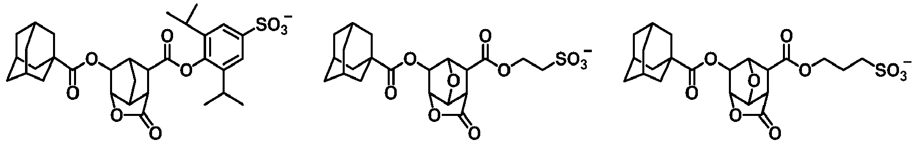

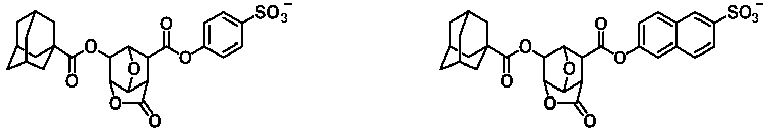

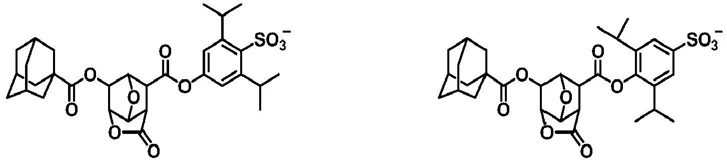

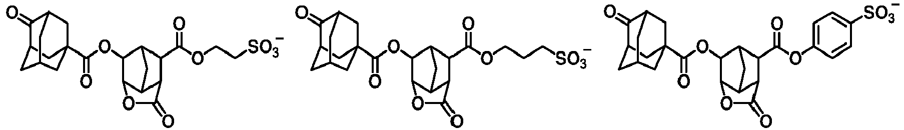

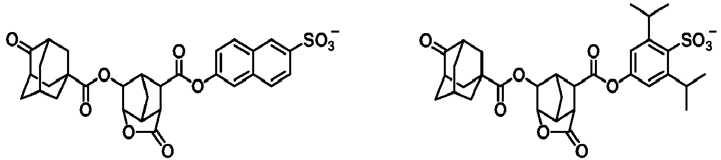

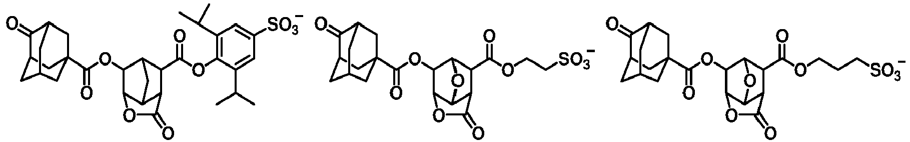

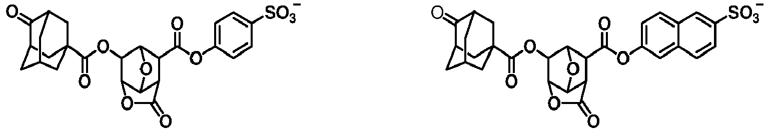

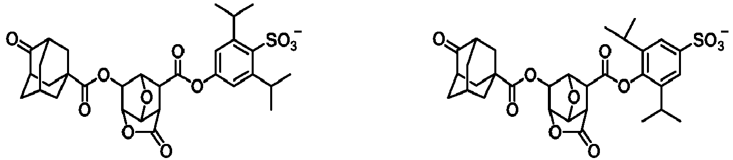

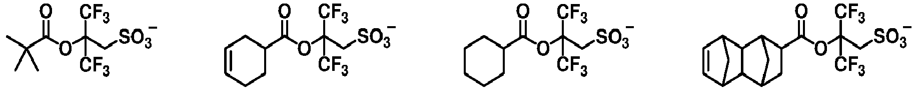

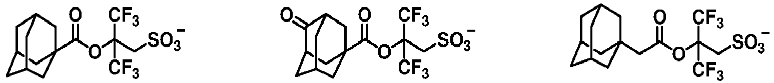

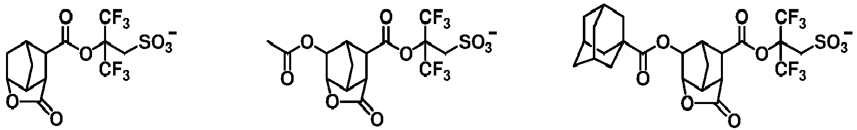

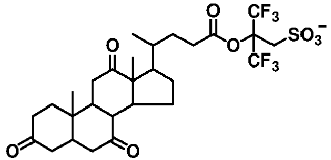

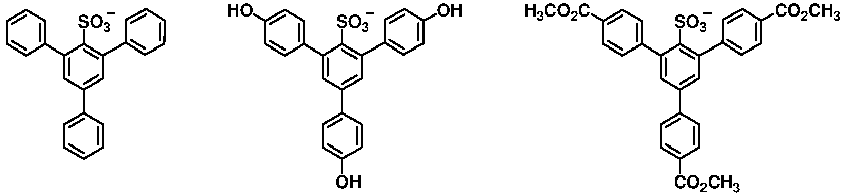

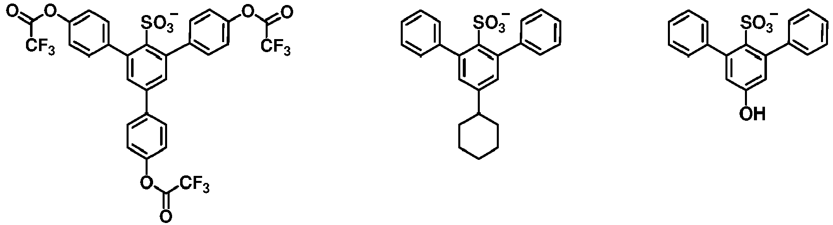

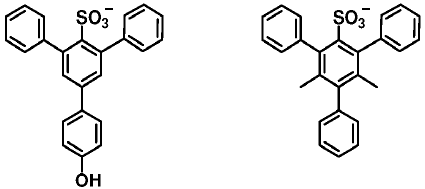

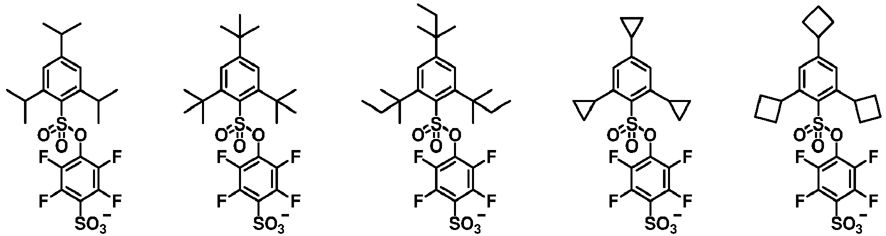

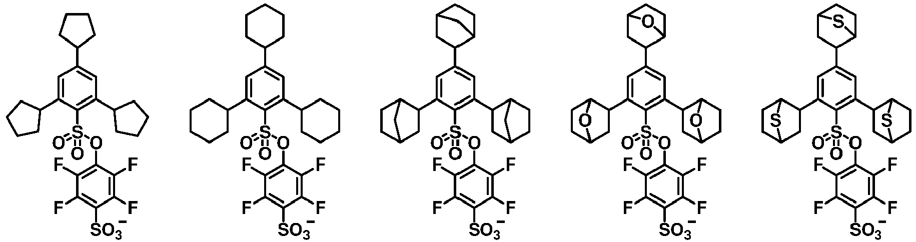

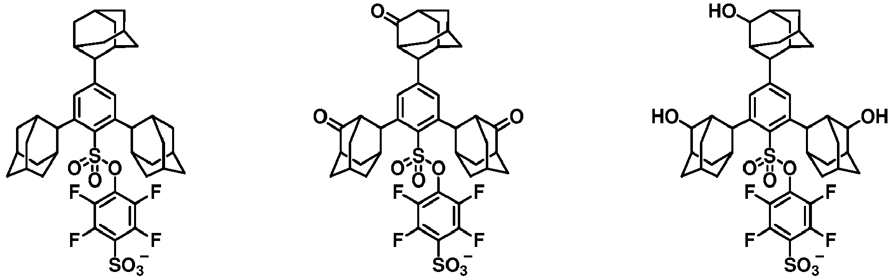

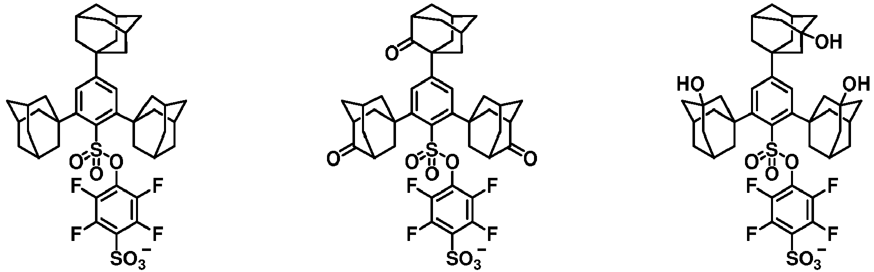

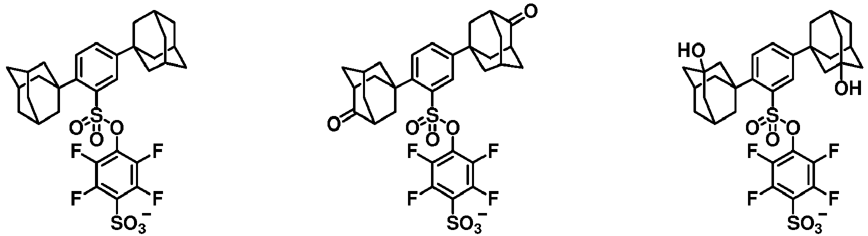

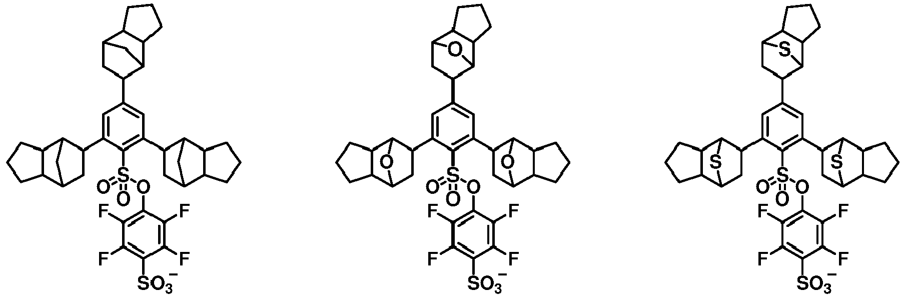

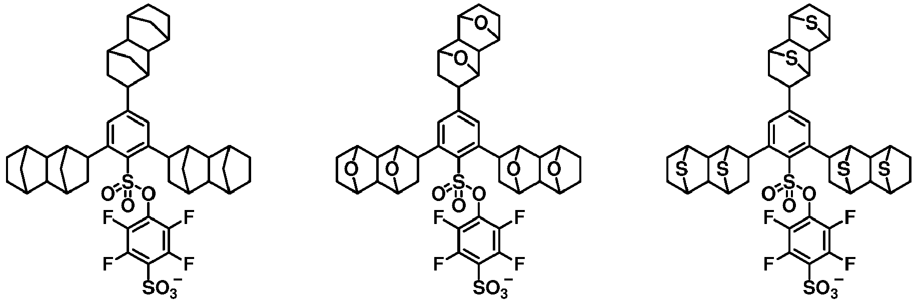

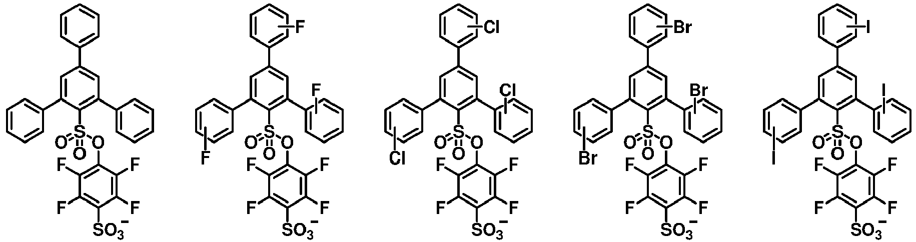

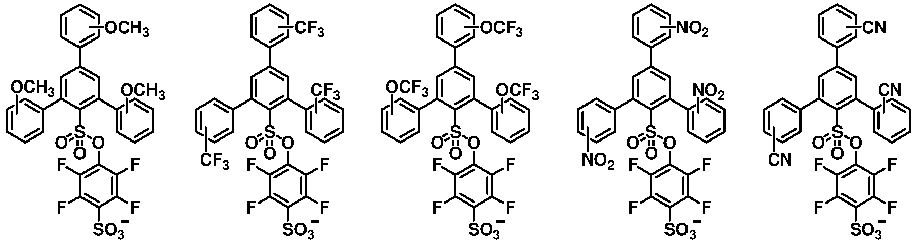

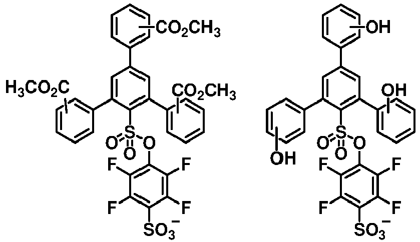

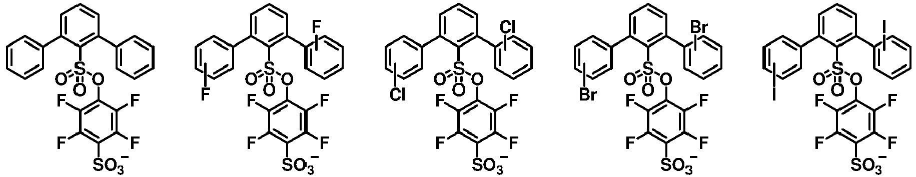

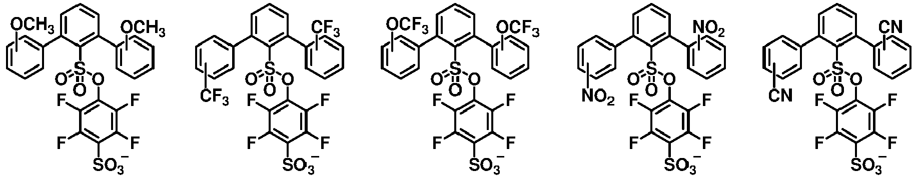

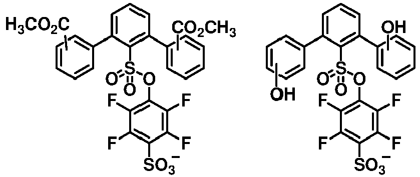

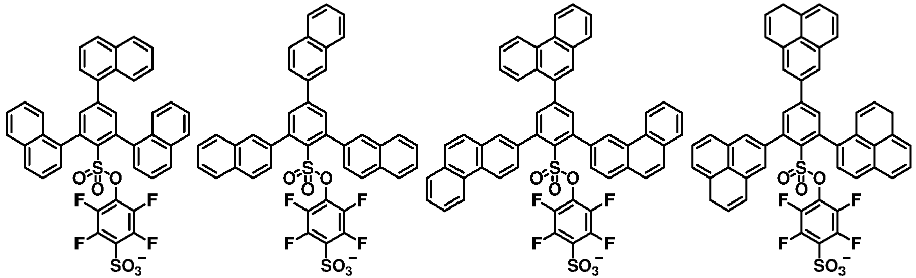

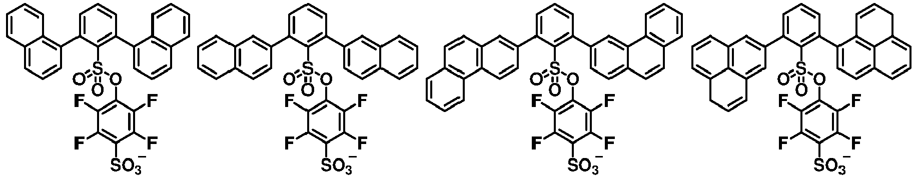

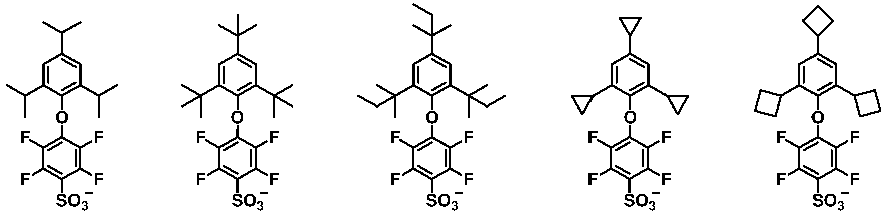

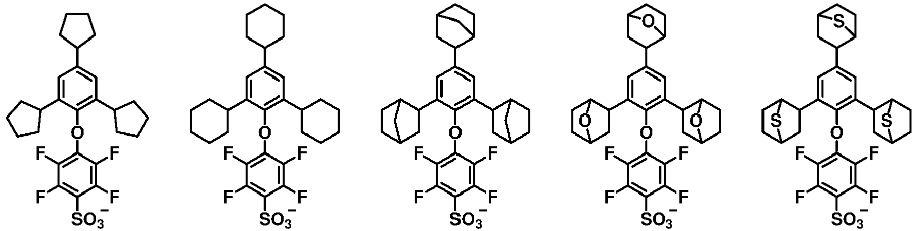

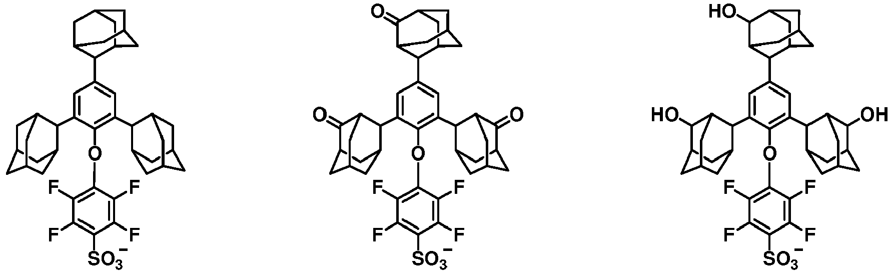

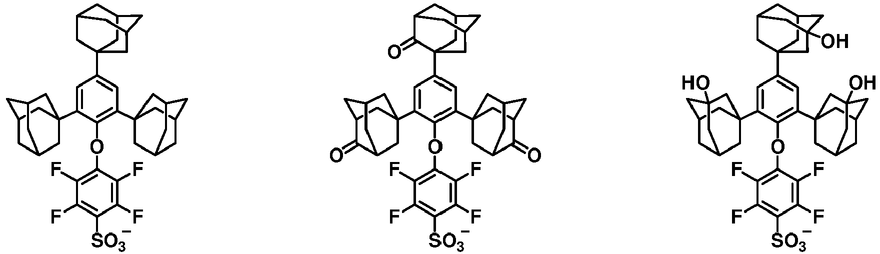

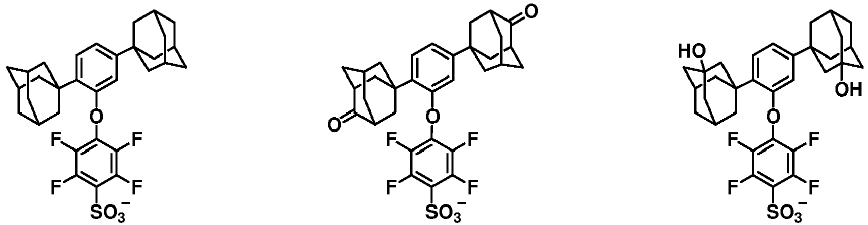

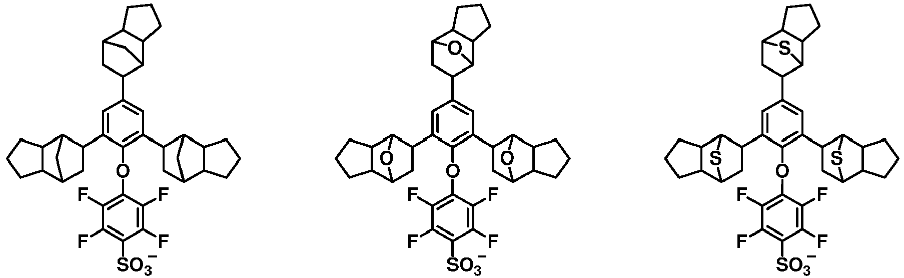

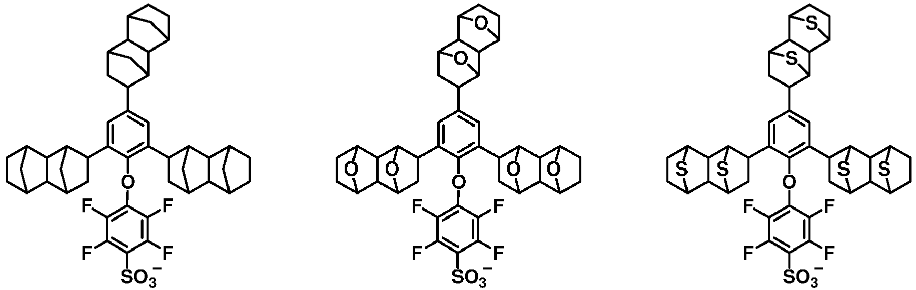

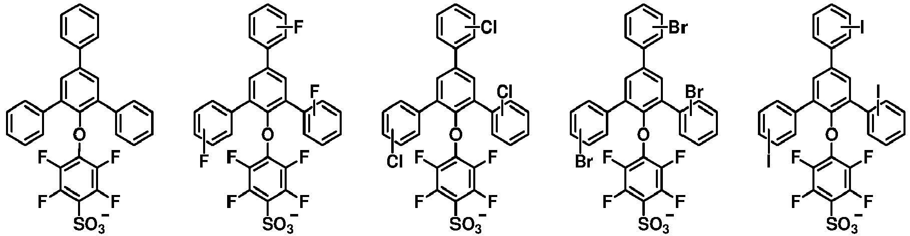

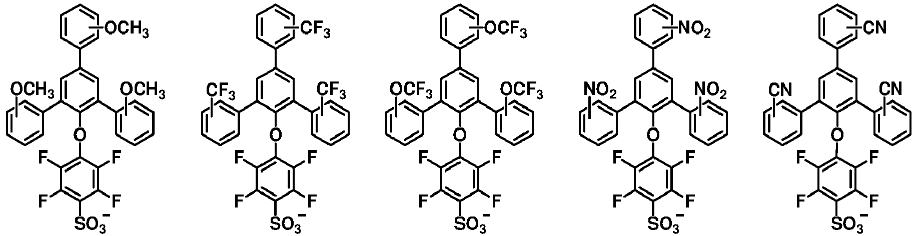

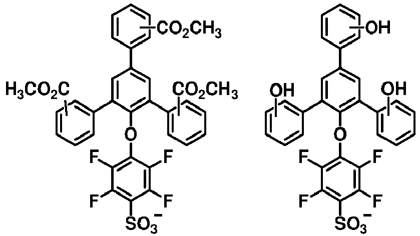

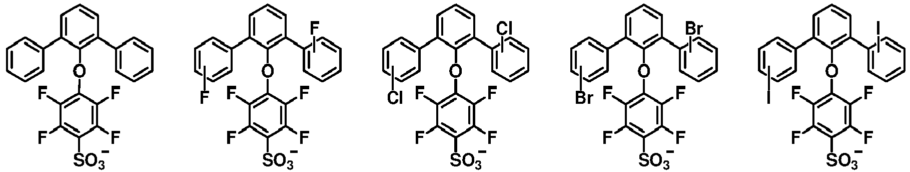

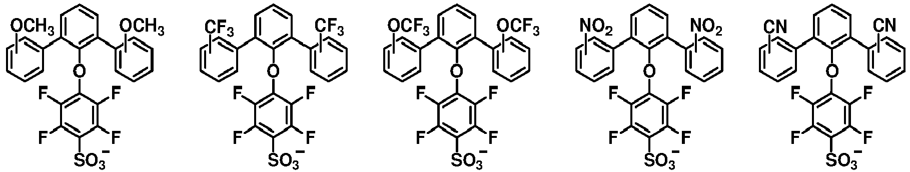

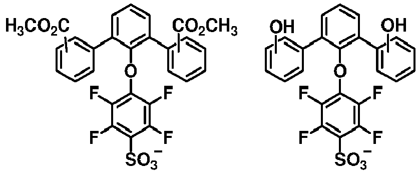

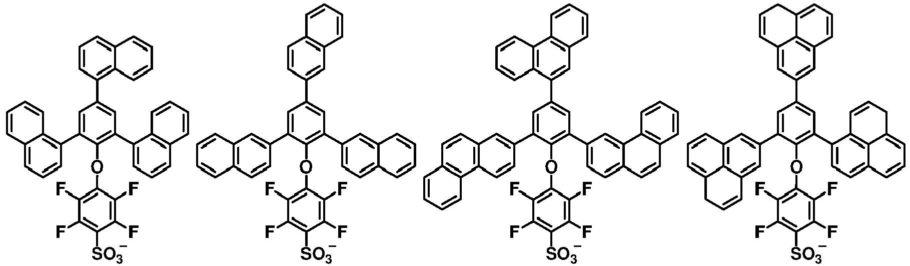

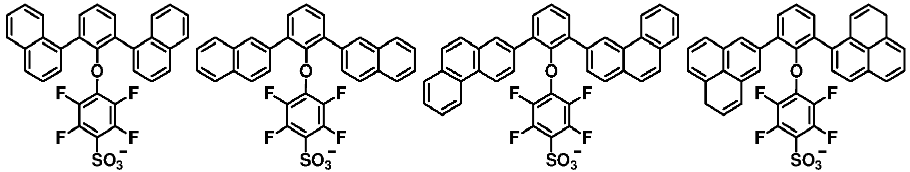

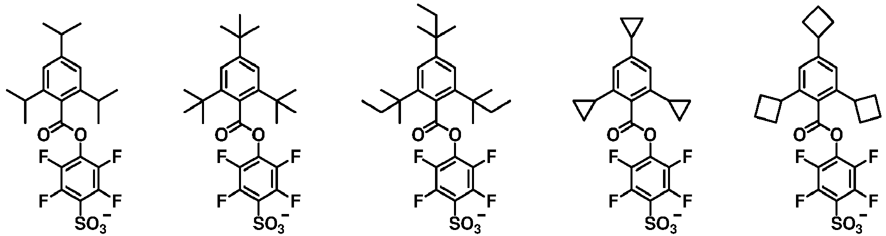

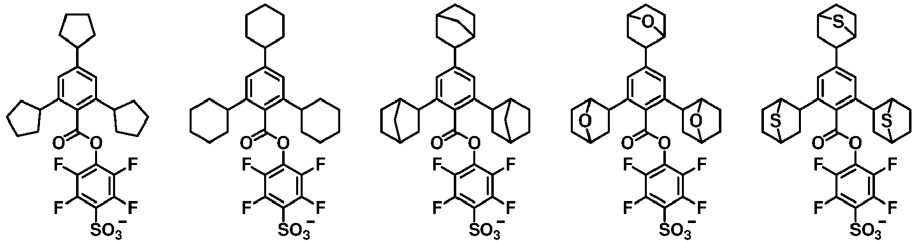

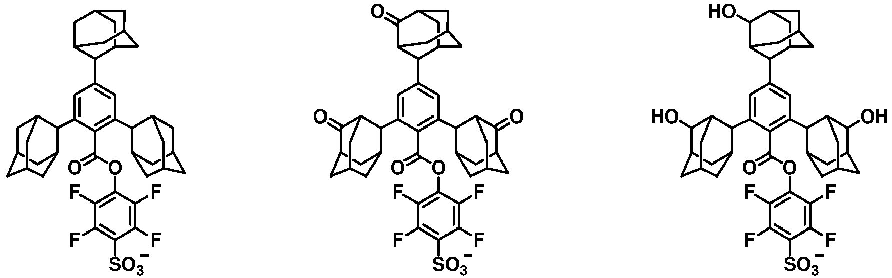

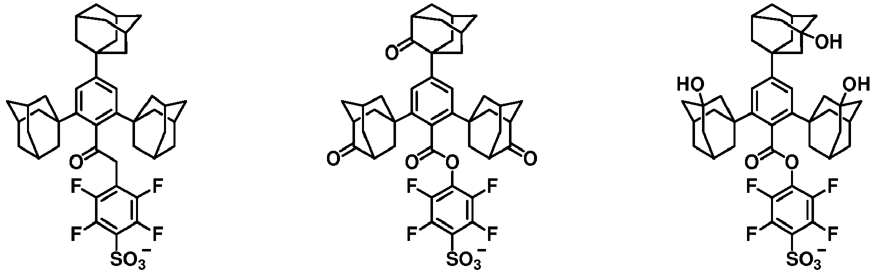

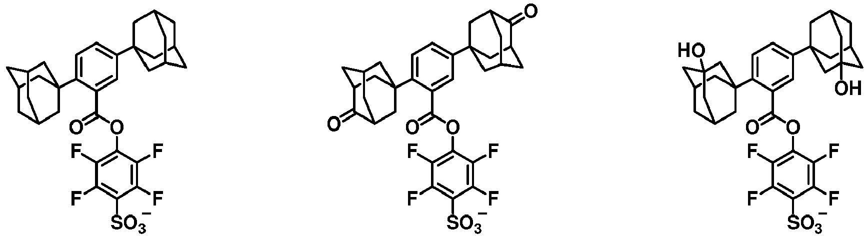

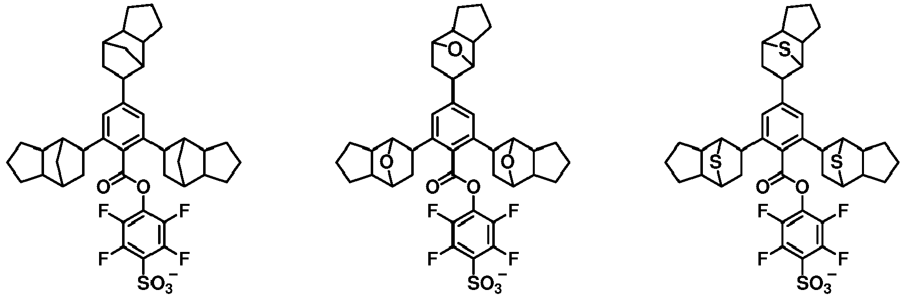

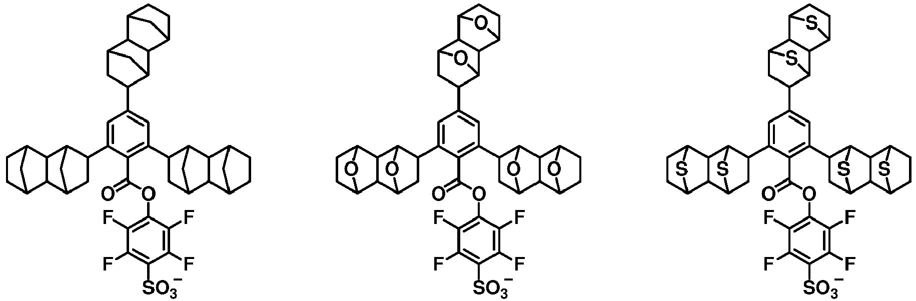

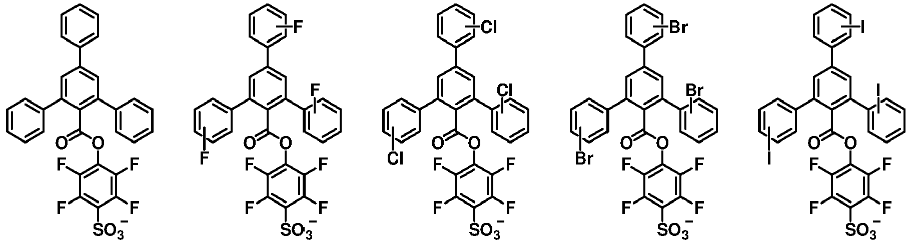

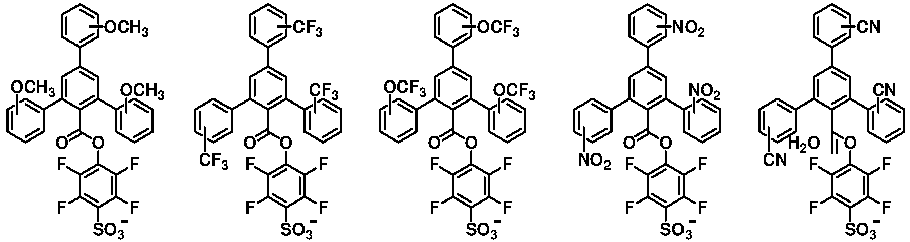

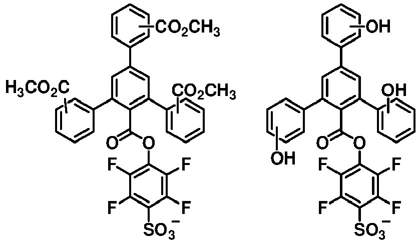

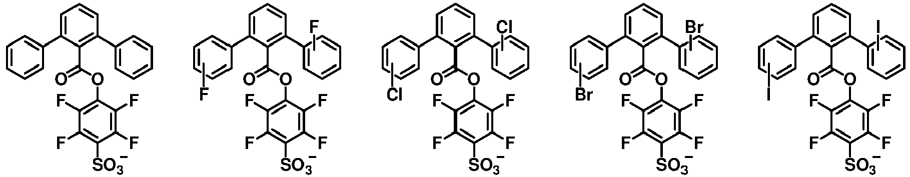

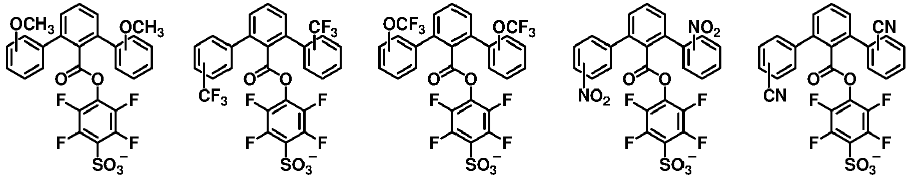

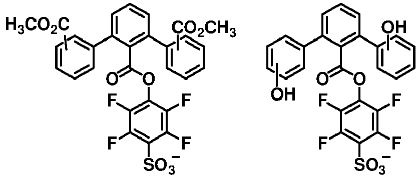

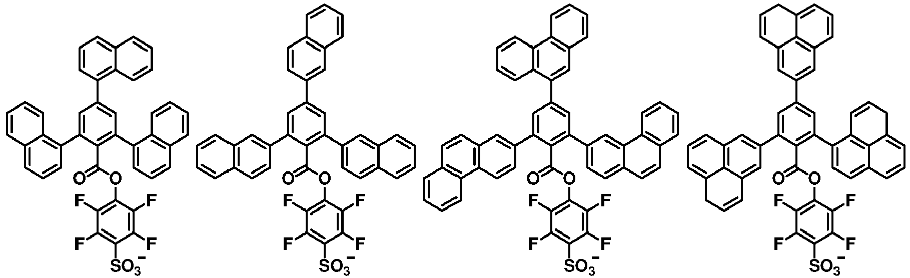

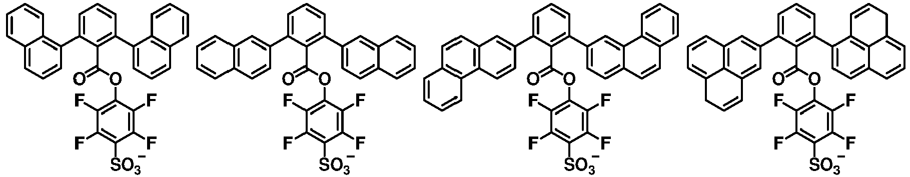

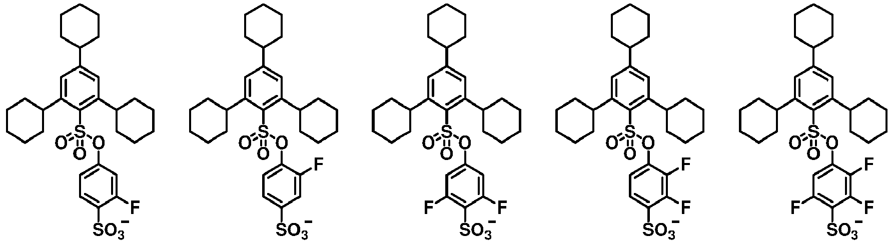

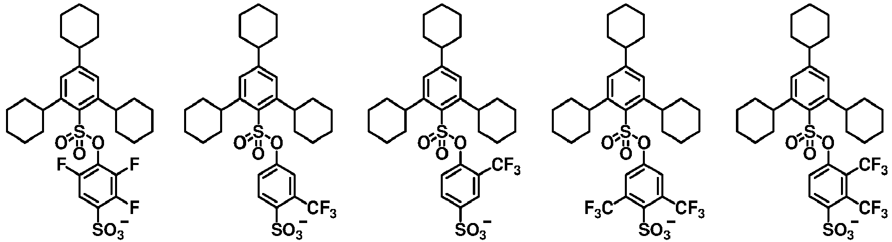

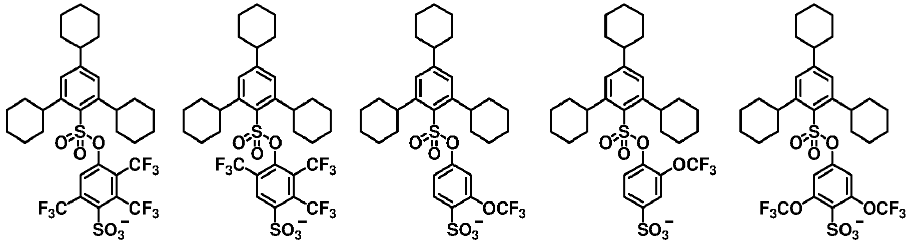

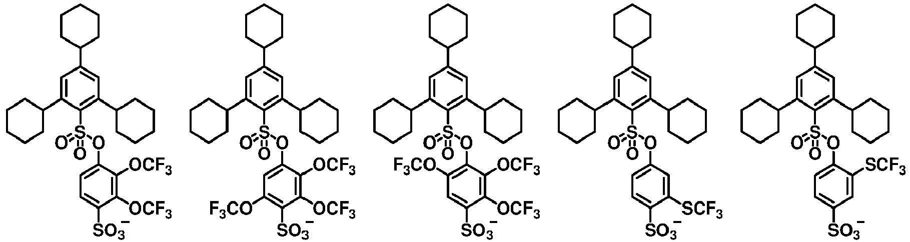

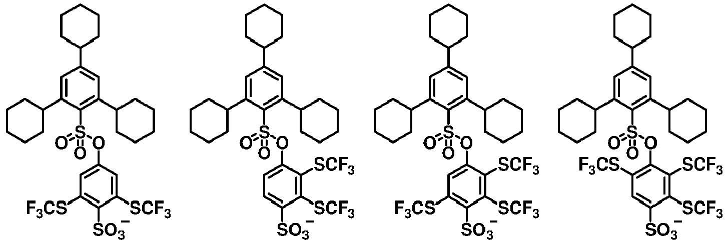

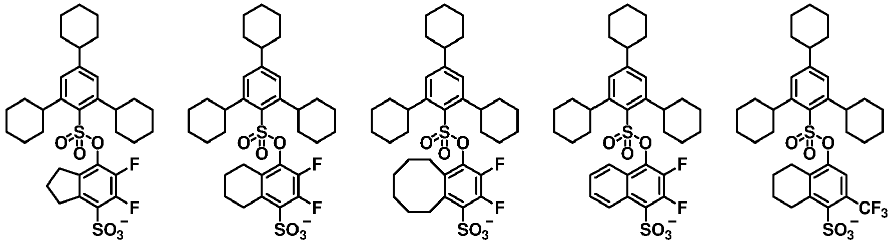

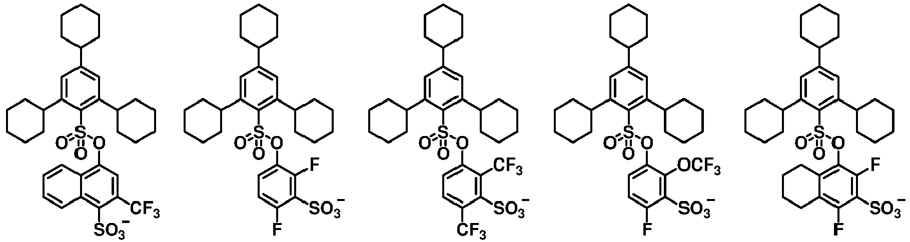

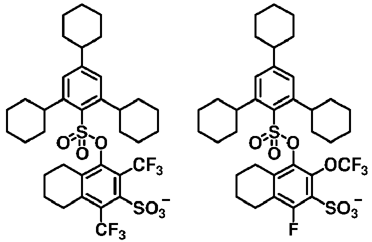

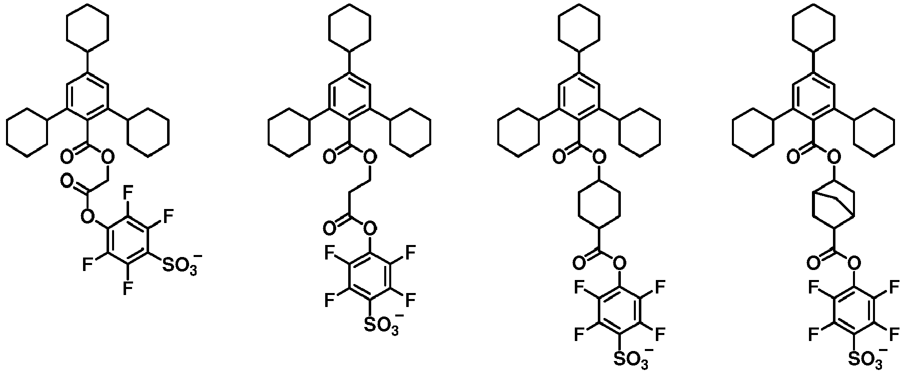

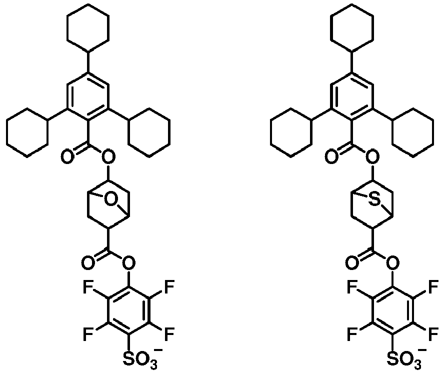

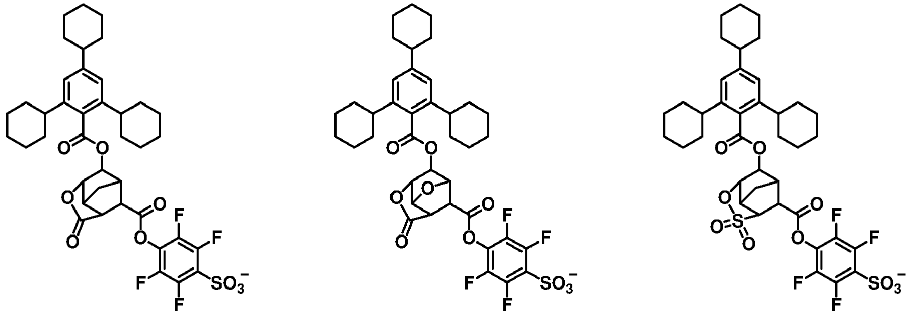

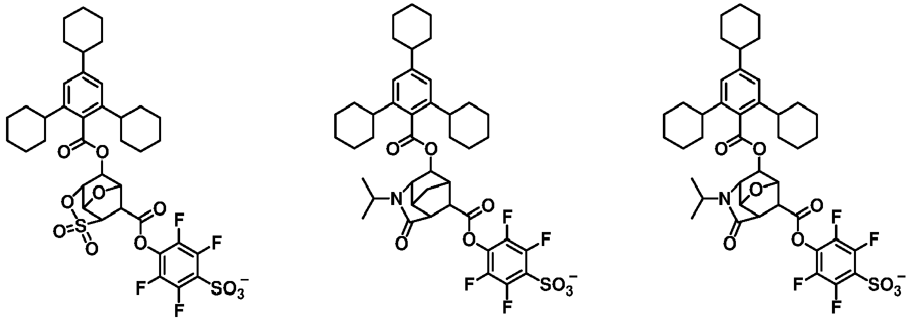

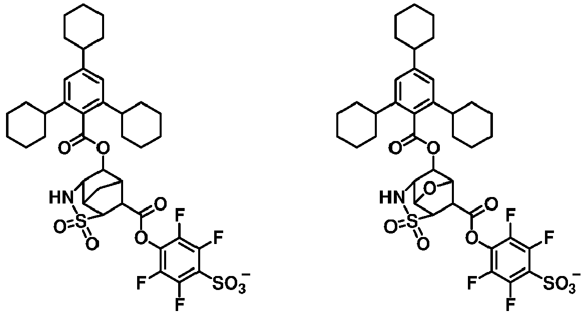

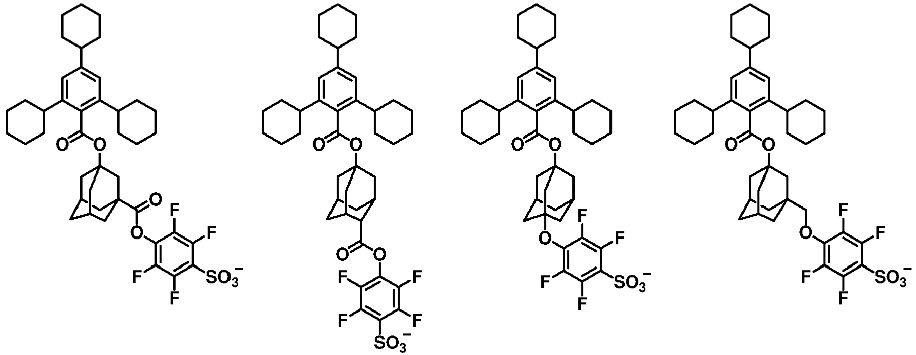

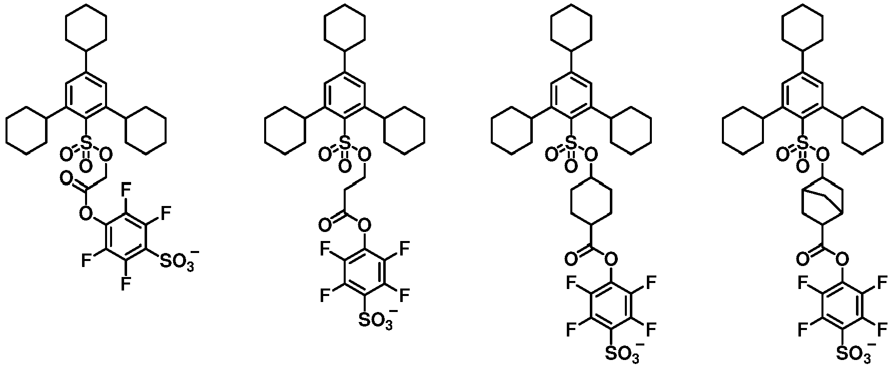

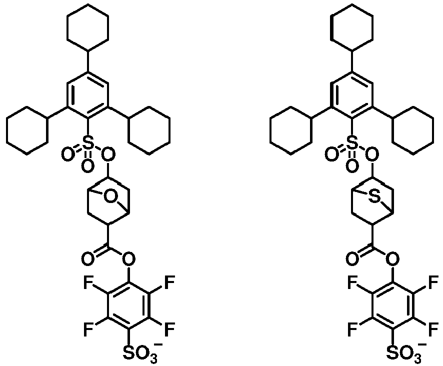

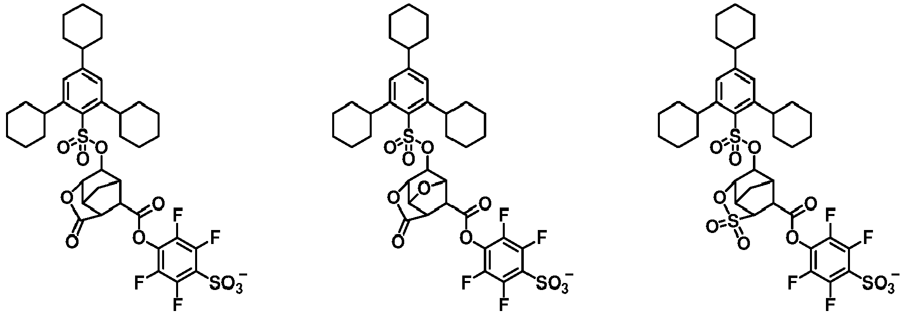

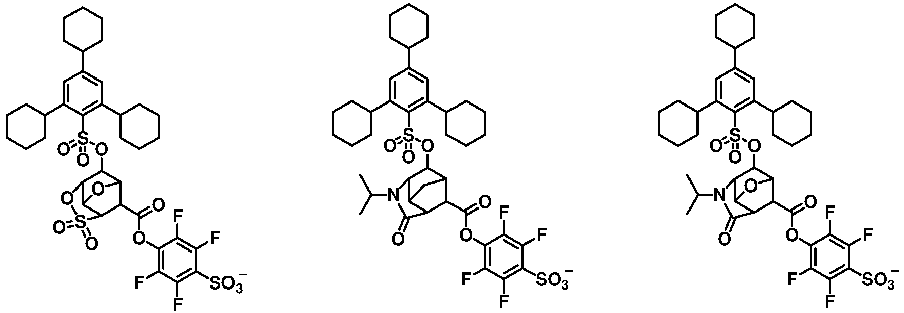

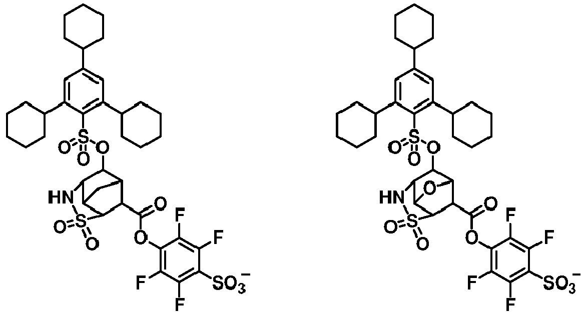

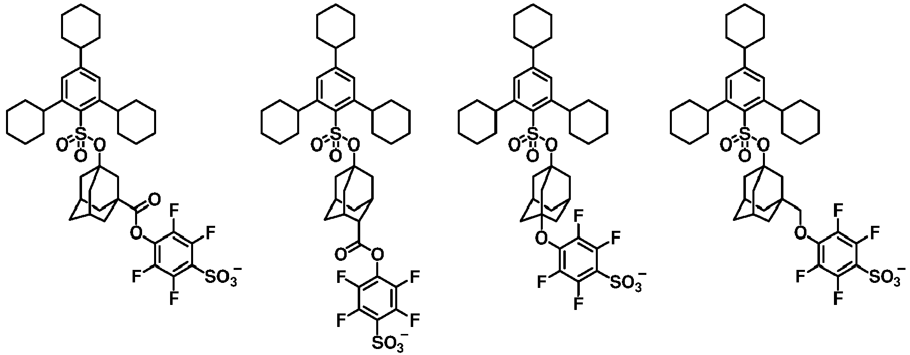

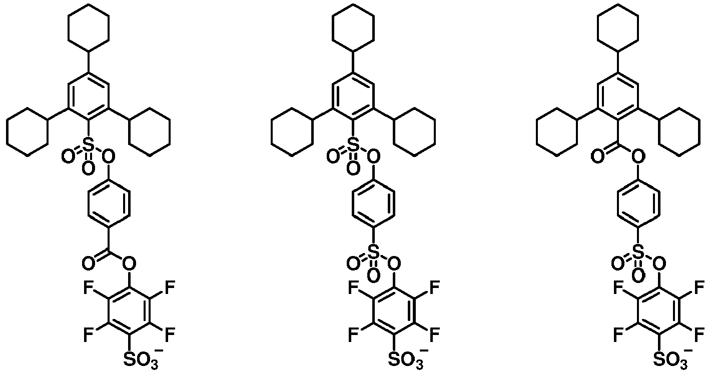

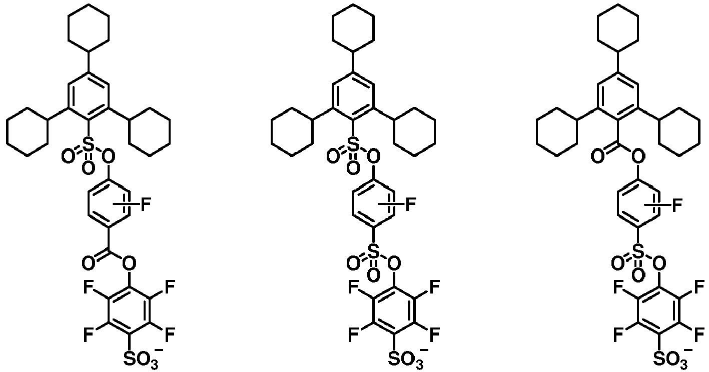

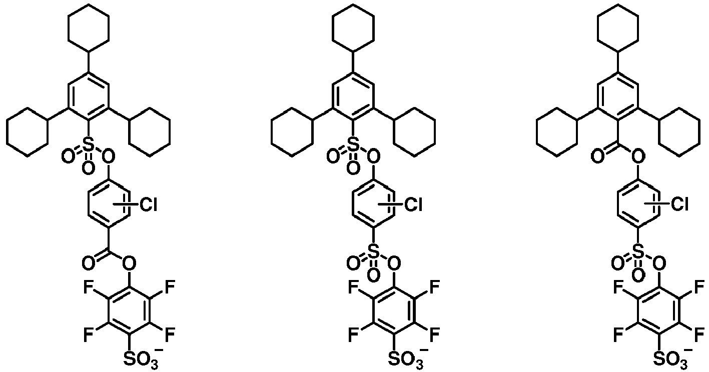

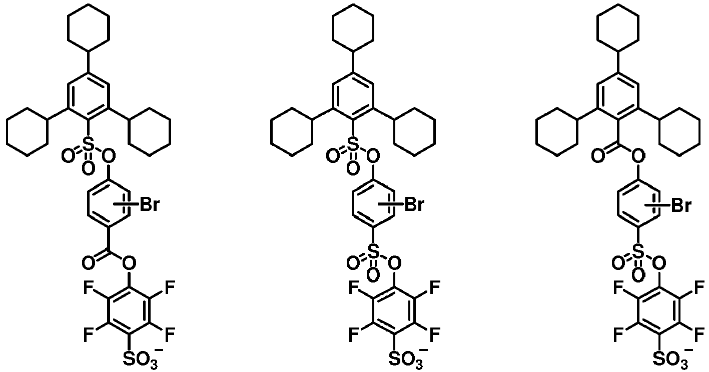

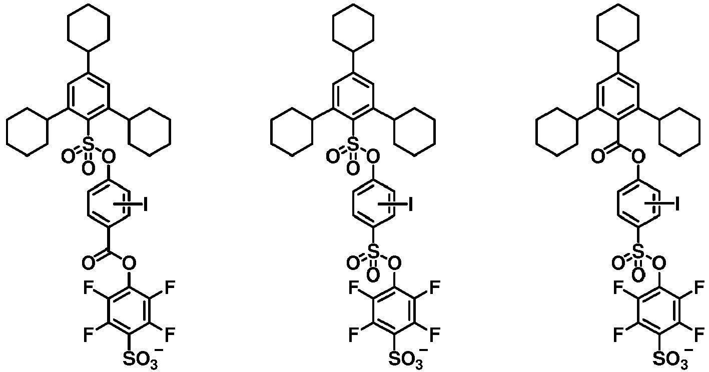

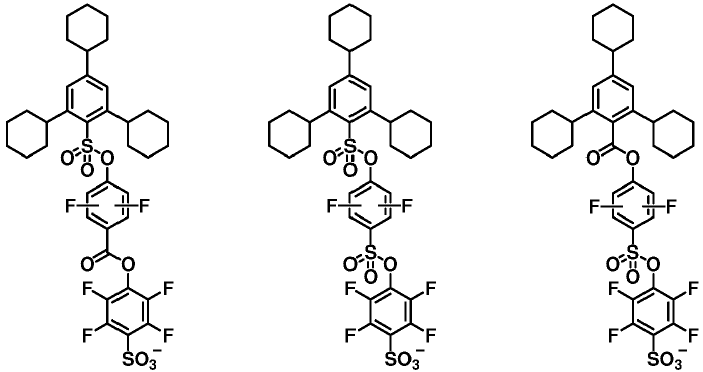

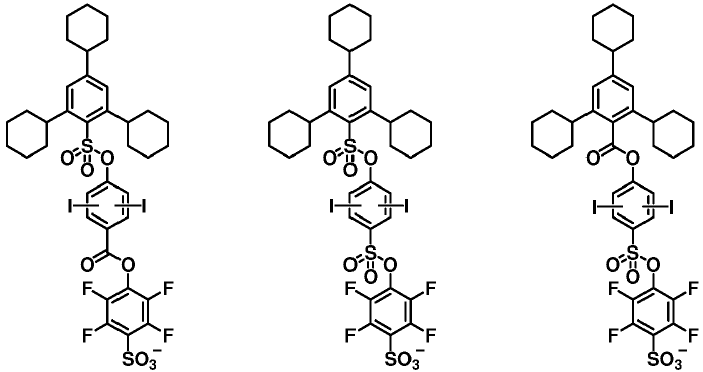

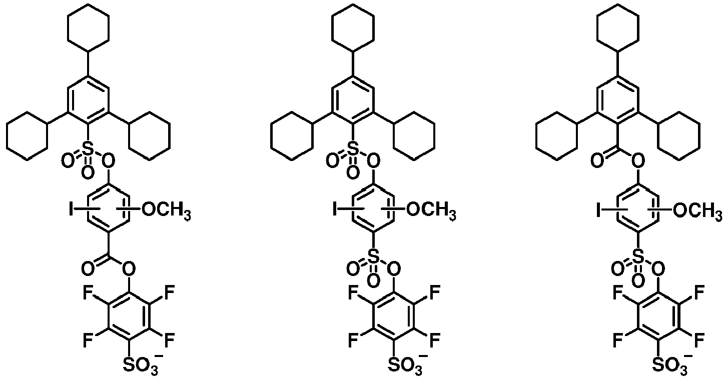

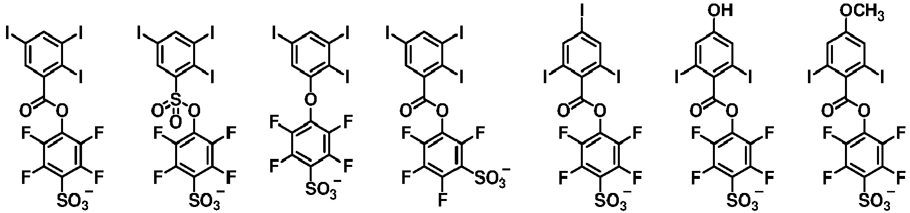

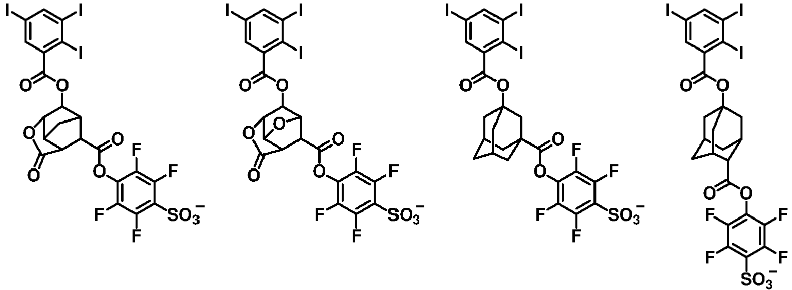

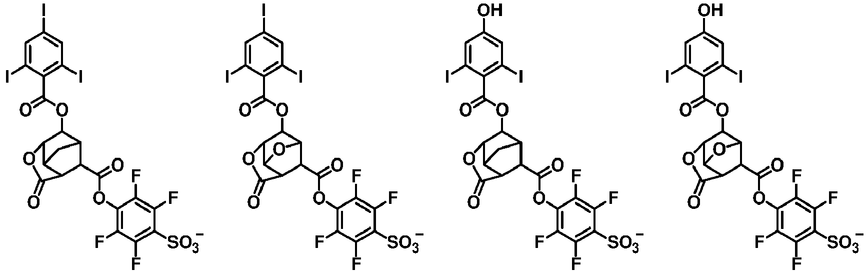

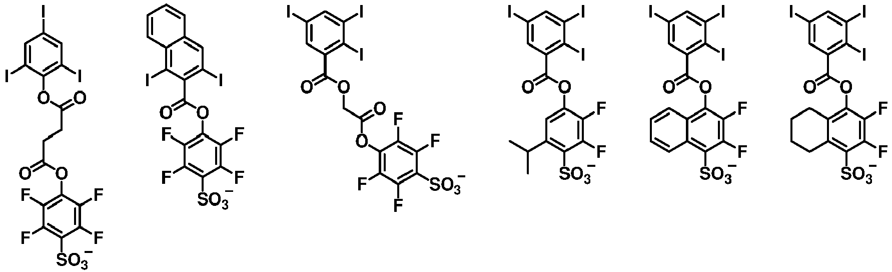

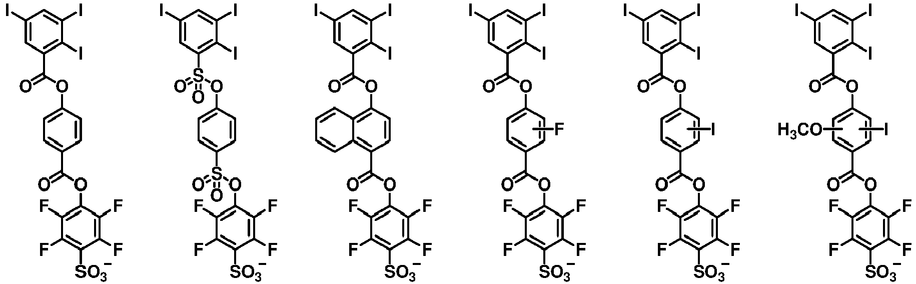

Also preferred as the PAG is a salt compound containing an anion having the formula (P1).

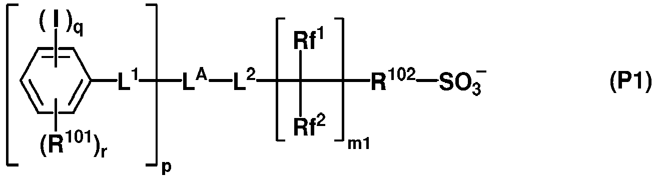

In formula (P1), m1 is 0 or 1, p is an integer of 1 to 3, q is an integer of 1 to 5, and r is an integer of 0 to 3.

In formula (P1), L¹ is a single bond, ether bond, ester bond, sulfonate ester bond, carbonate bond or carbamate bond.

In formula (P1), L² is an ether bond, ester bond, sulfonate ester bond, carbonate bond or carbamate bond.

In formula (P1), L^{A} is a single bond or a C₁-C₂₀ hydrocarbylene group when p is 1. L^{A} is a C₁-C₂₀ (p+1)-valent hydrocarbon group when p is 2 or 3. The hydrocarbylene group and (p+1)-valent hydrocarbon group may contain at least one moiety selected from ether bond, carbonyl moiety, ester bond, amide bond, sultone ring, lactam ring, carbonate bond, halogen, hydroxy moiety and carboxy moiety.

The C₁-C₂₀ hydrocarbylene group L^{A} may be saturated or unsaturated and straight, branched or cyclic. Examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (p+1)-valent hydrocarbon group L^{A} may be saturated or unsaturated and straight, branched or cyclic. Examples thereof include those exemplified above for the C₁-C₂₀ hydrocarbylene group, with one or two hydrogen atoms being eliminated.

In formula (P1), Rf¹ and Rf² are each independently hydrogen, fluorine or trifluoromethyl, at least one being fluorine or trifluoromethyl.

In formula (P1), R¹⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{101A})(R^{101B})-, -N(R^{101C})-C(=O)-R^{101D} or -N(R^{101C})-C(=O)-O-R^{101D}. R^{101A} and R^{101B} are each independently hydrogen or a C₁-C₆ saturated hydrocarbyl group. R^{101C} is hydrogen or a C₁-C₆ saturated hydrocarbyl group. R^{101D} is a C₁-C₆ saturated hydrocarbyl group or C₂-C₈ unsaturated aliphatic hydrocarbyl group.

The C₁-C₆ saturated hydrocarbyl group represented by R¹⁰¹, R^{101A}, R^{101B} and R^{101C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R¹⁰¹ are as exemplified above for the saturated hydrocarbyl group. Examples of the saturated hydrocarbyl moiety in the C₂-C₆ saturated hydrocarbylcarbonyloxy group represented by R¹⁰¹ are as exemplified above for the C₁-C₆ saturated hydrocarbyl group, but of 1 to 5 carbon atoms.

The C₂-C₈ unsaturated aliphatic hydrocarbyl group represented by R^{101D} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (P1), R¹⁰² is a C₁-C₂₀ saturated hydrocarbylene group or C₆-C₂₀ arylene group. Some or all of the hydrogen atoms in the saturated hydrocarbylene group may be substituted by halogen exclusive of fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₂₀ aryl groups, halogen, and hydroxy.

The C₁-C₂₀ hydrocarbylene group represented by R¹⁰² may be saturated or unsaturated and straight, branched or cyclic. Examples thereof are as exemplified above for the C₁-C₂₀ hydrocarbylene group L^{A}.

Examples of the C₆-C₂₀ arylene group represented by R¹⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

More preferably, the anion has the formula (P2).

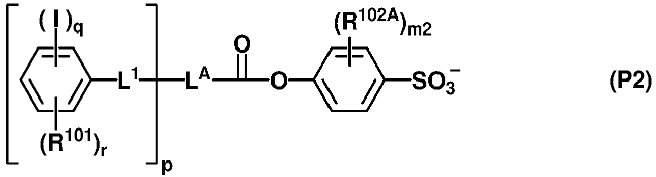

In formula (P2), p, q, r, L¹, L^{A}, and R¹⁰¹ are as defined above. The subscript m2 is an integer of 1 to 4. R^{102A} is a C₁-C₂₀ saturated hydrocarbyl group, C₁-C₂₀ saturated hydrocarbyloxy group, C₆-C₁₄ aryl group, halogen or hydroxy group. When m2 is 2, 3 or 4, a plurality of R^{102A} may be identical or different.

Examples of the anion having formula (P1) are shown below, but not limited thereto.

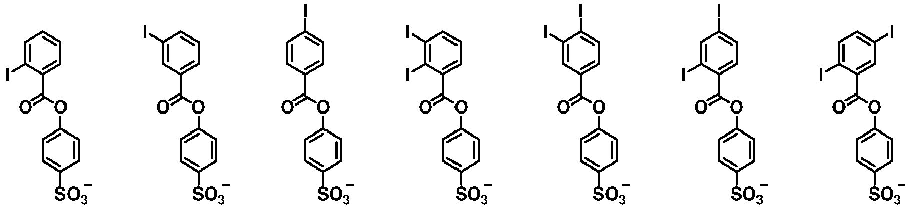

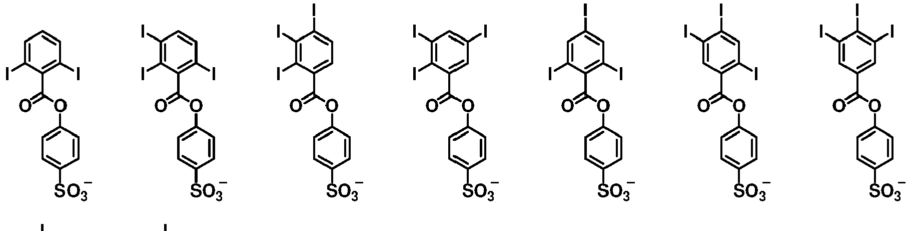

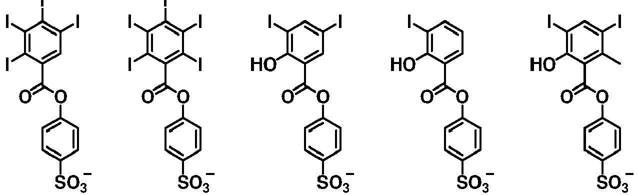

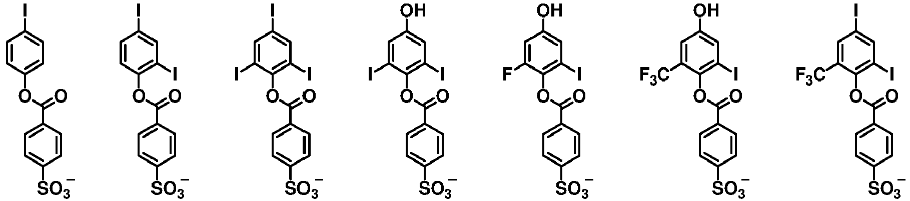

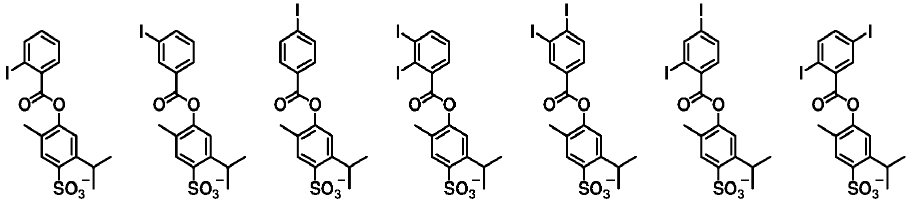

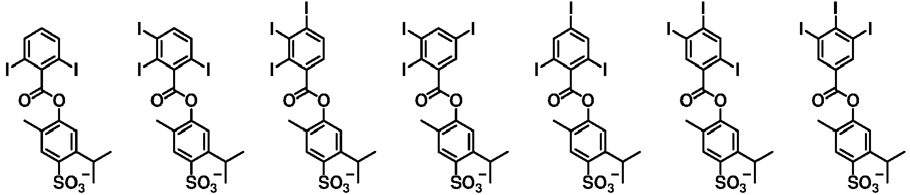

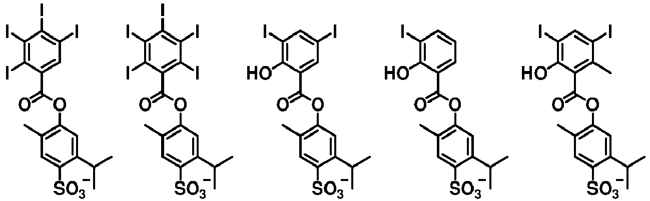

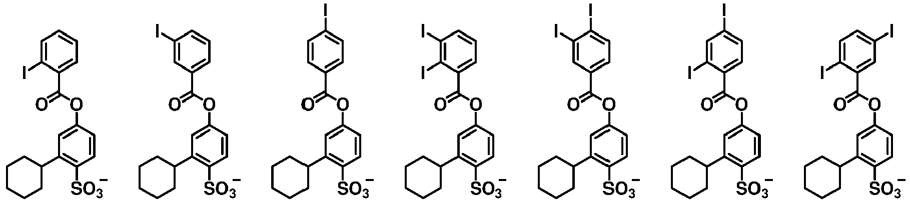

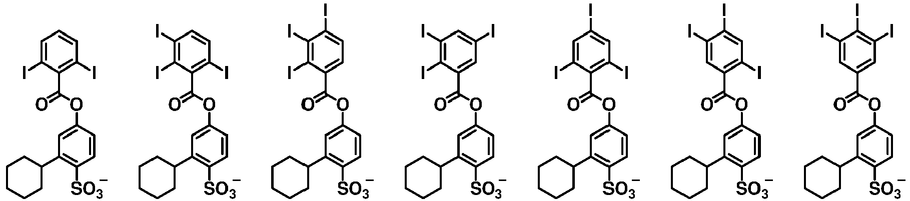

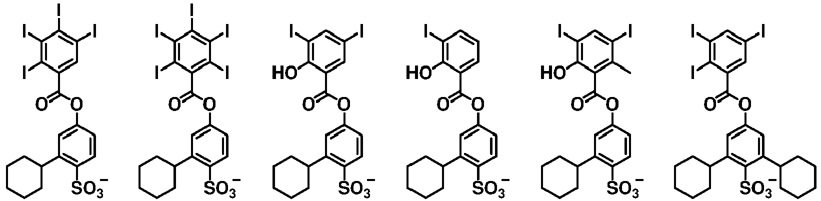

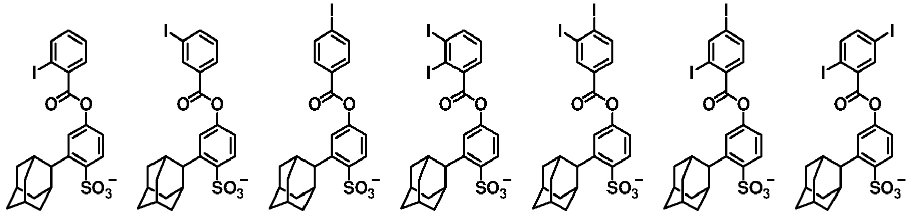

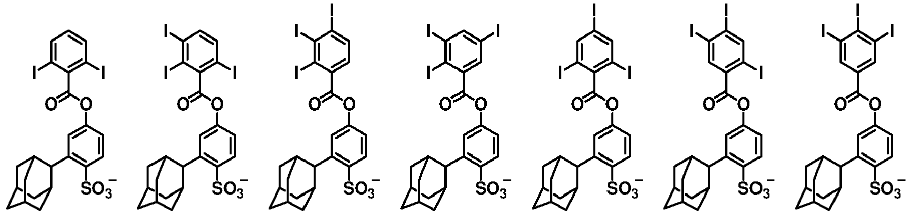

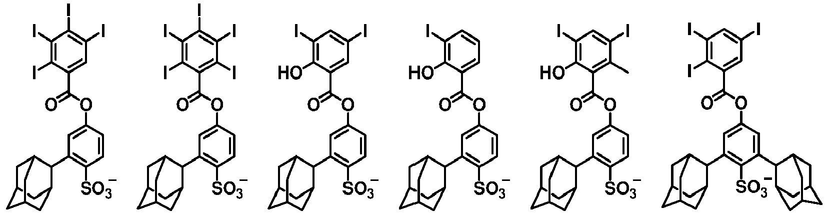

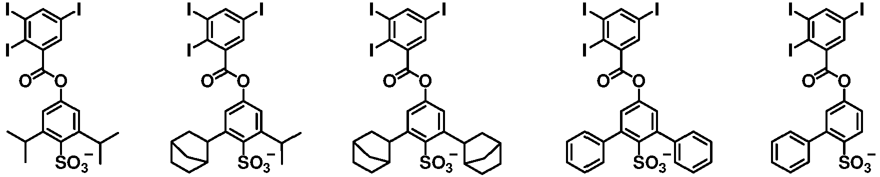

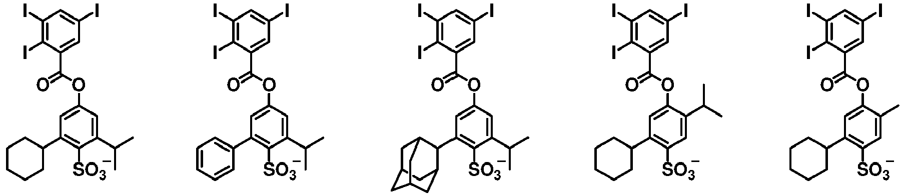

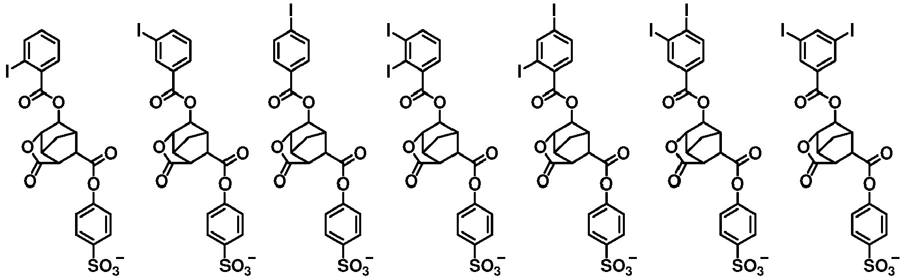

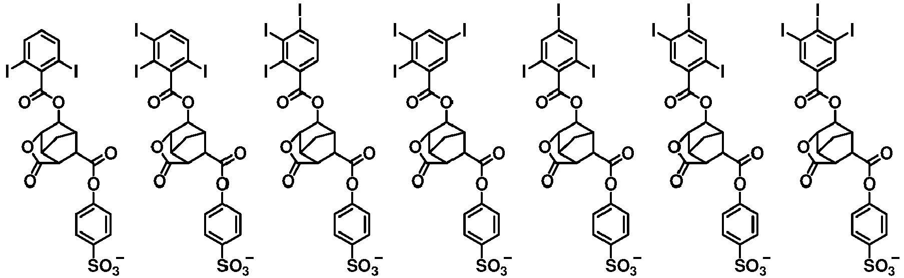

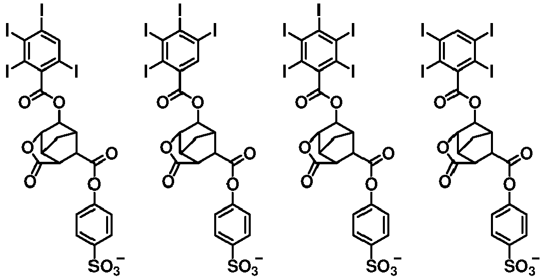

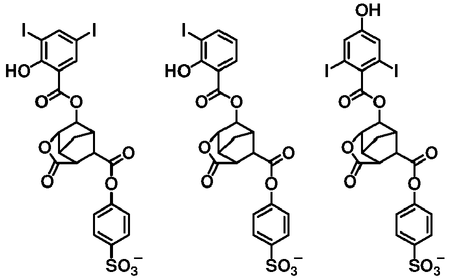

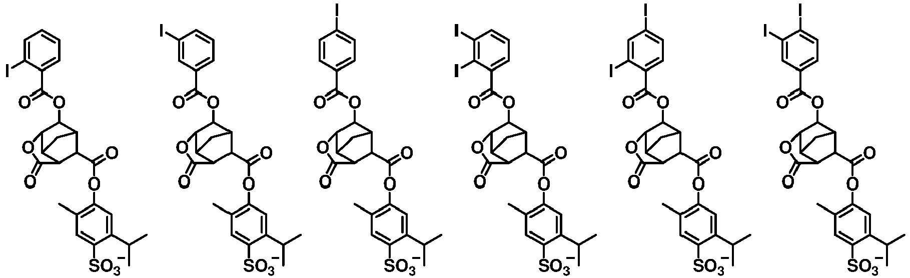

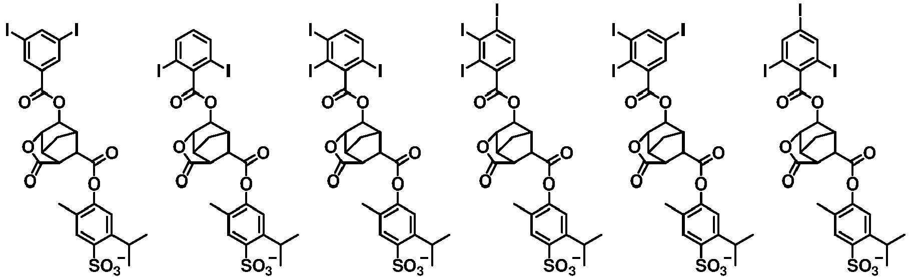

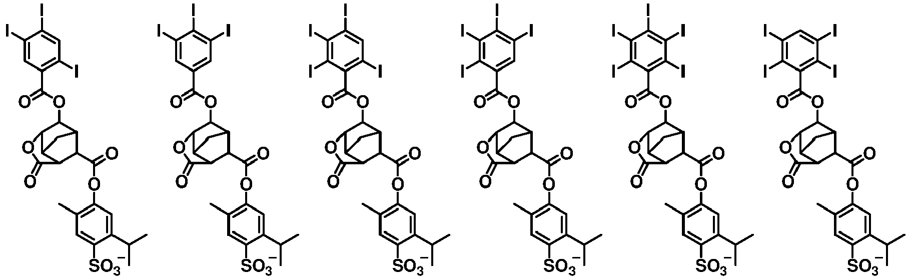

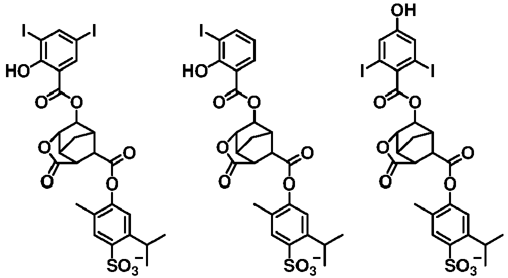

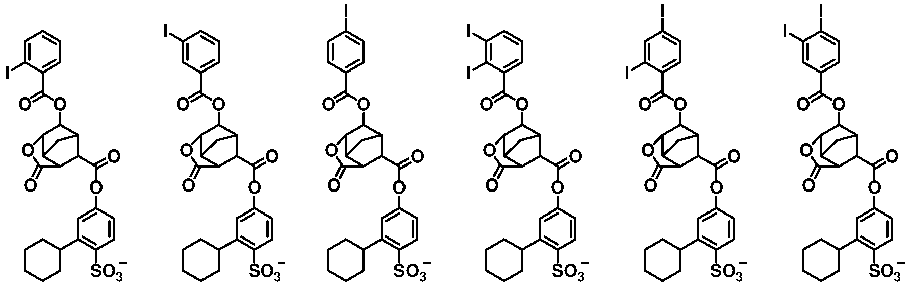

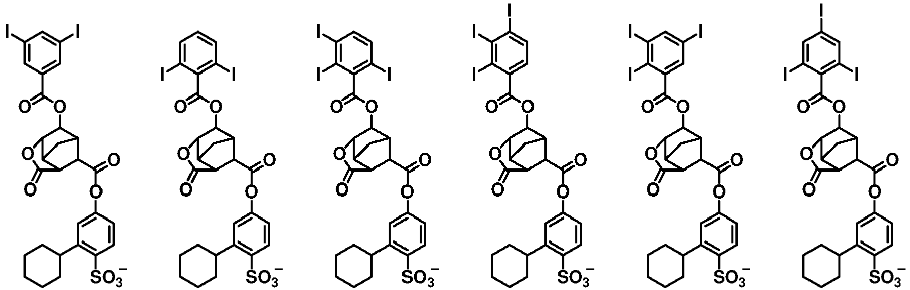

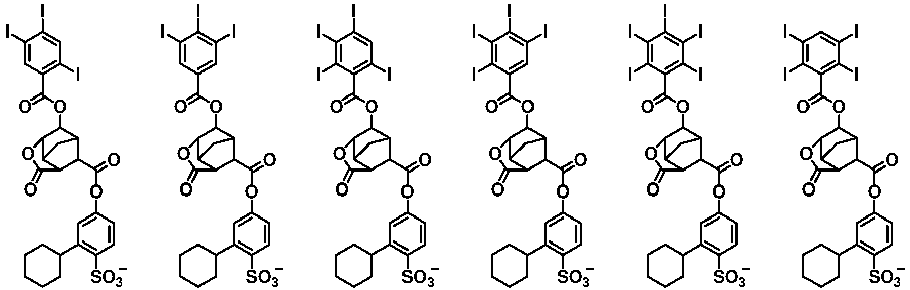

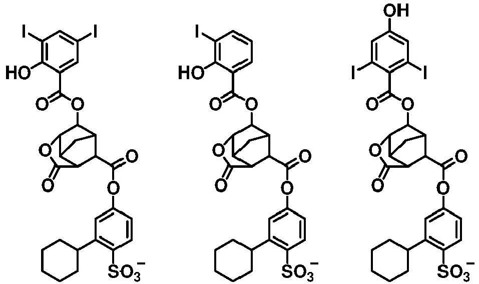

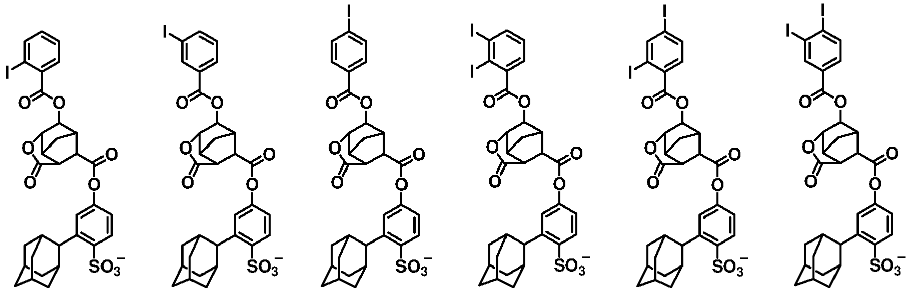

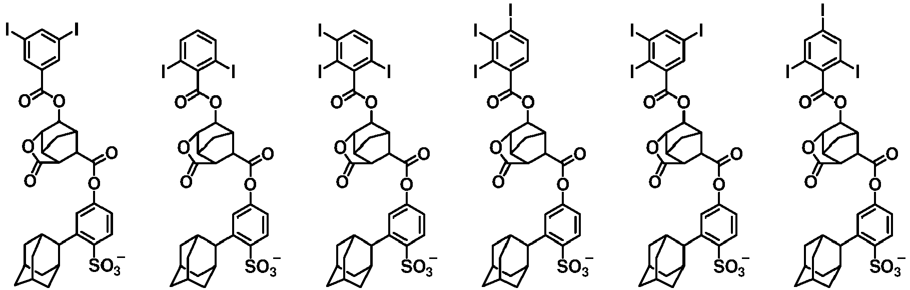

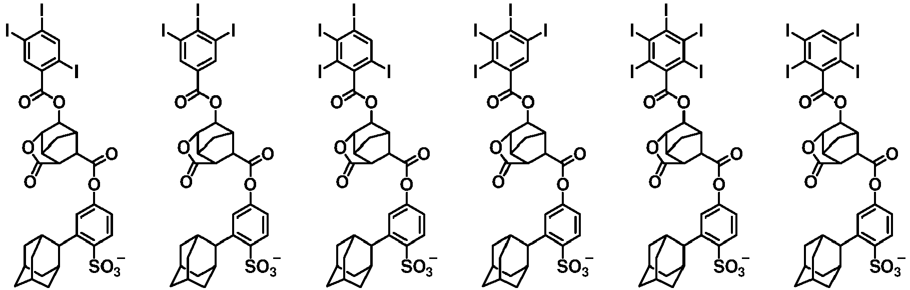

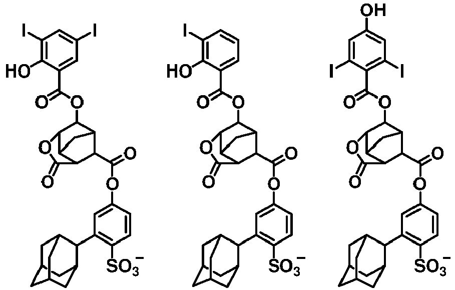

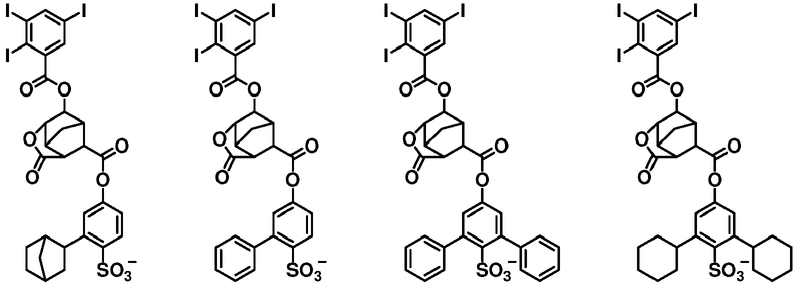

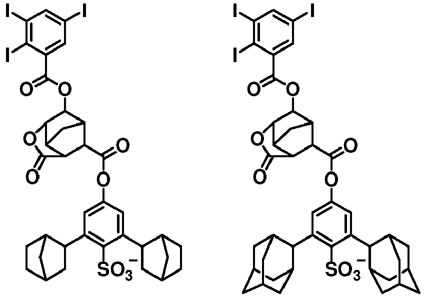

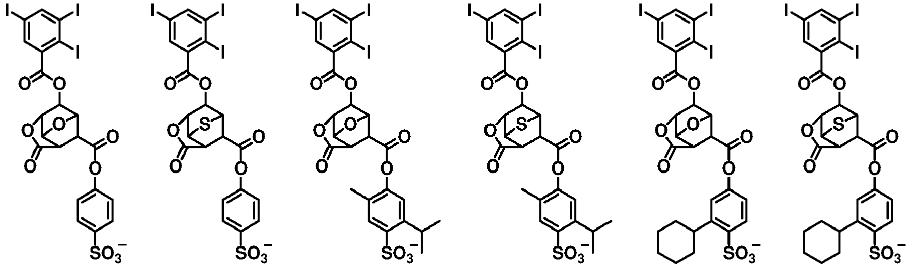

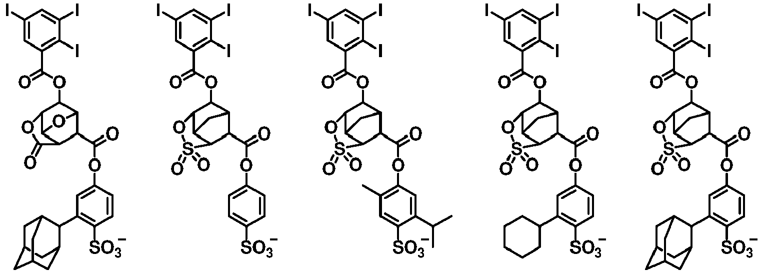

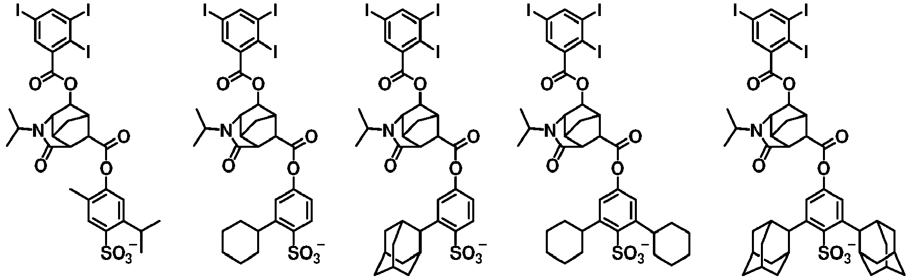

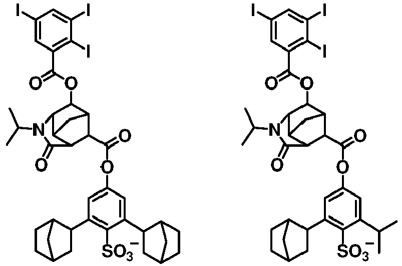

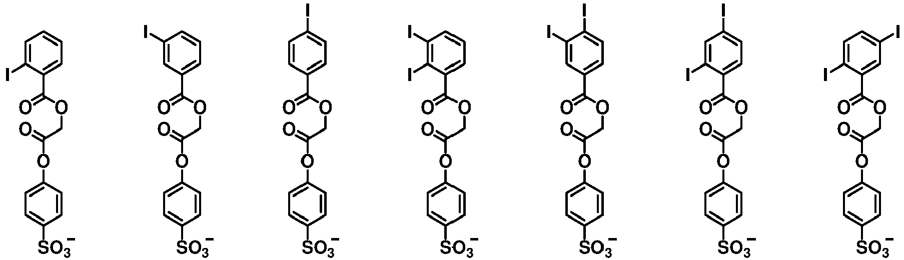

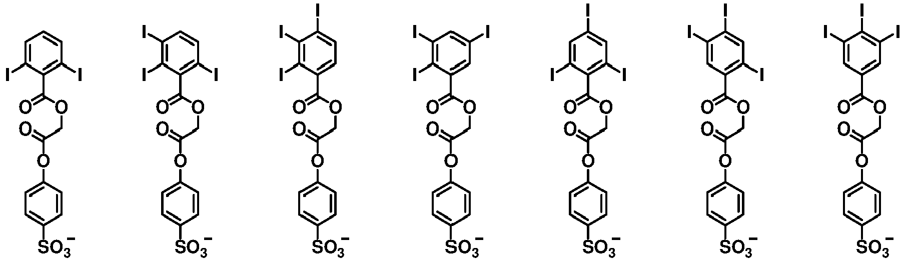

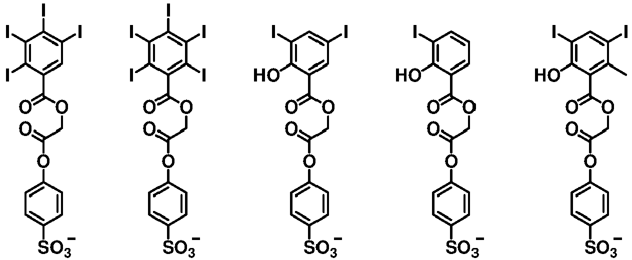

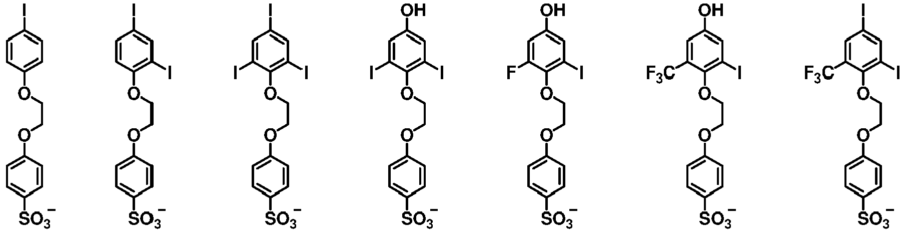

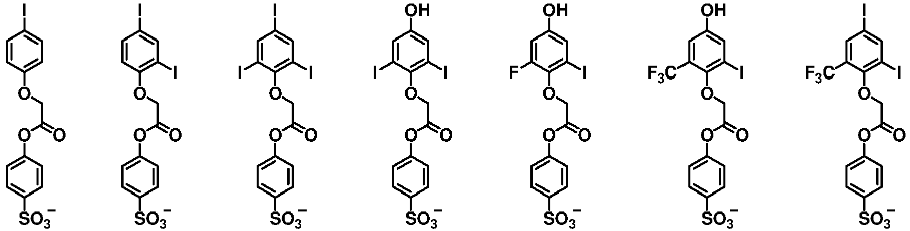

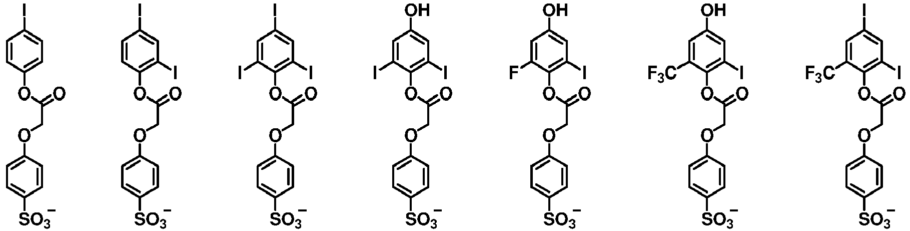

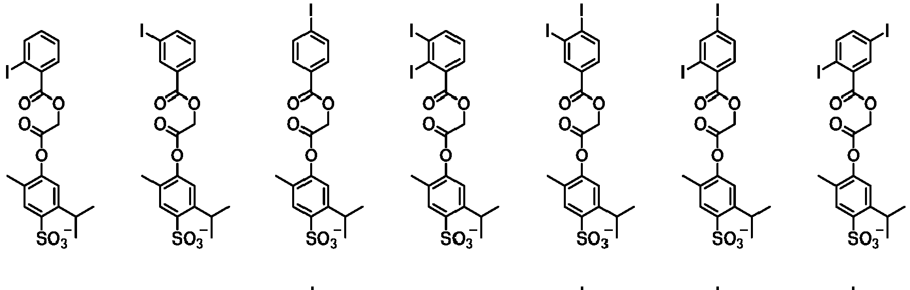

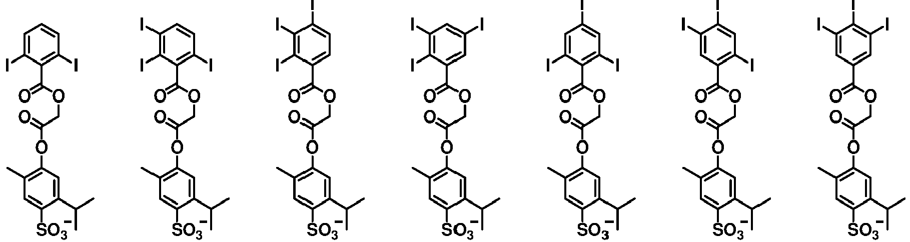

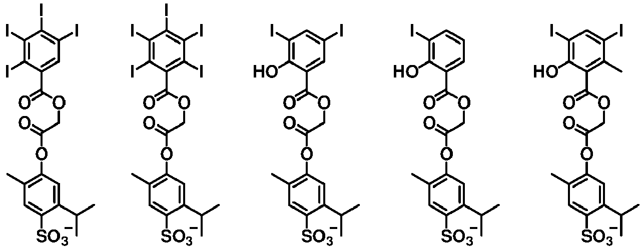

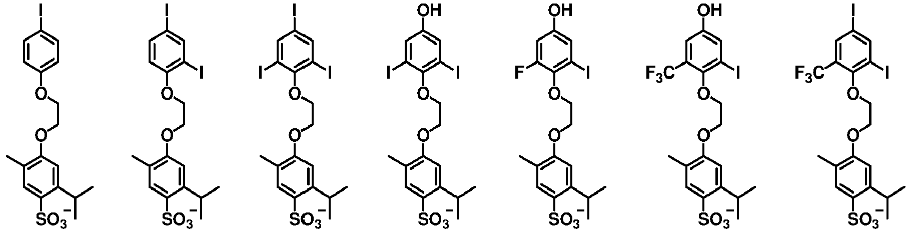

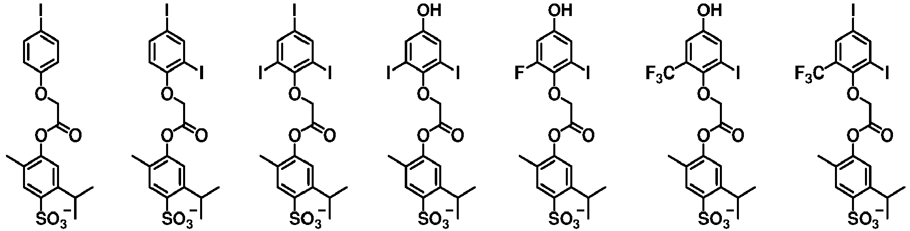

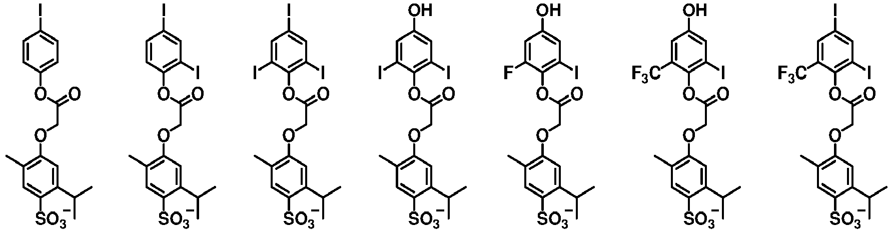

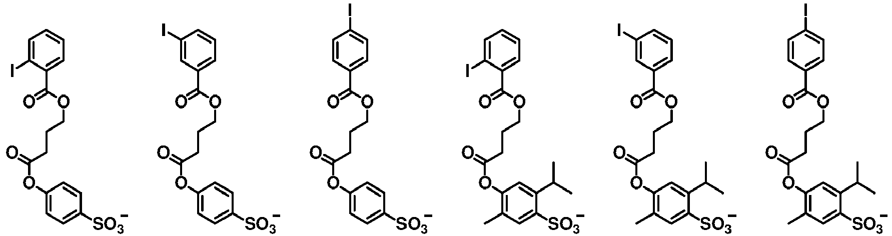

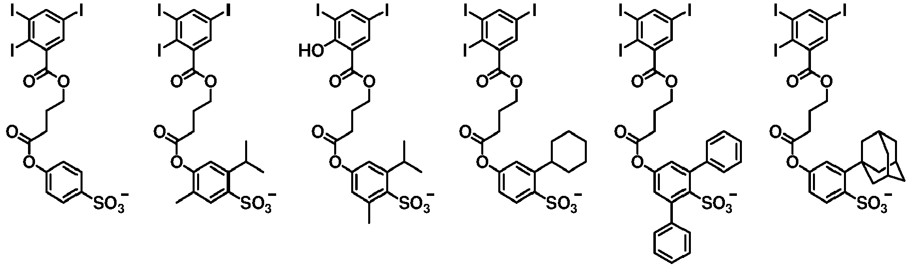

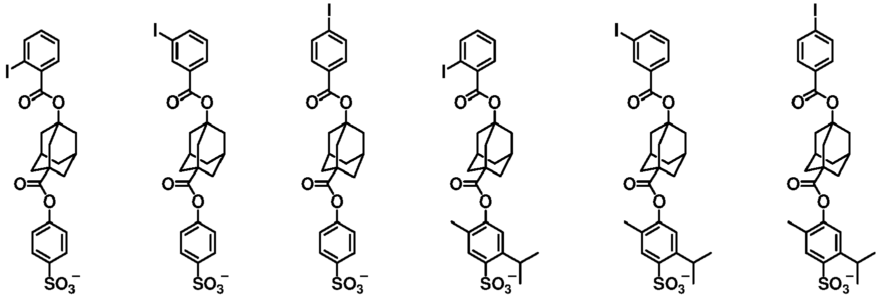

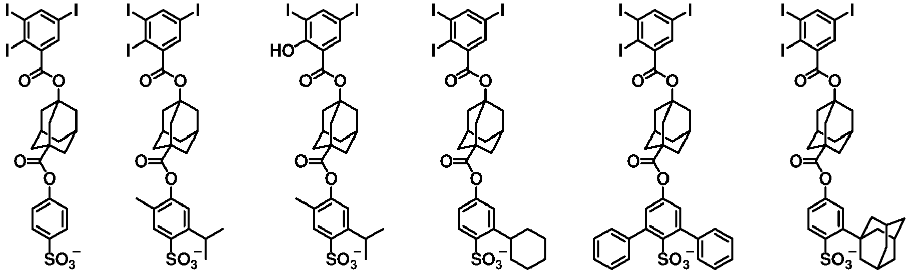

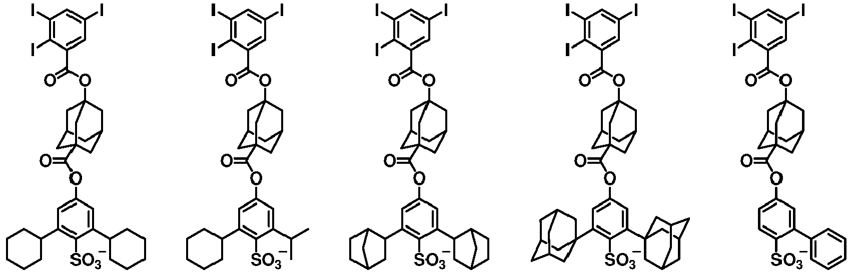

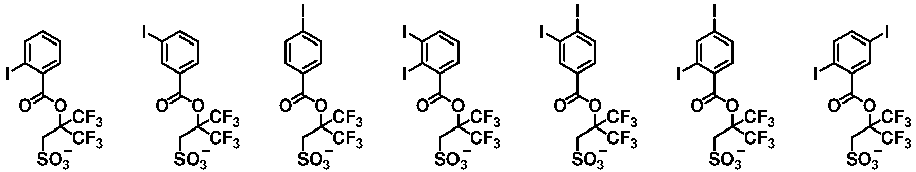

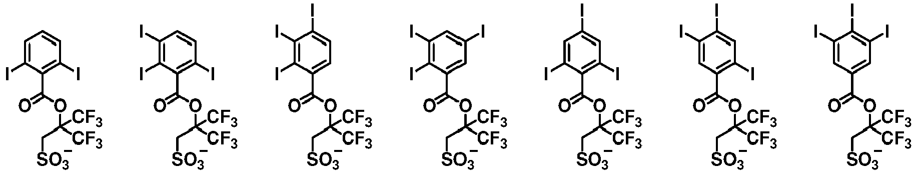

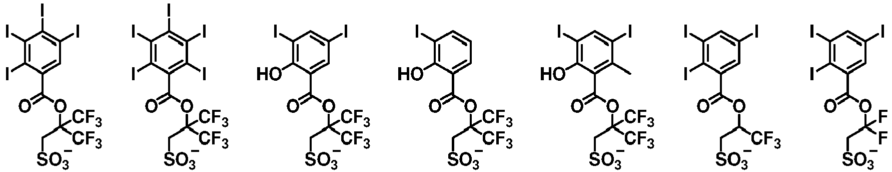

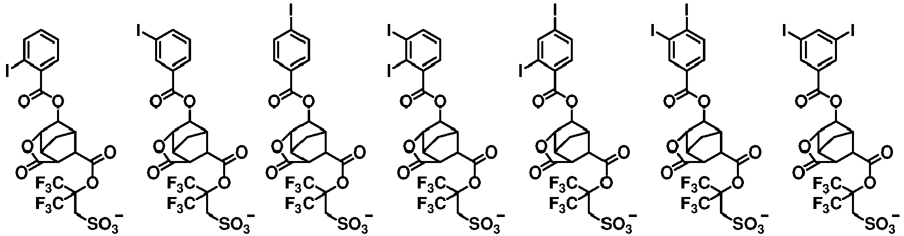

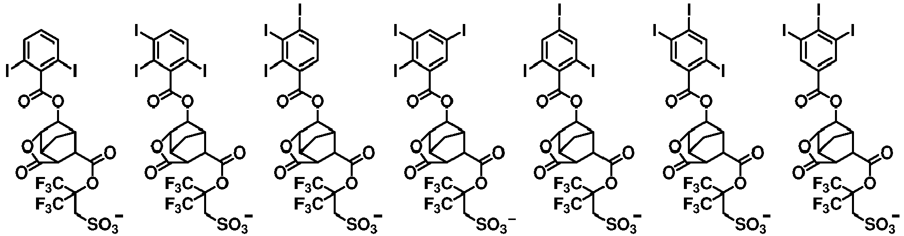

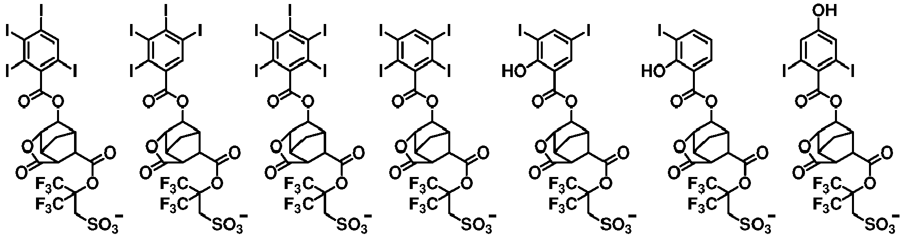

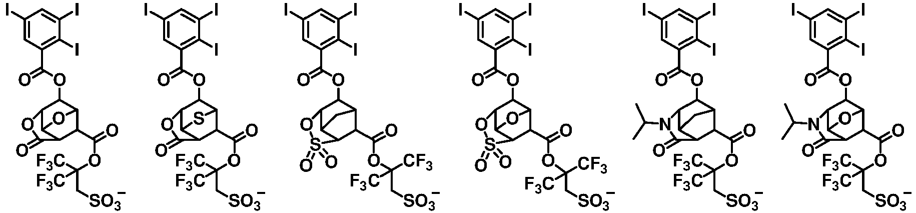

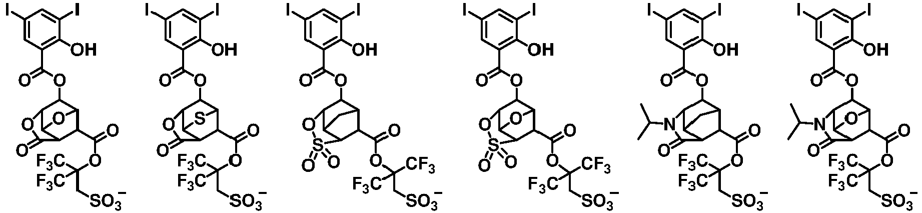

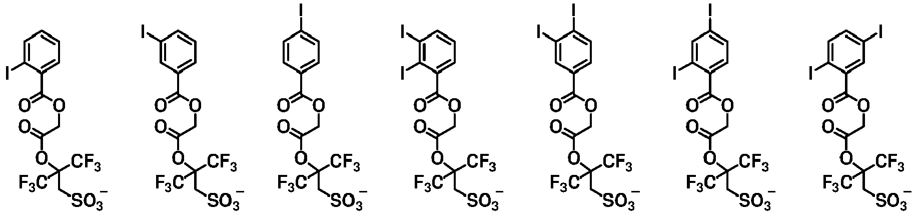

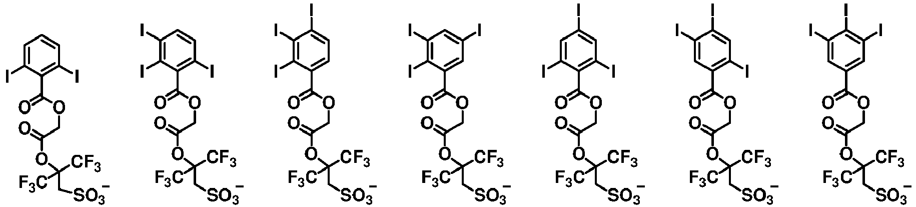

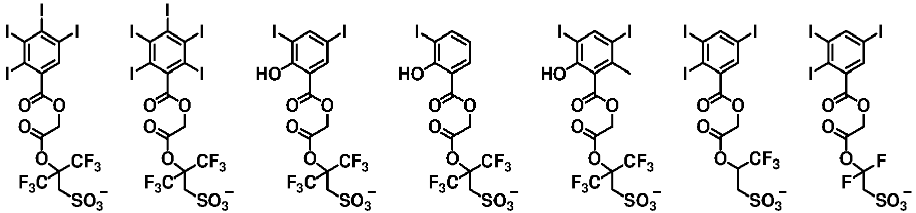

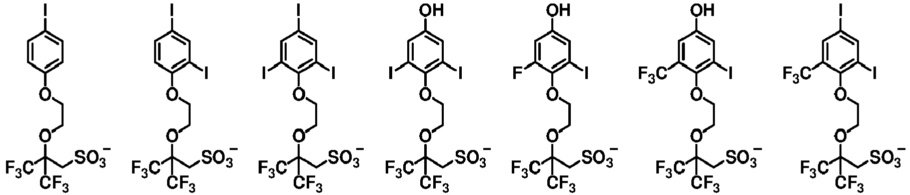

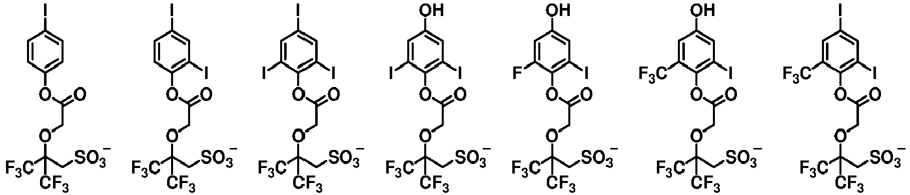

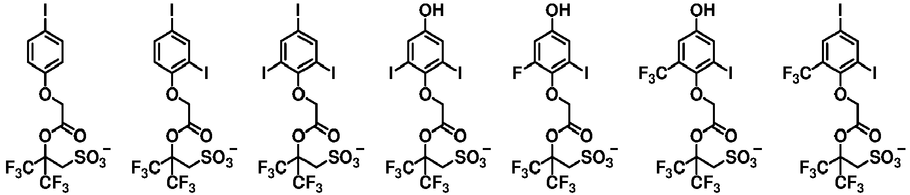

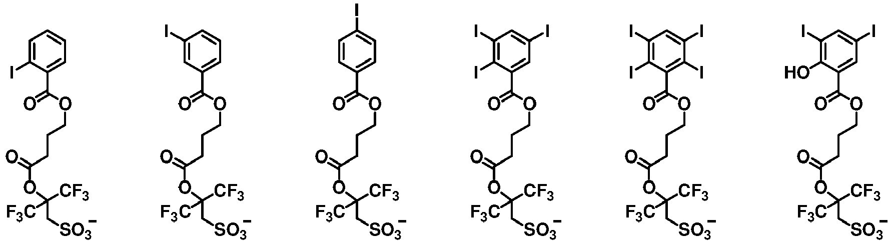

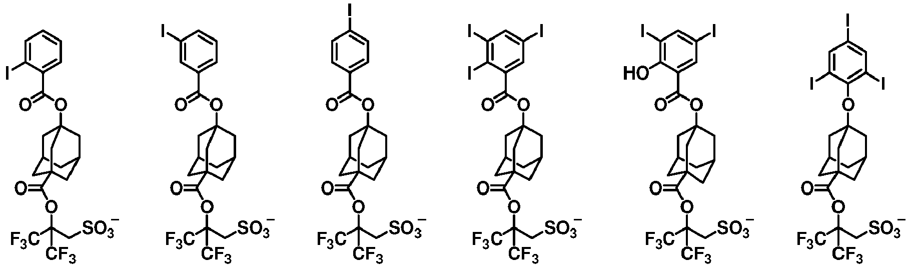

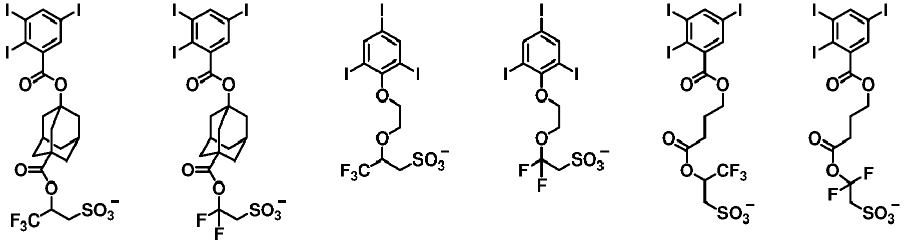

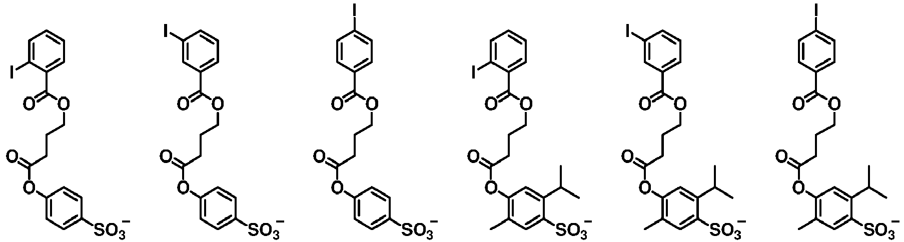

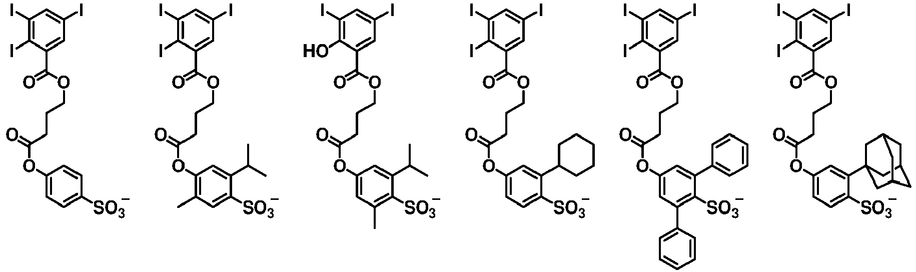

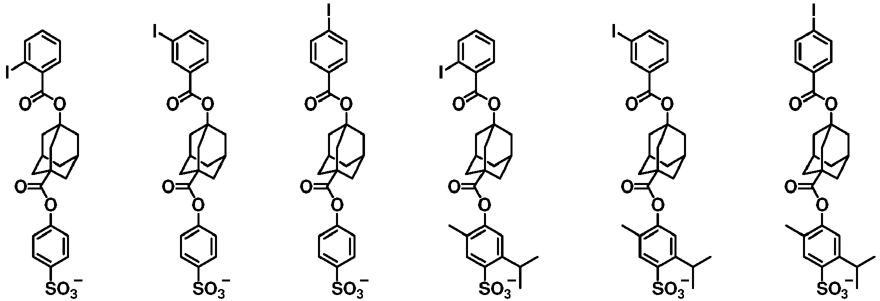

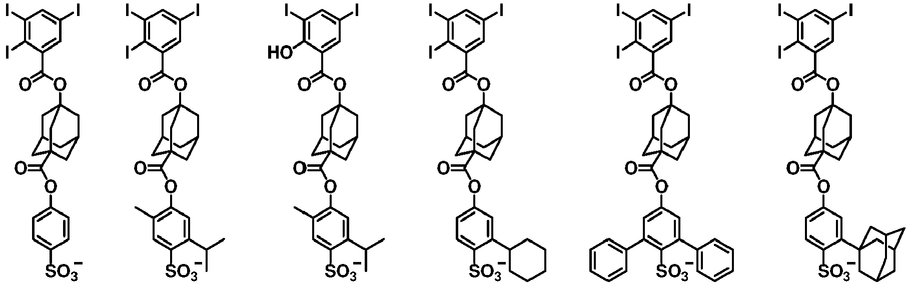

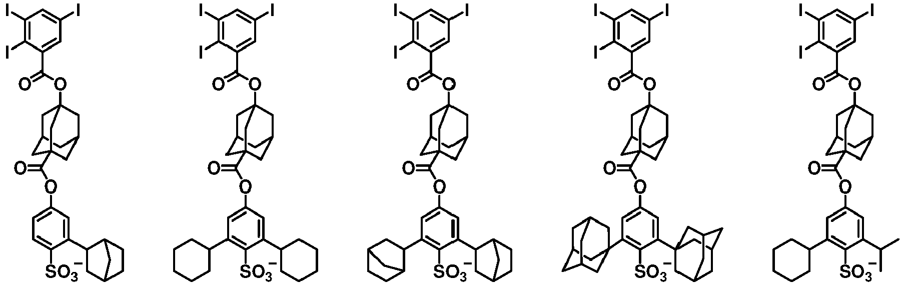

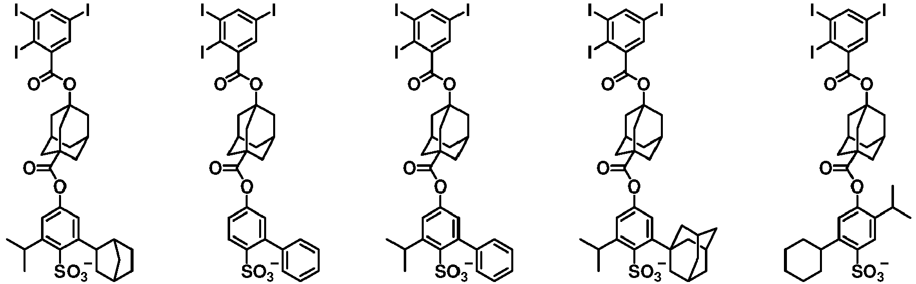

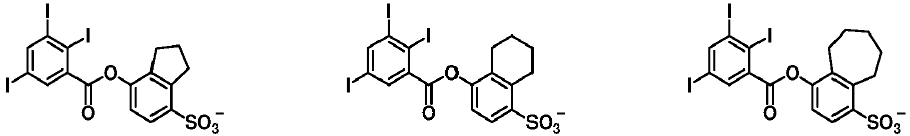

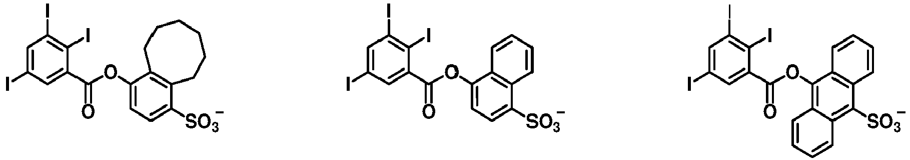

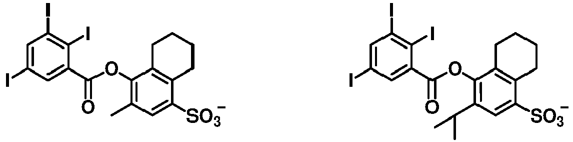

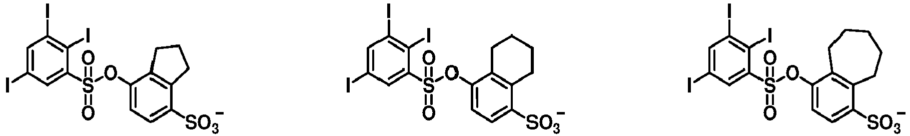

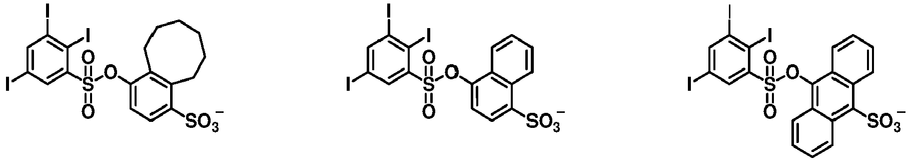

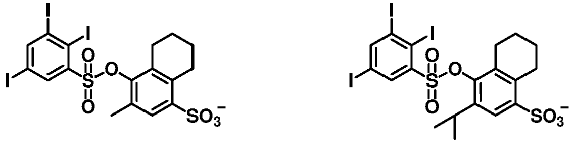

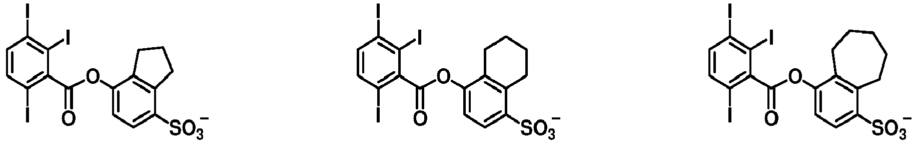

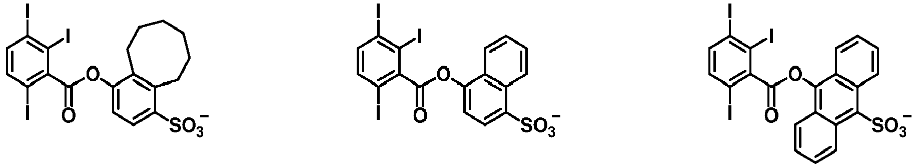

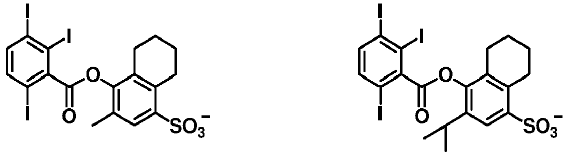

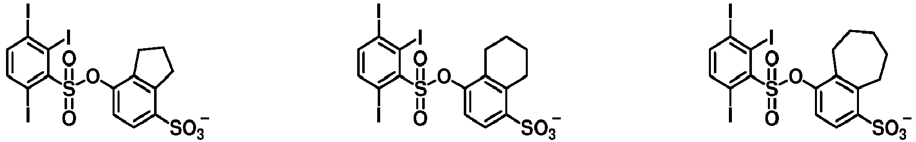

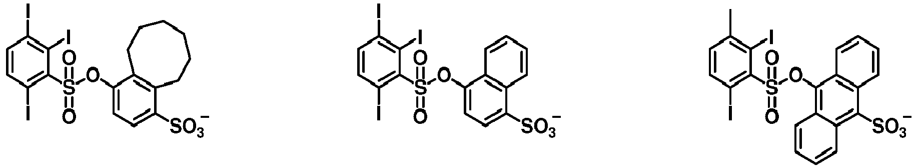

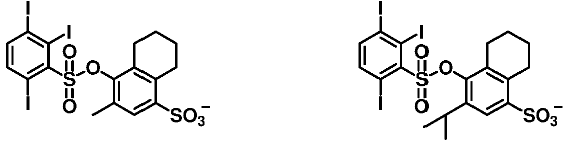

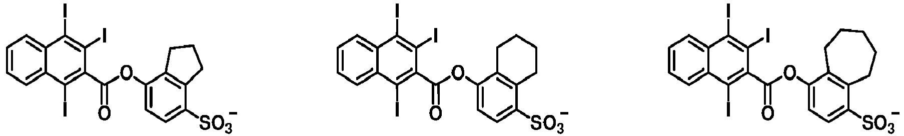

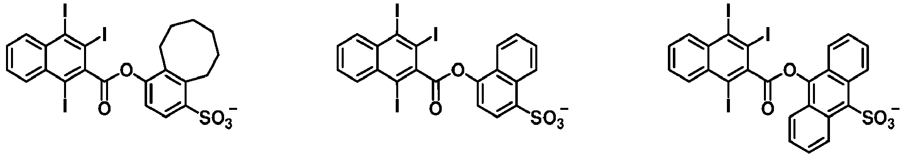

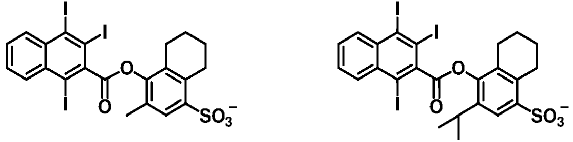

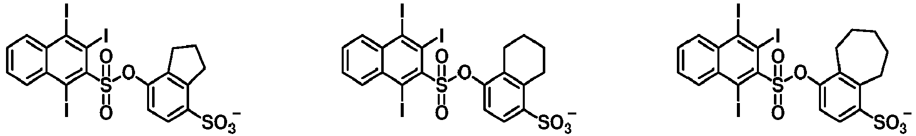

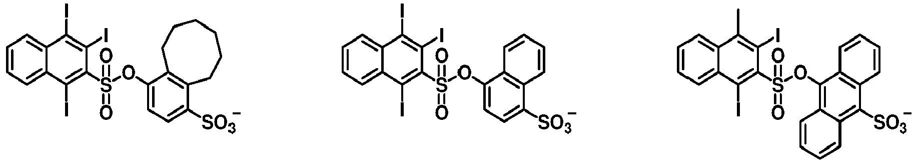

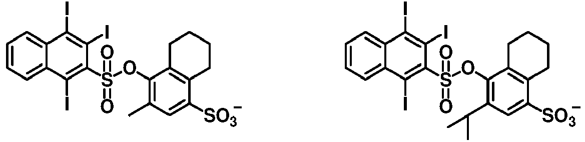

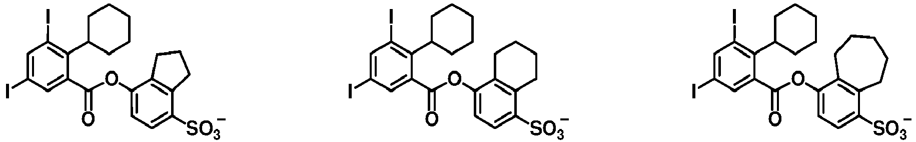

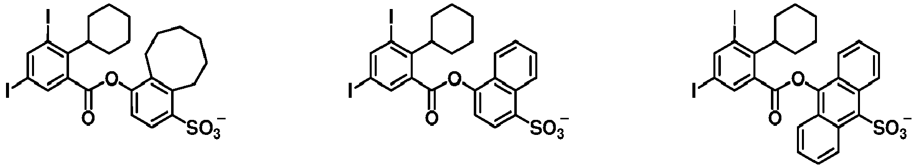

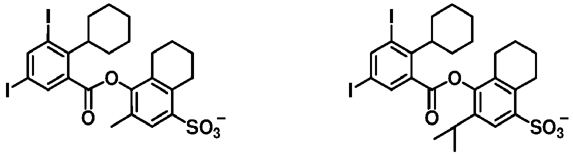

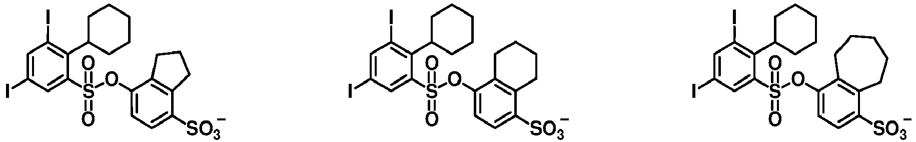

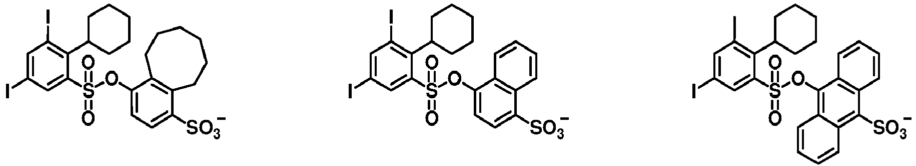

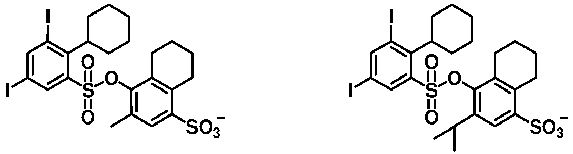

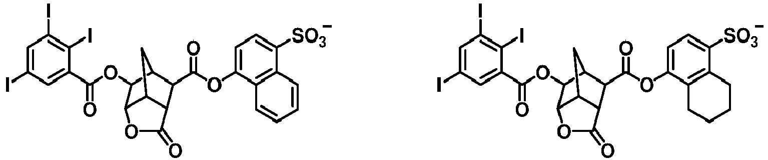

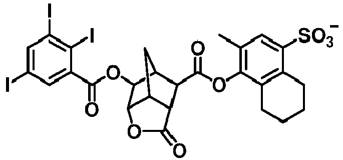

Preferred examples of the cation that pairs with the anion include sulfonium cations having the formula (P3) and iodonium cations having the formula (P4).

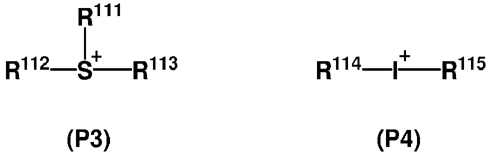

In formulae (P3) and (P4), R¹¹¹ to R¹¹⁵ are each independently halogen or a C₁-C₂₀ hydrocarbyl group which may contain a heteroatom.

Suitable halogen atoms include fluorine, chlorine, bromine and iodine.

The C₁-C₂₀ hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; and C₆-C₂₀ aryl groups such as phenyl, naphthyl and anthracenyl. In the hydrocarbyl groups, some or all of the hydrogen atoms may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

Also, R¹¹¹ and R¹¹² may bond together to form a ring with the sulfur atom to which they are attached. Examples of the ring are as exemplified above for the ring that R^{ct1} and R^{ct2} in formula (cation-1), taken together, form with the sulfur atom to which they are attached.

Exemplary structures of the sulfonium cation having formula (P3) are as exemplified above for the sulfonium cation having formula (cation-1), but not limited thereto. Exemplary structures of the iodonium cation having formula (P4) are as exemplified above for the iodonium cation having formula (cation-2), but not limited thereto.

The PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc. A chemically amplified positive resist composition comprising such a PAG exhibits a better resolution because the deprotection reaction of acid labile groups in the polymer is fully catalyzed.

An appropriate amount of the PAG used is 1 to 30 parts, more preferably 2 to 20 parts by weight per 80 parts by weight of the base polymer. The PAG may be used alone or in admixture.

### Quencher

The positive resist composition preferably contains a quencher or acid diffusion-suppressing agent. The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy group, ether bond, ester bond, lactone ring, cyano group, or sulfonate ester bond as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound is effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. An α-non-fluorinated carboxylic acid functions as a quencher because it does not induce substantial deprotection reaction.

Examples of the onium salt of α-non-fluorinated carboxylic acid include compounds having the formula (Q1).

### R²⁰¹-CO₂⁻Mq_{A}⁺ (Q1)

In formula (Q1), R²⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R²⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), dialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (Q1), Mq_{A}⁺ is an onium cation. The onium cation is preferably selected from sulfonium, iodonium and ammonium cations, more preferably sulfonium and iodonium cations. Exemplary sulfonium cations are as exemplified above for the sulfonium cation having formula (cation-1). Exemplary iodonium cations are as exemplified above for the iodonium cation having formula (cation-2).

Examples of the anion in the onium salt having formula (Q1) are shown below, but not limited thereto.

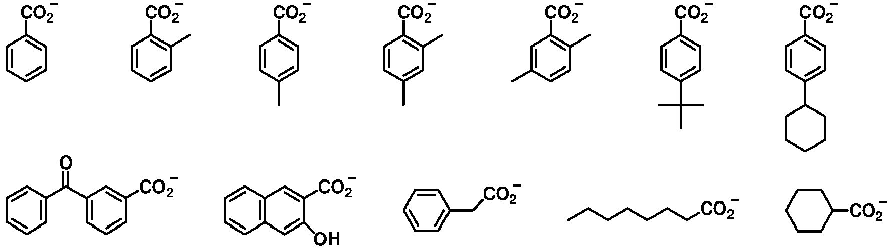

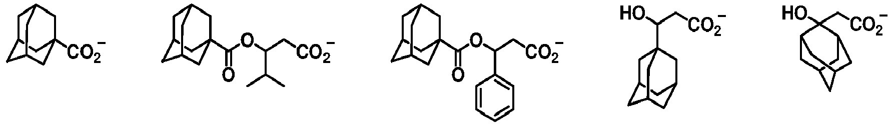

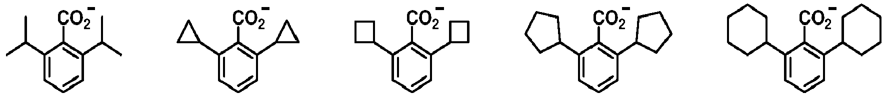

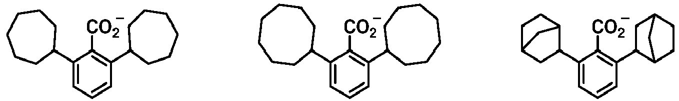

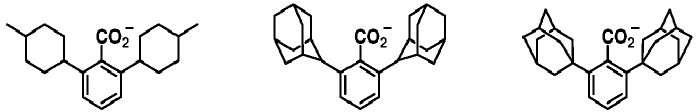

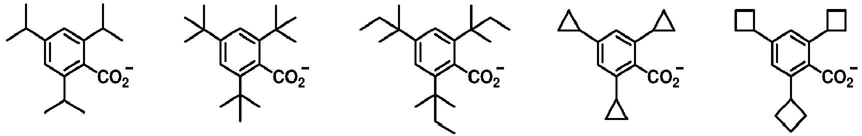

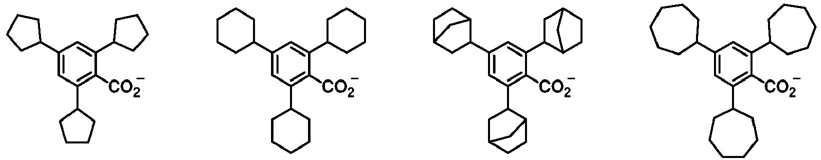

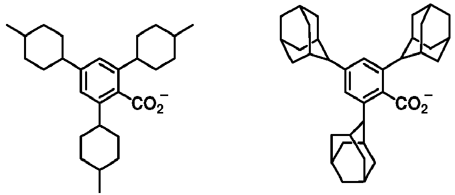

A sulfonium salt of iodized benzene ring-containing carboxylic acid having the formula (Q2) is also useful as the quencher.

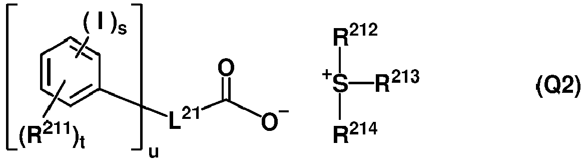

In formula (Q2), s is an integer of 1 to 5, t is an integer of 0 to 3, s+t is from 1 to 5, and u is an integer of 1 to 3.

In formula (Q2), R²¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{211A})-C(=O)-R^{211B}, or -N(R^{211A})-C(=O)-O-R^{211B}. R^{211A} is hydrogen or a C₁-C₆ saturated hydrocarbyl group. R^{211B} is a C₁-C₆ saturated hydrocarbyl or C₂-C₈ unsaturated aliphatic hydrocarbyl group. A plurality of R²¹¹ may be the same or different when t and/or u is 2 or 3.

In formula (Q2), L²¹ is a single bond, or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (Q2), R²¹², R²¹³ and R²¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also R²¹² and R²¹³ may bond together to form a ring with the sulfur atom to which they are attached.

Examples of the compound having formula (Q2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (Q3) is also useful as the quencher.

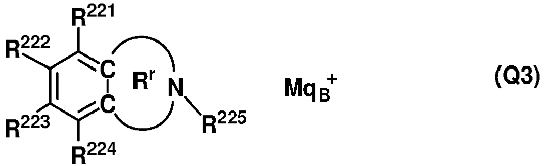

In formula (Q3), R²²¹ to R²²⁴ are each independently hydrogen, -L²²-CO₂⁻, or a C₁-C₂₀ hydrocarbyl group which may contain a heteroatom. R²²¹ and R²²², R²²² and R²²³, or R²²³ and R²²⁴ may bond together to form a ring with the carbon atom to which they are attached. L²² is a single bond or a C₁-C₂₀ hydrocarbylene group which may contain a heteroatom. R²²⁵ is hydrogen or a C₁-C₂₀ hydrocarbyl group which may contain a heteroatom.

In formula (Q3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²²-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (Q3) has at least one -L²²-CO₂⁻. That is, at least one of R²²¹ to R²²⁴ is -L²²-CO₂⁻, and/or at least one of hydrogen atoms bonded to carbon atoms in the ring R^{r} is substituted by -L²²-CO₂⁻.

In formula (Q3), Mq_{B}⁺ is a sulfonium, iodonium or ammonium cation, with the sulfonium cation being preferred. Examples of the sulfonium cation are as exemplified above for the sulfonium cation in formulae (C2) to (C4).

Examples of the anion in the compound having formula (Q3) are shown below, but not limited thereto.

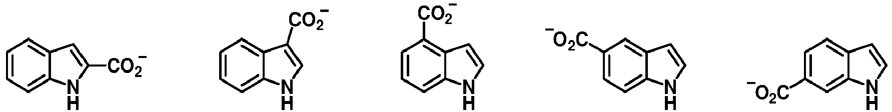

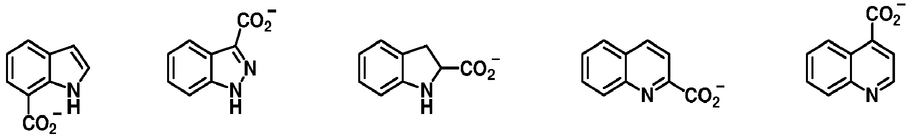

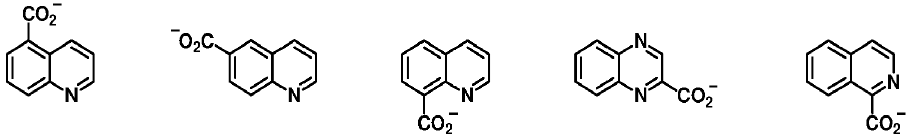

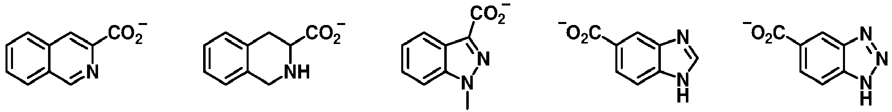

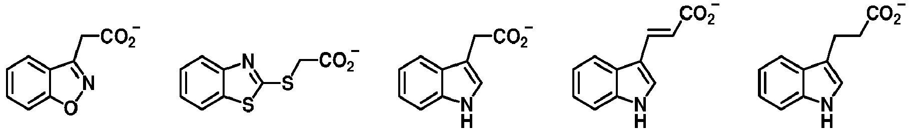

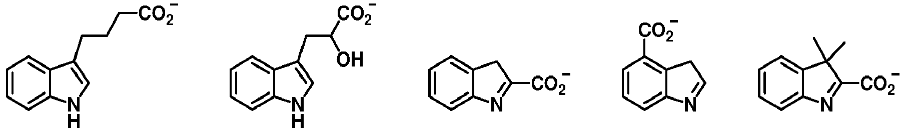

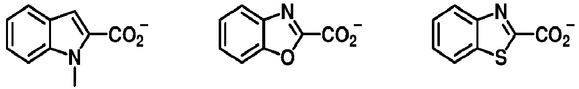

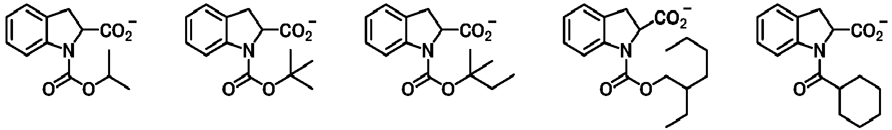

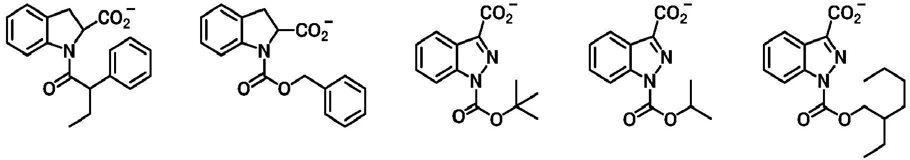

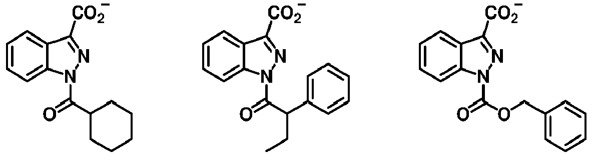

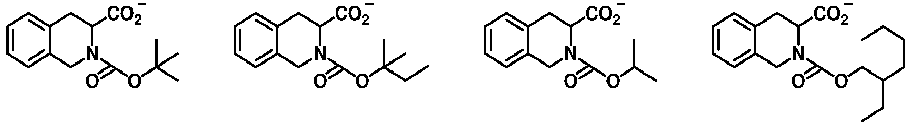

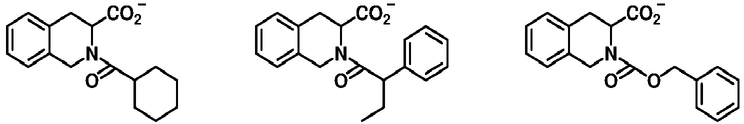

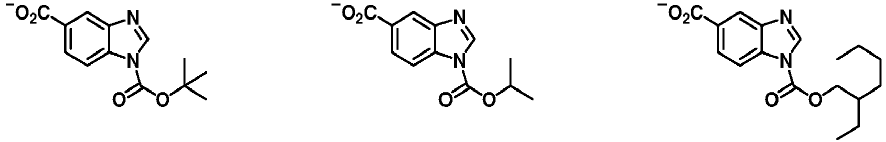

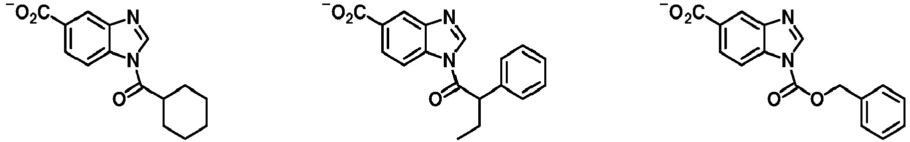

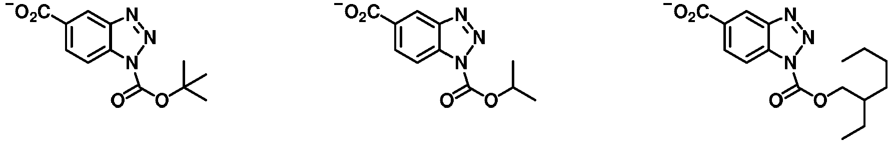

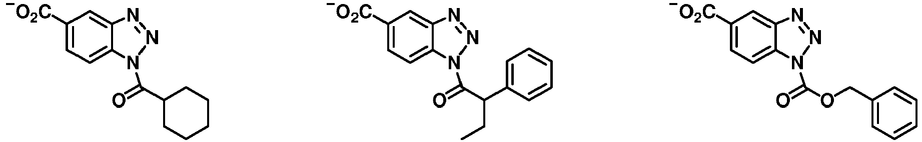

Weak acid betaine compounds are also useful as the quencher. Non-limiting examples thereof are shown below.

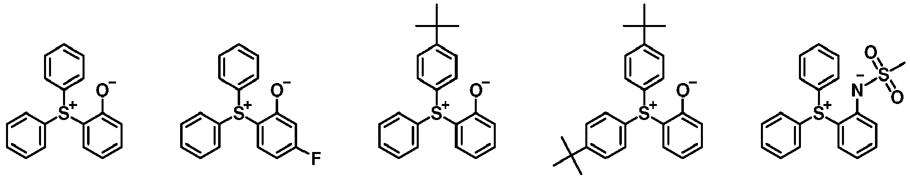

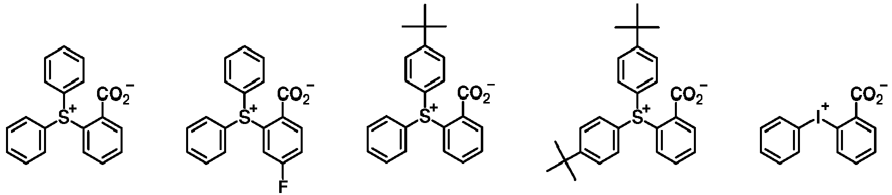

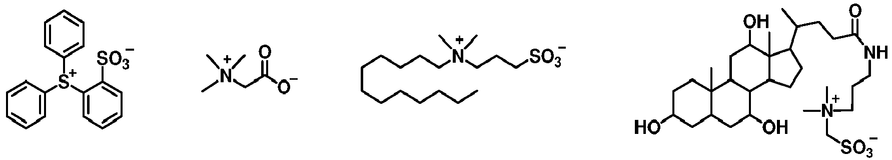

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the quencher is preferably added in an amount of 0 to 50 parts, more preferably 0.1 to 40 parts by weight per 80 parts by weight of the base polymer. The quencher may be used alone or in admixture.

When the chemically amplified positive resist composition contains both the PAG and the quencher, the weight ratio of the PAG to the quencher is preferably less than 3/1, more preferably less than 2.5/1, even more preferably less than 2/1. As long as the weight ratio of the PAG to the quencher is in the range, the resist composition is able to fully suppress acid diffusion, leading to improved resolution and dimensional uniformity.

### Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4). It is noted that repeat units having formulae (D1), (D2), (D3), and (D4) are also referred to as repeat units D1, D2, D3, and D4, respectively, hereinafter. Since the fluorinated polymer also has a surface active function, it can prevent insoluble residues from re-depositing onto the substrate during the development step and is thus effective for preventing development defects.

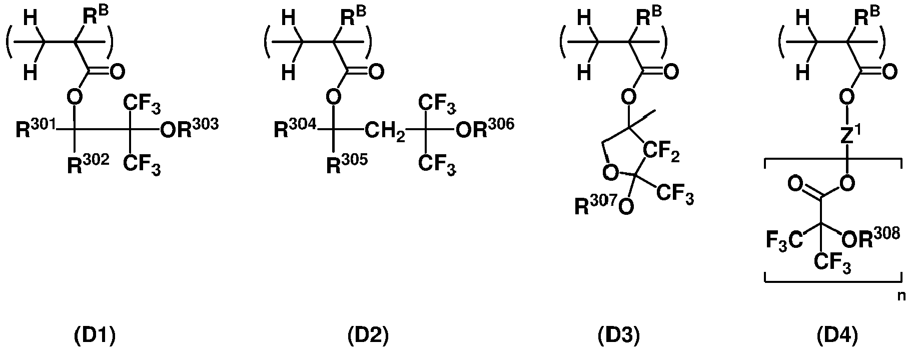

In formulae (D1) to (D4), R^{B} is each independently hydrogen, fluorine, methyl or trifluoromethyl. R³⁰¹, R³⁰², R³⁰⁴ and R³⁰⁵ are each independently hydrogen or a C₁-C₁₀ saturated hydrocarbyl group. R³⁰³, R³⁰⁶, R³⁰⁷ and R³⁰¹ are each independently hydrogen, a C₁-C₁₅ hydrocarbyl group or fluorinated hydrocarbyl group, or an acid labile group, with the proviso that an ether bond or carbonyl moiety may intervene in a carbon-carbon bond in the hydrocarbyl groups or fluorinated hydrocarbyl groups represented by R³⁰³, R³⁰⁶, R³⁰⁷ and R³⁰⁸. Z¹ is a C₁-C₂₀ (n+1)-valent hydrocarbon group or C₁-C₂₀ (n+1)-valent fluorinated hydrocarbon group, and n is an integer of 1 to 3.

Examples of the C₁-C₁₀ saturated hydrocarbyl group represented by R³⁰¹, R³⁰², R³⁰⁴ and R³⁰⁵ include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

Examples of the C₁-C₁₅ hydrocarbyl group represented by R³⁰³, R³⁰⁶, R³⁰⁷ and R³⁰⁸ include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

Examples of the C₁-C₂₀ (n+1)-valent hydrocarbon group Z¹ include the foregoing C₁-C₂₀ alkyl groups and C₃-C₂₀ cyclic saturated hydrocarbyl groups, with n number of hydrogen atoms being eliminated. Examples of the C₁-C₂₀ (n+1)-valent fluorinated hydrocarbon group Z¹ include the foregoing (n+1)-valent hydrocarbon groups in which one or more hydrogen atoms are substituted by fluorine.

Examples of the repeat units D1 to D4 are given below, but not limited thereto. Herein R^{B} is as defined above.

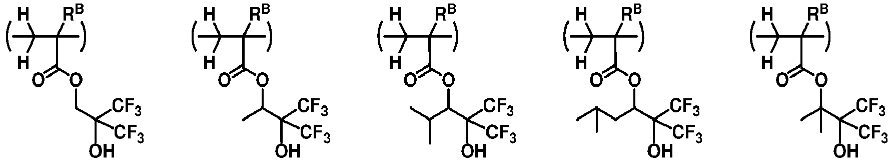

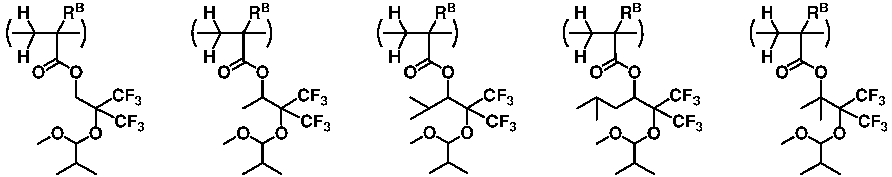

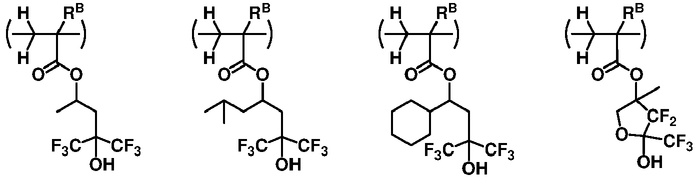

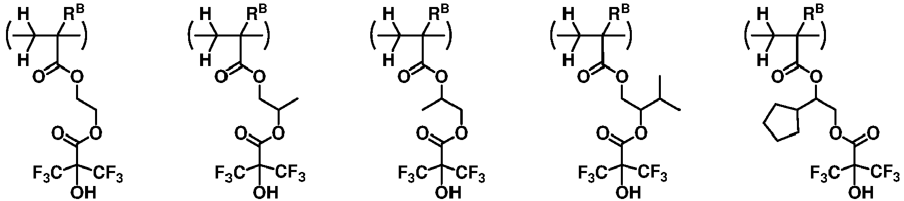

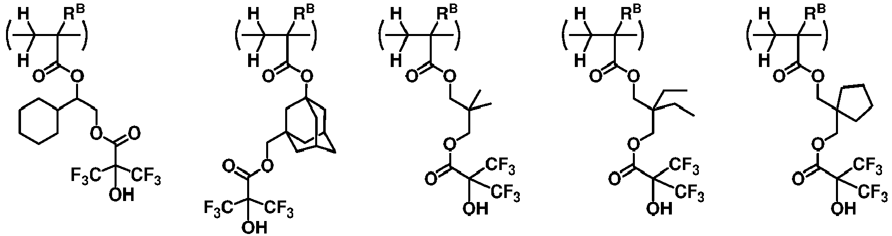

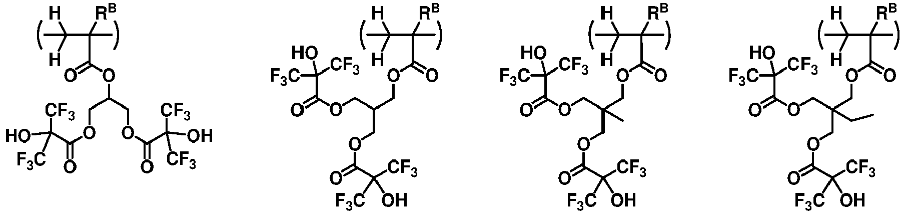

Preferably the fluorinated polymer further contains repeat units of at least one type selected from repeat units having the formula (D5) and repeat units having the formula (D6). It is noted that repeat units having formulae (D5) and (D6) are also referred to as repeat units D5 and D6, respectively, hereinafter.

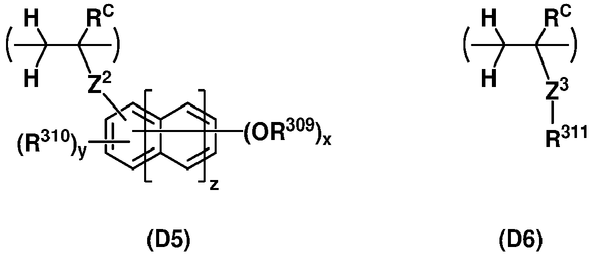

In formulae (D5) and (D6), R^{C} is each independently hydrogen or methyl. R³⁰⁹ is hydrogen or a C₁-C₅ straight or branched hydrocarbyl group in which a heteroatom-containing moiety may intervene in a carbon-carbon bond. R³¹⁰ is a C₁-C₅ straight or branched hydrocarbyl group in which a heteroatom-containing moiety may intervene in a carbon-carbon bond. R³¹¹ is a C₁-C₂₀ saturated hydrocarbyl group in which at least one hydrogen is substituted by fluorine and some constituent -CH₂- may be replaced by an ester bond or ether bond. The subscript x is an integer of 1 to 3, y is an integer satisfying: 0 <_ y ≤ 5+2z-x, and z is 0 or 1. Z² is a single bond, *-C(=O)-O- or *-C(=O)-NH-. Z³ is a single bond, -O-, *-C(=O)-O-Z³¹-Z³²- or *-C(=O)-NH-Z³¹-Z³²-, wherein Z³¹ is a single bond or a C₁-C₁₀ saturated hydrocarbylene group, Z³² is a single bond, ester bond, ether bond or sulfonamide bond, and * designates a point of attachment to the carbon atom in the backbone.

Examples of the C₁-C₅ hydrocarbyl groups R³⁰⁹ and R³¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

Preferably, -OR³⁰⁹ is a hydrophilic group. In this case, R³⁰⁹ is preferably hydrogen or a C₁-C₅ alkyl group in which oxygen intervenes in a carbon-carbon bond.

Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{C} is as defined above.

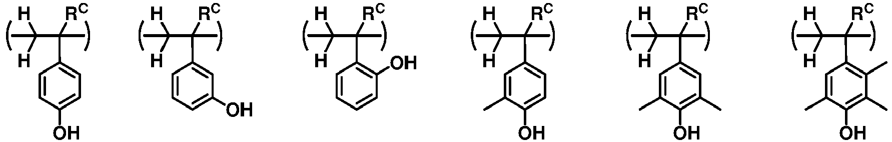

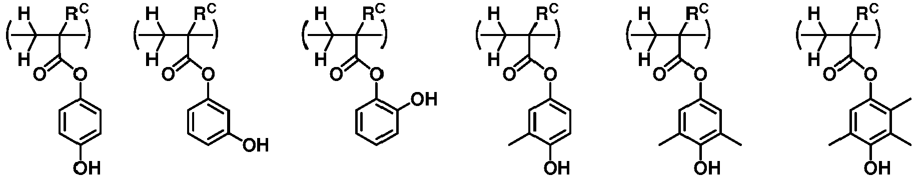

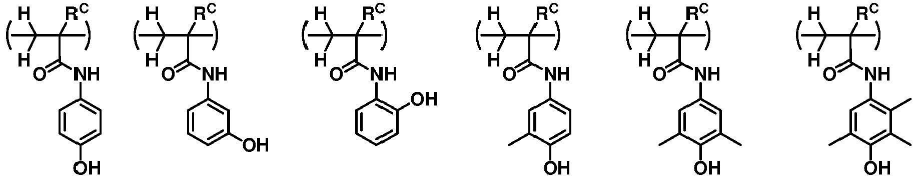

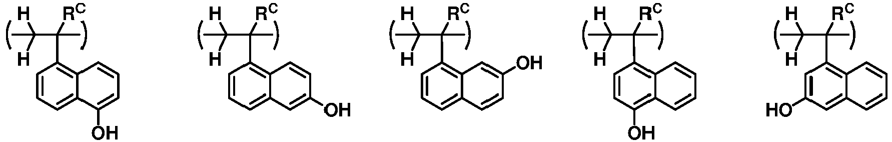

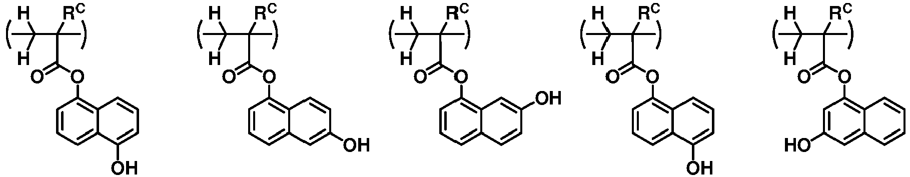

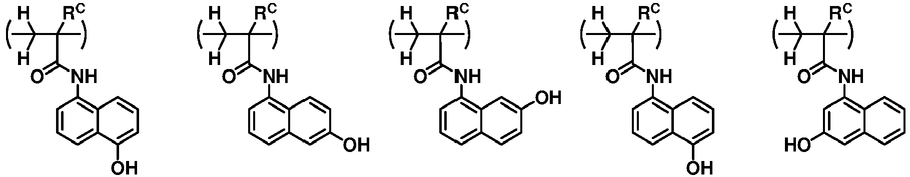

The C₁-C₁₀ saturated hydrocarbylene group Z³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R³¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{C} is as defined above.

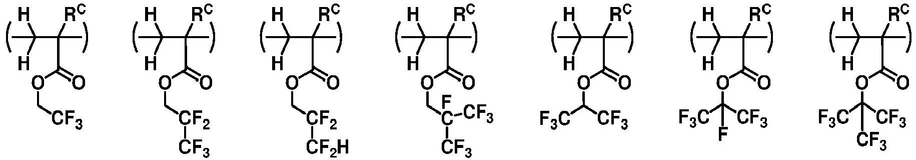

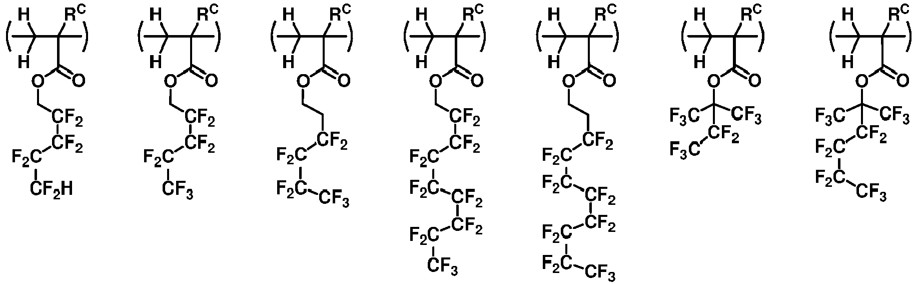

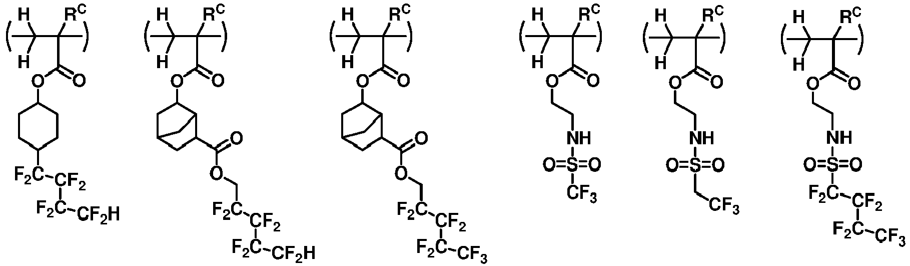

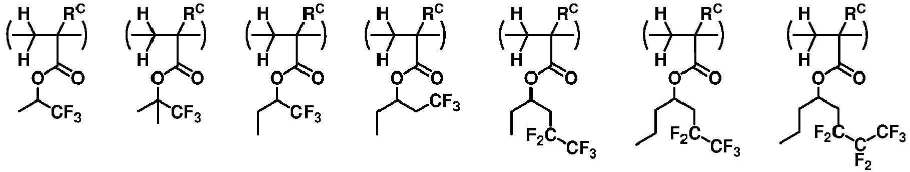

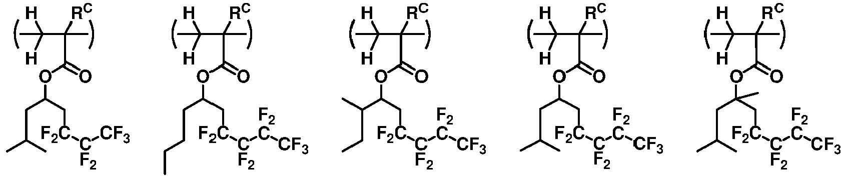

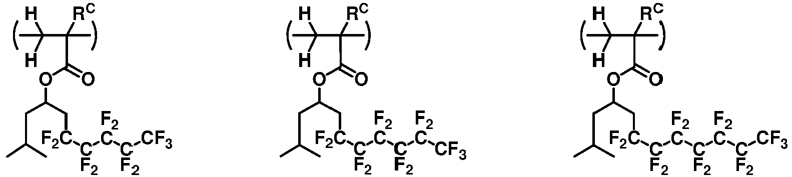

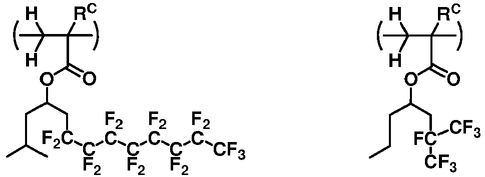

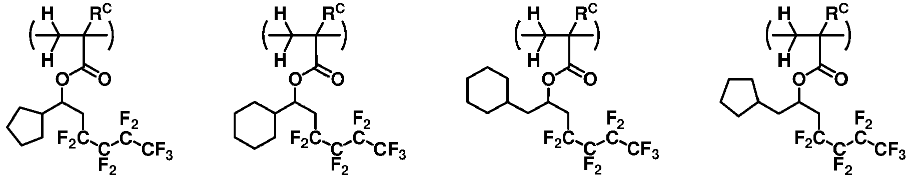

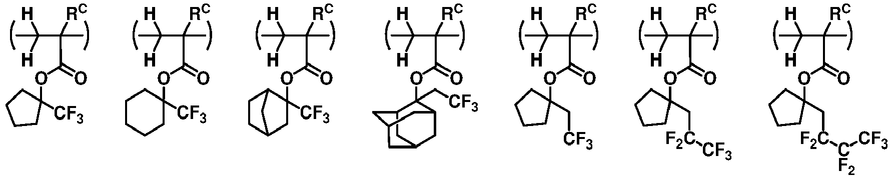

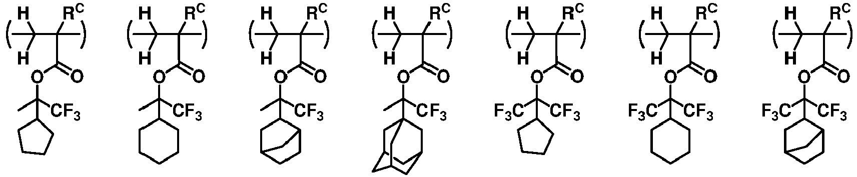

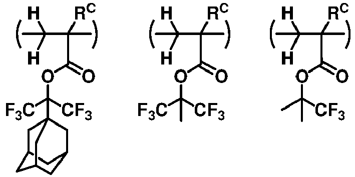

The repeat units D1 to D4 are preferably incorporated in an amount of 15 to 95 mol%, more preferably 20 to 85 mol% based on the overall repeat units of the fluorinated polymer. The repeat unit D5 and/or D6 is preferably incorporated in an amount of 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of at least 2,000 prevents extra acid diffusion and degradations of resolution and age stability. A polymer with a Mw of up to 5,000 has a sufficient solubility in solvent and leaves no coating defects. The fluorinated polymer preferably has a dispersity (Mw/Mn) of 1.0 to 2.2, more preferably 1.0 to 1.7.

In the resist composition, the fluorinated polymer is preferably used in an amount of 0.01 to 30 parts by weight, more preferably 0.1 to 20 parts by weight, even more preferably 0.5 to 10 parts by weight per 80 parts by weight of the base polymer.

### Surfactant

The positive resist composition may contain any conventional surfactants for facilitating to coat the composition to the substrate. A number of surfactants are known in the art as described in JP-A 2004-115630, and any suitable one may be chosen therefrom. The amount of surfactant added is preferably 0 to 5 parts by weight per 80 parts by weight of the base polymer. It is noted that the surfactant need not be added when the positive resist composition contains a fluorinated polymer as mentioned above, which also plays the role of a surfactant.

The positive resist composition may be prepared by dissolving the base polymer and optionally other components in an organic solvent at the same time or in any desired order to form a uniform resist solution. The resist solution is preferably filtered. Using a filter of nylon or polyethylene for filtration, gel fractions and particles can be effectively removed from the resist solution. Also preferably, a filter having a pore size of up to 20 nm is used so that the quality of the resist solution may be maintained adequate for the lithography of the advanced generation.

The resist film formed from the chemically amplified positive resist composition in an unexposed region preferably has a dissolution rate in alkaline developer of up to 10 nm/min, more preferably up to 9 nm/min, even more preferably up to 8 nm/min. Where the resist film is in the thin film range of up to 100 nm, the influence of pattern film thickness loss in alkaline developer becomes greater. When the dissolution rate in unexposed region is no more than 10 nm/min, pattern collapse does not occur, succeeding in forming a small-size pattern. This becomes outstanding in the fabrication of photomasks requiring to be defectless and having a tendency of strong development process. It is noted that the dissolution rate of an unexposed region is computed by spin coating the positive resist composition onto a 150-mm (6-inch) silicon wafer, baking at 110°C for 240 seconds to form a resist film of 80 nm thick, developing the film in a 2.38 wt% TMAH aqueous solution at 23°C for 80 seconds, and measuring a loss of film thickness.

From the standpoint of improving the develop loading effect, the positive resist composition is preferably designed such that a resist film formed therefrom in an exposed region may have a dissolution rate in alkaline developer of at least 50 nm/sec, more preferably at least 80 nm/sec. As long as the dissolution rate is at least 50 nm/sec, the resist film is uniformly dissolved in alkaline developer independent of a pattern layout difference in the case of a grouped/isolated pattern, and the variation of line width can be minimized. It is noted that the dissolution rate of an exposed region is computed by spin coating the positive resist composition onto a 200-mm (8-inch) silicon wafer, baking at 110°C for 60 seconds to form a resist film of 90 nm thick, exposing the resist film to KrF excimer laser radiation in a sufficient energy dose to complete deprotection reaction on the polymer, baking at 110°C for 60 seconds, developing the film in a 2.38 wt% TMAH aqueous solution at 23°C, and measuring a loss of film thickness by means of a resist development analyzer.

Since the acetal acid labile group is also effective for suppressing the influence of back scattering during EB image writing, the resist composition avoids the pattern profile from being inversely tapered in a sensitivity region of at least 50 µC, preferably at least 80 µC, more preferably at least 100 µC and displays excellent rectangular performance.

### Pattern forming process

A still further embodiment of the invention is a process for forming a resist pattern comprising the steps of applying the chemically amplified positive resist composition onto a substrate to form a resist film thereon, exposing patternwise the resist film to high-energy radiation, and developing the exposed resist film in an alkaline developer.

The resist composition is first applied onto a substrate on which an integrated circuit is to be formed (e.g., Si, SiO, SiO₂, SiN, SiON, TiN, WSi, BPSG, SOG, or organic antireflective coating) or a substrate on which a mask circuit is to be formed (e.g., Cr, CrO, CrON, MoSi₂, Si, SiO, SiO₂, SiON, SiONC, CoTa, NiTa, TaBN, or SnO₂) by a suitable coating technique such as spin coating. The coating is prebaked on a hot plate at a temperature of preferably 60 to 150°C for 1 to 20 minutes, more preferably at 80 to 140°C for 1 to 10 minutes. The resulting resist film is generally 0.03 to 2 µm thick.

The resist film is then exposed to a desired pattern of high-energy radiation such as excimer laser radiation (e.g., KrF or ArF), EUV of wavelength 3 to 15 nm, or EB. Exposure using EB is preferred. For forming the desired pattern, EB is preferably irradiated so as to give a dose of 50 to 400 µC/cm². Since the polymer containing acetal acid labile groups is also effective for suppressing the influence of back scattering during EB image writing, the resist composition avoids the pattern profile from being inversely tapered in a sensitivity region of at least 50 µC/cm², preferably at least 80 µC/cm², more preferably at least 100 µC/cm² and displays excellent rectangular performance.

The exposure may be performed by conventional lithography whereas the immersion lithography of holding a liquid, typically water between the resist film and the mask may be employed if desired. In the case of immersion lithography, a protective film which is insoluble in water may be formed on the resist film.

After the exposure, the resist film may be baked (PEB) on a hotplate preferably at 60 to 150°C for 1 to 20 minutes, more preferably at 80 to 140°C for 1 to 10 minutes.

After the exposure or PEB, the resist film is developed in a developer in the form of an aqueous alkaline solution for preferably 0.1 to 3 minutes, more preferably 0.5 to 2 minutes by conventional techniques such as dip, puddle and spray techniques. A typical developer is a 0.1 to 5 wt%, preferably 2 to 3 wt% aqueous solution of tetramethylammonium hydroxide (TMAH) or another alkali. In this way, the desired pattern is formed on the substrate.

From the positive resist composition, a pattern with a satisfactory isolated-space resolution and reduced LER can be formed. The resist composition is effectively applicable to a substrate, specifically a substrate having a surface layer of material to which a resist film is less adherent and which is likely to invite pattern stripping or pattern collapse. A typical substrate has an outermost surface of a material containing at least one element selected from chromium, silicon, tantalum, molybdenum, cobalt, nickel, tungsten, and tin. Preferred are substrates having sputter deposited on their outermost surface metallic chromium or a chromium compound containing at least one light element selected from oxygen, nitrogen and carbon and substrates having an outermost surface layer of SiO, SiOₓ, or a tantalum compound, molybdenum compound, cobalt compound, nickel compound, tungsten compound or tin compound. The substrate to which the positive resist composition is applied is most typically a photomask blank which may be either of transmission or reflection type. A mask blank of transmission or reflection type which is coated with the chemically amplified positive resist composition is also contemplated herein.

The mask blank of transmission type is typically a photomask blank having a light-shielding film of chromium-based material. It may be either a photomask blank for binary masks or a photomask blank for phase shift masks. In the case of the binary mask-forming photomask blank, the light-shielding film may include an antireflection layer of chromium-based material and a light-shielding layer. In one example, the antireflection layer on the surface layer side is entirely composed of a chromium-based material. In an alternative example, only a surface side portion of the antireflection layer on the surface layer side is composed of a chromium-based material and the remaining portion is composed of a silicon compound-based material which may contain a transition metal. In the case of the phase shift mask-forming photomask blank, it may include a phase shift film and a chromium-based light-shielding film thereon.

Photomask blanks having an outermost layer of chromium base material are well known as described in JP-A 2008-026500 and JP-A 2007-302873 and the references cited therein. Although the detail description is omitted herein, the following layer construction may be employed when a light-shielding film including an antireflective layer and a light-shielding layer is composed of chromium base materials.

In the example where a light-shielding film including an antireflective layer and a light-shielding layer is composed of chromium base materials, layers may be stacked in the order of an antireflective layer and a light-shielding layer from the outer surface side, or layers may be stacked in the order of an antireflective layer, a light-shielding layer, and an antireflective layer from the outer surface side. Each of the antireflective layer and the light-shielding layer may be composed of multiple sub-layers. When the sub-layers have different compositions, the composition may be graded discontinuously or continuously from sub-layer to sub-layer. The chromium base material used herein may be metallic chromium or a material consisting of metallic chromium and a light element such as oxygen, nitrogen or carbon. Examples used herein include metallic chromium, chromium oxide, chromium nitride, chromium carbide, chromium oxynitride, chromium oxycarbide, chromium nitride carbide, and chromium oxide nitride carbide.

The mask blank of reflection type includes a substrate, a multilayer reflective film formed on one major surface (front surface) of the substrate, for example, a multilayer reflective film of reflecting exposure radiation such as EUV radiation, and an absorber film formed on the multilayer reflective film, for example, an absorber film of absorbing exposure radiation such as EUV radiation to reduce reflectivity. From the reflection type mask blank (reflection type mask blank for EUV lithography), a reflection type mask (reflection type mask for EUV lithography) having an absorber pattern (patterned absorber film) formed by patterning the absorber film is produced. The EUV radiation used in the EUV lithography has a wavelength of 13 to 14 nm, typically about 13.5 nm.

The multilayer reflective film is preferably formed contiguous to one major surface of a substrate. An underlay film may be disposed between the substrate and the multilayer reflective film as long as the benefits of the invention are not lost. The absorber film may be formed contiguous to the multilayer reflective film while a protective film (protective film for the multilayer reflective film) may be disposed between the multilayer reflective film and the absorber film, preferably contiguous to the multilayer reflective film, more preferably contiguous to the multilayer reflective film and the absorber film. The protective film is used for protecting the multilayer reflective film in a cleaning, tailoring or otherwise processing step. Also preferably, the protective film has an additional function of protecting the multilayer reflective film or preventing the multilayer reflective film from oxidation during the step of patterning the absorber film by etching. Besides, an electroconductive film, which is used for electrostatic chucking of the reflection type mask to an exposure tool, may be disposed below the other major surface (back side surface) which is opposed to the one major surface of the substrate, preferably contiguous to the other major surface. It is provided herein that a substrate has one major surface which is a front or upper side surface and another major surface which is a back or lower side surface. The terms "front and back" sides or "upper and lower" sides are used for the sake of convenience. One or another major surface may be either of the two major surfaces (film-bearing surfaces) of a substrate, and in this sense, front and back or upper and lower are exchangeable. Specifically, the multilayer reflective film may be formed by any of the methods of JP-A 2021-139970 and the references cited therein.

The resist pattern forming process is successful in forming patterns having a high resolution, suppressed influences of develop loading and residue defects, and a small size difference independent of pattern density (grouped and isolated patterns), even on a substrate (typically mask blank of transmission or reflection type) whose outermost surface is made of a material tending to affect resist pattern profile such as a chromium, silicon or tantalum-containing material. EXAMPLES

Examples of the invention are given below by way of illustration and not by way of limitation. The abbreviation "pbw" is parts by weight. For copolymers, the compositional ratio is a molar ratio and Mw is determined by GPC versus polystyrene standards. For analysis, an IR spectrometer NICOLET 6700 by Thermo Fisher Scientific Inc. and a ¹H-NMR spectrometer ECA-500 by JEOL Ltd. were used.

### [1] Synthesis of acetal modifier

### Example 1-1

### Synthesis of acetal modifier AC-1

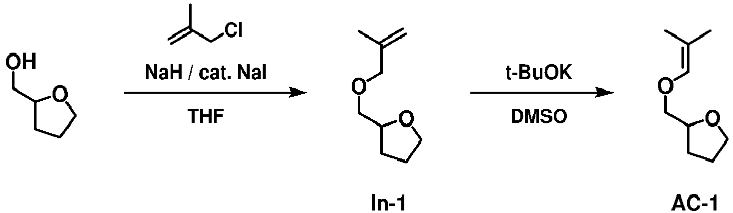

### (1) Synthesis of Intermediate In-1

In a reactor under nitrogen atmosphere, 91.6 g (purity 55 wt%) of sodium hydride was suspended in 530 g of THF. After the reactor was heated at a temperature of 50°C, a solution of 224.7 g of tetrahydrofurfuryl alcohol in 90 g of THF was added dropwise. At the end of addition, the solution was stirred for 2 hours while keeping the internal temperature of 50°C. At the end of stirring, 30.0 g of sodium iodide was added, and 180.7 g of methacrylic chloride was added dropwise while keeping the internal temperature of 50-60°C. The reactor was heated at a temperature of 65°C, after which the solution was aged for 5 hours. At the end of aging, the reaction solution was cooled and 620 g of water was added thereto to quench the reaction. The desired compound was extracted with 620 g of hexane, followed by ordinary aqueous work-up and solvent distillation. On purification by distillation, 281.2 g of Intermediate In-1 was obtained (yield 90%).

### (2) Synthesis of acetal modifier AC-1

In a reactor under nitrogen atmosphere, 281.2 g of Intermediate In-1 and 20.2 g of potassium t-butoxide were dissolved in 300 g of dimethylformamide (DMSO). The reactor was heated at a temperature of 80°C, after which the solution was aged for 12 hours. At the end of aging, the reaction solution was cooled and 300 g of water was added thereto to quench the reaction. The desired compound was extracted with 500 g of hexane, followed by ordinary aqueous work-up and solvent distillation. On purification by distillation, 268.1 g of the desired acetal modifier AC-1 was obtained as colorless oily matter (yield 95%).

The acetal modifier AC-1 was analyzed by IR and NMR spectroscopy.
IR (D-ATR): v = 2959, 2918, 2871, 1692, 1452, 1376, 1339, 1290, 1226, 1167, 1076, 1052, 1023, 922, 824, 444 cm⁻¹
¹H-NMR (600 MHz in DMSO-d6):
   δ = 5.90 (1H, m), 3.93 (1H, m), 3.71 (1H, m), 3.60 (3H, m), 1.89-1.75 (3H, m), 1.55 (1H, m), 1.49 (3H, s), 1.47 (3H, s) ppm

### Examples 1-2 to 1-8

### Synthesis of acetal modifiers AC-2 to AC-8

Acetal modifiers AC-2 to AC-8 were synthesized by a well-known organic synthesis method using corresponding reactants.

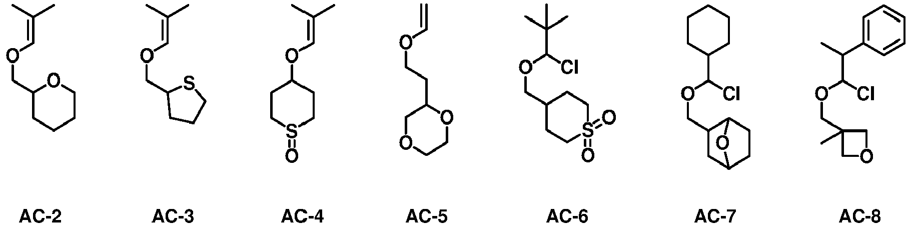

### [2] Synthesis of polymers

### Example 2-1

### Synthesis of Polymer P-1

A 100-ml flask was charged with 20 g of a hydroxystyrene-acenaphthylene copolymer and 46.7 g of THF solvent. In nitrogen atmosphere, 0.5 g of methanesulfonic acid was added at ~25°C, after which 4.4 g of acetal modifier AC-1 was added dropwise. The solution was held at room temperature for 4.5 hours for reaction. At the end of reaction, 1.0 g of triethylamine was added to the reaction solution, which was added dropwise to 500 g of hexane for precipitation. The copolymer precipitate was collected by filtration and washed twice with 120 g of hexane. The copolymer was dissolved in a mixture of 60 g of ethyl acetate and 20 g of water. The resulting solution was transferred to a separatory funnel. After 0.7 g of acetic acid was added to the solution, separatory operation was carried out. With the lower layer removed, 20 g of water and 0.9 g of pyridine were added to the organic layer, followed by separatory operation. With the lower layer removed, 20 g of water was added to the organic layer, followed by water washing and separatory operation. The water washing and separatory operation was carried out 5 times in total. Thereafter, the organic layer was concentrated, the concentrate was dissolved in 40 g of PGME, and the solution was added dropwise to 600 g of water. The resulting precipitate was collected by filtration, washed with water, and dried, obtaining 20.3 g of the target polymer P-1 as a white polymer. Polymer P-1 was analyzed by ¹H-NMR, ¹³C-NMR, and GPC, with the analytical results shown below.

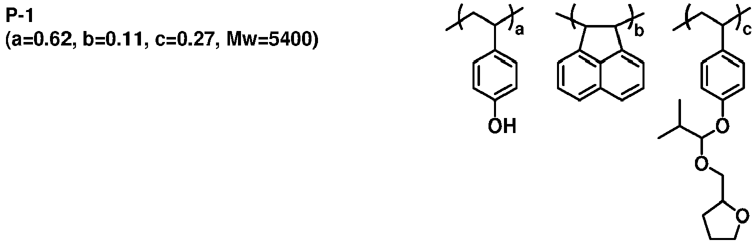

### Examples 2-2 to 2-15

### Synthesis of Polymers P-2 to P-15

Polymers P-2 to P-15 were synthesized by the same procedure as in Example 2-1 except that the polymer and/or acetal modifier was changed.

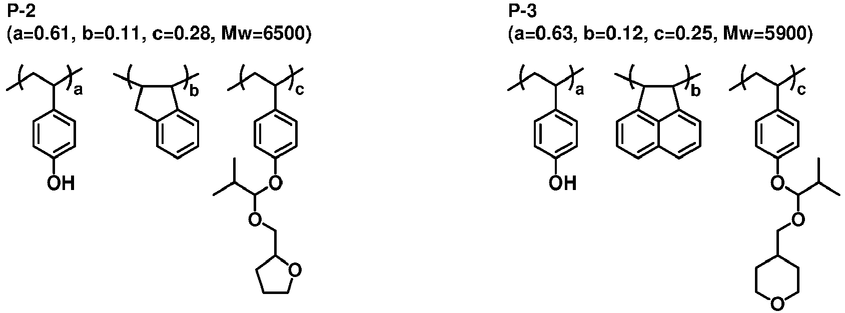

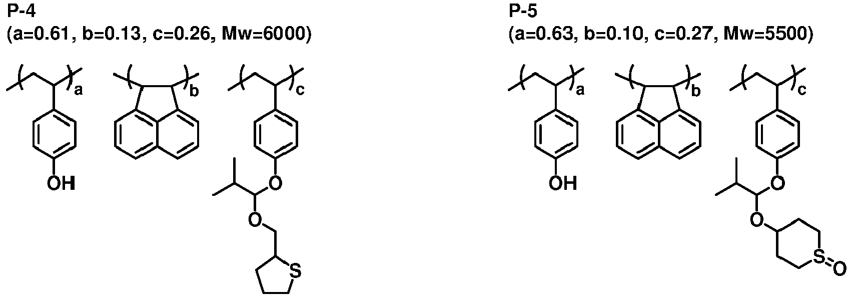

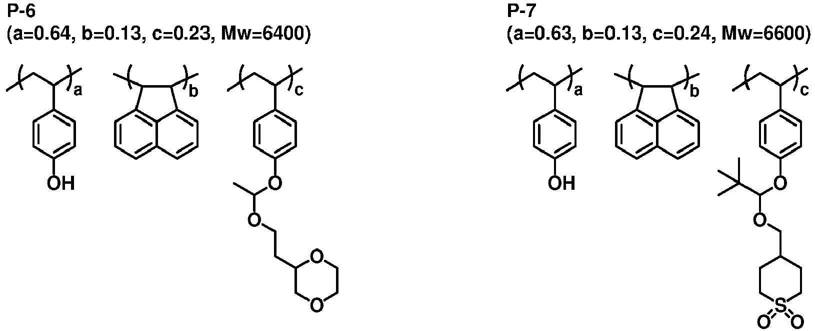

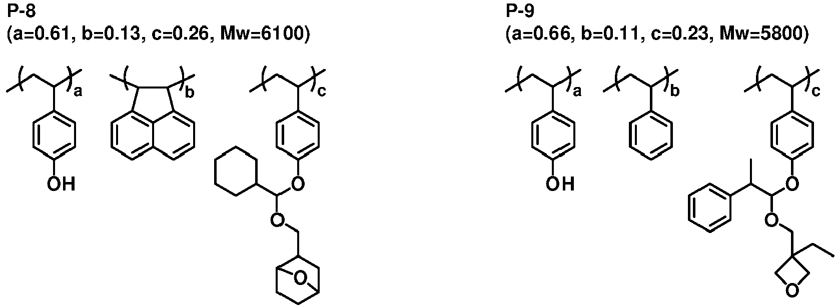

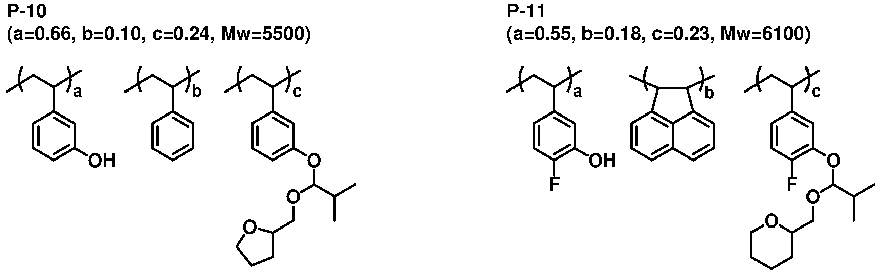

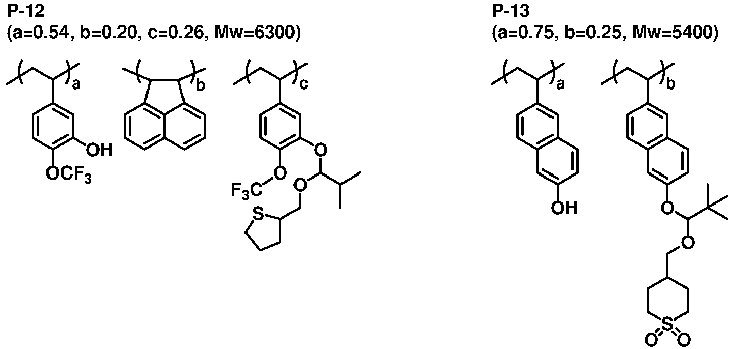

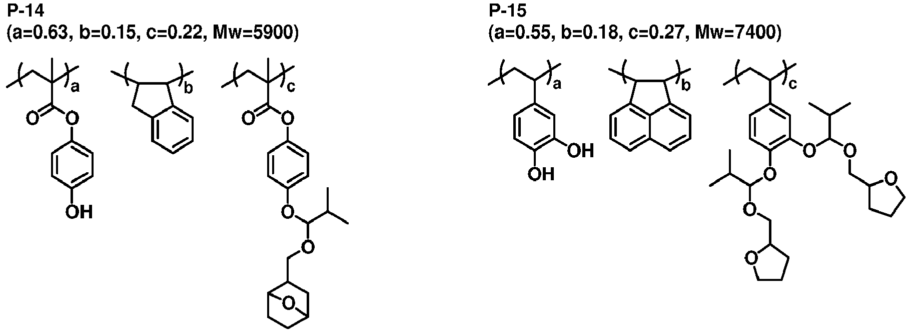

### Comparative Examples 1-1 to 1-6

### Synthesis of Polymers cP-1 to cP-6

Polymers cP-1 to cP-6 were synthesized by a well-known method using monomers in combination.

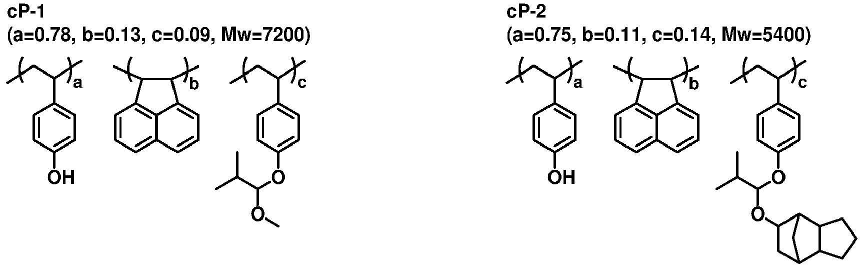

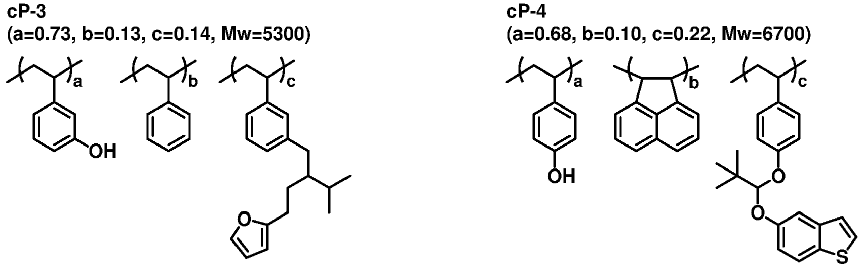

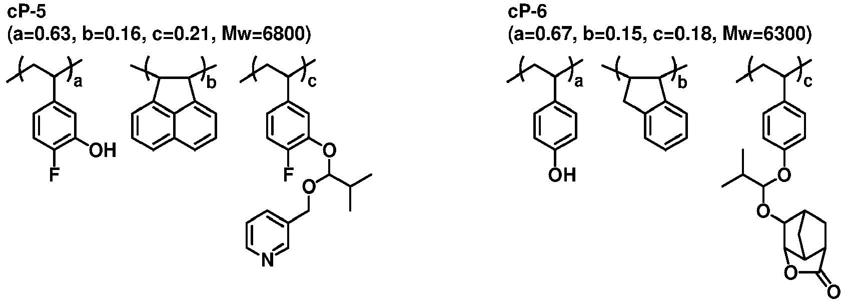

### Reference Synthesis Examples 1 to 6

### Synthesis of Polymers AP-1 to AP-6

Polymers AP-1 to AP-6 were synthesized by a well-known method using monomers in combination.

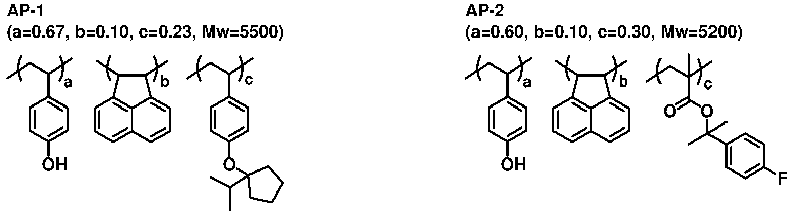

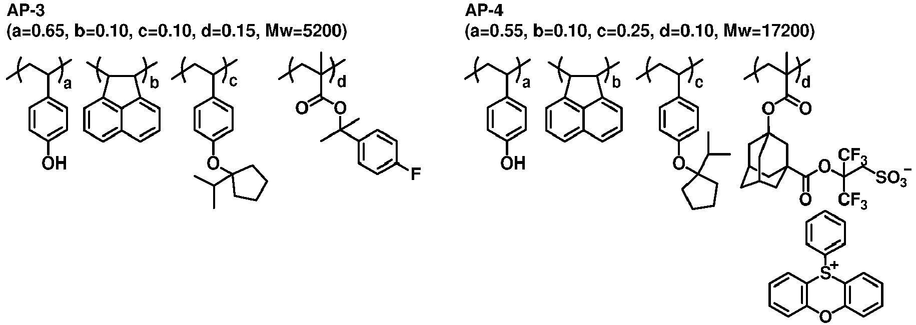

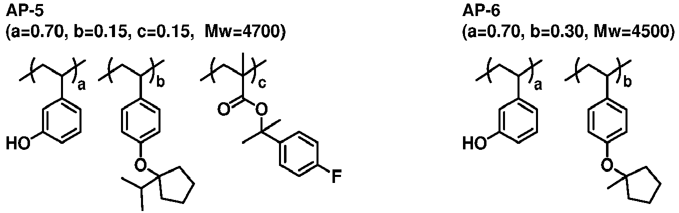

The dissolution rate of a polymer in alkaline developer was computed by spin coating a 16.7 wt% solution of a polymer in PGMEA solvent onto a 8-inch silicon wafer, baking at 100°C for 90 seconds to form a film of 1,000 nm thick, developing the film in a 2.38 wt% TMAH aqueous solution at 23°C for 100 seconds, and measuring a loss of film thickness. As a result, Polymers P-1 to P-15, AP-1 to AP-6 and cP-2 to cP-6 showed a dissolution rate of less than 5 nm/min and comparative Polymer cP-1 showed a dissolution rate of 14 nm/min.

### [3] Preparation of chemically amplified positive resist composition

### Examples 3-1 to 3-41 and Comparative Examples 2-1 to 2-10

A chemically amplified positive resist composition (R-1 to R-41, CR-1 to CR-10) was prepared by dissolving selected components in an organic solvent in accordance with the formulation shown in Tables 1 to 3 and filtering the solution through a nylon filter with a pore size of 5 nm and a UPE filter with a pore size of 1 nm. The organic solvent was a mixture of 940 pbw of PGMEA, 1,870 pbw of EL, and 1,870 pbw of PGME.

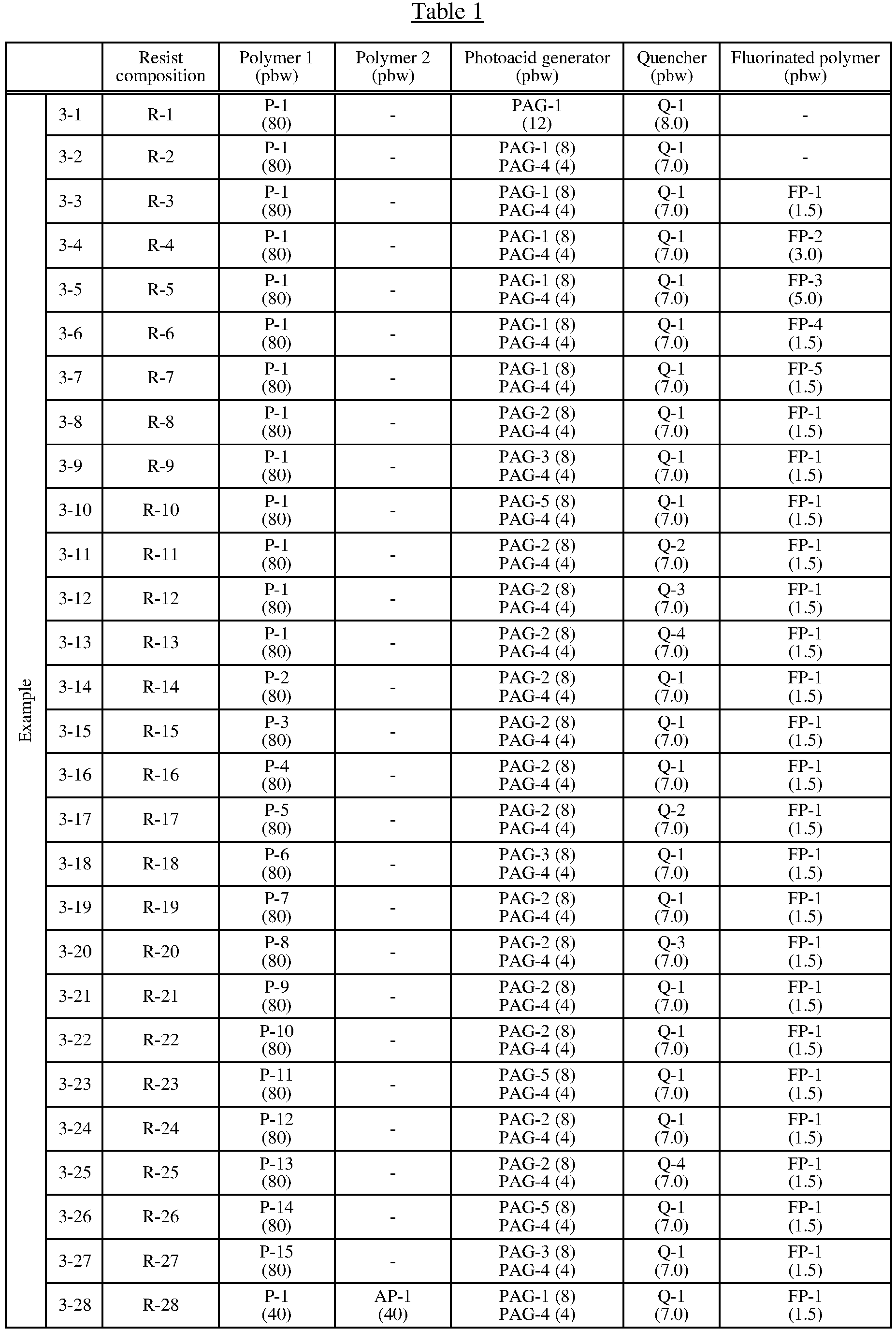

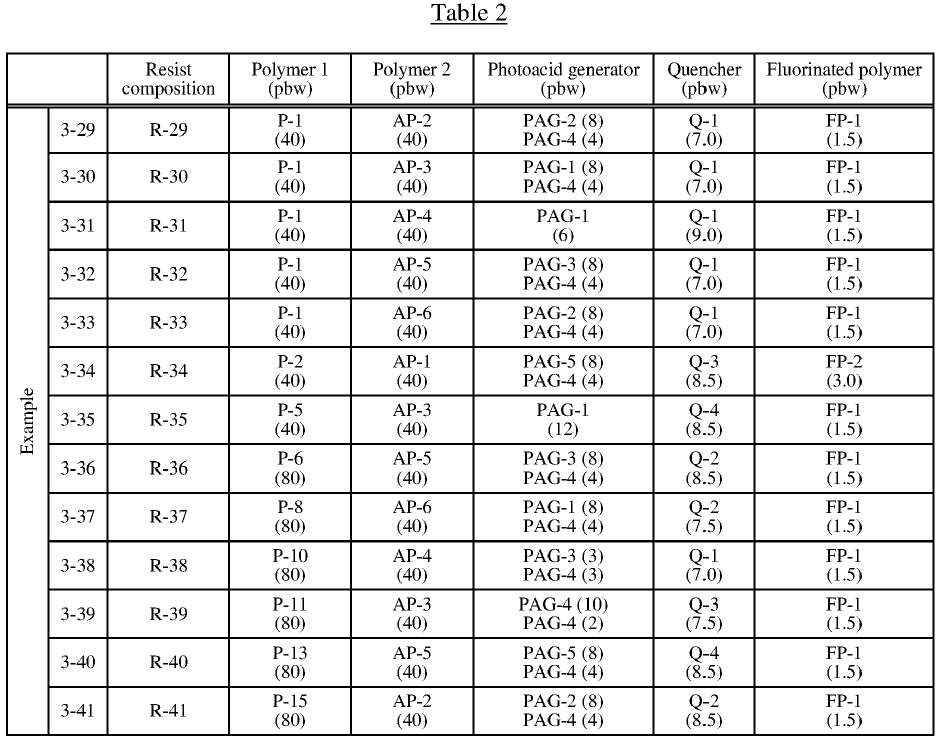

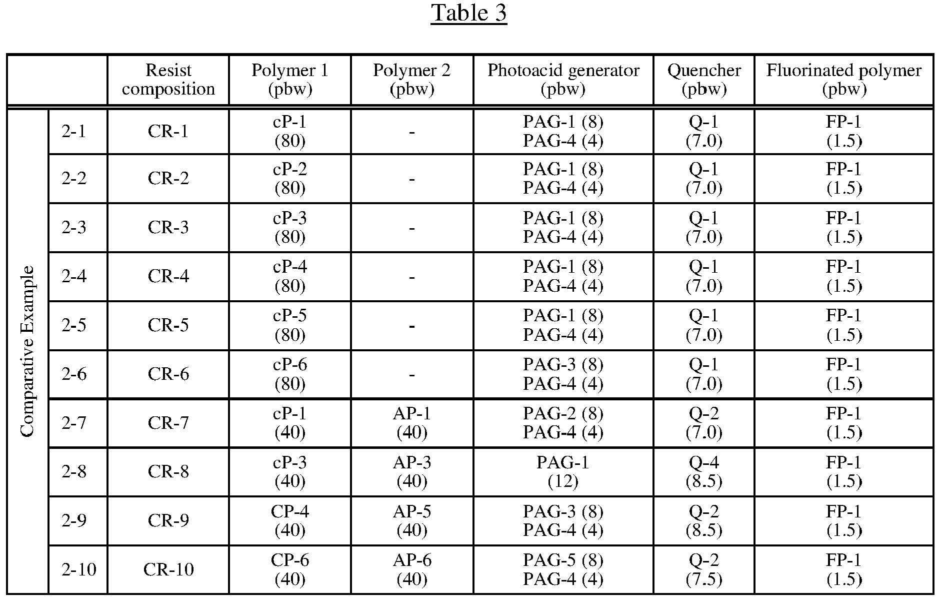

The components in Tables 1 to 3 are identified below.
Photoacid generators PAG-1 to PAG-5 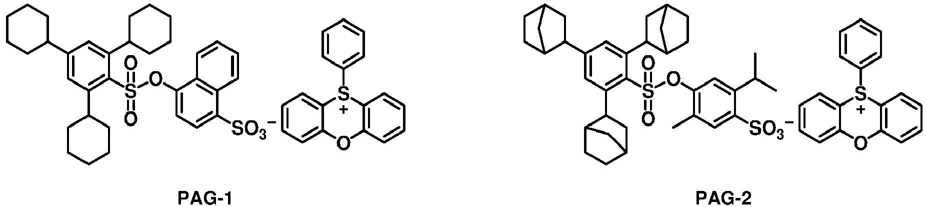 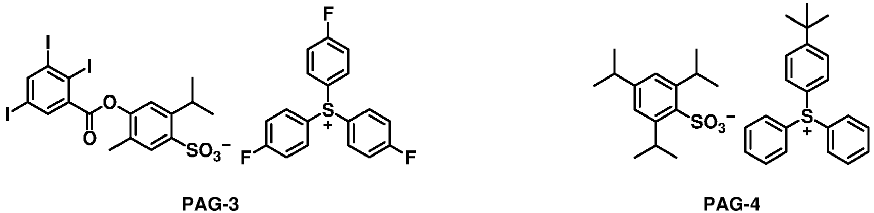 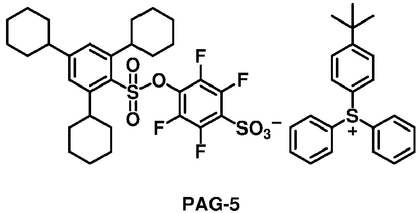
Quenchers Q-1 to Q-4 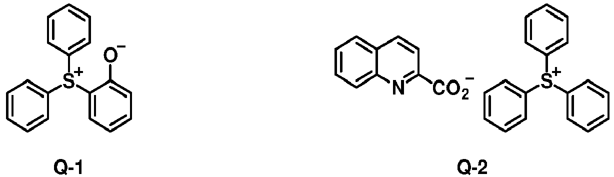 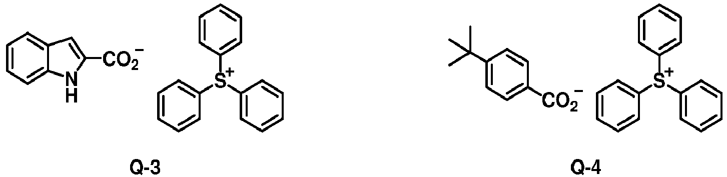
Fluorinated polymers FP-1 to FP-5

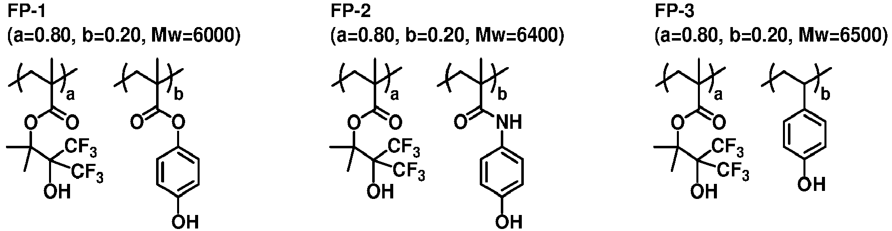

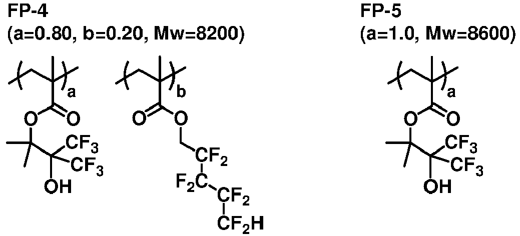

### [3] EB lithography test

### Examples 4-1 to 4-41 and Comparative Examples 3-1 to 3-10

There was furnished a reflection type mask blank for EUV lithography masks. Namely, a mask blank was furnished by forming a multilayer reflective film of 40 Mo/Si layers having a thickness of 284 nm on a low thermal expansion glass substrate of 6 inch squares, then successively depositing thereon a Ru film of 3.5 nm thick as a protective film, a TaN film of 70 nm thick as an absorber layer, and a CrN film of 6 nm thick as a hard mask.

Using a coater/developer system ACT-M (Tokyo Electron Ltd.), each of the positive resist compositions (R-1 to R-41, CR-1 to CR-10) was spin coated onto the reflection type photomask blank and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding an outer rim portion extending 10 mm inward from the periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum IS resolution) was defined as the minimum size at the dose which provided a 9:1 resolution for an isolated space (IS) of 200 nm. The edge roughness (LER) of a 200-nm LS pattern was measured under SEM. The develop loading was evaluated by forming a 200-nm LS pattern at the dose (µC/cm²) capable of resolving a 1:1 LS pattern of 200 nm design at a ratio 1: 1 and a 200-nm LS pattern including dummy patterns having a density of 15%, 25%, 33%, 45%, 50%, 55%, 66%, 75%, 85%, and 95% arranged around the center pattern, measuring the size of spaces under SEM, and comparing the size difference among grouped and isolated patterns. Also, the pattern was visually observed to judge whether or not the profile was rectangular.

The dissolution rate of an exposed region was computed by spin coating the resist solution onto a 8-inch silicon wafer, baking at 110°C for 60 seconds to form a resist film of 90 nm thick, exposing the resist film to KrF excimer laser radiation in a dose (mJ/cm²) capable of resolving a 200-nm 1:1 LS pattern at a ratio 1:1, baking at 110°C for 60 seconds, developing the film in a 2.38 wt% TMAH aqueous solution at 23°C, and measuring a loss of film thickness by means of a resist development rate analyzer (RDA-800 by Litho Tech Japan Corp.). The results are shown in Tables 4 to 6.

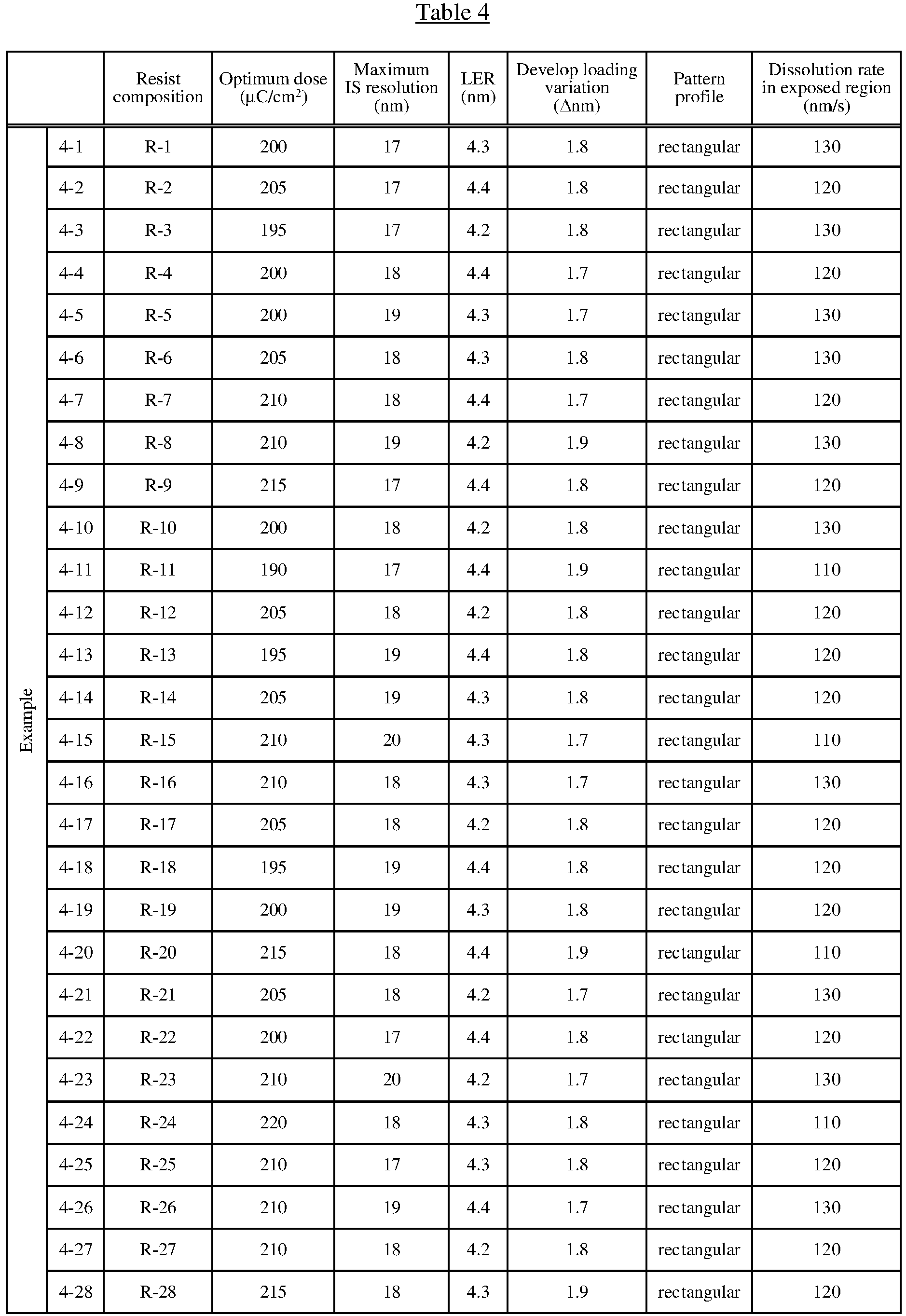

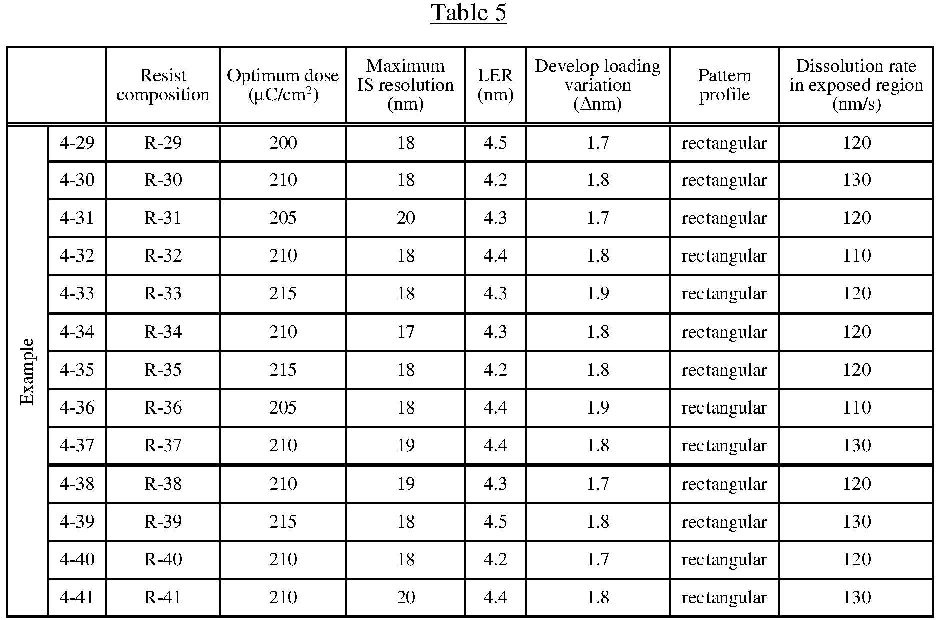

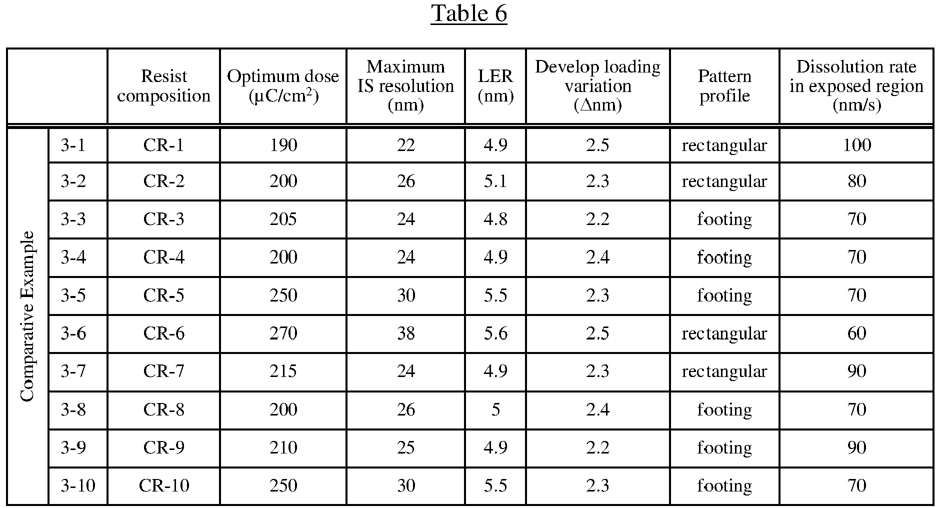

All the chemically amplified positive resist compositions (R-1 to R-41) within the scope of the invention show satisfactory IS resolution, LER and pattern rectangularity and reduced values of develop loading, as compared with comparative resist compositions (CR-1 to CR-10).

### [4] Evaluation of residue defects

### Examples 5-1 to 5-41 and Comparative Examples 4-1 to 4-10

Each of the resist compositions (R-1 to R-41, CR-1 to CR-10) was applied onto a reflection type mask blank for EUV lithography masks to form a resist film. Using an EB writer system EBM-5000Plus (NuFlare Technology Inc., accelerating voltage 50 kV), the resist film was exposed over its entire surface to EB in its optimum dose. The resist film was then baked (PEB) at 110°C for 600 seconds and developed in a 2.38 wt% TMAH aqueous solution. Using a mask defect inspection system M9650 (Laser Tech), development residues were evaluated. The total number of defects after development is shown in Tables 7 to 9.

**Table 7**

| | | Resist composition | Total number of defects after development |
|---|---|---|---|
| Example | 5-1 | R-1 | 230 |
| | 5-2 | R-2 | 240 |
| | 5-3 | R-3 | 290 |
| | 5-4 | R-4 | 250 |
| | 5-5 | R-5 | 250 |
| | 5-6 | R-6 | 240 |
| | 5-7 | R-7 | 250 |
| | 5-8 | R-8 | 230 |
| | 5-9 | R-9 | 250 |
| | 5-10 | R-10 | 260 |
| | 5-11 | R-11 | 250 |
| | 5-12 | R-12 | 260 |
| | 5-13 | R-13 | 270 |
| | 5-14 | R-14 | 240 |
| | 5-15 | R-15 | 260 |
| | 5-16 | R-16 | 250 |
| | 5-17 | R-17 | 260 |
| | 5-18 | R-18 | 270 |
| | 5-19 | R-19 | 250 |
| | 5-20 | R-20 | 260 |
| | 5-21 | R-21 | 280 |
| | 5-22 | R-22 | 270 |
| | 5-23 | R-23 | 280 |
| | 5-24 | R-24 | 260 |
| | 5-25 | R-25 | 260 |
| | 5-26 | R-26 | 250 |
| | 5-27 | R-27 | 260 |
| | 5-28 | R-28 | 260 |

**Table 8**

| | | Resist composition | Total number of defects after development |
|---|---|---|---|
| Example | 5-29 | R-29 | 250 |
| | 5-30 | R-30 | 270 |
| | 5-31 | R-31 | 280 |
| | 5-32 | R-32 | 250 |
| | 5-33 | R-33 | 270 |
| | 5-34 | R-34 | 280 |
| | 5-35 | R-35 | 270 |
| | 5-36 | R-36 | 260 |
| | 5-37 | R-37 | 250 |
| | 5-38 | R-38 | 280 |
| | 5-39 | R-39 | 260 |
| | 5-40 | R-40 | 270 |
| | 5-41 | R-41 | 260 |

**Table 9**

| | | Resist composition | Total number of defects after development |
|---|---|---|---|
| Comparative Example | 4-1 | CR-1 | 420 |
| | 4-2 | CR-2 | 960 |
| | 4-3 | CR-3 | 680 |
| | 4-4 | CR-4 | 570 |
| | 4-5 | CR-5 | 680 |
| | 4-6 | CR-6 | 970 |
| | 4-7 | CR-7 | 520 |
| | 4-8 | CR-8 | 580 |
| | 4-9 | CR-9 | 560 |
| | 4-10 | CR-10 | 870 |

All the chemically amplified positive resist compositions (R-1 to R-41) within the scope of the invention show smaller total numbers of defects after development than comparative resist compositions (CR-1 to CR-10).

### [5] Dry etching test

### Examples 6-1 to 6-41 and Comparative Examples 5-1 to 5-10

Each of the polymers (Polymers P-1 to P-41 and CP-1 to CP-10 in Tables 1 and 2), 2 g, was thoroughly dissolved in 10 g of cyclohexanone, and passed through a filter having a pore size of 0.2 µm, obtaining a polymer solution. The polymer solution was spin coated onto a mask blank of 152 mm squares having a chromium film as the outermost surface and baked to form a polymer film of 300 nm thick. Using a mask dry etching instrument Gen-4 (Plasma Thermo Ltd.), the polymer film was etched with chlorine gas under the following conditions.

| | |
|---|---|
| Chamber pressure: | 6.0 mTorr |
| RF power: | 700 V |
| Cl₂ gas flow rate: | 185 sccm |
| O₂ flow rate: | 55 sccm |
| He flow rate: | 9 sccm |
| Time: | 75 sec |

The difference in film thickness before and after etching was determined. A smaller value of film thickness difference, i.e., a smaller loss indicates better etching resistance. The results of dry etching resistance are shown in Tables 10 to 12.

**Table 10**

| | | Polymer | Cl₂/O₂ gas etching rate (nm/min) |
|---|---|---|---|
| Example | 6-1 | P-1 | 91 |
| | 6-2 | P-2 | 92 |
| | 6-3 | P-3 | 92 |
| | 6-4 | P-4 | 94 |
| | 6-5 | P-5 | 91 |
| | 6-6 | P-6 | 92 |
| | 6-7 | P-7 | 94 |
| | 6-8 | P-8 | 95 |
| | 6-9 | P-9 | 93 |
| | 6-10 | P-10 | 90 |
| | 6-11 | P-11 | 90 |
| | 6-12 | P-12 | 92 |
| | 6-13 | P-13 | 92 |
| | 6-14 | P-14 | 89 |
| | 6-15 | P-15 | 91 |
| | 6-16 | P-16 | 94 |
| | 6-17 | P-17 | 93 |
| | 6-18 | P-18 | 94 |
| | 6-19 | P-19 | 95 |
| | 6-20 | P-20 | 94 |
| | 6-21 | P-21 | 94 |
| | 6-22 | P-22 | 95 |
| | 6-23 | P-23 | 93 |
| | 6-24 | P-24 | 95 |
| | 6-25 | P-25 | 94 |
| | 6-26 | P-26 | 92 |
| | 6-27 | P-27 | 90 |
| | 6-28 | P-28 | 93 |

**Table 11**

| | | Polymer | Cl₂/O₂ gas etching rate (nm/min) |
|---|---|---|---|
| Example | 6-29 | P-29 | 90 |
| | 6-30 | P-30 | 93 |
| | 6-31 | P-31 | 91 |
| | 6-32 | P-32 | 94 |
| | 6-33 | P-33 | 95 |
| | 6-34 | P-34 | 92 |
| | 6-35 | P-35 | 94 |
| | 6-36 | P-36 | 93 |
| | 6-37 | P-37 | 91 |
| | 6-38 | P-38 | 94 |
| | 6-39 | P-39 | 93 |
| | 6-40 | P-40 | 93 |
| | 6-41 | P-41 | 90 |

**Table 12**

| | | Polymer | Cl₂/O₂ gas etching rate (nm/min) |
|---|---|---|---|
| Comparative Example | 5-1 | cP-1 | 143 |
| | 5-2 | cP-2 | 102 |
| | 5-3 | cP-3 | 100 |
| | 5-4 | cP-4 | 110 |
| | 5-5 | cP-5 | 120 |
| | 5-6 | cP-6 | 103 |
| | 5-7 | cP-7 | 108 |
| | 5-8 | cP-8 | 114 |
| | 5-9 | cP-9 | 115 |
| | 5-10 | cP-10 | 100 |

As is evident from the results shown in Tables 10 to 12, the inventive polymers display excellent etching resistance against Cl₂/O₂ gas.

It has been demonstrated that the polymers, chemically amplified positive resist compositions, and resist pattern forming process according to the invention are useful in the photolithography for the fabrication of semiconductor devices, especially the processing of photomask blanks of transmission and reflection types.

## Claims

1. An acetal modifier providing a group having an oxygen or sulfur-containing cyclic saturated heterocycle serving as a protective group for an aliphatic or aromatic hydroxy group.

2. The acetal modifier of claim 1, which is a compound having the formula (AC-1) or (AC-2): 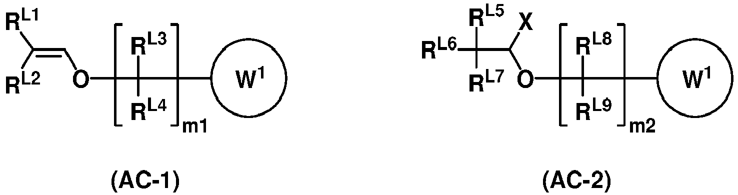 wherein R^{L1}, R^{L2}, R^{L5}, R^{L6} and R^{L7} are each independently hydrogen or a C₁-C₁₅ hydrocarbyl group, R^{L1} and R^{L2} may bond together to form a ring with the carbon atom to which they are attached, and any two of R^{L5} to R^{L7} may bond together to form a ring with the carbon atom to which they are attached,
R^{L3}, R^{L4}, R^{L8} and R^{L9} are each independently hydrogen or a C₁-C₂₀ hydrocarbyl group which may contain a heteroatom, R^{L3} and R^{L4} may bond together to form a ring with the carbon atom to which they are attached, and R^{L8} and R^{L9} may bond together to form a ring with the carbon atom to which they are attached,
W¹ is each independently a group having an oxygen or sulfur-containing cyclic saturated heterocycle,
X is chlorine, bromine or iodine, and
m1 and m2 are each independently an integer of 0 to 3.

3. A polymer adapted to turn alkali soluble as a result of deprotection under the action of acid, the polymer comprising repeat units A1 having on side chain a structure including an aromatic hydroxy group protected with an acid labile group having the formula (AL-1) or (AL-2): 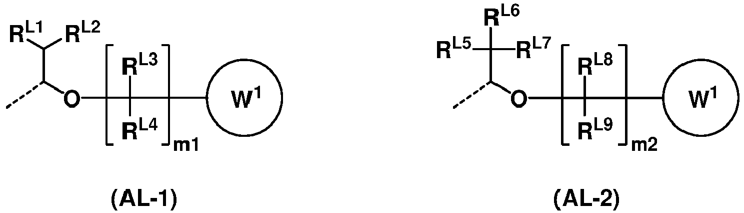 wherein R^{L1}, R^{L2}, R^{L5}, R^{L6} and R^{L7} are each independently hydrogen or a C₁-C₁₅ hydrocarbyl group, R^{L1} and R^{L2} may bond together to form a ring with the carbon atom to which they are attached, and any two of R^{L5} to R^{L7} may bond together to form a ring with the carbon atom to which they are attached,
R^{L3}, R^{L4}, R^{L8} and R^{L9} are each independently hydrogen or a C₁-C₂₀ hydrocarbyl group which may contain a heteroatom, R^{L3} and R^{L4} may bond together to form a ring with the carbon atom to which they are attached, and R^{L8} and R^{L9} may bond together to form a ring with the carbon atom to which they are attached,
W¹ is each independently a group having an oxygen or sulfur-containing cyclic saturated heterocycle,
m1 and m2 are each independently an integer of 0 to 3, and
the broken line designates a point of attachment to the oxygen atom of the aromatic hydroxy group.

4. The polymer of claim 3 wherein the repeat unit A1 has the formula (A1): 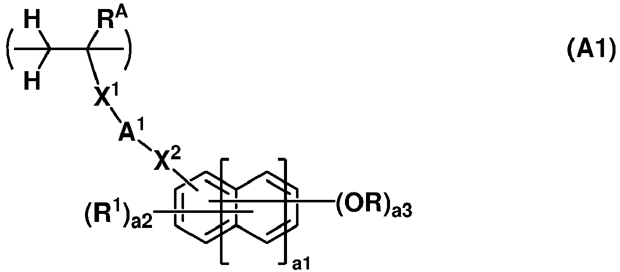 wherein a1 is 0 or 1, a2 is an integer of 0 to 4 in case of a1=0, and an integer of 0 to 6 in case of a1=1, a3 is an integer of 1 to 3, with the proviso that a2+a3 is from 1 to 5 in case of a1=0 and a2+a3 is from 1 to 7 in case of a1=1,
R^{A} is hydrogen, fluorine, methyl or trifluoromethyl,
X¹ is a single bond, *-C(=O)-O- or *-C(=O)-NH-, * designates a point of attachment to the carbon atom in the backbone,
X² is a single bond, ether bond, ester bond, carbonyl group, sulfonate ester bond, carbonate bond or carbamate bond,
A¹ is a single bond or a C₁-C₁₀ saturated hydrocarbylene group in which some -CH₂-may be replaced by -O-,
R¹ is halogen or a C₁-C₂₀ hydrocarbyl group which may contain a heteroatom, and
R is an acid labile group having formula (AL-1) or (AL-2).

5. The polymer of claim 3 or 4, further comprising phenolic hydroxy group-bearing repeat units A2 having the formula (A2): 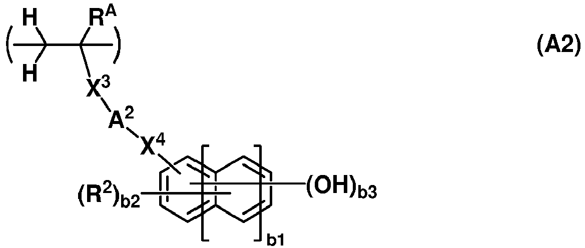 wherein b1 is 0 or 1, b2 is an integer of 0 to 4 in case of b1=0, and an integer of 0 to 6 in case of b1=1, b3 is an integer of 1 to 3, with the proviso that b2+b3 is from 1 to 5 in case of b1=0 and b2+b3 is from 1 to 7 in case of b1=1,
R^{A} is hydrogen, fluorine, methyl or trifluoromethyl,
X³ is a single bond, *-C(=O)-O- or *-C(=O)-NH-, * designates a point of attachment to the carbon atom in the backbone,
X⁴ is a single bond, ether bond, ester bond, carbonyl group, sulfonate ester bond, carbonate bond or carbamate bond,
A² is a single bond or a C₁-C₁₀ saturated hydrocarbylene group in which some -CH₂-may be replaced by -O-, and
R² is halogen or a C₁-C₂₀ hydrocarbyl group which may contain a heteroatom.

6. The polymer of any one of claims 3 to 5, further comprising repeat units of at least one type selected from repeat units having the formula (A3-1) and repeat units having the formula (A3-2): 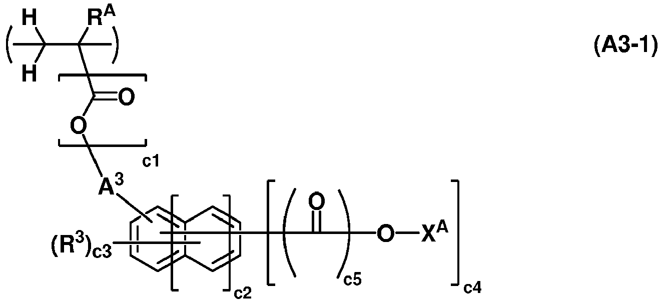 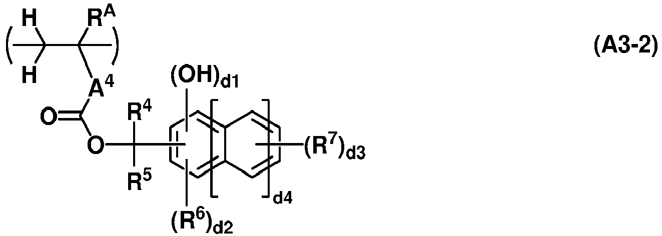 wherein c1 is 0 or 1, c2 is an integer of 0 to 2, c3 is an integer satisfying 0 ≤ c3 ≤ 5+2(c2)-c4, c4 is an integer of 1 to 3, c5 is 0 or 1,
d1 is an integer of 0 to 2, d2 is an integer of 0 to 2, d3 is an integer of 0 to 5, d4 is an integer of 0 to 2,
R^{A} is each independently hydrogen, fluorine, methyl or trifluoromethyl,
A³ is a single bond or a C₁-C₁₀ saturated hydrocarbylene group in which some -CH₂-may be replaced by -O-,
A⁴ is a single bond, phenylene group, naphthylene group or *-C(=O)-O-A⁴¹-, A⁴¹ is a C₁-C₂₀ aliphatic hydrocarbylene group which may contain hydroxy, ether bond, ester bond or lactone ring, or a phenylene group or naphthylene group, * designates a point of attachment to the carbon atom in the backbone,
R³ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R⁴ and R⁵ are each independently a C₁-C₁₀ hydrocarbyl group which may contain a heteroatom, R⁴ and R⁵ may bond together to form a ring with the carbon atom to which they are attached,
R⁶ is each independently fluorine or a C₁-C₅ fluorinated alkyl group or C₁-C₅ fluorinated alkoxy group,
R⁷ is each independently a C₁-C₁₀ hydrocarbyl group which may contain a heteroatom, and
X^{A} is an acid labile group in case of c4=1 and X^{A} is hydrogen or an acid labile group, at least one being an acid labile group, in case of c4=2 or 3.

7. The polymer of any one of claims 3 to 6, further comprising repeat units of at least one type selected from repeat units having the formula (B 1), repeat units having the formula (B2), and repeat units having the formula (B3): 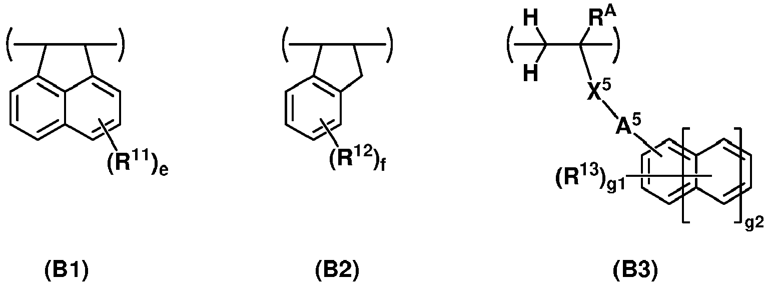 wherein e and f are each independently an integer of 0 to 4, g1 is an integer of 0 to 5, g2 is an integer of 0 to 2,
R^{A} is hydrogen, fluorine, methyl or trifluoromethyl,
X⁵ is a single bond, *-C(=O)-O- or *-C(=O)-NH-, * designates a point of attachment to the carbon atom in the backbone,
A⁵ is a single bond or a C₁-C₁₀ saturated hydrocarbylene group in which some -CH₂-may be replaced by -O-, and
R¹¹ and R¹² are each independently a hydroxy group, halogen, an optionally halogenated C₂-C₈ saturated hydrocarbylcarbonyloxy group, optionally halogenated C₁-C₈ saturated hydrocarbyl group, or optionally halogenated C₁-C₈ saturated hydrocarbyloxy group,
R¹³ is an acetyl group, C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro group or cyano group, R¹³ may also be a hydroxy group in case of g2=1 or 2.

8. The polymer of any one of claims 3 to 7, further comprising repeat units of at least one type selected from repeat units having the formulae (C1) to (C4): 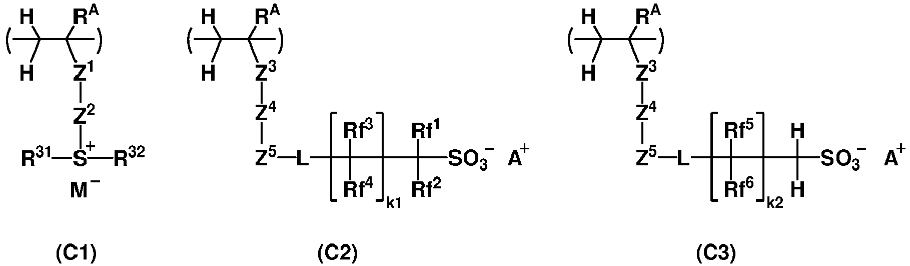 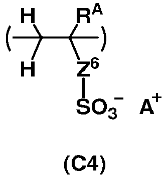 wherein R^{A} is each independently hydrogen, fluorine, methyl or trifluoromethyl,
Z¹ is a single bond or phenylene group,
Z² is *¹-C(=O)-O-Z²¹-, *¹-C(=O)-NH-Z²¹-, or *¹-O-Z²¹-, Z²¹ is a C₁-C₆ aliphatic hydrocarbylene group, phenylene group or divalent group obtained by combining the foregoing, which may contain a carbonyl moiety, ester bond, ether bond or hydroxy moiety, *¹ designates a point of attachment to Z¹,
Z³ is each independently a single bond, phenylene group, naphthylene group or *²-C(=O)-O-Z³¹-, Z³¹ is a C₁-C₁₀ aliphatic hydrocarbylene group, phenylene group or naphthylene group, the aliphatic hydrocarbylene group may contain a hydroxy moiety, ether bond, ester bond or lactone ring, *² designates a point of attachment to the carbon atom in the backbone,
Z⁴ is each independently a single bond, *³-Z⁴¹-C(=O)-O-, *³-C(=O)-NH-Z⁴¹-, or *³-O-Z⁴¹-, Z⁴¹ is a C₁-C₂₀ hydrocarbylene group which may contain a heteroatom, *³ designates a point of attachment to Z³,
Z⁵ is each independently a single bond, *⁴-Z⁵¹-C(=O)-O-, *⁴-C(=O)-NH-Z⁵¹-, or *⁴-O-Z⁵¹-, Z⁵¹ is a C₁-C₂₀ hydrocarbylene group which may contain a heteroatom, *⁴ designates a point of attachment to Z⁴,
Z⁶ is each independently a single bond, methylene, ethylene, phenylene, fluorinated phenylene, trifluoromethyl-substituted phenylene, *²-C(=O)-O-Z⁶¹-, *²-C(=O)-N(H)-Z⁶¹-, or *²-O-Z⁶¹-, Z⁶¹ is a C₁-C₆ aliphatic hydrocarbylene group, phenylene group, fluorinated phenylene group, or trifluoromethyl-substituted phenylene group, which may contain a carbonyl moiety, ester bond, ether bond or hydroxy moiety, *² designates a point of attachment to the carbon atom in the backbone,
R³¹ and R³² are each independently a C₁-C₂₀ hydrocarbyl group which may contain a heteroatom, R³¹ and R³² may bond together to form a ring with the sulfur atom to which they are attached,
L is a single bond, ether bond, ester bond, carbonyl group, sulfonate ester bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine or a C₁-C₆ fluorinated saturated hydrocarbyl group,
Rf⁵ and Rf⁶ are each independently hydrogen, fluorine or a C₁-C₆ fluorinated saturated hydrocarbyl group, excluding that all Rf⁵ and Rf⁶ are hydrogen at the same time,
M⁻ is a non-nucleophilic counter ion,
A⁺ is an onium cation,
k1 and k2 are each independently an integer of 0 to 3.

9. A chemically amplified positive resist composition comprising the polymer of any one of claims 3 to 8.

10. The resist composition of claim 9, further comprising an organic solvent.

11. The resist composition of claim 9 or 10, further comprising a photoacid generator capable of generating an acid having an acid strength (pKa) of -2.0 or larger.

12. The resist composition of any one of claims 9 to 11, further comprising a quencher.

13. The resist composition of any one of claims 9 to 12, further comprising a fluorinated polymer comprising repeat units of at least one type selected from repeat units having the formula (D1), repeat units having the formula (D2), repeat units having the formula (D3), and repeat units having the formula (D4) and optionally repeat units of at least one type selected from repeat units having the formula (D5) and repeat units having the formula (D6): 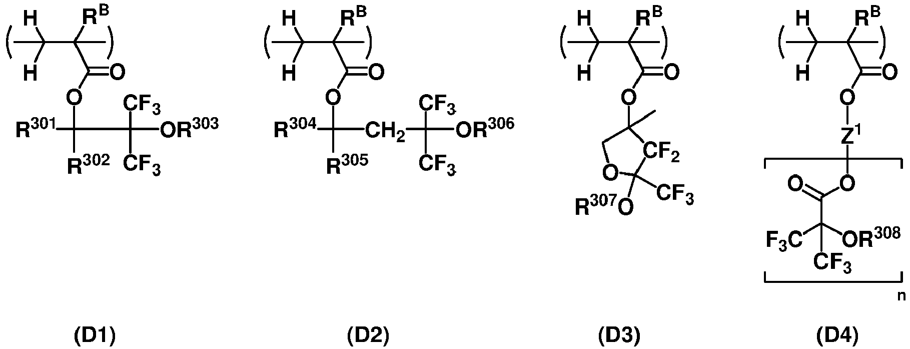 wherein R^{B} is each independently hydrogen, fluorine, methyl or trifluoromethyl,
R³⁰¹, R³⁰², R³⁰⁴ and R³⁰⁵ are each independently hydrogen or a C₁-C₁₀ saturated hydrocarbyl group,
R³⁰³, R³⁰⁶, R³⁰⁷ and R³⁰⁸ are each independently hydrogen, a C₁-C₁₅ hydrocarbyl group, C₁-C₁₅ fluorinated hydrocarbyl group, or acid labile group, with the proviso that when R³⁰³, R³⁰⁶, R³⁰⁷ and R³⁰⁸ each are a hydrocarbyl or fluorinated hydrocarbyl group, an ether bond or carbonyl moiety may intervene in a carbon-carbon bond,
n is an integer of 1 to 3, and
Z¹ is a C₁-C₂₀ (n+1)-valent hydrocarbon group or C₁-C₂₀ (n+1)-valent fluorinated hydrocarbon group. 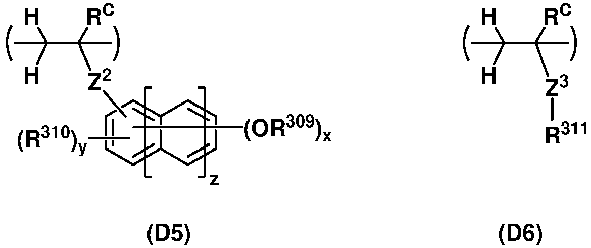
wherein R^{C} is each independently hydrogen or methyl,
R³⁰⁹ is hydrogen or a C₁-C₅ straight or branched hydrocarbyl group in which a heteroatom-containing moiety may intervene in a carbon-carbon bond,
R³¹⁰ is a C₁-C₅ straight or branched hydrocarbyl group in which a heteroatom-containing moiety may intervene in a carbon-carbon bond,
R³¹¹ is a C₁-C₂₀ saturated hydrocarbyl group in which at least one hydrogen is substituted by fluorine, and in which some -CH₂- may be replaced by an ester bond or ether bond,
x is an integer of 1 to 3, y is an integer satisfying 0 ≤ y ≤ 5+2z-x, z is 0 or 1,
Z² is a single bond, *-C(=O)-O- or *-C(=O)-NH-,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

14. A resist pattern forming process comprising the steps of:
applying the chemically amplified positive resist composition of any one of claims 9 to 13, onto a substrate to form a resist film thereon,
exposing the resist film to a pattern of high-energy radiation, and
developing the exposed resist film in an alkaline developer.

15. A mask blank of transmission or reflection type which is coated with the chemically amplified positive resist composition of any one of claims 9 to 13.
